# EUROPEAN PATENT SPECIFICATION

(11) **EP 2 837 636 B1**
(45) Date of publication and mention of the grant of the patent: **11.04.2018**
(21) Application number: 14184544.6
(22) Date of filing: 29.01.2008
(51) Int. Cl.: C07K 7/04, C07K 7/06, C07K 7/08

(54) **NOVEL MACROMOLECULE TRANSDUCTION DOMAINS AND METHODS FOR IDENTIFICATION AND USES THEREOF**
NEUE MAKROMOLEKÜLTRANSDUKTIONSDOMÄNEN UND IDENTIFIKATIONSVERFAHREN UND ANWENDUNGEN DAVON
NOUVEAUX DOMAINES DE TRANSDUCTION DE MACROMOLECULES ET PROCEDES D'IDENTIFICATION ET UTILISATIONS CORRESPONDANTES

(30) Priority: 29.01.2007 US 887060 P
(43) Date of publication of application: 18.02.2015
(62) Divisional of application: 08712219.8
(73) Proprietor: Procell Therapeutics Inc., Seoul 152-779 (KR)
(72) Inventor: Jo, Dae Woong, Seo-gu Gwangju 502-776 (KR); Ko, Jae Sun, Buk-gu Gwangju 500-200 (KR); Kim, Jin Sook, Gwangsan-gu Gwangju 506-708 (KR); Park, Kyung Mi, Yeosu-si Jeollanam-do 550-250 (KR); Song, Jin Kyung, Buk-gu Gwangju 500-921 (KR); Lim, Jung Hee, Gunsan-si Jeollabuk-do 573-923 (KR); Do, Thi Thuy Nga, Hwasun-gun Jeollanam-do 519-808 (KR); Do, Thi Lan Phuong, Hwasun-gun Jeollanam-do 519-808 (KR); Duong, Minh Tam, Hwasun-gun Jeollanam-do 519-808 (KR)
(74) Representative: V.O.

(56) References cited:
- WO-A1-95/34295
- WO-A1-99/49879
- WO-A1-03/097671
- WO-A2-03/106491
- US-A1- 2003 077 289
- US-A1- 2003 211 590
- US-A1- 2004 043 463
- HAWIGER J: "NONINVASIVE INTRACELLULAR DELIVERY OF FUNCTIONAL PEPTIDES AND PROTEINS", CURRENT OPINION IN CHEMICAL BIOLOGY, CURRENT BIOLOGY LTD, LONDON, GB, vol. 3, no. 1, 1 February 1999 (1999-02-01), pages 89-94, XP001009450, ISSN: 1367-5931, DOI: DOI:10.1016/S1367-5931(99)80016-7
- JOLIOT ALAIN ET AL: "Transduction peptides: From technology to physiology", NATURE CELL BIOLOGY, MACMILLAN MAGAZINES LTD, vol. 6, no. 3, 1 March 2004 (2004-03-01), pages 189-196, XP002513866, ISSN: 1465-7392, DOI: 10.1038/NCB0304-189

### SEQUENCE LISTINGS

<110> PROCELL THERAPEUTICS INC.
<120> NOVEL MACROMOLECULE TRANSDUCTION DOMAINS, METHOD FOR THE
   IDENDTIFICATION AND USES THEREOF
<150> US60/887,060
   <151> 2007-01-29
<160> 586
<170> KopatentIn 1.71
<210> 1
   <211> 12
   <212> PRT
   <213> Artificial Sequence
<220>
   <223> MTD JO-01
<400> 1
   Ala Val Val Val Cys Ala Ile Val Leu Ala Ala Pro
    1               5                   10
<210> 2
   <211> 12
   <212> PRT
   <213> Artificial Sequence
<220>
   <223> MTD JO-02
<400> 2
   Pro Leu Ala Leu Leu Val Leu Leu Leu Leu Gly Pro
    1               5                   10
<210> 3
   <211> 11
   <212> PRT
   <213> Artificial Sequence
<220>
   <223> MTD JO-03
<400> 3
   Leu Leu Leu Ala Phe Ala Leu Leu Cys Leu Pro
    1               5                   10
<210> 4
   <211> 13
   <212> PRT
   <213> Artificial Sequence
<220>
   <223> MTD JO-04
<400> 4
   Leu Leu Gly Ala Leu Ala Ala Val Leu Leu Ala Leu Ala
    1               5                   10
<210> 5
   <211> 17
   <212> PRT
   <213> Artificial Sequence
<220>
   <223> MTD JO-05
<400> 5
   Pro Val Leu Leu Ala Leu Gly Val Gly Leu Val Leu Leu Gly Leu Ala
   1               5                   10                  15
   Val
<210> 6
   <211> 9
   <212> PRT
   <213> Artificial Sequence
<220>
   <223> MTD JO-06
<400> 6
   Ala Ala Ala Ala Val Leu Leu Ala Ala
   1               5
<210> 7
   <211> 8
   <212> PRT
   <213> Artificial Sequence
<220>
   <223> MTD JO-07
<400> 7
   Ile Val Val Ala Val Val Val Ile
   1               5
<210> 8
   <211> 9
   <212> PRT
   <213> Artificial Sequence
<220>
   <223> MTD JO-08
<400> 8
   Ala Val Leu Ala Pro Val Val Ala Val
   1               5
<210> 9
   <211> 13
   <212> PRT
   <213> Artificial Sequence
<220>
   <223> MTD JO-09
<400> 9
   Leu Ala Val Cys Gly Leu Pro Val Val Ala Leu Leu Ala
    1               5                   10
<210> 10
   <211> 16
   <212> PRT
   <213> Artificial Sequence
<220>
   <223> MTD JO-10
<400> 10
   Leu Gly Gly Ala Val Val Ala Ala Pro Val Ala Ala Ala Val Ala Pro
    1               5                   10                  15
<210> 11
   <211> 13
   <212> PRT
   <213> Artificial Sequence
<220>
   <223> MTD JO-11
<400> 11
   Leu Leu Leu Val Leu Ala Val Leu Leu Ala Val Leu Pro
    1               5                   10
<210> 12
   <211> 12
   <212> PRT
   <213> Artificial Sequence
<220>
   <223> MTD JO-12
<400> 12
   Leu Leu Ile Leu Leu Leu Leu Pro Leu Leu Ile Val
    1               5                   10
<210> 13
   <211> 11
   <212> PRT
   <213> Artificial Sequence
<220>
   <223> MTD JO-13
<400> 13
   Leu Ala Ala Ala Ala Leu Ala Val Leu Pro Leu
   1 5 10
<210> 14
   <211> 14
   <212> PRT
   <213> Artificial Sequence
<220>
   <223> MTD JO-14
<400> 14
   Phe Leu Met Leu Leu Leu Pro Leu Leu Leu Leu Leu Val Ala
   1 5 10
<210> 15
   <211> 15
   <212> PRT
   <213> Artificial Sequence
<220>
   <223> MTD JO-15
<400> 15
   Ala Ala Ala Ala Ala Ala Leu Gly Leu Ala Ala Ala Val Pro Ala
   1 5 10 15
<210> 16
   <211> 14
   <212> PRT
   <213> Artificial Sequence
<220>
   <223> MTD JO-16
<400> 16
   Leu Leu Leu Ala Ala Leu Leu Leu Ile Ala Phe Ala Ala Val
   1 5 10
<210> 17
   <211> 14
   <212> PRT
   <213> Artificial Sequence
<220>
   <223> MTD JO-17
<400> 17
   Ala Leu Ala Ala Val Val Leu Ile Pro Leu Gly Ile Ala Ala
   1 5 10
<210> 18
   <211> 16
   <212> PRT
   <213> Artificial Sequence
<220>
   <223> MTD JO-18
<400> 18
   Ala Ala Leu Ala Leu Gly Val Ala Ala Ala Pro Ala Ala Ala Pro Ala
   1               5                   10                  15
<210> 19
   <211> 14
   <212> PRT
   <213> Artificial Sequence
<220>
   <223> MTD JO-19
<400> 19
   Ala Ala Leu Ile Gly Ala Val Leu Ala Pro Val Val Ala Val
   1               5                   10
<210> 20
   <211> 15
   <212> PRT
   <213> Artificial Sequence
<220>
   <223> MTD JO-20
<400> 20
   Ala Ala Gly Ile Ala Val Ala Ile Ala Ala Ile Val Pro Leu Ala
   1               5                   10                  15
<210> 21
   <211> 12
   <212> PRT
   <213> Artificial Sequence
<220>
   <223> MTD JO-21
<400> 21
   Ile Ala Val Ala Ile Ala Ala Ile Val Pro Leu Ala
   1               5                   10
<210> 22
   <211> 15
   <212> PRT
   <213> Artificial Sequence
<220>
   <223> MTD JO-22
<400> 22
   Val Ala Met Ala Ala Ala Ala Val Leu Ala Ala Pro Ala Leu Ala
   1 5 10 15
<210> 23
   <211> 11
   <212> PRT
   <213> Artificial Sequence
<220>
   <223> MTD JO-23
<400> 23
   Leu Ala Val Leu Val Leu Leu Val Leu Leu Pro
   1 5 10
<210> 24
   <211> 9
   <212> PRT
   <213> Artificial Sequence
<220>
   <223> MTD JO-24
<400> 24
   Val Val Ala Val Leu Ala Pro Val Leu
   1 5
<210> 25
   <211> 12
   <212> PRT
   <213> Artificial Sequence
<220>
   <223> MTD JO-25
<400> 25
   Ala Ala Leu Leu Leu Pro Leu Leu Leu Leu Leu Pro
   1 5 10
<210> 26
   <211> 10
   <212> PRT
   <213> Artificial Sequence
<220>
   <223> MTD JO-26
<400> 26
   Pro Ala Ala Val Ala Ala Leu Leu Val Ile
   1 5 10
<210> 27
   <211> 8
   <212> PRT
   <213> Artificial Sequence
<220>
   <223> MTD JO-27
<400> 27
   Leu Leu Ile Ala Ala Leu Leu Pro
    1               5
<210> 28
   <211> 12
   <212> PRT
   <213> Artificial Sequence
<220>
   <223> MTD JO-28
<400> 28
   Ala Ala Val Val Leu Leu Pro Leu Ala Ala Ala Pro
    1               5                  10
<210> 29
   <211> 10
   <212> PRT
   <213> Artificial Sequence
<220>
   <223> MTD JO-29
<400> 29
   Ala Ala Ala Ala Ala Ala Leu Leu Val Pro
    1               5                  10
<210> 30
   <211> 8
   <212> PRT
   <213> Artificial Sequence
<220>
   <223> MTD JO-30
<400> 30
   Leu Pro Val Val Ala Leu Leu Ala
    1               5
<210> 31
   <211> 11
   <212> PRT
   <213> Artificial Sequence
<220>
   <223> MTD JO-31
<400> 31
   Ala Ala Ala Leu Ala Ala Pro Leu Ala Leu Pro
   1 5 10
<210> 32
   <211> 9
   <212> PRT
   <213> Artificial Sequence
<220>
   <223> MTD JO-32
<400> 32
   Leu Leu Leu Ala Leu Leu Leu Ala Ala
   1 5
<210> 33
   <211> 8
   <212> PRT
   <213> Artificial Sequence
<220>
   <223> MTD JO-33
<400> 33
   Ala Val Ala Val Val Ala Leu Leu
   1 5
<210> 34
   <211> 11
   <212> PRT
   <213> Artificial Sequence
<220>
   <223> MTD JO-34
<400> 34
   Leu Leu Leu Ile Ile Val Leu Leu Ile Val Pro
   1 5 10
<210> 35
   <211> 9
   <212> PRT
   <213> Artificial Sequence
<220>
   <223> MTD JO-35
<400> 35
   Leu Ala Leu Ala Ala Ala Val Val Pro
   1 5
<210> 36
   <211> 10
   <212> PRT
   <213> Artificial Sequence
<220>
   <223> MTD JO-36
<400> 36
   Pro Ala Ala Leu Ala Leu Leu Leu Val Ala
    1               5                  10
<210> 37
   <211> 17
   <212> PRT
   <213> Artificial Sequence
<220>
   <223> MTD JO-37
<400> 37
   Ile Val Ala Leu Leu Leu Val Pro Leu Val Leu Ala Ile Ala Ala Val
    1               5                  10                  15
   Leu
<210> 38
   <211> 8
   <212> PRT
   <213> Artificial Sequence
<220>
   <223> MTD JO-38
<400> 38
   Ile Val Ala Leu Leu Leu Val Pro
    1               5
<210> 39
   <211> 10
   <212> PRT
   <213> Artificial Sequence
<220>
   <223> MTD JO-39
<400> 39
   Pro Leu Val Leu Ala Ile Ala Ala Val Leu
    1               5                  10
<210> 40
   <211> 9
   <212> PRT
   <213> Artificial Sequence
<220>
   <223> MTD JO-40
<400> 40
   Pro Leu Val Leu Ala Ala Leu Val Ala
   1               5
<210> 41
   <211> 8
   <212> PRT
   <213> Artificial Sequence
<220>
   <223> MTD JO-41
<400> 41
   Ala Ala Ala Leu Leu Ala Val Ala
   1               5
<210> 42
   <211> 8
   <212> PRT
   <213> Artificial Sequence
<220>
   <223> MTD JO-42
<400> 42
   Pro Leu Leu Leu Leu Ala Leu Ala
   1               5
<210> 43
   <211> 7
   <212> PRT
   <213> Artificial Sequence
<220>
   <223> MTD JO-43
<400> 43
   Ala Leu Ala Leu Val Val Ala
   1               5
<210> 44
   <211> 8
   <212> PRT
   <213> Artificial Sequence
<220>
   <223> MTD JO-44
<400> 44
   Val Ala Ala Val Val Val Ala Ala
   1 5
<210> 45
   <211> 9
   <212> PRT
   <213> Artificial Sequence
<220>
   <223> MTD JO-45
<400> 45
   Pro Leu Leu Pro Leu Leu Leu Leu Val
   1 5
<210> 46
   <211> 13
   <212> PRT
   <213> Artificial Sequence
<220>
   <223> MTD JO-46
<400> 46
   Val Val Leu Val Val Val Leu Pro Leu Ala Val Leu Ala
   1 5 10
<210> 47
   <211> 10
   <212> PRT
   <213> Artificial Sequence
<220>
   <223> MTD JO-47
<400> 47
   Ala Ala Ala Val Pro Val Leu Val Ala Ala
   1 5 10
<210> 48
   <211> 12
   <212> PRT
   <213> Artificial Sequence
<220>
   <223> MTD JO-48
<400> 48
   Pro Ala Leu Leu Leu Leu Leu Leu Ala Ala Val Val
   1 5 10
<210> 49
   <211> 12
   <212> PRT
   <213> Artificial Sequence
<220>
   <223> MTD JO-49
<400> 49
   Pro Leu Ala Ile Leu Leu Leu Leu Leu Ile Ala Pro
   1 5 10
<210> 50
   <211> 13
   <212> PRT
   <213> Artificial Sequence
<220>
   <223> MTD JO-50
<400> 50
   Pro Leu Leu Ala Leu Val Leu Leu Leu Ala Leu Ile Ala
   1 5 10
<210> 51
   <211> 12
   <212> PRT
   <213> Artificial Sequence
<220>
   <223> MTD JO-51
<400> 51
   Val Val Ala Val Leu Ala Leu Val Leu Ala Ala Leu
   1 5 10
<210> 52
   <211> 9
   <212> PRT
   <213> Artificial Sequence
<220>
   <223> MTD JO-52
<400> 52
   Pro Leu Leu Leu Leu Leu Pro Ala Leu
   1 5
<210> 53
   <211> 12
   <212> PRT
   <213> Artificial Sequence
<220>
   <223> MTD JO-53
<400> 53
   Leu Ala Ala Val Ala Ala Leu Ala Val Val Val Pro
   1 5 10
<210> 54
   <211> 12
   <212> PRT
   <213> Artificial Sequence
<220>
   <223> MTD JO-54
<400> 54
   Leu Leu Leu Leu Val Leu Ile Leu Pro Leu Ala Ala
   1 5 10
<210> 55
   <211> 9
   <212> PRT
   <213> Artificial Sequence
<220>
   <223> MTD JO-55
<400> 55
   Leu Ala Val Val Val Val Ala Ala Val
   1 5
<210> 56
   <211> 9
   <212> PRT
   <213> Artificial Sequence
<220>
   <223> MTD JO-56
<400> 56
   Val Leu Leu Ala Ala Ala Leu Ile Ala
   1 5
<210> 57
   <211> 10
   <212> PRT
   <213> Artificial Sequence
<220>
   <223> MTD JO-57
<400> 57
   Leu Ile Ala Leu Leu Ala Ala Pro Leu Ala
   1 5 10
<210> 58
   <211> 8
   <212> PRT
   <213> Artificial Sequence
<220>
   <223> MTD JO-58
<400> 58
   Leu Ala Leu Leu Leu Leu Ala Ala
   1 5
<210> 59
   <211> 12
   <212> PRT
   <213> Artificial Sequence
<220>
   <223> MTD JO-59
<400> 59
   Leu Leu Ala Ala Ala Leu Leu Leu Leu Leu Leu Ala
   1 5 10
<210> 60
   <211> 10
   <212> PRT
   <213> Artificial Sequence
<220>
   <223> MTD JO-60
<400> 60
   Val Ile Ile Ala Leu Ile Val Ile Val Ala
   1 5 10
<210> 61
   <211> 11
   <212> PRT
   <213> Artificial Sequence
<220>
   <223> MTD JO-61
<400> 61
   Val Val Leu Val Val Ala Ala Val Leu Ala Leu
   1 5 10
<210> 62
   <211> 9
   <212> PRT
   <213> Artificial Sequence
<220>
   <223> MTD JO-62
<400> 62
   Val Ala Val Ala Ile Ala Val Val Leu
   1 5
<210> 63
   <211> 13
   <212> PRT
   <213> Artificial Sequence
<220>
   <223> MTD JO-63
<400> 63
   Pro Leu Ile Val Val Val Ala Ala Ala Val Val Ala Val
   1 5 10
<210> 64
   <211> 11
   <212> PRT
   <213> Artificial Sequence
<220>
   <223> MTD JO-64
<400> 64
   Pro Leu Ala Val Ala Val Ala Ala Val Ala Ala
   1 5 10
<210> 65
   <211> 10
   <212> PRT
   <213> Artificial Sequence
<220>
   <223> MTD JO-65
<400> 65
   Ala Ala Ile Ala Leu Val Ala Val Val Leu
   1 5 10
<210> 66
   <211> 10
   <212> PRT
   <213> Artificial Sequence
<220>
   <223> MTD JO-66
<400> 66
   Ala Ala Ala Leu Ala Ala Ile Ala Val Ile
   1 5 10
<210> 67
   <211> 8
   <212> PRT
   <213> Artificial Sequence
<220>
   <223> MTD JO-67
<400> 67
   Ala Ala Ala Pro Ala Val Ala Ala
   1 5
<210> 68
   <211> 7
   <212> PRT
   <213> Artificial Sequence
<220>
   <223> MTD JO-68
<400> 68
   Leu Leu Leu Ala Ala Leu Pro
   1 5
<210> 69
   <211> 8
   <212> PRT
   <213> Artificial Sequence
<220>
   <223> MTD JO-69
<400> 69
   Ala Leu Leu Ala Val Val Ala Ala
   1 5
<210> 70
   <211> 10
   <212> PRT
   <213> Artificial Sequence
<220>
   <223> MTD JO-70
<400> 70
   Ala Val Val Val Val Leu Pro Ile Leu Leu
   1 5 10
<210> 71
   <211> 9
   <212> PRT
   <213> Artificial Sequence
<220>
   <223> MTD JO-71
<400> 71
   Ala Leu Ala Leu Leu Leu Leu Val Pro
   1 5
<210> 72
   <211> 11
   <212> PRT
   <213> Artificial Sequence
<220>
   <223> MTD JO-72
<400> 72
   Leu Val Val Leu Leu Ala Ala Leu Leu Val Leu
   1 5 10
<210> 73
   <211> 8
   <212> PRT
   <213> Artificial Sequence
<220>
   <223> MTD JO-73
<400> 73
   Pro Val Leu Leu Leu Leu Ala Pro
   1 5
<210> 74
   <211> 8
   <212> PRT
   <213> Artificial Sequence
<220>
   <223> MTD JO-74
<400> 74
   Ala Leu Ala Val Val Ala Ala Pro
   1 5
<210> 75
   <211> 8
   <212> PRT
   <213> Artificial Sequence
<220>
   <223> MTD JO-75
<400> 75
   Val Ile Val Ala Leu Leu Ala Val
   1 5
<210> 76
   <211> 8
   <212> PRT
   <213> Artificial Sequence
<220>
   <223> MTD JO-76
<400> 76
   Ala Leu Val Leu Pro Leu Ala Pro
   1 5
<210> 77
   <211> 9
   <212> PRT
   <213> Artificial Sequence
<220>
   <223> MTD JO-77
<400> 77
   Ala Val Ala Leu Leu Ile Leu Ala Val
   1 5
<210> 78
   <211> 7
   <212> PRT
   <213> Artificial Sequence
<220>
   <223> MTD JO-78
<400> 78
   Val Leu Leu Ala Val Ile Pro
   1 5
<210> 79
   <211> 13
   <212> PRT
   <213> Artificial Sequence
<220>
   <223> MTD JO-79
<400> 79
   Leu Ile Val Ala Ala Val Val Val Val Ala Val Leu Ile
   1 5 10
<210> 80
   <211> 7
   <212> PRT
   <213> Artificial Sequence
<220>
   <223> MTD JO-80
<400> 80
   Ala Val Val Val Ala Ala Pro
   1 5
<210> 81
   <211> 9
   <212> PRT
   <213> Artificial Sequence
<220>
   <223> MTD JO-81
<400> 81
   Leu Ala Ala Val Leu Leu Leu Ile Pro
   1 5
<210> 82
   <211> 10
   <212> PRT
   <213> Artificial Sequence
<220>
   <223> MTD JO-82
<400> 82
   Leu Leu Leu Leu Leu Leu Ala Val Val Pro
   1 5 10
<210> 83
   <211> 11
   <212> PRT
   <213> Artificial Sequence
<220>
   <223> MTD JO-83
<400> 83
   Ala Val Ala Leu Val Ala Val Val Ala Val Ala
   1 5 10
<210> 84
   <211> 9
   <212> PRT
   <213> Artificial Sequence
<220>
   <223> MTD JO-84
<400> 84
   Leu Val Ala Ala Leu Leu Ala Val Leu
   1               5
<210> 85
   <211> 11
   <212> PRT
   <213> Artificial Sequence
<220>
   <223> MTD JO-85
<400> 85
   Leu Leu Ala Ala Ala Ala Ala Leu Leu Leu Ala
   1               5                   10
<210> 86
   <211> 8
   <212> PRT
   <213> Artificial Sequence
<220>
   <223> MTD JO-86
<400> 86
   Leu Ala Val Leu Ala Ala Ala Pro
   1               5
<210> 87
   <211> 13
   <212> PRT
   <213> Artificial Sequence
<220>
   <223> MTD JO-87
<400> 87
   Val Val Val Leu Leu Val Leu Leu Ala Leu Val Val Val
   1               5                   10
<210> 88
   <211> 7
   <212> PRT
   <213> Artificial Sequence
<220>
   <223> MTD JO-88
<400> 88
   Val Val Ile Ala Val Val Pro
   1 5
<210> 89
   <211> 9
   <212> PRT
   <213> Artificial Sequence
<220>
   <223> MTD JO-89
<400> 89
   Val Leu Leu Val Leu Leu Ala Leu Val
   1 5
<210> 90
   <211> 9
   <212> PRT
   <213> Artificial Sequence
<220>
   <223> MTD JO-90
<400> 90
   Val Leu Leu Val Leu Leu Ala Leu Val
   1 5
<210> 91
   <211> 8
   <212> PRT
   <213> Artificial Sequence
<220>
   <223> MTD JO-91
<400> 91
   Pro Val Leu Val Pro Ala Val Pro
   1 5
<210> 92
   <211> 8
   <212> PRT
   <213> Artificial Sequence
<220>
   <223> MTD JO-92
<400> 92
   Pro Ala Leu Ala Leu Ala Leu Ala
   1 5
<210> 93
   <211> 8
   <212> PRT
   <213> Artificial Sequence
<220>
   <223> MTD JO-93
<400> 93
   Ala Ala Ala Ala Pro Ala Leu Ala
   1 5
<210> 94
   <211> 9
   <212> PRT
   <213> Artificial Sequence
<220>
   <223> MTD JO-94
<400> 94
   Ile Val Leu Pro Val Leu Ala Ala Pro
   1 5
<210> 95
   <211> 10
   <212> PRT
   <213> Artificial Sequence
<220>
   <223> MTD JO-95
<400> 95
   Leu Val Leu Leu Leu Leu Pro Leu Leu Ile
   1 5 10
<210> 96
   <211> 11
   <212> PRT
   <213> Artificial Sequence
<220>
   <223> MTD JO-96
<400> 96
   Leu Ala Ala Val Ala Pro Ala Leu Ala Val Val
   1 5 10
<210> 97
   <211> 8
   <212> PRT
   <213> Artificial Sequence
<220>
   <223> MTD JO-97
<400> 97
   Ile Leu Val Leu Val Leu Pro Ile
    1               5
<210> 98
   <211> 9
   <212> PRT
   <213> Artificial Sequence
<220>
   <223> MTD JO-98
<400> 98
   Ile Leu Leu Pro Leu Leu Leu Leu Pro
    1               5
<210> 99
   <211> 10
   <212> PRT
   <213> Artificial Sequence
<220>
   <223> MTD JO-99
<400> 99
   Ile Ala Pro Ala Val Val Ala Ala Leu Pro
    1               5                  10
<210> 100
   <211> 12
   <212> PRT
   <213> Artificial Sequence
<220>
   <223> MTD JO-100
<400> 100
   Leu Leu Leu Val Ala Val Val Pro Leu Leu Val Pro
    1               5                  10
<210> 101
   <211> 9
   <212> PRT
   <213> Artificial Sequence
<220>
   <223> MTD JO-101
<400> 101
   Leu Ile Leu Leu Leu Leu Pro Ile Ile
   1 5
<210> 102
   <211> 10
   <212> PRT
   <213> Artificial Sequence
<220>
   <223> MTD JO-102
<400> 102
   Ala Val Leu Ala Ala Pro Ala Val Leu Val
   1 5 10
<210> 103
   <211> 9
   <212> PRT
   <213> Artificial Sequence
<220>
   <223> MTD JO-103
<400> 103
   Leu Ala Leu Pro Val Leu Leu Leu Ala
   1 5
<210> 104
   <211> 7
   <212> PRT
   <213> Artificial Sequence
<220>
   <223> MTD JO-104
<400> 104
   Leu Ala Leu Ala Leu Leu Leu
   1 5
<210> 105
   <211> 9
   <212> PRT
   <213> Artificial Sequence
<220>
   <223> MTD JO-105
<400> 105
   Val Ala Val Pro Leu Leu Val Val Ala
   1 5
<210> 106
   <211> 11
   <212> PRT
   <213> Artificial Sequence
<220>
   <223> MTD JO-106
<400> 106
   Ala Val Ala Val Ala Pro Val Ala Ala Ala Ala
   1 5 10
<210> 107
   <211> 11
   <212> PRT
   <213> Artificial Sequence
<220>
   <223> MTD JO-107
<400> 107
   Ala Ala Ala Val Val Ala Ala Val Pro Ala Ala
   1 5 10
<210> 108
   <211> 9
   <212> PRT
   <213> Artificial Sequence
<220>
   <223> MTD JO-108
<400> 108
   Ala Leu Leu Ala Ala Leu Leu Ala Pro
   1 5
<210> 109
   <211> 7
   <212> PRT
   <213> Artificial Sequence
<220>
   <223> MTD JO-109
<400> 109
   Leu Leu Ala Leu Leu Val Pro
   1 5
<210> 110
   <211> 14
   <212> PRT
   <213> Artificial Sequence
<220>
   <223> MTD JO-110
<400> 110
   Ala Leu Leu Ala Ala Leu Leu Ala Leu Leu Ala Leu Leu Val
   1 5 10
<210> 111
   <211> 11
   <212> PRT
   <213> Artificial Sequence
<220>
   <223> MTD JO-111
<400> 111
   Ala Ala Ala Leu Pro Leu Leu Val Leu Leu Pro
   1 5 10
<210> 112
   <211> 10
   <212> PRT
   <213> Artificial Sequence
<220>
   <223> MTD JO-112
<400> 112
   Ala Ala Ala Val Pro Ala Ala Leu Ala Pro
   1 5 10
<210> 113
   <211> 10
   <212> PRT
   <213> Artificial Sequence
<220>
   <223> MTD JO-113
<400> 113
   Ala Ala Leu Ala Val Ala Ala Leu Ala Ala
   1 5 10
<210> 114
   <211> 8
   <212> PRT
   <213> Artificial Sequence
<220>
   <223> MTD JO-114
<400> 114
   Ala Val Leu Ala Ala Ala Val Pro
   1 5
<210> 115
   <211> 8
   <212> PRT
   <213> Artificial Sequence
<220>
   <223> MTD JO-115
<400> 115
   Val Ala Ala Leu Pro Ala Pro Ala
   1 5
<210> 116
   <211> 11
   <212> PRT
   <213> Artificial Sequence
<220>
   <223> MTD JO-116
<400> 116
   Ala Leu Ala Leu Ala Val Pro Ala Val Leu Pro
   1 5 10
<210> 117
   <211> 11
   <212> PRT
   <213> Artificial Sequence
<220>
   <223> MTD JO-117
<400> 117
   Ala Ala Leu Leu Pro Ala Ala Val Ala Val Pro
   1 5 10
<210> 118
   <211> 8
   <212> PRT
   <213> Artificial Sequence
<220>
   <223> MTD JO-118
<400> 118
   Ala Val Val Val Ala Leu Ala Pro
   1 5
<210> 119
   <211> 14
   <212> PRT
   <213> Artificial Sequence
<220>
   <223> MTD JO-119
<400> 119
   Ala Ala Ala Val Ala Leu Pro Ala Ala Ala Ala Leu Leu Ala
    1               5                  10
<210> 120
   <211> 13
   <212> PRT
   <213> Artificial Sequence
<220>
   <223> MTD JO-120
<400> 120
   Ala Val Val Leu Pro Leu Ala Leu Val Ala Val Ala Pro
    1               5                  10
<210> 121
   <211> 8
   <212> PRT
   <213> Artificial Sequence
<220>
   <223> MTD JO-121
<400> 121
   Leu Val Ala Leu Pro Leu Leu Pro
    1               5
<210> 122
   <211> 10
   <212> PRT
   <213> Artificial Sequence
<220>
   <223> MTD JO-122
<400> 122
   Val Val Val Pro Leu Leu Leu Ile Val Pro
    1               5                  10
<210> 123
   <211> 8
   <212> PRT
   <213> Artificial Sequence
<220>
   <223> MTD JO-123
<400> 123
   Leu Ala Val Val Leu Ala Val Pro
   1               5
<210> 124
   <211> 10
   <212> PRT
   <213> Artificial Sequence
<220>
   <223> MTD JO-124
<400> 124
   Leu Leu Ala Val Pro Ile Leu Leu Val Pro
   1               5                   10
<210> 125
   <211> 8
   <212> PRT
   <213> Artificial Sequence
<220>
   <223> MTD JO-125
<400> 125
   Leu Val Ala Leu Val Leu Leu Pro
   1               5
<210> 126
   <211> 14
   <212> PRT
   <213> Artificial Sequence
<220>
   <223> MTD JO-126
<400> 126
   Leu Val Leu Leu Leu Ala Val Leu Leu Leu Ala Val Leu Pro
   1               5                   10
<210> 127
   <211> 12
   <212> PRT
   <213> Artificial Sequence
<220>
   <223> MTD JO-127
<400> 127
   Leu Leu Ala Pro Val Val Ala Leu Val Ile Leu Pro
   1 5 10
<210> 128
   <211> 13
   <212> PRT
   <213> Artificial Sequence
<220>
   <223> MTD JO-128
<400> 128
   Val Leu Ala Val Leu Ala Val Pro Val Leu Leu Leu Pro
   1 5 10
<210> 129
   <211> 10
   <212> PRT
   <213> Artificial Sequence
<220>
   <223> MTD JO-129
<400> 129
   Val Val Ile Ala Val Val Pro Val Val Val
   1 5 10
<210> 130
   <211> 11
   <212> PRT
   <213> Artificial Sequence
<220>
   <223> MTD JO-130
<400> 130
   Leu Leu Val Leu Leu Ala Leu Val Val Val Pro
   1 5 10
<210> 131
   <211> 11
   <212> PRT
   <213> Artificial Sequence
<220>
   <223> MTD JO-131
<400> 131
   Val Leu Leu Ala Leu Pro Val Val Ala Ala Pro
   1 5 10
<210> 132
   <211> 12
   <212> PRT
   <213> Artificial Sequence
<220>
   <223> MTD JO-132
<400> 132
   Ala Val Val Val Pro Ala Ile Val Leu Ala Ala Pro
    1               5                  10
<210> 133
   <211> 11
   <212> PRT
   <213> Artificial Sequence
<220>
   <223> MTD JO-133
<400> 133
   Ala Val Leu Val Pro Ala Ala Ala Leu Val Pro
    1               5                  10
<210> 134
   <211> 10
   <212> PRT
   <213> Artificial Sequence
<220>
   <223> MTD JO-134
<400> 134
   Val Val Ala Ala Leu Pro Leu Val Leu Pro
    1               5                  10
<210> 135
   <211> 10
   <212> PRT
   <213> Artificial Sequence
<220>
   <223> MTD JO-135
<400> 135
   Ala Ala Val Ala Leu Pro Ala Ala Ala Pro
    1               5                  10
<210> 136
   <211> 8
   <212> PRT
   <213> Artificial Sequence
<220>
   <223> MTD JO-136
<400> 136
   Leu Ile Ala Leu Pro Leu Leu Pro
   1 5
<210> 137
   <211> 13
   <212> PRT
   <213> Artificial Sequence
<220>
   <223> MTD JO-137
<400> 137
   Leu Leu Ala Leu Pro Leu Val Leu Val Leu Ala Leu Pro
   1 5 10
<210> 138
   <211> 9
   <212> PRT
   <213> Artificial Sequence
<220>
   <223> MTD JO-138
<400> 138
   Ile Val Pro Leu Leu Leu Ala Ala Pro
   1 5
<210> 139
   <211> 10
   <212> PRT
   <213> Artificial Sequence
<220>
   <223> MTD JO-139
<400> 139
   Leu Leu Leu Ala Pro Leu Leu Leu Ala Pro
   1 5 10
<210> 140
   <211> 10
   <212> PRT
   <213> Artificial Sequence
<220>
   <223> MTD JO-140
<400> 140
   Leu Ala Ala Leu Pro Val Ala Ala Val Pro
   1 5 10
<210> 141
   <211> 10
   <212> PRT
   <213> Artificial Sequence
<220>
   <223> MTD JO-141
<400> 141
   Ala Leu Ala Val Ile Val Leu Val Leu Leu
   1 5 10
<210> 142
   <211> 10
   <212> PRT
   <213> Artificial Sequence
<220>
   <223> MTD JO-142
<400> 142
   Leu Ala Leu Leu Leu Pro Ala Ala Leu Ile
   1 5 10
<210> 143
   <211> 11
   <212> PRT
   <213> Artificial Sequence
<220>
   <223> MTD JO-143
<400> 143
   Ala Leu Leu Pro Leu Leu Ala Val Val Leu Pro
   1 5 10
<210> 144
   <211> 10
   <212> PRT
   <213> Artificial Sequence
<220>
   <223> MTD JO-144
<400> 144
   Ala Ile Ala Val Pro Val Leu Ala Ala Pro
   1 5 10
<210> 145
   <211> 10
   <212> PRT
   <213> Artificial Sequence
<220>
   <223> MTD JO-145
<400> 145
   Ala Ala Ala Pro Val Leu Leu Leu Leu Leu
    1               5                   10
<210> 146
   <211> 11
   <212> PRT
   <213> Artificial Sequence
<220>
   <223> MTD JO-146
<400> 146
   Ala Ala Ala Val Ala Val Leu Ala Leu Ala Pro
    1               5                   10
<210> 147
   <211> 11
   <212> PRT
   <213> Artificial Sequence
<220>
   <223> MTD JO-147
<400> 147
   Ala Ala Leu Ala Ala Leu Val Val Ala Ala Pro
    1               5                   10
<210> 148
   <211> 12
   <212> PRT
   <213> Artificial Sequence
<220>
   <223> MTD JO-148
<400> 148
   Ala Ala Leu Ala Ala Val Pro Leu Ala Leu Ala Pro
    1               5                   10
<210> 149
   <211> 13
   <212> PRT
   <213> Artificial Sequence
<220>
   <223> MTD JO-149
<400> 149
   Ala Leu Ala Val Ala Ala Pro Ala Leu Ala Leu Leu Pro
   1 5 10
<210> 150
   <211> 8
   <212> PRT
   <213> Artificial Sequence
<220>
   <223> MTD JO-150
<400> 150
   Ala Ala Leu Pro Ala Ala Ala Pro
   1 5
<210> 151
   <211> 9
   <212> PRT
   <213> Artificial Sequence
<220>
   <223> MTD JO-151
<400> 151
   Ala Ala Ala Pro Val Ala Ala Val Pro
   1 5
<210> 152
   <211> 10
   <212> PRT
   <213> Artificial Sequence
<220>
   <223> MTD JO-152
<400> 152
   Leu Leu Ala Val Leu Leu Ala Leu Leu Pro
   1 5 10
<210> 153
   <211> 10
   <212> PRT
   <213> Artificial Sequence
<220>
   <223> MTD JO-153
<400> 153
   Val Leu Ala Leu Leu Val Ala Val Val Pro
   1 5 10
<210> 154
   <211> 9
   <212> PRT
   <213> Artificial Sequence
<220>
   <223> MTD JO-154
<400> 154
   Ala Leu Val Val Pro Ala Ala Val Pro
   1               5
<210> 155
   <211> 9
   <212> PRT
   <213> Artificial Sequence
<220>
   <223> MTD JO-155
<400> 155
   Ala Val Val Leu Pro Leu Leu Leu Pro
   1               5
<210> 156
   <211> 10
   <212> PRT
   <213> Artificial Sequence
<220>
   <223> MTD JO-156
<400> 156
   Ala Val Ile Pro Val Ala Val Leu Val Pro
   1               5                   10
<210> 157
   <211> 11
   <212> PRT
   <213> Artificial Sequence
<220>
   <223> MTD JO-157
<400> 157
   Ala Ala Ala Val Pro Ala Ala Val Leu Ala Pro
   1               5                   10
<210> 158
   <211> 11
   <212> PRT
   <213> Artificial Sequence
<220>
   <223> MTD JO-158
<400> 158
   Val Ala Val Pro Val Val Leu Ala Ile Leu Pro
    1               5                   10
<210> 159
   <211> 12
   <212> PRT
   <213> Artificial Sequence
<220>
   <223> MTD JO-159
<400> 159
   Ile Ala Ile Ala Ala Ile Pro Ala Ile Leu Ala Leu
    1               5                   10
<210> 160
   <211> 10
   <212> PRT
   <213> Artificial Sequence
<220>
   <223> MTD JO-160
<400> 160
   Ala Leu Ile Ala Pro Ala Leu Ala Ala Pro
    1               5                   10
<210> 161
   <211> 10
   <212> PRT
   <213> Artificial Sequence
<220>
   <223> MTD JO-161
<400> 161
   Ala Ala Ile Ala Leu Val Ala Pro Ala Leu
    1               5                   10
<210> 162
   <211> 9
   <212> PRT
   <213> Artificial Sequence
<220>
   <223> MTD JO-162
<400> 162
   Leu Ala Pro Ala Val Ala Ala Ala Pro
   1 5
<210> 163
   <211> 15
   <212> PRT
   <213> Artificial Sequence
<220>
   <223> MTD JO-163
<400> 163
   Val Ala Ile Ile Val Pro Ala Val Val Ala Ile Ala Leu Ile Ile
   1 5 10 15
<210> 164
   <211> 9
   <212> PRT
   <213> Artificial Sequence
<220>
   <223> MTD JO-164
<400> 164
   Ala Val Val Ala Ile Ala Leu Ile Ile
   1 5
<210> 165
   <211> 9
   <212> PRT
   <213> Artificial Sequence
<220>
   <223> MTD JO-165
<400> 165
   Leu Ala Ala Val Pro Ala Ala Ala Pro
   1 5
<210> 166
   <211> 10
   <212> PRT
   <213> Artificial Sequence
<220>
   <223> MTD JO-166
<400> 166
   Ala Val Ala Ala Leu Pro Leu Ala Ala Pro
   1 5 10
<210> 167
   <211> 9
   <212> PRT
   <213> Artificial Sequence
<220>
   <223> MTD JO-167
<400> 167
   Leu Ala Ala Pro Ala Ala Ala Ala Pro
    1               5
<210> 168
   <211> 12
   <212> PRT
   <213> Artificial Sequence
<220>
   <223> MTD JO-168
<400> 168
   Leu Ala Ala Val Val Pro Val Ala Ala Ala Val Pro
    1               5                   10
<210> 169
   <211> 9
   <212> PRT
   <213> Artificial Sequence
<220>
   <223> MTD JO-169
<400> 169
   Val Ala Ala Pro Ala Ala Ala Ala Pro
    1               5
<210> 170
   <211> 8
   <212> PRT
   <213> Artificial Sequence
<220>
   <223> MTD JO-170
<400> 170
   Ala Val Pro Val Pro Val Pro Leu
    1               5
<210> 171
   <211> 10
   <212> PRT
   <213> Artificial Sequence
<220>
   <223> MTD JO-171
<400> 171
   Leu Leu Ile Leu Pro Ile Val Leu Leu Pro
    1               5                  10
<210> 172
   <211> 11
   <212> PRT
   <213> Artificial Sequence
<220>
   <223> MTD JO-172
<400> 172
   Ala Leu Ala Leu Pro Ala Leu Ala Ile Ala Pro
    1               5                  10
<210> 173
   <211> 9
   <212> PRT
   <213> Artificial Sequence
<220>
   <223> MTD JO-173
<400> 173
   Ala Val Ile Pro Ile Leu Ala Val Pro
    1               5
<210> 174
   <211> 11
   <212> PRT
   <213> Artificial Sequence
<220>
   <223> MTD JO-174
<400> 174
   Leu Ile Leu Leu Leu Pro Ala Val Ala Leu Pro
    1               5                  10
<210> 175
   <211> 10
   <212> PRT
   <213> Artificial Sequence
<220>
   <223> MTD JO-175
<400> 175
   Ile Val Leu Ala Pro Val Pro Ala Ala Ala
   1 5 10
<210> 176
   <211> 11
   <212> PRT
   <213> Artificial Sequence
<220>
   <223> MTD JO-176
<400> 176
   Val Val Val Val Pro Val Leu Ala Ala Ala Ala
   1 5 10
<210> 177
   <211> 7
   <212> PRT
   <213> Artificial Sequence
<220>
   <223> MTD JO-177
<400> 177
   Leu Val Ala Val Ala Ala Pro
   1 5
<210> 178
   <211> 10
   <212> PRT
   <213> Artificial Sequence
<220>
   <223> MTD JO-178
<400> 178
   Leu Val Leu Ala Ala Pro Ala Ala Leu Pro
   1 5 10
<210> 179
   <211> 9
   <212> PRT
   <213> Artificial Sequence
<220>
   <223> MTD JO-179
<400> 179
   Leu Ile Ala Pro Ala Ala Ala Val Pro
   1 5
<210> 180
   <211> 10
   <212> PRT
   <213> Artificial Sequence
<220>
   <223> MTD JO-180
<400> 180
   Ala Leu Ala Ala Leu Pro Ile Ala Leu Pro
    1               5                   10
<210> 181
   <211> 9
   <212> PRT
   <213> Artificial Sequence
<220>
   <223> MTD JO-181
<400> 181
   Ala Val Leu Leu Leu Pro Ala Ala Ala
    1               5
<210> 182
   <211> 9
   <212> PRT
   <213> Artificial Sequence
<220>
   <223> MTD JO-182
<400> 182
   Ile Ala Leu Ala Leu Leu Pro Leu Leu
    1               5
<210> 183
   <211> 10
   <212> PRT
   <213> Artificial Sequence
<220>
   <223> MTD JO-183
<400> 183
   Val Leu Leu Ala Ala Ala Leu Ile Ala Pro
    1               5                   10
<210> 184
   <211> 11
   <212> PRT
   <213> Artificial Sequence
<220>
   <223> MTD JO-184
<400> 184
   Ala Pro Ala Val Leu Pro Pro Val Val Val Ile
   1 5 10
<210> 185
   <211> 9
   <212> PRT
   <213> Artificial Sequence
<220>
   <223> MTD JO-185
<400> 185
   Val Val Gly Leu Leu Val Ala Ala Leu
   1 5
<210> 186
   <211> 11
   <212> PRT
   <213> Artificial Sequence
<220>
   <223> MTD JO-186
<400> 186
   Ala Ala Ile Ala Ala Ala Ala Pro Leu Ala Ala
   1 5 10
<210> 187
   <211> 7
   <212> PRT
   <213> Artificial Sequence
<220>
   <223> MTD JO-187
<400> 187
   Leu Leu Leu Ala Val Ala Pro
   1 5
<210> 188
   <211> 10
   <212> PRT
   <213> Artificial Sequence
<220>
   <223> MTD JO-188
<400> 188
   Leu Ile Leu Leu Leu Pro Leu Ala Ala Leu
   1 5 10
<210> 189
   <211> 8
   <212> PRT
   <213> Artificial Sequence
<220>
   <223> MTD JO-189
<400> 189
   Ala Leu Leu Leu Leu Val Leu Ala
   1               5
<210> 190
   <211> 9
   <212> PRT
   <213> Artificial Sequence
<220>
   <223> MTD JO-190
<400> 190
   Leu Leu Leu Leu Leu Leu Pro Leu Ala
   1               5
<210> 191
   <211> 8
   <212> PRT
   <213> Artificial Sequence
<220>
   <223> MTD JO-191
<400> 191
   Leu Ala Leu Pro Leu Leu Leu Pro
   1               5
<210> 192
   <211> 8
   <212> PRT
   <213> Artificial Sequence
<220>
   <223> MTD JO-192
<400> 192
   Leu Leu Val Leu Pro Leu Leu Ile
   1               5
<210> 193
   <211> 9
   <212> PRT
   <213> Artificial Sequence
<220>
   <223> MTD JO-193
<400> 193
   Leu Pro Leu Leu Pro Ala Ala Leu Val
   1 5
<210> 194
   <211> 36
   <212> DNA
   <213> Artificial Sequence
<220>
   <223> MTD JO-01
<400> 194
   gcggtggtgg tgtgcgcgat tgtgctggcg gcgccg 36
<210> 195
   <211> 36
   <212> DNA
   <213> Artificial Sequence
<220>
   <223> MTD JO-02
<400> 195
   ccgctggcgc tgctggtgct gctgctgctg ggcccg 36
<210> 196
   <211> 33
   <212> DNA
   <213> Artificial Sequence
<220>
   <223> MTD JO-03
<400> 196
   ctgctgctgg cgtttgcgct gctgtgcctg ccg 33
<210> 197
   <211> 39
   <212> DNA
   <213> Artificial Sequence
<220>
   <223> MTD JO-04
<400> 197
   ctgctgggcg cactggcggc ggtgctgctg gcgctggcg 39
<210> 198
   <211> 51
   <212> DNA
   <213> Artificial Sequence
<220>
   <223> MTD JO-05
<400> 198
   ccggtgctgc tggcgctggg cgtgggcctg gtgctgctgg gcctggcggt g 51
<210> 199
   <211> 27
   <212> DNA
   <213> Artificial Sequence
<220>
   <223> MTD JO-06
<400> 199
   gcggcggcgg cggtgctgct ggcggcg 27
<210> 200
   <211> 24
   <212> DNA
   <213> Artificial Sequence
<220>
   <223> MTD JO-07
<400> 200
   attgtggtgg cggtggtggt gatt 24
<210> 201
   <211> 27
   <212> DNA
   <213> Artificial Sequence
<220>
   <223> MTD JO-08
<400> 201
   gcggtgctgg cgccggtggt ggcggtg 27
<210> 202
   <211> 39
   <212> DNA
   <213> Artificial Sequence
<220>
   <223> MTD JO-09
<400> 202
   ctggcggtgt gcggcctgcc ggtggtggcg ctgctggcg 39
<210> 203
   <211> 48
   <212> DNA
   <213> Artificial Sequence
<220>
   <223> MTD JO-10
<400> 203
   ctgggcggcg cggtggtggc ggcgccggtg gcggcggcgg tggcgccg 48
<210> 204
   <211> 39
   <212> DNA
   <213> Artificial Sequence
<220>
   <223> MTD JO-11
<400> 204
   ctgctgctgg tgctggcggt gctgctggcg gtgctgccg 39
<210> 205
   <211> 36
   <212> DNA
   <213> Artificial Sequence
<220>
   <223> MTD JO-12
<400> 205
   ctgctgattc tgctgctgct gccgctgctg attgtg 36
<210> 206
   <211> 33
   <212> DNA
   <213> Artificial Sequence
<220>
   <223> MTD JO-13
<400> 206
   ctggcggcgg cggcgctggc ggtgctgccg ctg 33
<210> 207
   <211> 42
   <212> DNA
   <213> Artificial Sequence
<220>
   <223> MTD JO-14
<400> 207
   tttctgatgc tgctgctgcc gctgctgctg ctgctggtgg cg 42
<210> 208
   <211> 45
   <212> DNA
   <213> Artificial Sequence
<220>
   <223> MTD JO-15
<400> 208
   gcggcggcgg cggcggcgct gggcctggcg gcggcggtgc cggcg 45
<210> 209
   <211> 42
   <212> DNA
   <213> Artificial Sequence
<220>
   <223> MTD JO-16
<400> 209
   ctgctgctgg cggcgctgct gctgattgcg tttgcggcgg tg 42
<210> 210
   <211> 42
   <212> DNA
   <213> Artificial Sequence
<220>
   <223> MTD JO-17
<400> 210
   gcgctggcgg cggtggtgct gattccgctg ggcattgcgg cg 42
<210> 211
   <211> 48
   <212> DNA
   <213> Artificial Sequence
<220>
   <223> MTD JO-18
<400> 211
   gcggcgctgg cgctgggcgt ggcggcggcg ccggcggcgg cgccggcg 48
<210> 212
   <211> 42
   <212> DNA
   <213> Artificial Sequence
<220>
   <223> MTD JO-19
<400> 212
   gcggcgctga ttggcgcggt gctggcgccg gtggtggcgg tg 42
<210> 213
   <211> 45
   <212> DNA
   <213> Artificial Sequence
<220>
   <223> MTD JO-20
<400> 213
   gcggcgggca ttgcggtggc gattgcggcg attgtgccgc tggcg 45
<210> 214
   <211> 36
   <212> DNA
   <213> Artificial Sequence
<220>
   <223> MTD JO-21
<400> 214
   attgcggtgg cgattgcggc gattgtgccg ctggcg 36
<210> 215
   <211> 45
   <212> DNA
   <213> Artificial Sequence
<220>
   <223> MTD JO-22
<400> 215
   gtggcgatgg cggcggcggc ggtgctggcg gcgccggcgc tggcg 45
<210> 216
   <211> 33
   <212> DNA
   <213> Artificial Sequence
<220>
   <223> MTD JO-23
<400> 216
   ctggcggtgc tggtgctgct ggtgctgctg ccg 33
<210> 217
   <211> 27
   <212> DNA
   <213> Artificial Sequence
<220>
   <223> MTD JO-24
<400> 217
   gtggtggcgg tgctggcgcc ggtgctg 27
<210> 218
   <211> 36
   <212> DNA
   <213> Artificial Sequence
<220>
   <223> MTD JO-25
<400> 218
   gcggcgctgc tgctgccgct gctgctgctg ctgccg 36
<210> 219
   <211> 30
   <212> DNA
   <213> Artificial Sequence
<220>
   <223> MTD JO-26
<400> 219
   ccggcggcgg tggcggcgct gctggtgatt 30
<210> 220
   <211> 24
   <212> DNA
   <213> Artificial Sequence
<220>
   <223> MTD JO-27
<400> 220
   ctgctgattg cggcgctgct gccg 24
<210> 221
   <211> 36
   <212> DNA
   <213> Artificial Sequence
<220>
   <223> MTD JO-28
<400> 221
   gcggcggtgg tgctgctgcc gctggcggcg gcgccg 36
<210> 222
   <211> 30
   <212> DNA
   <213> Artificial Sequence
<220>
   <223> MTD JO-29
<400> 222
   gcggcggcgg cggcggcgct gctggtgccg 30
<210> 223
   <211> 24
   <212> DNA
   <213> Artificial Sequence
<220>
   <223> MTD JO-30
<400> 223
   ctgccggtgg tggcgctgct ggcg 24
<210> 224
   <211> 33
   <212> DNA
   <213> Artificial Sequence
<220>
   <223> MTD JO-31
<400> 224
   gcggcggcgc tggcggcgcc gctggcgctg ccg 33
<210> 225
   <211> 27
   <212> DNA
   <213> Artificial Sequence
<220>
   <223> MTD JO-32
<400> 225
   ctgctgctgg cgctgctgct ggcggcg 27
<210> 226
   <211> 24
   <212> DNA
   <213> Artificial Sequence
<220>
   <223> MTD JO-33
<400> 226
   gcggtggcgg tggtggcgct gctg 24
<210> 227
   <211> 33
   <212> DNA
   <213> Artificial Sequence
<220>
   <223> MTD JO-34
<400> 227
   ctgctgctga ttattgtgct gctgattgtg ccg 33
<210> 228
   <211> 27
   <212> DNA
   <213> Artificial Sequence
<220>
   <223> MTD JO-35
<400> 228
   ctggcgctgg cggcggcggt ggtgccg 27
<210> 229
   <211> 30
   <212> DNA
   <213> Artificial Sequence
<220>
   <223> MTD JO-36
<400> 229
   ccggcggcgc tggcgctgct gctggtggcg 30
<210> 230
   <211> 51
   <212> DNA
   <213> Artificial Sequence
<220>
   <223> MTD JO-37
<400> 230
   attgtggcgc tgctgctggt gccgctggtg ctggcgattg cggcggtgct g 51
<210> 231
   <211> 24
   <212> DNA
   <213> Artificial Sequence
<220>
   <223> MTD JO-38
<400> 231
   attgtggcgc tgctgctggt gccg 24
<210> 232
   <211> 30
   <212> DNA
   <213> Artificial Sequence
<220>
   <223> MTD JO-39
<400> 232
   ccgctggtgc tggcgattgc ggcggtgctg 30
<210> 233
   <211> 27
   <212> DNA
   <213> Artificial Sequence
<220>
   <223> MTD JO-40
<400> 233
   ccgctggtgc tggcggcgct ggtggcg 27
<210> 234
   <211> 24
   <212> DNA
   <213> Artificial Sequence
<220>
   <223> MTD JO-41
<400> 234
   gcggcggcgc tgctggcggt ggcg 24
<210> 235
   <211> 24
   <212> DNA
   <213> Artificial Sequence
<220>
   <223> MTD JO-42
<400> 235
   ccgctgctgc tgctggcgct ggcg 24
<210> 236
   <211> 21
   <212> DNA
   <213> Artificial Sequence
<220>
   <223> MTD JO-43
<400> 236
   gcgctggcgc tggtggtggc g 21
<210> 237
   <211> 24
   <212> DNA
   <213> Artificial Sequence
<220>
   <223> MTD JO-44
<400> 237
   gtggcggcgg tggtggtggc ggcg 24
<210> 238
   <211> 27
   <212> DNA
   <213> Artificial Sequence
<220>
   <223> MTD JO-45
<400> 238
   ccgctgctgc cgctgctgct gctggtg 27
<210> 239
   <211> 39
   <212> DNA
   <213> Artificial Sequence
<220>
   <223> MTD JO-46
<400> 239
   gtggtgctgg tggtggtgct gccgctggcg gtgctggcg 39
<210> 240
   <211> 30
   <212> DNA
   <213> Artificial Sequence
<220>
   <223> MTD JO-47
<400> 240
   gcggcggcgg tgccggtgct ggtggcggcg 30
<210> 241
   <211> 36
   <212> DNA
   <213> Artificial Sequence
<220>
   <223> MTD JO-48
<400> 241
   ccggcgctgc tgctgctgct gctggcggcg gtggtg 36
<210> 242
   <211> 36
   <212> DNA
   <213> Artificial Sequence
<220>
   <223> MTD JO-49
<400> 242
   ccgctggcga ttctgctgct gctgctgatt gcgccg 36
<210> 243
   <211> 39
   <212> DNA
   <213> Artificial Sequence
<220>
   <223> MTD JO-50
<400> 243
   ccgctgctgg cgctggtgct gctgctggcg ctgattgcg 39
<210> 244
   <211> 36
   <212> DNA
   <213> Artificial Sequence
<220>
   <223> MTD JO-51
<400> 244
   gtggtggcgg tgctggcgct ggtgctggcg gcgctg 36
<210> 245
   <211> 27
   <212> DNA
   <213> Artificial Sequence
<220>
   <223> MTD JO-52
<400> 245
   ccgctgctgc tgctgctgcc ggcgctg 27
<210> 246
   <211> 36
   <212> DNA
   <213> Artificial Sequence
<220>
   <223> MTD JO-53
<400> 246
   ctggcggcgg tggcggcgct ggcggtggtg gtgccg 36
<210> 247
   <211> 36
   <212> DNA
   <213> Artificial Sequence
<220>
   <223> MTD JO-54
<400> 247
   ctgctgctgc tggtgctgat tctgccgctg gcggcg 36
<210> 248
   <211> 27
   <212> DNA
   <213> Artificial Sequence
<220>
   <223> MTD JO-55
<400> 248
   ctggcggtgg tggtggtggc ggcggtg 27
<210> 249
   <211> 27
   <212> DNA
   <213> Artificial Sequence
<220>
   <223> MTD JO-56
<400> 249
   gtgctgctgg cggcggcgct gattgcg 27
<210> 250
   <211> 30
   <212> DNA
   <213> Artificial Sequence
<220>
   <223> MTD JO-57
<400> 250
   ctgattgcgc tgctggcggc gccgctggcg 30
<210> 251
   <211> 24
   <212> DNA
   <213> Artificial Sequence
<220>
   <223> MTD JO-58
<400> 251
   ctggcgctgc tgctgctggc ggcg 24
<210> 252
   <211> 36
   <212> DNA
   <213> Artificial Sequence
<220>
   <223> MTD JO-59
<400> 252
   ctgctggcgg cggcgctgct gctgctgctg ctggcg 36
<210> 253
   <211> 30
   <212> DNA
   <213> Artificial Sequence
<220>
   <223> MTD JO-60
<400> 253
   gtgattattg cgctgattgt gattgtggcg 30
<210> 254
   <211> 33
   <212> DNA
   <213> Artificial Sequence
<220>
   <223> MTD JO-61
<400> 254
   gtggtgctgg tggtggcggc ggtgctggcg ctg 33
<210> 255
   <211> 27
   <212> DNA
   <213> Artificial Sequence
<220>
   <223> MTD JO-62
<400> 255
   gtggcggtgg cgattgcggt ggtgctg 27
<210> 256
   <211> 39
   <212> DNA
   <213> Artificial Sequence
<220>
   <223> MTD JO-63
<400> 256
   ccgctgattg tggtggtggc ggcggcggtg gtggcggtg 39
<210> 257
   <211> 33
   <212> DNA
   <213> Artificial Sequence
<220>
   <223> MTD JO-64
<400> 257
   ccgctggcgg tggcggtggc ggcggtggcg gcg 33
<210> 258
   <211> 30
   <212> DNA
   <213> Artificial Sequence
<220>
   <223> MTD JO-65
<400> 258
   gcggcgattg cgctggtggc ggtggtgctg 30
<210> 259
   <211> 30
   <212> DNA
   <213> Artificial Sequence
<220>
   <223> MTD JO-66
<400> 259
   gcggcggcgc tggcggcgat tgcggtgatt 30
<210> 260
   <211> 24
   <212> DNA
   <213> Artificial Sequence
<220>
   <223> MTD JO-67
<400> 260
   gcggcggcgc cggcggtggc ggcg 24
<210> 261
   <211> 21
   <212> DNA
   <213> Artificial Sequence
<220>
   <223> MTD JO-68
<400> 261
   ctgctgctgg cggcgctgcc g 21
<210> 262
   <211> 24
   <212> DNA
   <213> Artificial Sequence
<220>
   <223> MTD JO-69
<400> 262
   gcgctgctgg cggtggtggc ggcg 24
<210> 263
   <211> 30
   <212> DNA
   <213> Artificial Sequence
<220>
   <223> MTD JO-70
<400> 263
   gcggtggtgg tggtgctgcc gattctgctg 30
<210> 264
   <211> 27
   <212> DNA
   <213> Artificial Sequence
<220>
   <223> MTD JO-71
<400> 264
   gcgctggcgc tgctgctgct ggtgccg 27
<210> 265
   <211> 33
   <212> DNA
   <213> Artificial Sequence
<220>
   <223> MTD JO-72
<400> 265
   ctggtggtgc tgctggcggc gctgctggtg ctg 33
<210> 266
   <211> 24
   <212> DNA
   <213> Artificial Sequence
<220>
   <223> MTD JO-73
<400> 266
   ccggtgctgc tgctgctggc gccg 24
<210> 267
   <211> 24
   <212> DNA
   <213> Artificial Sequence
<220>
   <223> MTD JO-74
<400> 267
   gcgctggcgg tggtggcggc gccg 24
<210> 268
   <211> 24
   <212> DNA
   <213> Artificial Sequence
<220>
   <223> MTD JO-75
<400> 268
   gtgattgtgg cgctgctggc ggtg 24
<210> 269
   <211> 24
   <212> DNA
   <213> Artificial Sequence
<220>
   <223> MTD JO-76
<400> 269
   gcgctggtgc tgccgctggc gccg 24
<210> 270
   <211> 27
   <212> DNA
   <213> Artificial Sequence
<220>
   <223> MTD JO-77
<400> 270
   gcggtggcgc tgctgattct ggcggtg 27
<210> 271
   <211> 21
   <212> DNA
   <213> Artificial Sequence
<220>
   <223> MTD JO-78
<400> 271
   gtgctgctgg cggtgattcc g 21
<210> 272
   <211> 39
   <212> DNA
   <213> Artificial Sequence
<220>
   <223> MTD JO-79
<400> 272
   ctgattgtgg cggcggtggt ggtggtggcg gtgctgatt 39
<210> 273
   <211> 21
   <212> DNA
   <213> Artificial Sequence
<220>
   <223> MTD HO-80
<400> 273
   gcggtggtgg tggcggcgcc g 21
<210> 274
   <211> 27
   <212> DNA
   <213> Artificial Sequence
<220>
   <223> MTD JO-81
<400> 274
   ctggcggcgg tgctgctgct gattccg 27
<210> 275
   <211> 30
   <212> DNA
   <213> Artificial Sequence
<220>
   <223> MTD JO-82
<400> 275
   ctgctgctgc tgctgctggc ggtggtgccg 30
<210> 276
   <211> 33
   <212> DNA
   <213> Artificial Sequence
<220>
   <223> MTD JO-83
<400> 276
   gcggtggcgc tggtggcggt ggtggcggtg gcg 33
<210> 277
   <211> 27
   <212> DNA
   <213> Artificial Sequence
<220>
   <223> MTD JO-84
<400> 277
   ctggtggcgg cgctgctggc ggtgctg 27
<210> 278
   <211> 33
   <212> DNA
   <213> Artificial Sequence
<220>
   <223> MTD JO-85
<400> 278
   ctgctggcgg cggcggcggc gctgctgctg gcg 33
<210> 279
   <211> 24
   <212> DNA
   <213> Artificial Sequence
<220>
   <223> MTD JO-86
<400> 279
   ctggcggtgc tggcggcggc gccg 24
<210> 280
   <211> 39
   <212> DNA
   <213> Artificial Sequence
<220>
   <223> MTD JO-87
<400> 280
   gtggtggtgc tgctggtgct gctggcgctg gtggtggtg 39
<210> 281
   <211> 21
   <212> DNA
   <213> Artificial Sequence
<220>
   <223> MTD JO-88
<400> 281
   gtggtgattg cggtggtgcc g 21
<210> 282
   <211> 27
   <212> DNA
   <213> Artificial Sequence
<220>
   <223> MTD JO-89
<400> 282
   gtgctgctgg tgctgctggc gctggtg 27
<210> 283
   <211> 27
   <212> DNA
   <213> Artificial Sequence
<220>
   <223> MTD JO-90
<400> 283
   gtgctgctgg tgctgctggc gctggtg 27
<210> 284
   <211> 24
   <212> DNA
   <213> Artificial Sequence
<220>
   <223> MTD JO-91
<400> 284
   ccggtgctgg tgccggcggt gccg 24
<210> 285
   <211> 24
   <212> DNA
   <213> Artificial Sequence
<220>
   <223> MTD JO-92
<400> 285
   ccggcgctgg cgctggcgct ggcg 24
<210> 286
   <211> 24
   <212> DNA
   <213> Artificial Sequence
<220>
   <223> MTD JO-93
<400> 286
   gcggcggcgg cgccggcgct ggcg 24
<210> 287
   <211> 27
   <212> DNA
   <213> Artificial Sequence
<220>
   <223> MTD JO-94
<400> 287
   attgtgctgc cggtgctggc ggcgccg 27
<210> 288
   <211> 30
   <212> DNA
   <213> Artificial Sequence
<220>
   <223> MTD JO-95
<400> 288
   ctggtgctgc tgctgctgcc gctgctgatt 30
<210> 289
   <211> 33
   <212> DNA
   <213> Artificial Sequence
<220>
   <223> MTD JO-96
<400> 289
   ctggcggcgg tggcgccggc gctggcggtg gtg 33
<210> 290
   <211> 24
   <212> DNA
   <213> Artificial Sequence
<220>
   <223> MTD JO-97
<400> 290
   attctggtgc tggtgctgcc gatt 24
<210> 291
   <211> 27
   <212> DNA
   <213> Artificial Sequence
<220>
   <223> MTD JO-98
<400> 291
   attctgctgc cgctgctgct gctgccg 27
<210> 292
   <211> 30
   <212> DNA
   <213> Artificial Sequence
<220>
   <223> MTD JO-99
<400> 292
   attgcgccgg cggtggtggc ggcgctgccg 30
<210> 293
   <211> 36
   <212> DNA
   <213> Artificial Sequence
<220>
   <223> MTD JO-100
<400> 293
   ctgctgctgg tggcggtggt gccgctgctg gtgccg 36
<210> 294
   <211> 27
   <212> DNA
   <213> Artificial Sequence
<220>
   <223> MTD JO-101
<400> 294
   ctgattctgc tgctgctgcc gattatt 27
<210> 295
   <211> 30
   <212> DNA
   <213> Artificial Sequence
<220>
   <223> MTD JO-102
<400> 295
   gcggtgctgg cggcgccggc ggtgctggtg 30
<210> 296
   <211> 27
   <212> DNA
   <213> Artificial Sequence
<220>
   <223> MTD JO-103
<400> 296
   ctggcgctgc cggtgctgct gctggcg 27
<210> 297
   <211> 21
   <212> DNA
   <213> Artificial Sequence
<220>
   <223> MTD JO-104
<400> 297
   ctggcgctgg cgctgctgct g 21
<210> 298
   <211> 27
   <212> DNA
   <213> Artificial Sequence
<220>
   <223> MTD JO-105
<400> 298
   gtggcggtgc cgctgctggt ggtggcg 27
<210> 299
   <211> 33
   <212> DNA
   <213> Artificial Sequence
<220>
   <223> MTD JO-106
<400> 299
   gcggtggcgg tggcgccggt ggcggcggcg gcg 33
<210> 300
   <211> 33
   <212> DNA
   <213> Artificial Sequence
<220>
   <223> MTD JO-107
<400> 300
   gcggcggcgg tggtggcggc ggtgccggcg gcg 33
<210> 301
   <211> 27
   <212> DNA
   <213> Artificial Sequence
<220>
   <223> MTD JO-108
<400> 301
   gcgctgctgg cggcgctgct ggcgccg 27
<210> 302
   <211> 21
   <212> DNA
   <213> Artificial Sequence
<220>
   <223> MTD JO-109
<400> 302
   ctgctggcgc tgctggtgcc g 21
<210> 303
   <211> 42
   <212> DNA
   <213> Artificial Sequence
<220>
   <223> MTD JO-110
<400> 303
   gcgctgctgg cggcgctgct ggcgctgctg gcgctgctgg tg 42
<210> 304
   <211> 33
   <212> DNA
   <213> Artificial Sequence
<220>
   <223> MTD JO-111
<400> 304
   gcggcggcgc tgccgctgct ggtgctgctg ccg 33
<210> 305
   <211> 30
   <212> DNA
   <213> Artificial Sequence
<220>
   <223> MTD JO-112
<400> 305
   gcggcggcgg tgccggcggc gctggcgccg 30
<210> 306
   <211> 30
   <212> DNA
   <213> Artificial Sequence
<220>
   <223> MTD JO-113
<400> 306
   gcggcgctgg cggtggcggc gctggcggcg 30
<210> 307
   <211> 24
   <212> DNA
   <213> Artificial Sequence
<220>
   <223> MTD JO-114
<400> 307
   gcggtgctgg cggcggcggt gccg 24
<210> 308
   <211> 24
   <212> DNA
   <213> Artificial Sequence
<220>
   <223> MTD JO-115
<400> 308
   gtggcggcgc tgccggcgcc ggcg 24
<210> 309
   <211> 33
   <212> DNA
   <213> Artificial Sequence
<220>
   <223> MTD JO-116
<400> 309
   gcgctggcgc tggcggtgcc ggcggtgctg ccg 33
<210> 310
   <211> 33
   <212> DNA
   <213> Artificial Sequence
<220>
   <223> MTD JO-117
<400> 310
   gcggcgctgc tgccggcggc ggtggcggtg ccg 33
<210> 311
   <211> 24
   <212> DNA
   <213> Artificial Sequence
<220>
   <223> MTD JO-118
<400> 311
   gcggtggtgg tggcgctggc gccg 24
<210> 312
   <211> 42
   <212> DNA
   <213> Artificial Sequence
<220>
   <223> MTD JO-119
<400> 312
   gcggcggcgg tggcgctgcc ggcggcggcg gcgctgctgg cg 42
<210> 313
   <211> 39
   <212> DNA
   <213> Artificial Sequence
<220>
   <223> MTD JO-120
<400> 313
   gcggtggtgc tgccgctggc gctggtggcg gtggcgccg 39
<210> 314
   <211> 24
   <212> DNA
   <213> Artificial Sequence
<220>
   <223> MTD JO-121
<400> 314
   ctggtggcgc tgccgctgct gccg 24
<210> 315
   <211> 30
   <212> DNA
   <213> Artificial Sequence
<220>
   <223> MTD JO-122
<400> 315
   gtggtggtgc cgctgctgct gattgtgccg 30
<210> 316
   <211> 24
   <212> DNA
   <213> Artificial Sequence
<220>
   <223> MTD JO-123
<400> 316
   ctggcggtgg tgctggcggt gccg 24
<210> 317
   <211> 30
   <212> DNA
   <213> Artificial Sequence
<220>
   <223> MTD JO-124
<400> 317
   ctgctggcgg tgccgattct gctggtgccg 30
<210> 318
   <211> 24
   <212> DNA
   <213> Artificial Sequence
<220>
   <223> MTD JO-125
<400> 318
   ctggtggcgc tggtgctgct gccg 24
<210> 319
   <211> 42
   <212> DNA
   <213> Artificial Sequence
<220>
   <223> MTD JO-126
<400> 319
   ctggtgctgc tgctggcggt gctgctgctg gcggtgctgc cg 42
<210> 320
   <211> 36
   <212> DNA
   <213> Artificial Sequence
<220>
   <223> MTD JO-127
<400> 320
   ctgctggcgc cggtggtggc gctggtgatt ctgccg 36
<210> 321
   <211> 39
   <212> DNA
   <213> Artificial Sequence
<220>
   <223> MTD JO-128
<400> 321
   gtgctggcgg tgctggcggt gccggtgctg ctgctgccg 39
<210> 322
   <211> 30
   <212> DNA
   <213> Artificial Sequence
<220>
   <223> MTD JO-129
<400> 322
   gtggtgattg cggtggtgcc ggtggtggtg 30
<210> 323
   <211> 33
   <212> DNA
   <213> Artificial Sequence
<220>
   <223> MTD JO-130
<400> 323
   ctgctggtgc tgctggcgct ggtggtggtg ccg 33
<210> 324
   <211> 33
   <212> DNA
   <213> Artificial Sequence
<220>
   <223> MTD JO-131
<400> 324
   gtgctgctgg cgctgccggt ggtggcggcg ccg 33
<210> 325
   <211> 36
   <212> DNA
   <213> Artificial Sequence
<220>
   <223> MTD JO-132
<400> 325
   gcggtggtgg tgccggcgat tgtgctggcg gcgccg 36
<210> 326
   <211> 33
   <212> DNA
   <213> Artificial Sequence
<220>
   <223> MTD JO-133
<400> 326
   gcggtgctgg tgccggcggc ggcgctggtg ccg 33
<210> 327
   <211> 30
   <212> DNA
   <213> Artificial Sequence
<220>
   <223> MTD JO-134
<400> 327
   gtggtggcgg cgctgccgct ggtgctgccg 30
<210> 328
   <211> 30
   <212> DNA
   <213> Artificial Sequence
<220>
   <223> MTD JO-135
<400> 328
   gcggcggtgg cgctgccggc ggcggcgccg 30
<210> 329
   <211> 24
   <212> DNA
   <213> Artificial Sequence
<220>
   <223> MTD JO-136
<400> 329
   ctgattgcgc tgccgctgct gccg 24
<210> 330
   <211> 39
   <212> DNA
   <213> Artificial Sequence
<220>
   <223> MTD JO-137
<400> 330
   ctgctggcgc tgccgctggt gctggtgctg gcgctgccg 39
<210> 331
   <211> 27
   <212> DNA
   <213> Artificial Sequence
<220>
   <223> MTD JO-138
<400> 331
   attgtgccgc tgctgctggc ggcgccg 27
<210> 332
   <211> 30
   <212> DNA
   <213> Artificial Sequence
<220>
   <223> MTD JO-139
<400> 332
   ctgctgctgg cgccgctgct gctggcgccg 30
<210> 333
   <211> 30
   <212> DNA
   <213> Artificial Sequence
<220>
   <223> MTD JO-140
<400> 333
   ctggcggcgc tgccggtggc ggcggtgccg 30
<210> 334
   <211> 30
   <212> DNA
   <213> Artificial Sequence
<220>
   <223> MTD JO-141
<400> 334
   gcgctggcgg tgattgtgct ggtgctgctg 30
<210> 335
   <211> 30
   <212> DNA
   <213> Artificial Sequence
<220>
   <223> MTD JO-142
<400> 335
   ctggcgctgc tgctgccggc ggcgctgatt 30
<210> 336
   <211> 33
   <212> DNA
   <213> Artificial Sequence
<220>
   <223> MTD JO-143
<400> 336
   gcgctgctgc cgctgctggc ggtggtgctg ccg 33
<210> 337
   <211> 30
   <212> DNA
   <213> Artificial Sequence
<220>
   <223> MTD JO-144
<400> 337
   gcgattgcgg tgccggtgct ggcggcgccg 30
<210> 338
   <211> 30
   <212> DNA
   <213> Artificial Sequence
<220>
   <223> MTD JO-145
<400> 338
   gcggcggcgc cggtgctgct gctgctgctg 30
<210> 339
   <211> 33
   <212> DNA
   <213> Artificial Sequence
<220>
   <223> MTD JO-146
<400> 339
   gcggcggcgg tggcggtgct ggcgctggcg ccg 33
<210> 340
   <211> 33
   <212> DNA
   <213> Artificial Sequence
<220>
   <223> MTD JO-147
<400> 340
   gcggcgctgg cggcgctggt ggtggcggcg ccg 33
<210> 341
   <211> 36
   <212> DNA
   <213> Artificial Sequence
<220>
   <223> MTD JO-148
<400> 341
   gcggcgctgg cggcggtgcc gctggcgctg gcgccg 36
<210> 342
   <211> 39
   <212> DNA
   <213> Artificial Sequence
<220>
   <223> MTD JO-149
<400> 342
   gcgctggcgg tggcggcgcc ggcgctggcg ctgctgccg 39
<210> 343
   <211> 24
   <212> DNA
   <213> Artificial Sequence
<220>
   <223> MTD JO-150
<400> 343
   gcggcgctgc cggcggcggc gccg 24
<210> 344
   <211> 27
   <212> DNA
   <213> Artificial Sequence
<220>
   <223> MTD JO-151
<400> 344
   gcggcggcgc cggtggcggc ggtgccg 27
<210> 345
   <211> 30
   <212> DNA
   <213> Artificial Sequence
<220>
   <223> MTD JO-152
<400> 345
   ctgctggcgg tgctgctggc gctgctgccg 30
<210> 346
   <211> 30
   <212> DNA
   <213> Artificial Sequence
<220>
   <223> MTD JO-153
<400> 346
   gtgctggcgc tgctggtggc ggtggtgccg 30
<210> 347
   <211> 27
   <212> DNA
   <213> Artificial Sequence
<220>
   <223> MTD JO-154
<400> 347
   gcgctggtgg tgccggcggc ggtgccg 27
<210> 348
   <211> 27
   <212> DNA
   <213> Artificial Sequence
<220>
   <223> MTD JO-155
<400> 348
   gcggtggtgc tgccgctgct gctgccg 27
<210> 349
   <211> 30
   <212> DNA
   <213> Artificial Sequence
<220>
   <223> MTD JO-156
<400> 349
   gcggtgattc cggtggcggt gctggtgccg 30
<210> 350
   <211> 33
   <212> DNA
   <213> Artificial Sequence
<220>
   <223> MTD JO-157
<400> 350
   gcggcggcgg tgccggcggc ggtgctggcg ccg 33
<210> 351
   <211> 33
   <212> DNA
   <213> Artificial Sequence
<220>
   <223> MTD JO-158
<400> 351
   gtggcggtgc cggtggtgct ggcgattctg ccg 33
<210> 352
   <211> 36
   <212> DNA
   <213> Artificial Sequence
<220>
   <223> MTD JO-159
<400> 352
   attgcgattg cggcgattcc ggcgattctg gcgctg 36
<210> 353
   <211> 30
   <212> DNA
   <213> Artificial Sequence
<220>
   <223> MTD JO-160
<400> 353
   gcgctgattg cgccggcgct ggcggcgccg 30
<210> 354
   <211> 30
   <212> DNA
   <213> Artificial Sequence
<220>
   <223> MTD JO-161
<400> 354
   gcggcgattg cgctggtggc gccggcgctg 30
<210> 355
   <211> 27
   <212> DNA
   <213> Artificial Sequence
<220>
   <223> MTD JO-162
<400> 355
   ctggcgccgg cggtggcggc ggcgccg 27
<210> 356
   <211> 45
   <212> DNA
   <213> Artificial Sequence
<220>
   <223> MTD JO-163
<400> 356
   gtggcgatta ttgtgccggc ggtggtggcg attgcgctga ttatt 45
<210> 357
   <211> 27
   <212> DNA
   <213> Artificial Sequence
<220>
   <223> MTD JO-164
<400> 357
   gcggtggtgg cgattgcgct gattatt 27
<210> 358
   <211> 27
   <212> DNA
   <213> Artificial Sequence
<220>
   <223> MTD JO-165
<400> 358
   ctggcggcgg tgccggcggc ggcgccg 27
<210> 359
   <211> 30
   <212> DNA
   <213> Artificial Sequence
<220>
   <223> MTD JO-166
<400> 359
   gcggtggcgg cgctgccgct ggcggcgccg 30
<210> 360
   <211> 27
   <212> DNA
   <213> Artificial Sequence
<220>
   <223> MTD JO-167
<400> 360
   ctggcggcgc cggcggcggc ggcgccg 27
<210> 361
   <211> 36
   <212> DNA
   <213> Artificial Sequence
<220>
   <223> MTD JO-168
<400> 361
   ctggcggcgg tggtgccggt ggcggcggcg gtgccg 36
<210> 362
   <211> 27
   <212> DNA
   <213> Artificial Sequence
<220>
   <223> MTD JO-169
<400> 362
   gtggcggcgc cggcggcggc ggcgccg 27
<210> 363
   <211> 24
   <212> DNA
   <213> Artificial Sequence
<220>
   <223> MTD JO-170
<400> 363
   gcggtgccgg tgccggtgcc gctg 24
<210> 364
   <211> 30
   <212> DNA
   <213> Artificial Sequence
<220>
   <223> MTD JO-171
<400> 364
   ctgctgattc tgccgattgt gctgctgccg 30
<210> 365
   <211> 33
   <212> DNA
   <213> Artificial Sequence
<220>
   <223> MTD JO-172
<400> 365
   gcgctggcgc tgccggcgct ggcgattgcg ccg 33
<210> 366
   <211> 27
   <212> DNA
   <213> Artificial Sequence
<220>
   <223> MTD JO-173
<400> 366
   gcggtgattc cgattctggc ggtgccg 27
<210> 367
   <211> 33
   <212> DNA
   <213> Artificial Sequence
<220>
   <223> MTD JO-174
<400> 367
   ctgattctgc tgctgccggc ggtggcgctg ccg 33
<210> 368
   <211> 30
   <212> DNA
   <213> Artificial Sequence
<220>
   <223> MTD JO-175
<400> 368
   attgtgctgg cgccggtgcc ggcggcggcg 30
<210> 369
   <211> 33
   <212> DNA
   <213> Artificial Sequence
<220>
   <223> MTD JO-176
<400> 369
   gtggtggtgg tgccggtgct ggcggcggcg gcg 33
<210> 370
   <211> 21
   <212> DNA
   <213> Artificial Sequence
<220>
   <223> MTD JO-177
<400> 370
   ctggtggcgg tggcggcgcc g 21
<210> 371
   <211> 30
   <212> DNA
   <213> Artificial Sequence
<220>
   <223> MTD JO-178
<400> 371
   ctggtgctgg cggcgccggc ggcgctgccg 30
<210> 372
   <211> 27
   <212> DNA
   <213> Artificial Sequence
<220>
   <223> MTD JO-179
<400> 372
   ctgattgcgc cggcggcggc ggtgccg 27
<210> 373
   <211> 30
   <212> DNA
   <213> Artificial Sequence
<220>
   <223> MTD JO-180
<400> 373
   gcgctggcgg cgctgccgat tgcgctgccg 30
<210> 374
   <211> 27
   <212> DNA
   <213> Artificial Sequence
<220>
   <223> MTD JO-181
<400> 374
   gcggtgctgc tgctgccggc ggcggcg 27
<210> 375
   <211> 27
   <212> DNA
   <213> Artificial Sequence
<220>
   <223> MTD JO-182
<400> 375
   attgcgctgg cgctgctgcc gctgctg 27
<210> 376
   <211> 30
   <212> DNA
   <213> Artificial Sequence
<220>
   <223> MTD JO-183
<400> 376
   gtgctgctgg cggcggcgct gattgcgccg 30
<210> 377
   <211> 33
   <212> DNA
   <213> Artificial Sequence
<220>
   <223> MTD JO-184
<400> 377
   gcgccggcgg tgctgccgcc ggtggtggtg att 33
<210> 378
   <211> 27
   <212> DNA
   <213> Artificial Sequence
<220>
   <223> MTD JO-185
<400> 378
   gtggtgggcc tgctggtggc ggcgctg 27
<210> 379
   <211> 33
   <212> DNA
   <213> Artificial Sequence
<220>
   <223> MTD JO-186
<400> 379
   gcggcgattg cggcggcggc gccgctggcg gcg 33
<210> 380
   <211> 21
   <212> DNA
   <213> Artificial Sequence
<220>
   <223> MTD JO-187
<400> 380
   ctgctgctgg cggtggcgcc g 21
<210> 381
   <211> 30
   <212> DNA
   <213> Artificial Sequence
<220>
   <223> MTD JO-188
<400> 381
   ctgattctgc tgctgccgct ggcggcgctg 30
<210> 382
   <211> 24
   <212> DNA
   <213> Artificial Sequence
<220>
   <223> MTD JO-189
<400> 382
   gcgctgctgc tgctggtgct ggcg 24
<210> 383
   <211> 27
   <212> DNA
   <213> Artificial Sequence
<220>
   <223> MTD JO-190
<400> 383
   ctgctgctgc tgctgctgcc gctggcg 27
<210> 384
   <211> 24
   <212> DNA
   <213> Artificial Sequence
<220>
   <223> MTD JO-191
<400> 384
   ctggcgctgc cgctgctgct gccg 24
<210> 385
   <211> 24
   <212> DNA
   <213> Artificial Sequence
<220>
   <223> MTD JO-192
<400> 385
   ctgctggtgc tgccgctgct gatt 24
<210> 386
   <211> 27
   <212> DNA
   <213> Artificial Sequence
<220>
   <223> MTD JO-193
<400> 386
   ctgccgctgc tgccggcggc gctggtg 27
<210> 387
   <211> 10
   <212> PRT
   <213> Artificial Sequence
<220>
   <223> Scramble peptide
<400> 387
   Ser Ala Asn Val Glu Pro Leu Glu Arg Leu
    1               5                  10
<210> 388
   <211> 30
   <212> DNA
   <213> Artificial Sequence
<220>
   <223> Scramble peptide
<400> 388
   tcagcgaatg tcgaccccct agaccgacta 30
<210> 389
   <211> 12
   <212> PRT
   <213> Artificial Sequence
<220>
   <223> kFGF4-derived MTD
<400> 389
   Ala Ala Val Leu Leu Pro Val Leu Leu Ala Ala Pro
   1 5 10
<210> 390
   <211> 36
   <212> DNA
   <213> Artificial Sequence
<220>
   <223> kFGF4-derived MTD
<400> 390
   gccgcggtac tgctcccggt cctgctggcc gcgccc 36
<210> 391
   <211> 265
   <212> PRT
   <213> Human EGFP
<400> 391
   Met Val Ser Lys Gly Glu Glu Leu Phe Thr Gly Val Val Pro Ile Leu
   1               5                   10                  15
   Val Glu Leu Asp Gly Asp Val Asn Gly His Lys Phe Ser Val Ser Gly
               20                  25                  30
   Glu Gly Glu Gly Asp Ala Thr Tyr Gly Lys Leu Thr Leu Lys Phe Ile
           35                  40                  45
   Cys Thr Thr Gly Lys Leu Pro Val Pro Trp Pro Thr Leu Val Thr Thr
       50                  55                  60
   Leu Thr Tyr Gly Val Gln Cys Phe Ser Arg Tyr Pro Asp His Met Lys
   65                  70                  75                  80
   Gln His Asp Phe Phe Lys Ser Ala Met Pro Glu Gly Tyr Val Gln Glu
                   85                  90                  95
   Arg Thr Ile Phe Phe Lys Asp Asp Gly Asn Tyr Lys Thr Arg Ala Glu
               100                 105                 110
   Val Lys Phe Glu Gly Asp Thr Leu Val Asn Arg Ile Glu Leu Lys Gly
           115                 120                 125
   Ile Asp Phe Lys Glu Asp Gly Asn Ile Leu Gly His Lys Leu Glu Tyr
       130                 135                 140
   Asn Tyr Asn Ser His Asn Val Tyr Ile Met Ala Asp Lys Gln Lys Asn
   145                 150                 155                 160
   Gly Ile Lys Val Asn Phe Lys Ile Arg His Asn Ile Glu Asp Gly Ser
                   165                 170                 175
   Val Gln Leu Ala Asp His Tyr Gln Gln Asn Thr Pro Ile Gly Asp Gly
               180                 185                 190
   Pro Val Leu Leu Pro Asp Asn His Tyr Leu Ser Thr Gln Ser Ala Leu
           195                 200                 205
   Ser Lys Asp Pro Asn Glu Lys Arg Asp His Met Val Leu Leu Glu Phe
       210                 215                 220
   Val Thr Ala Ala Gly Ile Thr Leu Gly Met Asp Glu Leu Tyr Lys Ser
   225                 230                 235                 240
   Gly Leu Arg Ser Arg Ala Gln Ala Ser Asn Ser Ala Val Asp Gly Thr
                   245                 250                 255
   Ala Gly Pro Gly Ser Thr Gly Ser Arg
               260                 265
<210> 392
   <211> 798
   <212> DNA
   <213> Human EGFP
<400> 392
   atggtgagca agggcgagga gctgttcacc ggggtggtgc ccatcctggt cgagctggac        60
   ggcgacgtaa acggccacaa gttcagcgtg tccggcgagg gcgagggcga tgccacctac 120
   ggcaagctga ccctgaagtt catctgcacc accggcaagc tgcccgtgcc ctggcccacc 180
   ctcgtgacca ccctgaccta cggcgtgcag tgcttcagcc gctaccccga ccacatgaag 240
   cagcacgact tcttcaagtc cgccatgccc gaaggctacg tccaggagcg caccatcttc 300
   ttcaaggacg acggcaacta caagacccgc gccgaggtga agttcgaggg cgacaccctg 360
   gtgaaccgca tcgagctgaa gggcatcgac ttcaaggagg acggcaacat cctggggcac 420
   aagctggagt acaactacaa cagccacaac gtctatatca tggccgacaa gcagaagaac 480
   ggcatcaagg tgaacttcaa gatccgccac aacatcgagg acggcagcgt gcagctcgcc 540
   gaccactacc agcagaacac ccccatcggc gacggccccg tgctgctgcc cgacaaccac 600
   tacctgagca cccagtccgc cctgagcaaa gaccccaacg agaagcgcga tcacatggtc 660
   ctgctggagt tcgtgaccgc cgccgggatc actctcggca tggacgagct gtacaagtcc 720
   ggactcagat ctcgagctca agcttcgaat tctgcagtcg acggtaccgc gggcccggga 780
   tccaccggat ctagataa 798
<210> 393
   <211> 66
   <212> DNA
   <213> Artificial Sequence
<220>
   <223> forward primer for JO-01
<400> 393
   ccgcatatgg cggtggtggt gtgcgcgatt gtgctggcgg cgccggtgag caagggcgag 60
   gagctg 66
<210> 394
   <211> 66
   <212> DNA
   <213> Artificial Sequence
<220>
   <223> forward primer for JO-02
<400> 394
   ccgcatatgc cgctggcgct gctggtgctg ctgctgctgg gcccggtgag caagggcgag 60
   gagctg 66
<210> 395
   <211> 63
   <212> DNA
   <213> Artificial Sequence
<220>
   <223> forward primer for JO-03
<400> 395
   ccgcatatgc tgctgctggc gtttgcgctg ctgtgcctgc cggtgagcaa gggcgaggag 60
   ctg 63
<210> 396
   <211> 69
   <212> DNA
   <213> Artificial Sequence
<220>
   <223> forward primer for Jo-04
<400> 396
   ccgcatatgc tgctgggcgc actggcggcg gtgctgctgg cgctggcggt gagcaagggc 60
   gaggagctg 69
<210> 397
   <211> 81
   <212> DNA
   <213> Artificial Sequence
<220>
   <223> forward primer for JO-05
<400> 397
   ccgcatatgc cggtgctgct ggcgctgggc gtgggcctgg tgctgctggg cctggcggtg 60
   gtgagcaagg gcgaggagct g 81
<210> 398
   <211> 57
   <212> DNA
   <213> Artificial Sequence
<220>
   <223> forward primer for JO-06
<400> 398
   ccgcatatgg cggcggcggc ggtgctgctg gcggcggtga gcaagggcga ggagctg 57
<210> 399
   <211> 54
   <212> DNA
   <213> Artificial Sequence
<220>
   <223> forward primer for JO-07
<400> 399
   ccgcatatga ttgtggtggc ggtggtggtg attgtgagca agggcgagga gctg 54
<210> 400
   <211> 57
   <212> DNA
   <213> Artificial Sequence
<220>
   <223> forward primer for JO-08
<400> 400
   ccgcatatgg cggtgctggc gccggtggtg gcggtggtga gcaagggcga ggagctg 57
<210> 401
   <211> 69
   <212> DNA
   <213> Artificial Sequence
<220>
   <223> forward primer for JO-09
<400> 401
   ccgcatatgc tggcggtgtg cggcctgccg gtggtggcgc tgctggcggt gagcaagggc 60
   gaggagctg 69
<210> 402
   <211> 77
   <212> DNA
   <213> Artificial Sequence
<220>
   <223> forward primer for JO-10
<400> 402
   ccgcatatgc tgggcggcgc ggtggtggcg gcgccggtgg cgcggcggtg gcgccggtga 60
   gcaagggcga ggagctg 77
<210> 403
   <211> 69
   <212> DNA
   <213> Artificial Sequence
<220>
   <223> forward primer for JO-11
<400> 403
   ccgcatatgc tgctgctggt gctggcggtg ctgctggcgg tgctgccggt gagcaagggc 60
   gaggagctg 69
<210> 404
   <211> 66
   <212> DNA
   <213> Artificial Sequence
<220>
   <223> forward primer for JO-12
<400> 404
   ccgcatatgc tgctgattct gctgctgctg ccgctgctga ttgtggtgag caagggcgag 60
   gagctg 66
<210> 405
   <211> 63
   <212> DNA
   <213> Artificial Sequence
<220>
   <223> forward primer for JO-13
<400> 405
   ccgcatatgc tggcggcggc ggcgctggcg gtgctgccgc tggtgagcaa gggcgaggag 60
   ctg 63
<210> 406
   <211> 72
   <212> DNA
   <213> Artificial Sequence
<220>
   <223> forward primer for JO-14
<400> 406
   ccgcatatgt ttctgatgct gctgctgccg ctgctgctgc tgctggtggc ggtgagcaag 60
   ggcgaggagc tg 72
<210> 407
   <211> 75
   <212> DNA
   <213> Artificial Sequence
<220>
   <223> forward primer for JP-15
<400> 407
   ccgcatatgg cggcggcggc ggcggcgctg ggtctggcgg cggcggtgcc ggcggtgagc 60
   aagggcgagg agctg 75
<210> 408
   <211> 72
   <212> DNA
   <213> Artificial Sequence
<220>
   <223> forward primer for JO-16
<400> 408
   ccgcatatgc tgctgctggc ggcgctgctg ctgattgcgt ttgcggcggt ggtgagcaag 60
   ggcgaggagc tg 72
<210> 409
   <211> 72
   <212> DNA
   <213> Artificial Sequence
<220>
   <223> forward primer for JO-17
<400> 409
   ccgcatatgg cgctggcggc ggtggtgctg attccgctgg gcattgcggc ggtgagcaag 60
   ggcgaggagc tg 72
<210> 410
   <211> 72
   <212> DNA
   <213> Artificial Sequence
<220>
   <223> forward primer for JO-18
<400> 410
   ccgcatatgg cggcgctgat tggcgcggtg ctggcgccgg tggtggcggt ggtgagcaag 60
   ggcgaggagc tg 72
<210> 411
   <211> 72
   <212> DNA
   <213> Artificial Sequence
<220>
   <223> forward primer for JO-19
<400> 411
   ccgcatatgg cggcggcggt ggcggtggcg ggcctggcgc cgctggcgct ggtgagcaag 60
   ggcgaggagc tg 72
<210> 412
   <211> 66
   <212> DNA
   <213> Artificial Sequence
<220>
   <223> forward primer for JO-20
<400> 412
   ccgcatatga ttgcggtggc gattgcggcg attgtgccgc tggcggtgag caagggcgag 60
   gagctg 66
<210> 413
   <211> 66
   <212> DNA
   <213> Artificial Sequence
<220>
   <223> forward primer for JO-21
<400> 413
   ccgcatatgg cggcggcggc ggtgctggcg gcgccggcgc tggcggtgag caagggcgag 60
   gagctg 66
<210> 414
   <211> 78
   <212> DNA
   <213> Artificial Sequence
<220>
   <223> forward primer for JO-22
<400> 414
   ccgcatatgg cggcgctggc gctgggcgtg gcggcggcgc cggcggcggc gccggcggtg 60
   agcaagggcg aggagctg 78
<210> 415
   <211> 63
   <212> DNA
   <213> Artificial Sequence
<220>
   <223> forward primer for JO-23
<400> 415
   ccgcatatgc tggcggtgct ggtgctgctg gtgctgctgc cggtgagcaa gggcgaggag 60
   ctg 63
<210> 416
   <211> 57
   <212> DNA
   <213> Artificial Sequence
<220>
   <223> forward primer for JO-24
<400> 416
   ccgcatatgg tggtggcggt gctggcgccg gtgctggtga gcaagggcga ggagctg 57
<210> 417
   <211> 66
   <212> DNA
   <213> Artificial Sequence
<220>
   <223> forward primer for JO-25
<400> 417
   ccgcatatgg cggcgctgct gctgccgctg ctgctgctgc tgccggtgag caagggcgag 60
   gagctg 66
<210> 418
   <211> 60
   <212> DNA
   <213> Artificial Sequence
<220>
   <223> forward primer for JO-26
<400> 418
<210> 419
   <211> 54
   <212> DNA
   <213> Artificial Sequence
<220>
   <223> forward primer for JO-27
<400> 419
   ccgcatatgc tgctgattgc ggcgctgctg ccggtgagca agggcgagga gctg 54
<210> 420
   <211> 66
   <212> DNA
   <213> Artificial Sequence
<220>
   <223> forward primer for JO-28
<400> 420
   ccgcatatgg cggcggtggt gctgctgccg ctggcggcgg cgccggtgag caagggcgag 60
   gagctg 66
<210> 421
   <211> 60
   <212> DNA
   <213> Artificial Sequence
<220>
   <223> forward primer for JO-29
<400> 421
<210> 422
   <211> 54
   <212> DNA
   <213> Artificial Sequence
<220>
   <223> forward primer for JO-30
<400> 422
   ccgcatatgc tgccggtggt ggcgctgctg gcggtgagca agggcgagga gctg 54
<210> 423
   <211> 63
   <212> DNA
   <213> Artificial Sequence
<220>
   <223> forward primer for JO-31
<400> 423
   ccgcatatgg cggcggcgct ggcggcgccg ctggcgctgc cggtgagcaa gggcgaggag 60
   ctg 63
<210> 424
   <211> 57
   <212> DNA
   <213> Artificial Sequence
<220>
   <223> forward primer for JO-32
<400> 424
   ccgcatatgc tgctgctggc gctgctgctg gcggcggtga gcaagggcga ggagctg 57
<210> 425
   <211> 54
   <212> DNA
   <213> Artificial Sequence
<220>
   <223> forward primer for JO-33
<400> 425
   ccgcatatgg cggtggcggt ggtggcgctg ctggtgagca agggcgagga gctg 54
<210> 426
   <211> 63
   <212> DNA
   <213> Artificial Sequence
<220>
   <223> forward primer for JO-34
<400> 426
   ccgcatatgc tgctgctgat tattgtgctg ctgattgtgc cggtgagcaa gggcgaggag 60
   ctg 63
<210> 427
   <211> 57
   <212> DNA
   <213> Artificial Sequence
<220>
   <223> forward primer for JO-35
<400> 427
   ccgcatatgc tggcgctggc ggcggcggtg gtgccggtga gcaagggcga ggagctg 57
<210> 428
   <211> 60
   <212> DNA
   <213> Artificial Sequence
<220>
   <223> forward primer for JO-36
<400> 428
<210> 429
   <211> 81
   <212> DNA
   <213> Artificial Sequence
<220>
   <223> forward primer for JO-37
<400> 429
   ccgcatatga ttgtggcgct gctgctggtg ccgctggtgc tggcgattgc ggcggtgctg 60
   gtgagcaagg gcgaggagct g 81
<210> 430
   <211> 54
   <212> DNA
   <213> Artificial Sequence
<220>
   <223> forward primer for JO-38
<400> 430
   ccgcatatga ttgtggcgct gctgctggtg ccggtgagca agggcgagga gctg 54
<210> 431
   <211> 60
   <212> DNA
   <213> Artificial Sequence
<220>
   <223> forward primer for JO-39
<400> 431
<210> 432
   <211> 57
   <212> DNA
   <213> Artificial Sequence
<220>
   <223> forward primer for JO-40
<400> 432
   ccgcatatgc cgctggtgct ggcggcgctg gtggcggtga gcaagggcga ggagctg 57
<210> 433
   <211> 54
   <212> DNA
   <213> Artificial Sequence
<220>
   <223> forward primer for JO-41
<400> 433
   ccgcatatgg cggcggcgct gctggcggtg gcggtgagca agggcgagga gctg 54
<210> 434
   <211> 54
   <212> DNA
   <213> Artificial Sequence
<220>
   <223> forward primer for JO-42
<400> 434
   ccgcatatgc cgctgctgct gctggcgctg gcggtgagca agggcgagga gctg 54
<210> 435
   <211> 51
   <212> DNA
   <213> Artificial Sequence
<220>
   <223> forward primer for JO-43
<400> 435
   ccgcatatgg cgctggcgct ggtggtggcg gtgagcaagg gcgaggagct g 51
<210> 436
   <211> 54
   <212> DNA
   <213> Artificial Sequence
<220>
   <223> forward primer for JO-44
<400> 436
   ccgcatatgg tggcggcggt ggtggtggcg gcggtgagca agggcgagga gctg 54
<210> 437
   <211> 57
   <212> DNA
   <213> Artificial Sequence
<220>
   <223> forward primer for JO-45
<400> 437
   ccgcatatgc cgctgctgcc gctgctgctg ctggtggtga gcaagggcga ggagctg 57
<210> 438
   <211> 69
   <212> DNA
   <213> Artificial Sequence
<220>
   <223> forward primer for JO-46
<400> 438
   ccgcatatgg tggtgctggt ggtggtgctg ccgctggcgg tgctggcggt gagcaagggc 60
   gaggagctg 69
<210> 439
   <211> 60
   <212> DNA
   <213> Artificial Sequence
<220>
   <223> forward primer for JO-47
<400> 439
<210> 440
   <211> 66
   <212> DNA
   <213> Artificial Sequence
<220>
   <223> forward primer for JO-48
<400> 440
   ccgcatatgc cggcgctgct gctgctgctg ctggcggcgg tggtggtgag caagggcgag 60
   gagctg 66
<210> 441
   <211> 66
   <212> DNA
   <213> Artificial Sequence
<220>
   <223> forward primer for JO-49
<400> 441
   ccgcatatgc cgctggcgat tctgctgctg ctgctgattg cgccggtgag caagggcgag 60
   gagctg 66
<210> 442
   <211> 69
   <212> DNA
   <213> Artificial Sequence
<220>
   <223> forward primer for JO-50
<400> 442
   ccgcatatgc cgctgctggc gctggtgctg ctgctggcgc tgattgcggt gagcaagggc 60
   gaggagctg 69
<210> 443
   <211> 66
   <212> DNA
   <213> Artificial Sequence
<220>
   <223> forward primer for JO-51
<400> 443
   ccgcatatgg tggtggcggt gctggcgctg gtgctggcgg cgctggtgag caagggcgag 60
   gagctg 66
<210> 444
   <211> 57
   <212> DNA
   <213> Artificial Sequence
<220>
   <223> forward primer for JO-52
<400> 444
   ccgcatatgc cgctgctgct gctgctgccg gcgctggtga gcaagggcga ggagctg 57
<210> 445
   <211> 66
   <212> DNA
   <213> Artificial Sequence
<220>
   <223> forward primer for JO-53
<400> 445
   ccgcatatgc tggcggcggt ggcggcgctg gcggtggtgg tgccggtgag caagggcgag 60
   gagctg 66
<210> 446
   <211> 66
   <212> DNA
   <213> Artificial Sequence
<220>
   <223> forward primer for JO-54
<400> 446
   ccgcatatgc tgctgctgct ggtgctgatt ctgccgctgg cggcggtgag caagggcgag 60
   gagctg 66
<210> 447
   <211> 57
   <212> DNA
   <213> Artificial Sequence
<220>
   <223> forward primer for JO-55
<400> 447
   ccgcatatgc tggcggtggt ggtggtggcg gcggtggtga gcaagggcga ggagctg 57
<210> 448
   <211> 57
   <212> DNA
   <213> Artificial Sequence
<220>
   <223> forward primer for JO-56
<400> 448
   ccgcatatgg tgctgctggc ggcggcgctg attgcggtga gcaagggcga ggagctg 57
<210> 449
   <211> 60
   <212> DNA
   <213> Artificial Sequence
<220>
   <223> forward primer for JO-57
<400> 449
<210> 450
   <211> 54
   <212> DNA
   <213> Artificial Sequence
<220>
   <223> forward primer for JO-58
<400> 450
   ccgcatatgc tggcgctgct gctgctggcg gcggtgagca agggcgagga gctg 54
<210> 451
   <211> 66
   <212> DNA
   <213> Artificial Sequence
<220>
   <223> forward primer for JO-59
<400> 451
   ccgcatatgc tgctggcggc ggcgctgctg ctgctgctgc tggcggtgag caagggcgag 60
   gagctg 66
<210> 452
   <211> 60
   <212> DNA
   <213> Artificial Sequence
<220>
   <223> forward primer for JO-60
<400> 452
<210> 453
   <211> 63
   <212> DNA
   <213> Artificial Sequence
<220>
   <223> forward primer for JO-61
<400> 453
   ccgcatatgg tggtgctggt ggtggcggcg gtgctggcgc tggtgagcaa gggcgaggag 60
   ctg 63
<210> 454
   <211> 57
   <212> DNA
   <213> Artificial Sequence
<220>
   <223> forward primer for JO-62
<400> 454
   ccgcatatgg tggcggtggc gattgcggtg gtgctggtga gcaagggcga ggagctg 57
<210> 455
   <211> 69
   <212> DNA
   <213> Artificial Sequence
<220>
   <223> forward primer for JO-63
<400> 455
   ccgcatatgc cgctgattgt ggtggtggcg gcggcggtgg tggcggtggt gagcaagggc 60
   gaggagctg 69
<210> 456
   <211> 63
   <212> DNA
   <213> Artificial Sequence
<220>
   <223> forward primer for JO-64
<400> 456
   ccgcatatgc cgctggcggt ggcggtggcg gcggtggcgg cggtgagcaa gggcgaggag 60
   ctg 63
<210> 457
   <211> 60
   <212> DNA
   <213> Artificial Sequence
<220>
   <223> forward primer for JO-65
<400> 457
<210> 458
   <211> 60
   <212> DNA
   <213> Artificial Sequence
<220>
   <223> forward primer for JO-66
<400> 458
<210> 459
   <211> 54
   <212> DNA
   <213> Artificial Sequence
<220>
   <223> forward primer for JO-67
<400> 459
   ccgcatatgg cggcggcgcc ggcggtggcg gcggtgagca agggcgagga gctg 54
<210> 460
   <211> 51
   <212> DNA
   <213> Artificial Sequence
<220>
   <223> forward primer for JO-68
<400> 460
   ccgcatatgc tgctgctggc ggcgctgccg gtgagcaagg gcgaggagct g 51
<210> 461
   <211> 54
   <212> DNA
   <213> Artificial Sequence
<220>
   <223> forward primer for JO-69
<400> 461
   ccgcatatgg cgctgctggc ggtggtggcg gcggtgagca agggcgagga gctg 54
<210> 462
   <211> 60
   <212> DNA
   <213> Artificial Sequence
<220>
   <223> forward primer for JO-70
<400> 462
<210> 463
   <211> 57
   <212> DNA
   <213> Artificial Sequence
<220>
   <223> forward primer for JO-71
<400> 463
   ccgcatatgg cgctggcgct gctgctgctg gtgccggtga gcaagggcga ggagctg 57
<210> 464
   <211> 63
   <212> DNA
   <213> Artificial Sequence
<220>
   <223> forward primer for JO-72
<400> 464
   ccgcatatgc tggtggtgct gctggcggcg ctgctggtgc tggtgagcaa gggcgaggag 60
   ctg 63
<210> 465
   <211> 54
   <212> DNA
   <213> Artificial Sequence
<220>
   <223> forward primer for JO-73
<400> 465
   ccgcatatgc cggtgctgct gctgctggcg ccggtgagca agggcgagga gctg 54
<210> 466
   <211> 54
   <212> DNA
   <213> Artificial Sequence
<220>
   <223> forward primer for JO-74
<400> 466
   ccgcatatgg cgctggcggt ggtggcggcg ccggtgagca agggcgagga gctg 54
<210> 467
   <211> 54
   <212> DNA
   <213> Artificial Sequence
<220>
   <223> forward primer for JO-75
<400> 467
   ccgcatatgg tgattgtggc gctgctggcg gtggtgagca agggcgagga gctg 54
<210> 468
   <211> 54
   <212> DNA
   <213> Artificial Sequence
<220>
   <223> forward primer for JO-76
<400> 468
   ccgcatatgg cgctggtgct gccgctggcg ccggtgagca agggcgagga gctg 54
<210> 469
   <211> 57
   <212> DNA
   <213> Artificial Sequence
<220>
   <223> forward primer for JO-77
<400> 469
   ccgcatatgg cggtggcgct gctgattctg gcggtggtga gcaagggcga ggagctg 57
<210> 470
   <211> 51
   <212> DNA
   <213> Artificial Sequence
<220>
   <223> forward primer for JO-78
<400> 470
   ccgcatatgg tgctgctggc ggtgattccg gtgagcaagg gcgaggagct g 51
<210> 471
   <211> 69
   <212> DNA
   <213> Artificial Sequence
<220>
   <223> forward primer for JO-79
<400> 471
   ccgcatatgc tgattgtggc ggcggtggtg gtggtggcgg tgctgattgt gagcaagggc 60
   gaggagctg 69
<210> 472
   <211> 51
   <212> DNA
   <213> Artificial Sequence
<220>
   <223> forward primer for JO-80
<400> 472
   ccgcatatgg cggtggtggt ggcggcgccg gtgagcaagg gcgaggagct g 51
<210> 473
   <211> 57
   <212> DNA
   <213> Artificial Sequence
<220>
   <223> forward primer for JO-81
<400> 473
   ccgcatatgc tggcggcggt gctgctgctg attccggtga gcaagggcga ggagctg 57
<210> 474
   <211> 60
   <212> DNA
   <213> Artificial Sequence
<220>
   <223> forward primer for JO-82
<400> 474
<210> 475
   <211> 63
   <212> DNA
   <213> Artificial Sequence
<220>
   <223> forward primer for JO-83
<400> 475
   ccgcatatgg cggtggcgct ggtggcggtg gtggcggtgg cggtgagcaa gggcgaggag 60
   ctg 63
<210> 476
   <211> 57
   <212> DNA
   <213> Artificial Sequence
<220>
   <223> forward primer for JO-84
<400> 476
   ccgcatatgc tggtggcggc gctgctggcg gtgctggtga gcaagggcga ggagctg 57
<210> 477
   <211> 63
   <212> DNA
   <213> Artificial Sequence
<220>
   <223> forward primer for JO-85
<400> 477
   ccgcatatgc tgctggcggc ggcggcggcg ctgctgctgg cggtgagcaa gggcgaggag 60
   ctg 63
<210> 478
   <211> 54
   <212> DNA
   <213> Artificial Sequence
<220>
   <223> forward primer for JO-86
<400> 478
   ccgcatatgc tggcggtgct ggcggcggcg ccggtgagca agggcgagga gctg 54
<210> 479
   <211> 69
   <212> DNA
   <213> Artificial Sequence
<220>
   <223> forward primer for JO-87
<400> 479
   ccgcatatgg tggtggtgct gctggtgctg ctggcgctgg tggtggtggt gagcaagggc 60
   gaggagctg 69
<210> 480
   <211> 51
   <212> DNA
   <213> Artificial Sequence
<220>
   <223> forward primer for JO-88
<400> 480
   ccgcatatgg tggtgattgc ggtggtgccg gtgagcaagg gcgaggagct g 51
<210> 481
   <211> 57
   <212> DNA
   <213> Artificial Sequence
<220>
   <223> forward primer for JO-89
<400> 481
   ccgcatatgg tgctgctggt gctgctggcg ctggtggtga gcaagggcga ggagctg 57
<210> 482
   <211> 57
   <212> DNA
   <213> Artificial Sequence
<220>
   <223> forward primer for JO-90
<400> 482
   ccgcatatgg tgctgctggt gctgctggcg ctggtggtga gcaagggcga ggagctg 57
<210> 483
   <211> 54
   <212> DNA
   <213> Artificial Sequence
<220>
   <223> forward primer for JO-91
<400> 483
   ccgcatatgc cggtgctggt gccggcggtg ccggtgagca agggcgagga gctg 54
<210> 484
   <211> 54
   <212> DNA
   <213> Artificial Sequence
<220>
   <223> forward primer for JO-92
<400> 484
   ccgcatatgc cggcgctggc gctggcgctg gcggtgagca agggcgagga gctg 54
<210> 485
   <211> 54
   <212> DNA
   <213> Artificial Sequence
<220>
   <223> forward primer for JO-93
<400> 485
   ccgcatatgg cggcggcggc gccggcgctg gcggtgagca agggcgagga gctg 54
<210> 486
   <211> 57
   <212> DNA
   <213> Artificial Sequence
<220>
   <223> forward primer for JO-94
<400> 486
   ccgcatatga ttgtgctgcc ggtgctggcg gcgccggtga gcaagggcga ggagctg 57
<210> 487
   <211> 60
   <212> DNA
   <213> Artificial Sequence
<220>
   <223> forward primer for JO-95
<400> 487
<210> 488
   <211> 63
   <212> DNA
   <213> Artificial Sequence
<220>
   <223> forward primer for JO-96
<400> 488
   ccgcatatgc tggcggcggt ggcgccggcg ctggcggtgg tggtgagcaa gggcgaggag 60
   ctg 63
<210> 489
   <211> 54
   <212> DNA
   <213> Artificial Sequence
<220>
   <223> forward primer for JO-97
<400> 489
   ccgcatatga ttctggtgct ggtgctgccg attgtgagca agggcgagga gctg 54
<210> 490
   <211> 57
   <212> DNA
   <213> Artificial Sequence
<220>
   <223> forward primer for JO-98
<400> 490
   ccgcatatga ttctgctgcc gctgctgctg ctgccggtga gcaagggcga ggagctg 57
<210> 491
   <211> 60
   <212> DNA
   <213> Artificial Sequence
<220>
   <223> forward primer for JO-99
<400> 491
<210> 492
   <211> 66
   <212> DNA
   <213> Artificial Sequence
<220>
   <223> forward primer for JO-100
<400> 492
   ccgcatatgc tgctgctggt ggcggtggtg ccgctgctgg tgccggtgag caagggcgag 60
   gagctg 66
<210> 493
   <211> 57
   <212> DNA
   <213> Artificial Sequence
<220>
   <223> forward primer for JO-101
<400> 493
   ccgcatatgc tgattctgct gctgctgccg attattgtga gcaagggcga ggagctg 57
<210> 494
   <211> 60
   <212> DNA
   <213> Artificial Sequence
<220>
   <223> forward primer for JO-102
<400> 494
<210> 495
   <211> 57
   <212> DNA
   <213> Artificial Sequence
<220>
   <223> forward primer for JO-103
<400> 495
   ccgcatatgc tggcgctgcc ggtgctgctg ctggcggtga gcaagggcga ggagctg 57
<210> 496
   <211> 51
   <212> DNA
   <213> Artificial Sequence
<220>
   <223> forward primer for JO-104
<400> 496
   ccgcatatgc tggcgctggc gctgctgctg gtgagcaagg gcgaggagct g 51
<210> 497
   <211> 57
   <212> DNA
   <213> Artificial Sequence
<220>
   <223> forward primer for JO-105
<400> 497
   ccgcatatgg tggcggtgcc gctgctggtg gtggcggtga gcaagggcga ggagctg 57
<210> 498
   <211> 63
   <212> DNA
   <213> Artificial Sequence
<220>
   <223> forward primer for JO-106
<400> 498
   ccgcatatgg cggtggcggt ggcgccggtg gcggcggcgg cggtgagcaa gggcgaggag 60
   ctg 63
<210> 499
   <211> 62
   <212> DNA
   <213> Artificial Sequence
<220>
   <223> forward primer for JO-107
<400> 499
   cccatatggc ggcggcggtg gtggcggcgg tgccggcggc ggtgagcaag ggcgaggagc 60
   tg 62
<210> 500
   <211> 56
   <212> DNA
   <213> Artificial Sequence
<220>
   <223> forward primer for JO-108
<400> 500
   cccatatggc gctgctggcg gcgctgctgg cgccggtgag caagggcgag gagctg 56
<210> 501
   <211> 48
   <212> DNA
   <213> Artificial Sequence
<220>
   <223> forward primer for JO-109
<400> 501
   cccattgcgc tggcgctgct ggtgccggtg agcaagggcg aggagctg 48
<210> 502
   <211> 72
   <212> DNA
   <213> Artificial Sequence
<220>
   <223> forward primer for JO-110
<400> 502
   ccgcatatgg cgctgctggc ggcgctgctg gcgctgctgg cgctgctggt ggtgagcaag 60
   ggcgaggagc tg 72
<210> 503
   <211> 63
   <212> DNA
   <213> Artificial Sequence
<220>
   <223> forward primer for JO-111
<400> 503
   ccgcatatgg cggcggcgct gccgctgctg gtgctgctgc cggtgagcaa gggcgaggag 60
   ctg 63
<210> 504
   <211> 59
   <212> DNA
   <213> Artificial Sequence
<220>
   <223> forward primer for JO-112
<400> 504
   cccatatggc ggcggcggtg ccggcggcgc tggcgccggt gagcaagggc gaggagctg 59
<210> 505
   <211> 58
   <212> DNA
   <213> Artificial Sequence
<220>
   <223> forward primer for JO-113
<400> 505
   cccattggcg gcgctggcgg tggcggcgct ggcggcggtg agcaagggcg aggagctg 58
<210> 506
   <211> 52
   <212> DNA
   <213> Artificial Sequence
<220>
   <223> forward primer for JO-114
<400> 506
   cccattggcg gtgctggcgg cggcggtgcc ggtgagcaag ggcgaggagc tg 52
<210> 507
   <211> 54
   <212> DNA
   <213> Artificial Sequence
<220>
   <223> forward primer for JO-115
<400> 507
   ccgcatatgg tggcggcgct gccggcgccg gcggtgagca agggcgagga gctg 54
<210> 508
   <211> 63
   <212> DNA
   <213> Artificial Sequence
<220>
   <223> forward primer for JO-116
<400> 508
   ccgcatatgg cgctggcgct ggcggtgccg gcggtgctgc cggtgagcaa gggcgaggag 60
   ctg 63
<210> 509
   <211> 63
   <212> DNA
   <213> Artificial Sequence
<220>
   <223> forward primer for JO-117
<400> 509
   ccgcatatgg cggcgctgct gccggcggcg gtggcggtgc cggtgagcaa gggcgaggag 60
   ctg 63
<210> 510
   <211> 54
   <212> DNA
   <213> Artificial Sequence
<220>
   <223> forward primer for JO-118
<400> 510
   ccgcatatgg cggtggtggt ggcgctggcg ccggtgagca agggcgagga gctg 54
<210> 511
   <211> 72
   <212> DNA
   <213> Artificial Sequence
<220>
   <223> forward primer for JO-119
<400> 511
   ccgcatatgg cggcggcggt ggcgctgccg gcggcggcgg cgctgctggc ggtgagcaag 60
   ggcgaggagc tg 72
<210> 512
   <211> 69
   <212> DNA
   <213> Artificial Sequence
<220>
   <223> forward primer for JO-120
<400> 512
   ccgcatatgg cggtggtgct gccgctggcg ctggtggcgg tggcgccggt gagcaagggc 60
   gaggagctg 69
<210> 513
   <211> 54
   <212> DNA
   <213> Artificial Sequence
<220>
   <223> forward primer for JO-121
<400> 513
   ccgcatatgc tggtggcgct gccgctgctg ccggtgagca agggcgagga gctg 54
<210> 514
   <211> 60
   <212> DNA
   <213> Artificial Sequence
<220>
   <223> forward primer for JO-122
<400> 514
<210> 515
   <211> 54
   <212> DNA
   <213> Artificial Sequence
<220>
   <223> forward primer for JO-123
<400> 515
   ccgcatatgc tggcggtggt gctggcggtg ccggtgagca agggcgagga gctg 54
<210> 516
   <211> 60
   <212> DNA
   <213> Artificial Sequence
<220>
   <223> forward primer for JO-124
<400> 516
<210> 517
   <211> 54
   <212> DNA
   <213> Artificial Sequence
<220>
   <223> forward primer for JO-125
<400> 517
   ccgcatatgc tggtggcgct ggtgctgctg ccggtgagca agggcgagga gctg 54
<210> 518
   <211> 72
   <212> DNA
   <213> Artificial Sequence
<220>
   <223> forward primer for JO-126
<400> 518
   ccgcatatgc tggtgctgct gctggcggtg ctgctgctgg cggtgctgcc ggtgagcaag 60
   ggcgaggagc tg 72
<210> 519
   <211> 66
   <212> DNA
   <213> Artificial Sequence
<220>
   <223> forward primer for JO-127
<400> 519
   ccgcatatgc tgctggcgcc ggtggtggcg ctggtgattc tgccggtgag caagggcgag 60
   gagctg 66
<210> 520
   <211> 69
   <212> DNA
   <213> Artificial Sequence
<220>
   <223> forward primer for JO-128
<400> 520
   ccgcatatgg tgctggcggt gctggcggtg ccggtgctgc tgctgccggt gagcaagggc 60
   gaggagctg 69
<210> 521
   <211> 60
   <212> DNA
   <213> Artificial Sequence
<220>
   <223> forward primer for JO-129
<400> 521
<210> 522
   <211> 63
   <212> DNA
   <213> Artificial Sequence
<220>
   <223> forward primer for JO-130
<400> 522
   ccgcatatgc tgctggtgct gctggcgctg gtggtggtgc cggtgagcaa gggcgaggag 60
   ctg 63
<210> 523
   <211> 63
   <212> DNA
   <213> Artificial Sequence
<220>
   <223> forward primer for JO-131
<400> 523
   ccgcatatgg tgctgctggc gctgccggtg gtggcggcgc cggtgagcaa gggcgaggag 60
   ctg 63
<210> 524
   <211> 66
   <212> DNA
   <213> Artificial Sequence
<220>
   <223> forward primer for JO-132
<400> 524
   ccgcatatgg cggtggtggt gccggcgatt gtgctggcgg cgccggtgag caagggcgag 60
   gagctg 66
<210> 525
   <211> 63
   <212> DNA
   <213> Artificial Sequence
<220>
   <223> forward primer for JO-133
<400> 525
   ccgcatatgg cggtgctggt gccggcggcg gcgctggtgc cggtgagcaa gggcgaggag 60
   ctg 63
<210> 526
   <211> 60
   <212> DNA
   <213> Artificial Sequence
<220>
   <223> forward primer for JO-134
<400> 526
<210> 527
   <211> 60
   <212> DNA
   <213> Artificial Sequence
<220>
   <223> forward primer for JO-135
<400> 527
<210> 528
   <211> 54
   <212> DNA
   <213> Artificial Sequence
<220>
   <223> forward primer for JO-136
<400> 528
   ccgcatatgc tgattgcgct gccgctgctg ccggtgagca agggcgagga gctg 54
<210> 529
   <211> 69
   <212> DNA
   <213> Artificial Sequence
<220>
   <223> forward primer for JO-137
<400> 529
   ccgcatatgc tgctggcgct gccgctggtg ctggtgctgg cgctgccggt gagcaagggc 60
   gaggagctg 69
<210> 530
   <211> 57
   <212> DNA
   <213> Artificial Sequence
<220>
   <223> forward primer for JO-138
<400> 530
   ccgcatatga ttgtgccgct gctgctggcg gcgccggtga gcaagggcga ggagctg 57
<210> 531
   <211> 60
   <212> DNA
   <213> Artificial Sequence
<220>
   <223> forward primer for JO-139
<400> 531
<210> 532
   <211> 60
   <212> DNA
   <213> Artificial Sequence
<220>
   <223> forward primer for JO-140
<400> 532
<210> 533
   <211> 60
   <212> DNA
   <213> Artificial Sequence
<220>
   <223> forward primer for JO-141
<400> 533
<210> 534
   <211> 63
   <212> DNA
   <213> Artificial Sequence
<220>
   <223> forward primer for JO-142
<400> 534
   ccgcatatgc tggcgctgct gctgccggcg gcgctgattc cggtgagcaa gggcgaggag 60
   ctg 63
<210> 535
   <211> 66
   <212> DNA
   <213> Artificial Sequence
<220>
   <223> forward primer for JO-143
<400> 535
   ccgcatatgg cgctgctgcc gctgctggcg gtggtgctgc cgccggtgag caagggcgag 60
   gagctg 66
<210> 536
   <211> 60
   <212> DNA
   <213> Artificial Sequence
<220>
   <223> forward primer for JO-144
<400> 536
<210> 537
   <211> 63
   <212> DNA
   <213> Artificial Sequence
<220>
   <223> forward primer for JO-145
<400> 537
   ccgcatatgg cggcggcgcc ggtgctgctg ctgctgctgc cggtgagcaa gggcgaggag 60
   ctg 63
<210> 538
   <211> 63
   <212> DNA
   <213> Artificial Sequence
<220>
   <223> forward primer for JO-146
<400> 538
   ccgcatatgg cggcggcgcc ggtgctgctg ctgctgctgc cggtgagcaa gggcgaggag 60
   ctg 63
<210> 539
   <211> 66
   <212> DNA
   <213> Artificial Sequence
<220>
   <223> forward primer for JO-147
<400> 539
   ccgcatatgg cggcgctggc ggcgctggtg gtggcggcgc cgccggtgag caagggcgag 60
   gagctg 66
<210> 540
   <211> 66
   <212> DNA
   <213> Artificial Sequence
<220>
   <223> forward primer for JO-148
<400> 540
   ccgcatatgg cggcgctggc ggcggtgccg ctggcgctgg cgccggtgag caagggcgag 60
   gagctg 66
<210> 541
   <211> 72
   <212> DNA
   <213> Artificial Sequence
<220>
   <223> forward primer for JO-149
<400> 541
   ccgcatatgg cgctggcggt ggcggcgccg gcgctggcgc tgctgccgcc ggtgagcaag 60
   ggcgaggagc tg 72
<210> 542
   <211> 54
   <212> DNA
   <213> Artificial Sequence
<220>
   <223> forward primer for JO-150
<400> 542
   ccgcatatgc tggtggcgct ggtgctgctg ccggtgagca agggcgagga gctg 54
<210> 543
   <211> 72
   <212> DNA
   <213> Artificial Sequence
<220>
   <223> forward primer for JO-151
<400> 543
   ccgcatatgc tggtgctgct gctggcggtg ctgctgctgg cggtgctgcc ggtgagcaag 60
   ggcgaggagc tg 72
<210> 544
   <211> 66
   <212> DNA
   <213> Artificial Sequence
<220>
   <223> forward primer for JO-152
<400> 544
   ccgcatatgc tgctggcgcc ggtggtggcg ctggtgattc tgccggtgag caagggcgag 60
   gagctg 66
<210> 545
   <211> 69
   <212> DNA
   <213> Artificial Sequence
<220>
   <223> forward primer for JO-153
<400> 545
   ccgcatatgg tgctggcggt gctggcggtg ccggtgctgc tgctgccggt gagcaagggc 60
   gaggagctg 69
<210> 546
   <211> 60
   <212> DNA
   <213> Artificial Sequence
<220>
   <223> forward primer for JO-154
<400> 546
<210> 547
   <211> 63
   <212> DNA
   <213> Artificial Sequence
<220>
   <223> forward primer for JO-155
<400> 547
   ccgcatatgc tgctggtgct gctggcgctg gtggtggtgc cggtgagcaa gggcgaggag 60
   ctg 63
<210> 548
   <211> 63
   <212> DNA
   <213> Artificial Sequence
<220>
   <223> forward primer for JO-156
<400> 548
   ccgcatatgg tgctgctggc gctgccggtg gtggcggcgc cggtgagcaa gggcgaggag 60
   ctg 63
<210> 549
   <211> 66
   <212> DNA
   <213> Artificial Sequence
<220>
   <223> forward primer for JO-157
<400> 549
   ccgcatatgg cggtggtggt gccggcgatt gtgctggcgg cgccggtgag caagggcgag 60
   gagctg 66
<210> 550
   <211> 63
   <212> DNA
   <213> Artificial Sequence
<220>
   <223> forward primer for JO-158
<400> 550
   ccgcatatgg cggtgctggt gccggcggcg gcgctggtgc cggtgagcaa gggcgaggag 60
   ctg 63
<210> 551
   <211> 60
   <212> DNA
   <213> Artificial Sequence
<220>
   <223> forward primer for JO-159
<400> 551
<210> 552
   <211> 60
   <212> DNA
   <213> Artificial Sequence
<220>
   <223> forward primer for JO-160
<400> 552
<210> 553
   <211> 54
   <212> DNA
   <213> Artificial Sequence
<220>
   <223> forward primer for JO-161
<400> 553
   ccgcatatgc tgattgcgct gccgctgctg ccggtgagca agggcgagga gctg 54
<210> 554
   <211> 69
   <212> DNA
   <213> Artificial Sequence
<220>
   <223> forward primer for JO-162
<400> 554
   ccgcatatgc tgctggcgct gccgctggtg ctggtgctgg cgctgccggt gagcaagggc 60
   gaggagctg 69
<210> 555
   <211> 57
   <212> DNA
   <213> Artificial Sequence
<220>
   <223> forward primer for JO-163
<400> 555
   ccgcatatga ttgtgccgct gctgctggcg gcgccggtga gcaagggcga ggagctg 57
<210> 556
   <211> 60
   <212> DNA
   <213> Artificial Sequence
<220>
   <223> forward primer for JO-164
<400> 556
<210> 557
   <211> 60
   <212> DNA
   <213> Artificial Sequence
<220>
   <223> forward primer for JO-165
<400> 557
<210> 558
   <211> 60
   <212> DNA
   <213> Artificial Sequence
<220>
   <223> forward primer for JO-166
<400> 558
<210> 559
   <211> 63
   <212> DNA
   <213> Artificial Sequence
<220>
   <223> forward primer for JO-167
<400> 559
   ccgcatatgc tggcgctgct gctgccggcg gcgctgattc cggtgagcaa gggcgaggag 60
   ctg 63
<210> 560
   <211> 66
   <212> DNA
   <213> Artificial Sequence
<220>
   <223> forward primer for JO-168
<400> 560
   ccgcatatgc tggcggcggt ggtgccggtg gcggcggcgg tgccggtgag caagggcgag 60
   gagctg 66
<210> 561
   <211> 57
   <212> DNA
   <213> Artificial Sequence
<220>
   <223> forward primer for JO-169
<400> 561
   ccgcatatgg tggcggcgcc ggcggcggcg gcgccggtga gcaagggcga ggagctg 57
<210> 562
   <211> 54
   <212> DNA
   <213> Artificial Sequence
<220>
   <223> forward primer for JO-170
<400> 562
   ccgcatatgg cggtgccggt gccggtgccg ctggtgagca agggcgagga gctg 54
<210> 563
   <211> 60
   <212> DNA
   <213> Artificial Sequence
<220>
   <223> forward primer for JO-171
<400> 563
<210> 564
   <211> 63
   <212> DNA
   <213> Artificial Sequence
<220>
   <223> forward primer for JO-172
<400> 564
   ccgcatatgg cgctggcgct gccggcgctg gcgattgcgc cggtgagcaa gggcgaggag 60
   ctg 63
<210> 565
   <211> 57
   <212> DNA
   <213> Artificial Sequence
<220>
   <223> forward primer for JO-173
<400> 565
   ccgcatatgg cggtgattcc gattctggcg gtgccggtga gcaagggcga ggagctg 57
<210> 566
   <211> 63
   <212> DNA
   <213> Artificial Sequence
<220>
   <223> forward primer for JO-174
<400> 566
   ccgcatatgc tgattctgct gctgccggcg gtggcgctgc cggtgagcaa gggcgaggag 60
   ctg 63
<210> 567
   <211> 60
   <212> DNA
   <213> Artificial Sequence
<220>
   <223> forward primer for JO-175
<400> 567
<210> 568
   <211> 63
   <212> DNA
   <213> Artificial Sequence
<220>
   <223> forward primer for JO-176
<400> 568
   ccgcatatgg tggtggtggt gccggtgctg gcggcggcgg cggtgagcaa gggcgaggag 60
   ctg 63
<210> 569
   <211> 51
   <212> DNA
   <213> Artificial Sequence
<220>
   <223> forward primer for JO-177
<400> 569
   ccgcatatgc tggtggcggt ggcggcgccg gtgagcaagg gcgaggagct g 51
<210> 570
   <211> 60
   <212> DNA
   <213> Artificial Sequence
<220>
   <223> forward primer for JO-178
<400> 570
<210> 571
   <211> 57
   <212> DNA
   <213> Artificial Sequence
<220>
   <223> forward primer for JO-179
<400> 571
   ccgcatatgc tgattgcgcc ggcggcggcg gtgccggtga gcaagggcga ggagctg 57
<210> 572
   <211> 60
   <212> DNA
   <213> Artificial Sequence
<220>
   <223> forward primer for JO-180
<400> 572
<210> 573
   <211> 57
   <212> DNA
   <213> Artificial Sequence
<220>
   <223> forward primer for JO-181
<400> 573
   ccgcatatgg cggtgctgct gctgccggcg gcggcggtga gcaagggcga ggagctg 57
<210> 574
   <211> 57
   <212> DNA
   <213> Artificial Sequence
<220>
   <223> forward primer for JO-182
<400> 574
   ccgcatatga ttgcgctggc gctgctgccg ctgctggtga gcaagggcga ggagctg 57
<210> 575
   <211> 60
   <212> DNA
   <213> Artificial Sequence
<220>
   <223> forward primer for JO-183
<400> 575
<210> 576
   <211> 63
   <212> DNA
   <213> Artificial Sequence
<220>
   <223> forward primer for JO-184
<400> 576
   ccgcatatgg cgccggcggt gctgccgccg gtggtggtga ttgtgagcaa gggcgaggag 60
   ctg 63
<210> 577
   <211> 57
   <212> DNA
   <213> Artificial Sequence
<220>
   <223> forward primer for JO-185
<400> 577
   ccgcatatgg tggtgggcct gctggtggcg gcgctggtga gcaagggcga ggagctg 57
<210> 578
   <211> 63
   <212> DNA
   <213> Artificial Sequence
<220>
   <223> forward primer for JO-186
<400> 578
   ccgcatatgg cggcgattgc ggcggcggcg ccgctggcgg cggtgagcaa gggcgaggag 60
   ctg 63
<210> 579
   <211> 51
   <212> DNA
   <213> Artificial Sequence
<220>
   <223> forward primer for JO-187
<400> 579
   ccgcatatgc tgctgctggc ggtggcgccg gtgagcaagg gcgaggagct g 51
<210> 580
   <211> 60
   <212> DNA
   <213> Artificial Sequence
<220>
   <223> forward primer for JO-188
<400> 580
<210> 581
   <211> 54
   <212> DNA
   <213> Artificial Sequence
<220>
   <223> forward primer for JO-189
<400> 581
   ccgcatatgg cgctgctgct gctggtgctg gcggtgagca agggcgagga gctg 54
<210> 582
   <211> 57
   <212> DNA
   <213> Artificial Sequence
<220>
   <223> forward primer for JO-190
<400> 582
   ccgcatatgc tgctgctgct gctgctgccg ctggcggtga gcaagggcga ggagctg 57
<210> 583
   <211> 54
   <212> DNA
   <213> Artificial Sequence
<220>
   <223> forward primer for JO-191
<400> 583
   ccgcatatgc tggcgctgcc gctgctgctg ccggtgagca agggcgagga gctg 54
<210> 584
   <211> 54
   <212> DNA
   <213> Artificial Sequence
<220>
   <223> forward primer for JO-192
<400> 584
   ccgcatatgc tgctggtgct gccgctgctg attgtgagca agggcgagga gctg 54
<210> 585
   <211> 57
   <212> DNA
   <213> Artificial Sequence
<220>
   <223> forward primer for JO-193
<400> 585
   ccgcatatgc tgccgctgct gccggcggcg ctggtggtga gcaagggcga ggagctg 57
<210> 586
   <211> 27
   <212> DNA
   <213> Artificial Sequence
<220>
   <223> reverse primer for JO-01 to JO-193
<400> 586
   ttatctagat ccggtggatc ccgggcc 27

## Description

### [Technical Field]

The present invention relates to novel macromolecule transduction domain (MTD) peptides comprising an amino acid sequence of SEQ ID NO: 173 which facilitate the traverse of a biologically active molecule across the cell membrane, polynucleotides encoding the same, methods of identifying the same, methods of genetically engineering a biologically active molecule having cell permeability by using the same, methods of importing a biologically active molecule into a cell by using the same, and uses thereof.

### [Background Art]

Cellular internalization of macromolecules, such as DNA, RNA, proteins, oligonucleotides, and peptides, is still a challenging task because of the presence of the plasma membrane, which constitutes an impermeable barrier for such molecules. Numerous difficulties have been encountered in delivering such molecules to a desired target, including poor penetration into a tissue or cell, toxicity when delivered systemically due to the insufficient specificity of targeting to a particular tissue or cell, side effects when delivered in a high concentration in order to achieve an adequate local concentration at a particular target cell or tissue, and degradation such that inadequate amounts are delivered to the target and/or such that byproducts of degradation result in undesirable side effects.

In order to circumvent these problems, several carrier-mediated delivery systems have been developed. Among them, much attention has recently been given to the use of peptide-based delivery systems. The use of peptides with cell permeability has several advantages, which are mainly due to the various modifications that can be made to the peptide sequence. This allows the engineering of carriers that can address different cellular subdomains and/or are able to transport various types of cargo molecules.

Many cell permeable peptides are designed from sequences of membrane-interacting proteins, such as recombinant proteins, signal peptides, transmembrane domains, and antimicrobial peptides. Within these sequences, short sequences called protein transduction domains (PTDs) have been proved to efficiently cross biological membranes without the need of a carrier or a receptor and to deliver peptides or proteins into intracellular compartments. A number of studies have suggested that the use of PTD-based peptides could be of major importance for therapies against viral diseases or cancers. Among the PTD-based peptides, the third helix of the homeodomain of antennapedia called penetratin (Joliot, A. and A. Prochiantz, Nat. Cell Biol. 6(3): 189-96 (2004)), the Tat peptide derived from the transactivating protein Tat of HIV-1 (Wadia, J.S. and S.F. Dowdy, Curr. Opin. Biotechnol. 13(1):52-6 (2002)), transportan (Pooga et al., Faseb J. 12(1):67-77 (1998)), and VP22 (Elliott, G. and P. O'Hare, Cell 88(2):223-33 (1997)) have been shown to improve the cellular uptake of peptides, proteins, and oligonucleotides. WO 99/49879 describes artificial PTDs, wherein N- and C-termini vary, e.g., by additional amino acids

A second category of cell-penetrating peptides, called amphipathic peptides, has also been described. An amphipathic molecule can be defined, in short, as consisting of two domains: a hydrophilic (polar) domain and a hydrophobic (non-polar) domain. For peptides, the amphipathic character can arise from either the primary structure or the secondary structure. Primary amphipathic peptides can be defined as the sequential assembly of a domain of hydrophobic residues with a domain of hydrophilic residues. Secondary amphipathic peptides are generated by the conformational state which allows the positioning of the hydrophobic and hydrophilic residues on opposite sides of the molecule.

Other peptides, such as polyarginine-based peptides, calcitonin-derived peptides, and oligomers, have also been proposed as tools for intracellular delivery of therapeutics.

However, the currently known delivery systems appear to be limited due to their lack of efficiency and/or their toxicity, and little is known about the pathway of their cellular uptake, constituting a handicap for improving their efficiency. In addition, a number of delivery systems are limited in their ability to cross cellular and nuclear membranes. Even where such delivery peptides do cross cell membranes, they are often limited in their efficacies due to their entrapment in endosomes.

The present invention is directed to overcoming these deficiencies in the art.

### [Disclosure]

### [Technical Solution]

The present invention relates to isolated macromolecule transduction domain (MTD) peptides comprising an amino acid sequence of SEQ ID NO: 173 capable of mediating the transport of a biologically active molecule into a cell.

Another aspect of the present invention relates to isolated polynucleotides encoding such MTD peptides.

The present invention also relates to a method of identifying a MTD peptide comprising an amino acid sequence of SEQ ID NO: 173 having cell permeability.

Another aspect of the present invention relates to a method of genetically engineering a biologically active molecule having cell permeability by attaching a MTD peptide comprising an amino acid sequence of SEQ ID NO: 173 to a biologically active molecule.

Still another aspect of the present invention relates to an isolated recombinant protein having cell permeability comprising a MTD peptide of SEQ ID NO: 173 having cell permeability and a biologically active molecule.

The present invention also relates to a pharmaceutical composition for delivery of a biologically active molecule to a cell comprising a cell permeable recombinant protein where a MTD is attached to a biologically active molecule.

Another aspect of the present invention relates to a method of transporting a biologically active molecule into a cell in a subject comprising administering to a subject a cell permeable recombinant protein comprising a MTD peptide comprising an amino acid sequence of SEQ ID NO: 173 attached to a biologically active molecule.

Other aspects of the present invention relate to the use of MTD peptides comprising an amino acid sequence of SEQ ID NO: 173 for drug delivery, vaccine administration, protein therapy, and gene therapy.

### [Advantageous Effects]

The present invention provides MTD peptides comprising an amino acid sequence of SEQ ID NO: 173 having cell permeability which can facilitate the transport of a biologically active molecule into a cell and, thus, can be effectively used for drug delivery, vaccine administration, peptide therapy, and gene therapy.

### [Description of Drawings]

Figs. 1a to 1o show tables illustrating the amino acid sequences and structural characteristics of macromolecule transduction domain (MTD) peptides including the MTD peptide comprising an amino acid sequence of SEQ ID NO: 173 identified according to the method of the present invention.
Fig. 2 is a schematic diagram illustrating the His-MTD-EGFP recombinant protein having cell permeability and comprising an amino acid sequence of SEQ ID NO: 173 constructed according to the present invention.
Figs. 3a to 3c are photographs of an agarose gel electrophoresis analysis showing DNA fragments encoding MTDs-EGFP amplified by PCR including an MTD comprising an amino acid sequence of SEQ ID NO: 173 according to the present invention.
Fig. 4a is a schematic diagram illustrating the subcloning of a DNA fragment encoding MTD-EGFP into the pGEM-Teasy vector, whereas Figs. 4b to 4d are photographs of an agarose gel electrophoresis analysis showing DNA fragments encoding MTDs-EGFP subcloned into pGEM-Teasy vector including an MTD comprising an amino acid sequence of SEQ ID NO: 173 according to the present invention.
Fig. 5a is a schematic diagram illustrating the cloning of a DNA fragment encoding MTD-EGFP into the pET 28(+) vector, whereas Figs. 5b to 5c are photographs of an agarose gel electrophoresis analysis showing DNA fragments encoding MTDs-EGFP cloned into the pET 28(+) vector including an MTD comprising an amino acid sequence of SEQ ID NO: 173 according to the present invention.
Figs. 6a and 6b are photographs of a SDS-PAGE analysis illustrating the inducible expression of His-MTD-EGFP recombinant proteins including an MTD comprising an amino acid sequence of SEQ ID NO: 173 according to the present invention.
Figs. 7a and 7b are photographs of a SDS-PAGE analysis showing the purity of His-MTD-EGFP recombinant proteins under denaturing conditions including an MTD comprising an amino acid sequence of SEQ ID NO: 173 according to the present invention.
Fig. 8 is a photograph of a SDS-PAGE analysis showing the purification of His-MTD-EGFP recombinant proteins under renaturing conditions including an MTD comprising an amino acid sequence of SEQ ID NO: 173 according to the present invention.
Figs. 9a to 9g are graphs illustrating the cell permeabilities of His-MTD-EGFP recombinant proteins analyzed by flow cytometry including an MTD comprising an amino acid sequence of SEQ ID NO: 173 according to the present invention.
Figs. 10a to 10i are photographs visualizing the cell permeabilities of His-MTD-EGFP recombinant proteins by confocal laser scanning microscopy including an MTD comprising an amino acid sequence of SEQ ID NO: 173 according to the present invention.
Figs. 11a and 11b are graphs comparing the cell permeabilities of His-MTD-EGFP recombinant proteins including an MTD comprising an amino acid sequence of SEQ ID NO: 173 according to the present invention with a positive control.
Figs. 12a to 12i are photographs visualizing the *in vivo* distribution of His-MTD-EGFP recombinant proteins by fluorescence Figs. 13a to 13k show tables summarizing the characteristics of the MTD peptides including an MTD comprising an amino acid sequence of SEQ ID NO: 173 identified according to the method of the present invention.

### [Detailed Description]

The present invention relates to novel macromolecule transduction domain (MTD) peptides comprising an amino acid sequence of SEQ ID NO: 173 having cell permeability that facilitate the traverse of a biologically active molecule across the cell membrane. The MTD peptides of the present invention are cell permeable polypeptides capable of mediating the import of a biologically active molecule, including polypeptides, protein domains, or full-length proteins, through the cell membrane. The MTD peptides of the present invention are characterized as having a single hydrophobic region at their N-terminus, forming a helix structure, exhibiting flexibility, and having relatively short amino acids (7 to 17 amino acids) in length (see Figs. In).

One embodiment of the present invention relates to isolated MTD peptides having cell permeability, where the MTD peptide has an amino acid sequence of SEQ ID NO: 173, analogs, derivatives, amidated variations, and conservative variations thereof.

A person having ordinary skill in the art can make similar substitutions to obtain peptides having greater cell permeability and a broader host range. For example, the present invention provides peptides corresponding to amino acid sequence SEQ ID NO:173, as well as analogues, derivatives, and amidated derivatives thereof, as long as the cell permeability of the peptide remains. Minor modifications to the primary amino acid sequence of the peptides of the present invention may result in peptides which have substantially equivalent or enhanced cell permeability, as compared to the specific peptides described herein. Such modifications may be deliberate, as by site-directed mutagenesis, or may be spontaneous. All of the peptides produced by these modifications are included herein, as long as the cell permeability of the original peptide still exists or, in the case of amidated versions of the peptide, the cell permeability of the original peptide is enhanced or altered such that the amidated peptide is therapeutically useful. It is envisioned that such modifications are useful for altering or enhancing the cell permeability of a particular peptide.

Further, the deletion of one or more amino acids can also result in a modification to the structure of the resultant molecule without any significant change in its cell permeability. This can lead to the development of a smaller active molecule which may also have utility. For example, amino- or carboxy-terminal amino acids which may not be required for the cell permeability of a particular peptide can be removed. The peptides of the present invention include any analog, homolog, mutant, isomer, or derivative of the peptides disclosed in the present application, so long as the cell permeability, as described herein, remains. All peptides were synthesized using L-amino acids; however, D forms of all of the peptides can be synthetically produced. In addition, C-terminal derivatives, such as C-terminal methyl esters and C-terminal amidates, can be produced, in order to increase the cell permeability of the peptide of the present invention.

A "peptide" of the present invention includes amino acid sequences that are conservative variations of those peptides specifically exemplified herein. The term "conservative variation" as used herein denotes the replacement of an amino acid residue by another, biologically similar residue. Examples of conservative variations include the substitution of one hydrophobic residue, such as isoleucine, valine, leucine, alanine, cysteine, glycine, phenylalanine, proline, tryptophan, tyrosine, norleucine, or methionine for another, or the substitution of one polar residue for another, such as the substitution of arginine for lysine, glutamic for aspartic acids, or glutamine for asparagine, and the like. Neutral hydrophilic amino acids which can be substituted for one another include asparagine, glutamine, serine, and threonine. The term "conservative variation" also includes the use of a substituted amino acid in place of an unsubstituted parent amino acid, provided that antibodies raised to the substituted polypeptide also immunoreact with the unsubstituted polypeptide. Such conservative substitutions are within the definition of the classes of the peptides of the present invention.

Another aspect of the present invention relates to isolated polynucleotides encoding the above MTD peptides comprising an amino acid sequence of SEQ ID NO: 173 of the present invention. The polynucleotides encode the MTD peptides having an amino acid sequence of SEQ ID NO: 173 and analogs, derivatives, amidated variations, and conservative variations thereof. The polynucleotides encoding the MTD peptides of the present invention comprise a nucleotide sequence represented by SEQ ID NO: 366.

The polynucleotides of the present invention may be in the form of a RNA or DNA, where DNA includes cDNA and synthetic DNA. The DNA may be single stranded or double stranded. If it is single stranded, it may be the coding strand or non-coding (antisense) strand. The coding sequence may be identical to the nucleotide sequence of SEQ ID NO: 366 or may be a different coding sequence, where the coding sequence, as a result of degeneracy or redundancy of the genetic code, encodes for the same polypeptide.

The polynucleotides of the present invention also include variants of the above-described polynucleotides, which encode fragments, analogs, and derivatives of the polynucleotides characterized by the deduced amino acid sequence of SEQ ID NO: 173. The variant of the polynucleotide may be a naturally occurring allelic variant of the polynucleotide or a non-naturally occurring variant of the polynucleotide.

The polynucleotide of the present invention may have a coding sequence which is a naturally occurring allelic variant of the coding sequence of SEQ ID NO: 366. An allelic variant is an alternate form of a polynucleotide sequence which may have a substitution, deletion, or addition of one or more nucleotides, which does not substantially alter the function of the encoded polypeptide.

Sequences similar or homologous (e.g., at least about 70% sequence identity) to the sequences disclosed herein are also part of the present invention. In other embodiments of the present invention, the sequence identity at the amino acid level can be about 80%, 85%, 90%, 91%, 92%, 93%, 94%, 95%, 96%, 97%, 98%, 99%, or higher. At the polynucleotide level, the sequence identity can be about 70%, 75%, 80%, 85%, 90%, 91%, 92%, 93%, 94%, 95%, 96%, 97%, 98%, 99%, or higher. Alternatively, substantial identity exists when the polynucleotide segments hybridize under selective hybridization conditions (e.g., very high stringency hybridization conditions), to the complement of the strand. The polynucleotides may be present in whole cells, in a cell lysate, or in a partially purified or substantially pure form.

It is well known in the art that a single amino acid may be encoded by more than one nucleotide codon and that the polynucleotide may be easily modified to produce an alternate polynucleotide that encodes the same peptide. Therefore, other embodiments of the present invention include alternate nucleotide sequences encoding peptides containing the amino acid sequences as previously described. Nucleic acid molecules encoding peptides containing the claimed amino acid sequence include nucleotide sequences which encode any combination of the claimed sequence and any other amino acids located N-terminal or C-terminal to the claimed amino acid sequence.

It is to be understood that amino acid and nucleic acid sequence of the present invention may include additional residues, particularly N- or C-terminal amino acids or 5' or 3' polynucleotides, and still be essentially as set forth in the sequence disclosed herein, as long as the sequence confers membrane permeability upon the polypeptide or protein moiety of the recombinant protein.

The present invention also relates to a method of identifying the MTD peptides having cell permeability.

The method of the present invention involves the following steps:
1) identifying secreted proteins having a signal sequence-like domain from multiple amino acid sequence databases;
2) selecting from said identified secreted proteins hydrophobic peptides having a single hydrophobic region at their N-terminus and forming a helix structure; and
3) optimizing and minimizing said selected hydrophobic peptides under conditions effective to produce peptides suitable for use as an MTD peptide.

In order to identify certain candidate peptides that can potentially penetrate the plasma membrane, secreted proteins that have a signal sequence-like domain are identified from multiple amino acid sequence databases, as described in step 1). In a specific embodiment of the present invention, these secreted proteins are selected from the PubMed Entrez Protein Database by using several key words, such as "hydrophobic region of signal sequence," "signal sequence hydrophobic region," "signal sequence of secreted protein," "hydrophobic signal sequence," and "hydrophobic domain of secreted protein." As a result, more than 1,500 secreted proteins that have a signal sequence-like domain are identified.

As used herein, the term "signal sequence-like domain" refers to a peptide capable of mediating macromolecule translocation across the plasma membrane.

The term "signal peptide" as used herein is a short (3-60 amino acids long) peptide chain that directs the post-translational transport of a protein. Signal peptides may also be called targeting signals, signal sequences, transit peptides, or localization signals. The amino acid sequences of signal peptides direct proteins (which are synthesized in the cytoplasm) to certain organelles such as the nucleus, mitochondrial matrix, endoplasmic reticulum, chloroplast, apoplast and peroxisome. Some signal peptides are cleaved from the protein by signal peptidase after the proteins are transported. The N-terminal sequence of a secreted protein, which is required for transport through the cell membrane.

Examples of signal peptides may include, but are not limited to, transport to the nucleus (NLS: -Pro-Pro-Lys-Lys-Lys-Arg-Lys-Val-), transport to the endoplasmic reticulum (H₂N-Met-Met-Ser-Phe-Val-Ser-Leu-Leu-Leu-Val-Gly-Ile-Leu-Phe-Trp-Ala-Thr-Glu-Ala-Glu-Gln-Leu-Thr-Lys-Cys-Glu-Val-Phe-Gln-), retention to the endoplasmic reticulum (-Lys-Asp-Glu-Leu-COOH), transport to the mitochondrial matrix (H₂N-Met-Leu-Ser-Leu-Arg-Gln-Ser-Ile-Arg-Phe-Phe-Lys-Pro-Ala-Thr-Arg-Thr-Leu-Cys-Ser-Ser-Arg-Tyr-Leu-Leu-), transport to the peroxisome (PTS1: -Ser-Lys-Leu-COOH), transport to the peroxisome (PTS2: H₂N-----Arg-Leu-X5-His-Leu-) (wherein, H₂N is the N-terminus of a protein, and COOH is the C-Terminus of a protein).

Signal sequence-like domains are for example penetratin (Arg-Gln-Ile-Lys-Ile-Trp-Phe-Gln-Asn-Arg-Arg-Met-Lys-Trp-Lys-Lys), the Tat peptide derived from the transactivating protein Tat of HIV-1 (Arg-Lys-Lys-Arg-Arg-Gln-Arg-Arg-Pro), transportan (Gly-Trp-Thr-Leu-Asn-Ser-Ala-Gly-Tyr-Leu-Leu-Gly-Lys-Ile-Asn-Lys-Ala-Leu-Ala-Ala-Leu-Ala-Lys-Lys-Ile-Leu), Buforin II (Thr-Arg-Ser-Ser-Arg-Ala-Gly-Leu-Gln-Phe-Arg-Val-Gly-Arg-Val-His-Arg-Leu-Leu-Arg-Lys), MAP (model amphiphatic peptide: Lys-Leu-Ala-Leu-Lys-Ala-Ser-Leu-Lys-Ala-Leu-Lys-Ala-Ala-Leu-Lys-Leu-Ala), k-FGF (Ala-Ala-Val-Ala-Leu-Leu-Pro-Ala-Val-Leu-Leu-Ala-Leu-Leu-Ala-Pro), Ku 70 (Val-Pro-Met-Leu-Lys-Pro-Met-Leu-Lys-Glu), prion (Met-Ala-Asn-Leu-Gly-Tyr-Trp-Leu-Leu-Ala-Leu-Phe-Val-Thr-Met-Trp-Thr-Asp-Val-Gly-Leu-Cys-Lys-Lys-Arg-Pro-Lys-Pro), pVEC (Leu-Leu-Ile-Ile-Leu-Arg-Arg-Arg-Ile-Arg-Lys-Gln-Ala-His-Ala-His-Ser-Lys), pep-1 (Lys-Glu-Thr-Trp-Trp-Glu-Thr-Trp-Trp-Thr-Glu-Trp-Ser-Gln-Pro-Lys-Lys-Lys-Arg-Lys-Val), SynB1 (Arg-Gly-Gly-Arg-Leu-Ser-Tyr-Ser-Arg-Arg-Arg-Phe-Ser-Thr-Ser-Thr-Gly-Arg), pep-7 (Ser-Asp-Leu-Trp-Glu-Met-Met-Met-Val-Ser-Leu-Ala-Cys-Gln-Tyr), and HN-1 (Thr-Ser-Pro-Leu-Leu-Ile-His-Asn-Gly-Gln-Lys-Leu).

Next, as described in step 2), hydrophobic peptides that have a single hydrophobic region at their N-terminus and form a helix structure are selected from the secreted proteins identified in step 1). All of the secreted proteins having a signal sequence-like domain that were identified in step 1) are subjected to hydropathy analysis to determine whether they contain a single hydrophobic region (H-region) at their N-terminus, followed by computer-aided genomic and proteomic information analysis to determine whether they form a helix structure. The hydrophobic region usually forms a helix structure, which imparts the protein with membrane-translocating activity.

In one embodiment of the present invention, the SOSUI system, a classification and secondary prediction system for membrane proteins, can be used for the computer-aided genomic and proteomic information analysis. The SOSUI system is a useful tool for secondary structure prediction of membrane proteins from a protein sequence and is freely available on-line (see http://bp.nuap.nagoya-u.ac.jp/sosui). Since the SOSUI system currently requires peptides to be analyzed to have at least about 20 amino acids in length, additional amino acids derived from the N-terminal domain of enhanced green fluorescent protein are added to the end of the signal sequence-like domain. When the SOSUI system is used, 220 hydrophobic peptides that have a single hydrophobic region at their N-terminus and form a transmembrane helix structure are selected.

Finally, the hydrophobic peptides selected in step 2) are optimized and minimized so that they are suitable for use as an MTD peptide, as described in step 3). To optimize the selected hydrophobic peptides, empirical modifications are made to them as follows: i) hydrophilic, nonpolar, and positively or negatively charged amino acids are removed from the selected hydrophobic peptides; and ii) various combinations of five hydrophobic amino acids, i.e., alanine (Ala or A), valine (Val or V), proline (Pro or P), leucine (Leu or L), and isoleucine (Ile or I) are created empirically. Said hydrophobic amino acids are known to provide flexibility to the H-region which may allow the hydrophobic region of the signal sequences to form a hairpin-like loop, resulting in destabilized conformational change of the phospholipid bilayer. Such local and transient formation of the non-bilayer lipid structure induced by contact with the flexible H-region leads to topological transformation.

To minimize the length of the hydrophobic peptides optimized as described above, the following principles are employed for determining which amino acids should be eliminated therefrom: i) non-hydrophobic amino acids at the left or the right side of the hydrophobic peptides are eliminated to minimize the size, while maintaining the hydrophobic region thereof; ii) non-hydrophobic amino acids at the middle or the right side of the hydrophobic peptides are replaced with a hydrophobic amino acid, proline (Pro or P) to provide flexibility or a bending potential; and iii) the number of repeated hydrophobic amino acids is minimized to reduce the size of the hydrophobic peptides. Thus, the MTD candidates are characterized as modified peptides exhibiting hydrophobicity and flexibility, having relatively short amino acids (7 to 17) in length, and forming a helix structure.

According to the method of the present invention described above, novel MTD peptides comprising an amino acid sequence of SEQ ID NO: 173 and capable of facilitating the traverse of biologically active molecules across the cell membrane can be identified.

For understanding of the invention it is described that one of the novel MTDs, JO-98 (SEQ ID NO: 98) can be identified by the following process:
i) immunoglobulin domain containing 4 from *Homo sapiens* is selected from the PubMed Entrez Protein Database as a secreted protein having a signal sequence-like domain;
ii) through a hydropathy analysis, it is confirmed that said protein contains a single N-terminal hydrophobic region;
iii) the hydrophobic region (H-region) is selected from the full-length signal sequences of said protein, which has the amino acid sequence represented by MGILLGLLLLGHLTVDTY
   GRPIL (23 amino acids in length);
iv) through a structural analysis utilizing the SOSUI system, it is confirmed that the selected hydrophobic region forms a helix structure;
v) non-hydrophobic amino acids (methionine: M; glycine: G) at the left side of the hydrophobic region are deleted to make the first amino acid of the hydrophobic region a hydrophobic amino acid, hydrophobic peptide that has an amino acid sequence represented by ILLGLLLLGHLTVDTYGRPIL (21 amino acids in length);
vi) basic (histidine: H; arginine: R) and hydrophilic (threonine: T; aspartic acid: D; tyrosine: Y) amino acids are removed from the hydrophobic region to reduce the peptide length to no more than 15 amino acids, resulting in a peptide that has an amino acid sequence represented by ILLGLLLLGLVGPIL (15 amino acids in length);
vii) the structural analysis shows that the modified peptide, as described above, forms a helix structure;
viii) in order to impart flexibility to the peptide, nonpolar amino acids (glycine: G) at the middle and the right sides of the peptide are replaced with a hydrophobic amino acid, proline (P), respectively, resulting in a peptide has an amino acid sequence represented by ILLPLLLLGLVPPIL (15 amino acids in length); and
ix) some of the repeated hydrophobic amino acids at the far right side of the peptide sequence are deleted to shorten the peptide length to 10 or less amino acids.

Therefore, the finally optimized peptide as a macromolecule transduction domain has an amino acid sequence represented by AVIPILAVP (9 amino acids in length; SEQ ID NO: 98) and forms a helix structure, which has been designated as "JO-173." The last amino acid, proline, of the JO-173 peptide is a flexible site for interaction with a biologically active molecule to form a recombinant protein.

A further aspect of the present invention relates to a method of genetically engineering a biologically active molecule to have cell permeability by using the MTD peptides.

The therapeutic use of biologically active molecules is often hindered by their low cell permeability. Although biologically active molecules have been shown to be taken up by cells via an endocytic process, the molecules that enter the cell in this manner are usually trapped in endocytic vesicles and degraded in lysosomes.

The MTD of the present invention provides efficient transport of biologically active molecules having a high molecular weight across the cell membrane, whereas other membrane transport peptides previously tested have not been shown to transport molecules of a size greater than approximately 25 amino acids. The MTD of the present invention can be attached to a peptide or polypeptide using conventional methods in the art to enhance the peptide's or polypeptide's membrane permeability. The MTD can be provided in the form of a kit, which includes the necessary components known to those skilled in the art to facilitate linkage of a peptide to a target polypeptide. A target protein attached to the MTD in this manner can then be delivered to the cell either *in vitro* or *in vivo* for intracellular import.

According to the method of the present invention, the polynucleotide described above can be inserted into a protein expression vector to produce a protein which can be imported from the exterior to the interior of a cell by the action of the MTD peptides described herein.

An expression vector is genetically engineered to incorporate a nucleic acid sequence encoding a MTD in an orientation either N-terminal and/or C-terminal to a nucleic acid sequence encoding a peptide, polypeptide, protein domain, or full-length protein of interest as a biologically active molecule, and in the correct reading frame so that a recombinant protein consisting of the macromolecule transduction domain and the target biologically active molecule may be expressed. Expression vectors may be chosen from among those readily available for use in prokaryotic or eukaryotic expression systems.

As used herein, an MTD is a macromolecule transduction domain of the present invention, which directs cellular transport of a target protein from the exterior to the interior of a cell. In another embodiment of the present invention, the MTD may comprise an alternate sequence which mediates the import of a peptide or polypeptide through the cell membrane to the interior of a cell.

A target protein is a protein which normally exhibits less than optimal permeability through the cell membrane, but which, when attached either N-terminal and/or C-terminal to an MTD of the present invention, is transported from the exterior to the interior of the cell.

In another embodiment of the present invention, a cleavage site is located between the MTD and the target polypeptide, protein domain, or full-length protein. This site may alternatively be a factor X site or another site that is known to those skilled in the art to relate to the cleavage of the recombinant protein to physically remove the MTD from the subject peptide or polypeptide.

The method of the present invention provides a means for producing proteins having cell permeability for introduction into the interior of the cell, where their actions help to further elucidate cellular control and biosynthesis mechanisms. This method also provides a means for introducing intracellular proteins into cells to produce targeted cellular changes, such as the inhibition of apoptosis by the introduction of Bcl-2. Cell cycle control, for example, can be altered by the introduction of a functional p53 protein product into those cells that have become tumorigenic due to an abnormal p53 protein.

As used herein, the term "biologically active molecule" includes any molecule which, if imported into a cell, is capable of exhibiting a biological effect. Since very large proteins having molecular weights ranging from about 100,000 to about 1 million are exported by cells (e.g., antibodies, fibrinogen, and macroglobulin), very large proteins can be imported into cells by the method of the present invention. Therefore, proteins having sizes ranging from a few amino acids to about a thousand amino acids can be used. A preferable size range for proteins is from a few amino acids to about 250 amino acids. For any molecule, the size can be up to a molecular weight of about 1 million, specifically up to a molecular weight of about 25,000, and more specifically up to a molecular weight of about 3,000. In addition, only those molecules which can be attached to an MTD peptide as a signal peptide, either directly or indirectly, are within the scope of the present invention.

Examples of biologically active molecules include, but are not limited to, proteins, polypeptides, and peptides, which include functional domains of biologically active molecules, such as growth factors, enzymes, transcription factors, toxins, antigenic peptides (for vaccines), antibodies, and antibody fragments. Additional examples of biologically active molecules include nucleic acids, such as plasmids, coding nucleic acid sequences, mRNAs and antisense RNA molecules, carbohydrates, lipids, and glycolipids. Further examples of biologically active molecules include therapeutic agents, in particular those with low cell membrane permeability. Some examples of these therapeutic agents include cancer drugs, such as Daunorubicin, and toxic chemicals which, because of the lower dosage that can be used when administered by this method, can now be more safely administered. Yet another example of a biologically active molecule is an antigenic peptide. Antigenic peptides can be administered to provide immunological protection when imported by cells involved in the immune response. Other examples include immunosuppressive peptides (e.g., peptides that block autoreactive T cells, which are known in the art). Numerous other examples will be apparent to the skilled artisan.

Representative examples of biologically active molecules suitable for the present invention may include enzymes, hormones, transport proteins, immunoglobulin or antibodies, structural proteins, motor function proteins, receptors, signaling proteins and storage proteins in terms of their function; and membrane or transmembrane proteins, internal proteins, external or secret proteins, virus proteins, native proteins, glycoproteins, cleaved proteins, proteins with disulfide bonds, protein complexes, chemically modified proteins and prions in terms of their location and roles.

Standard recombinant nucleic acid methods can be used to express a genetically engineered recombinant protein. In one embodiment of the present invention, a nucleic acid sequence encoding the macromolecule transduction domain is cloned into a nucleic acid expression vector, e.g., with appropriate signal and processing sequences and regulatory sequences for transcription and translation. In another embodiment, the protein can be synthesized using automated organic synthetic methods. Synthetic methods for producing proteins are described, for example, in Methods in Enzymology, Volume 289: Solid-Phase Peptide Synthesis by Gregg B. Fields (Editor), Sidney P. Colowick, Melvin I. Simon (Editor), Academic Press (1997).

In order to obtain high level expression of a cloned gene or nucleic acid, such as a cDNA encoding a MTD peptide, a MTD sequence is typically subcloned into an expression vector that contains a strong promoter for directing transcription, a transcription/translation terminator, and, in the case of a nucleic acid encoding a protein, a ribosome binding site for translational initiation. Suitable bacterial promoters are well known in the art and are described, e.g., in Sambrook & Russell, Molecular Cloning: A Laboratory Manual, 3d Edition, Cold Spring Harbor Laboratory, N.Y. (2001) and Ausubel et al., Current Protocols in Molecular Biology, Greene Publishing Associates and Wiley Interscience, N. Y. (1989). Bacterial expression systems for expressing the MTD peptide of the present invention are available in, e.g., *E. coli, Bacillus* sp., and *Salmonella* (Palva et al., Gene 22: 229-235 (1983); Mosbach et al., Nature 302: 543-545 (1983)). Kits for such expression systems are commercially available. Eukaryotic expression systems for mammalian cells, yeast, and insect cells are well known in the art and are also commercially available. In another embodiment of the present invention, the eukaryotic expression vector is an adenoviral vector, an adeno-associated vector, or a retroviral vector.

The expression vector for expressing the cell permeable recombinant protein can include regulatory sequences, including for example, a promoter, operably attached to a sequence encoding the macromolecule transduction domain. Non-limiting examples of inducible promoters that can be used include steroid-hormone responsive promoters (e.g., ecdysone-responsive, estrogen-responsive, and glutacorticoid-responsive promoters), the tetracycline "Tet-On" and "Tet-Off" systems, and metal-responsive promoters. The construct can be introduced into an appropriate host cell, e.g., a bacterial cell, yeast cell, insect cell, or tissue culture cell. The construct can also be introduced into embryonic stem cells to generate a transgenic organism as a model subject. Large numbers of suitable vectors and promoters are known to those skilled in the art and are commercially available for generating the recombinant constructs of the present invention.

Known methods can be used to construct vectors containing the polynucleotide of the present invention and appropriate transcriptional/translational control signals. These methods include *in vitro* recombinant DNA techniques, synthetic techniques, and *in vivo* recombination/genetic recombination. See, for example, the techniques described in Sambrook & Russell, Molecular Cloning: A Laboratory Manual, 3d Edition, Cold Spring Harbor Laboratory, N. Y. (2001) and Ausubel et al., Current Protocols in Molecular Biology Greene Publishing Associates and Wiley Interscience, N.Y. (1989).

Host cells suitable for producing a cell permeable recombinant protein include bacterial cells and eukaryotic cells (e.g., fungal, insect, plant, and mammalian cells). Host cells can be disrupted by any convenient method, including freeze-thaw cycling, sonication, mechanical disruption, or the use of cell lysing agents. Scopes, Protein Purification: Principles and Practice, New York: Springer-Verlag (1994) describes a number of general methods for purifying recombinant (and non-recombinant) proteins. The methods can include, e.g., ion-exchange chromatography, size-exclusion chromatography, affinity chromatography, selective precipitation, dialysis, and hydrophobic interaction chromatography. These methods can be adapted to devise a purification strategy for the cell permeable recombinant protein. If the cell permeable recombinant protein includes a purification handle, such as an epitope tag or a metal chelating sequence, affinity chromatography can be used to purify the protein greatly.

The amount of protein produced can be evaluated by detecting the macromolecule transduction domain directly (e.g., using Western analysis) or indirectly (e.g., by assaying materials from the cells for specific DNA binding activity, such as by electrophoretic mobility shift assay). Proteins can be detected prior to purification, during any stage of purification, or after purification. In some implementations, purification or complete purification may not be necessary.

In a specific embodiment of the present invention, the expression vector is pEGFP-C1 (Clontech, Mountain View, CA), which comprises a polynucleotide encoding a recombinant protein including an enhanced green fluorescent protein (EGFP). The insertion of a polynucleotide encoding an MTD according to the present invention into vector pEGFP-C1, 5' and/or 3' to the EGFP gene of the vector enables the expression of a recombinant protein incorporating both the MTD and the EGFP. The MTD, connected N-terminal and/or C-terminal to the EGFP, carries the EGFP protein to the interior of the cell.

The genetically engineered recombinant proteins prepared by the method of the present invention are cell permeable proteins and can be used as protein-based vaccines, particularly where killed or attenuated whole organism vaccines are impractical. The cell permeable proteins prepared by the method of the present invention can also be used for the treatment of diseases, particularly cancer. Cell permeable proteins can be delivered to the interior of the cell, eliminating the need to transfect or transform the cell with a recombinant vector. The cell permeable proteins of the present invention can be used *in vitro* to investigate protein function or can be used to maintain cells in a desired state.

The MTD of the present invention can be used to deliver peptides, polypeptides, protein domains, or proteins to the interior of a target cell either *in vitro* or *in vivo.* The MTD can be attached to the target protein through a peptide linkage formed by the expression of the recombinant protein from a recombinant DNA or RNA molecule or can be attached to the target protein by means of a linker covalently attached to the MTD. A covalent linkage can be used to attach the MTD of the present invention to a non-protein molecule, such as a polynucleotide, for import into the cell.

Another aspect of the present invention relates to a pharmaceutical composition comprising the cell permeable recombinant proteins of the present invention, where the MTD peptide is operably attached to a biologically active molecule.

The cell permeable recombinant protein produced by the method of the present invention may be administered *in vitro* by any of the standard methods known to those skilled in the art, such as by addition of the recombinant protein to culture medium. Furthermore, it will be appreciated by those skilled in the art that recombinant proteins produced by this method may be delivered *in vivo* by standard methods utilized for protein/drug delivery, including parenteral administration, intravenous administration, topical administration, aerosol administration or inhalation, oral administration (particularly when provided in an encapsulated form), or by rectal or vaginal administration (particularly when provided in a suppository form).

Examples of administration include, but are not limited to, parenteral administration, e.g., by intravenous injection including regional perfusion, inhalation of an aerosol, subcutaneous or intramuscular injection, topical administration, such as to skin wounds and lesions, direct transfection into, e.g., bone marrow cells prepared for transplantation and subsequent transplantation into the subject, and direct transfection into an organ that is subsequently transplanted into the subject. Parenteral administration, e.g., regional perfusion, if used, is generally characterized by injection. Injectables can be prepared in conventional forms, such as liquid solutions, suspensions, or emulsions. A slow release or sustained release system can also be used, allowing the maintenance of a constant level of dosage.

Further administration methods include oral administration, particularly when the complex is encapsulated, or rectal administration, particularly when the complex is in a suppository form. A pharmaceutically acceptable carrier includes any material that is not biologically or otherwise undesirable, i.e., the material may be administered to an individual along with the selected complex without causing any undesirable biological effects or interacting in a deleterious manner with any of the other components of the pharmaceutical composition in which it is administered.

Depending on the intended mode of administration (e.g., including, but not limited to, intravenous, parenteral, transcutaneous, subcutaneous, intramuscular, intracranial, intraorbital, ophthalmic, intraventricular, intracapsular, intraspinal, intracisternal, intraperitoneal, intranasal, intrarectal, intravaginal, aerosol, or oral), the pharmaceutical compositions may be in the form of solid, semi-solid or liquid dosage forms, such as, for example, tablets, suppositories, pills, capsules, powders, liquids, suspensions, lotions, creams, gels, or the like, preferably in unit dosage form suitable for single administration of a precise dosage. The pharmaceutical compositions of the present invention will include, as noted above, an effective amount of the cell permeable recombinant protein in combination with a pharmaceutically acceptable carrier and, in addition, may include other medicinal agents, pharmaceutical agents, carriers, adjuvants, diluents, etc.

For solid compositions, conventional nontoxic solid carriers include, for example, pharmaceutical grades of mannitol, lactose, starch, magnesium stearate, sodium saccharin, talc, cellulose, glucose, sucrose, magnesium carbonate, and the like. Liquid pharmaceutically administrable compositions can, for example, be prepared by dissolving or dispersing an active compound as described herein, and optional pharmaceutical adjuvants in an excipient, such as, for example, water, saline, aqueous dextrose, glycerol, ethanol, and the like, to thereby form a solution or suspension. If desired, the pharmaceutical composition to be administered may also contain minor amounts of nontoxic auxiliary substances, such as wetting or emulsifying agents, pH buffering agents, and the like. Actual methods of preparing such dosage forms are known, or will be apparent, to those skilled in the art.

Administration of a cell permeable recombinant protein produced by the method of the present invention may be performed for a period of time ranging from 10 minutes to 72 hours , particularly when the administration is carried out by the addition of recombinant protein to culture media for *in vitro* use. For *in vivo* or *in vitro* use, the effective administration time for a cell permeable recombinant protein produced by the method of the present invention may be readily determined by those skilled in the relevant art.

For either *in vitro* or *in vivo* use, the cell permeable recombinant protein can be administered at any effective concentration. An effective concentration is that amount that results in the importation of the biologically active molecule into the cell. Such a concentration - culture medium concentration (*in vitro*) or blood serum concentration (*in vivo*) - will typically be from about 0.1 nM to about 500 µM. Optimal concentrations for the specific recombinant protein and/or the specific target cell can be readily determined according to the teachings herein. Thus, the *in vivo* dosage for the recombinant protein is the concentration which will cause the blood serum concentration of the recombinant protein to be about 0.1 nM to about 500 µM, more specifically, about 0.5 nM to about 100 µM. The amount of the recombinant protein administered will, of course, depend upon the subject being treated, the subject's age and weight, the manner of administration, and the judgment of the skilled administrator. The exact amount of the complex will further depend upon the general condition of the subject, the severity of the disease/condition being treated by the administration, and the particular complex chosen. However, the appropriate amount can be determined by one of ordinary skill in the art, using routine optimization, given the teachings provided herein.

Further, the cell permeable recombinant proteins produced by the method of the present invention can be used for vaccine administration (Linqnan et al., Expert. Rev. Vaccines 6: 741-746, 2007; O' Haqan et al., Methods 40: 10-19, 2009).

Vaccines using cell permeable recombinant proteins provide an advantage over the typical peptide vaccines. The immune system recognition of an antigen depends upon appropriate antigen processing. Previously, entire proteins or protein domains could not be delivered to the interior of the cell for processing to occur. As a consequence, peptides representing antigenic epitopes had to be identified prior to the delivery to the cell of small peptides representing those epitopes. The method of the present invention allows whole proteins or protein domains to be imported into the cell, where antigenic processing can occur. This provides multiple antigenic epitopes in one administration, and eliminates the need for experimental identification of specific epitopes for vaccine development.

Typically, such vaccines are prepared for injection into a human or mammalian subject. Injectable vaccines can be prepared as liquid solutions or suspensions. Solid forms can be prepared which are suitable for solution in, or suspension in, a liquid, prior to injection. The preparation may also be emulsified. The active immunogenic ingredient is often mixed with a pharmaceutically acceptable carrier which is compatible with the active ingredient. Suitable carriers include, but are not limited to, water, dextrose, glycerol, saline, ethanol, and combinations thereof. The vaccine may contain additional agents, such as wetting or emulsifying agents, pH buffering agents, or adjuvants which enhance the effectiveness of the vaccine.

The vaccine may be conventionally administered parenterally. Either subcutaneous or intramuscular injection is appropriate. Other modes of administration may include oral administration, nasal administration, rectal administration, and vaginal administration, which may involve combining the peptide immunogen with pharmaceutically acceptable carriers, such as mannitol, lactose, starch, magnesium stearate, sodium saccharine, cellulose, magnesium carbonate, or other carriers. Compositions for oral administration may form solutions, suspensions, tablets, pills, capsules, sustained release formulations, or powders. A protein-based vaccine of the present invention can be administered by an enteric-coated capsule for release of the polypeptide into the lumen of the intestine.

The cell permeable recombinant proteins of the present invention may be formulated into the vaccine as neutral or salt forms. Pharmaceutically acceptable salts include the acid addition salts (formed with the free amino groups of the polypeptide) and which are formed with inorganic acids, such as, for example, hydrochloric or phosphoric acids, or such organic acids as acetic, mandelic, oxalic, and tartaric acids. Salts formed with the free carboxyl groups of the polypeptide may also be derived from inorganic bases such as, for example, sodium, potassium ammonium, calcium, or ferric hydroxides, and such organic bases as isopropylamine, trimethylamine, 2-ethylamino ethanol, and histidine.

The vaccine is administered in a manner compatible with the dosage formulation, and in such an amount as will be therapeutically effective and immunogenic. The quantity to be administered depends on the subject to be treated, taking into account, for example, the capacity of the individual's immune system to synthesize antibodies and the degree of protection desired. The precise amounts of the active ingredient (peptide immunogen) to be administered depend on the judgment of the practitioner. Suitable dosage ranges generally require several hundred micrograms of active ingredient per vaccination. Also variable are the regimes for initial administration and booster vaccinations, which should be determined according to the judgment of the practitioner. The dosage of the vaccine will depend on the route of administration and will vary according to the size of the host.

Furthermore, the cell permeable recombinant proteins of the present invention can be effectively used in drug delivery systems (Spencer and Verma, Proc. Natl. Acad. Sci. USA 104: 7594-7599, 2007; Choi et al., Nat. Med. 12: 574-579, 2006; Vives et al., J. Biol. Chem. 272: 16010-16017, 1997; Kumar et al., Nature 448: 39-43, 2007; Jo et al., Nat. Med. 11: 892-898, 2005).

The method of genetically engineering proteins with cell membrane permeability according to the present invention provides a means for delivering therapeutic protein products into a cell. Combination of the present invention with previously described methods of extracellular protein delivery provides a method of delivering proteins for import into a cell in a stabilized, functional form in a controlled-release fashion.

Polypeptides are produced using an appropriate expression vector and an expression system. Cell permeability is conferred upon the protein or polypeptide by the expression of a recombinant protein with the MTD located N-terminal and/or C-terminal to the expressed polypeptide. The less stable proteins are stabilized by methods known to those skilled in the art and described previously. Delivery to the extracellular environment is accomplished by providing the stabilized recombinant protein in an appropriate carrier, such as microsphere carriers. The protein of choice will dictate the appropriate vector and expression system, as well as the appropriate stabilization and delivery techniques. A person of ordinary skill in the art of drug delivery systems can choose the appropriate techniques from among those described.

The method of the present invention provides a means for producing cell permeable proteins for the treatment of cancer. Regulators of apoptosis and cell cycle control have been found to play a key role in oncogenesis, while gene therapy techniques (Balaggan et al., Gene Therapy 13(15): 1153-1165, 2006; Kuo et al., Proc. Natl. Acad. Sci. USA 10; 98(8): 4605-4610, 2001), using intratumoral injection of an adenoviral expression vector encoding the p53 gene, have shown promise for the control of some tumors. However, the delivery of specific protein products through the use of viral vectors has proven to be problematic. The MTDs and methods of the present invention provide a means for producing cell permeable proteins from among the cell cycle regulators and regulators of apoptosis, as well as other proteins identified to play a role in the development of the cancer state.

For example, in one embodiment of the present invention, the polynucleotide encoding the p53 gene can be inserted into a suitable vector, either 5' or 3' to the sequence of the MTD of the present invention. Under expression conditions appropriate for the vector of choice and known to those skilled in the art, a recombinant protein comprising an MTD and the p53 protein can be expressed. The attachment of the MTD to the p53 protein renders the p53 protein cell permeable, and protein can be administered to tumor cells to inhibit tumor development. Administration of cell permeable proteins can be accomplished in various ways, including, but not limited to, intratumoral injection, infusion, and intravenous administration. Bax and Bcl-x_{L} are other examples of proteins from among a wide variety of proteins that have been determined to affect cell cycle control and apoptosis and, therefore, be effective for cancer therapy. The method of the present invention provides a more efficient, less labor-intensive, and less costly method for the delivery of anti-oncogenic proteins to tumor cells.

The term "cell membrane" as used herein refers to a lipid-containing barrier which separates cells or groups of cells from the extracellular space. Cell membranes include, but are not limited to, plasma membranes, cell walls, intracellular organelle membranes, such as the mitochondrial membrane, nuclear membranes, and the like.

The term "biologically active molecule" as used herein refers to compounds or molecules that are capable of eliciting or modifying a biological response in a system. Non-limiting examples of biologically active molecules include antibodies (e.g., monoclonal, chimeric, humanized etc.), cholesterol, hormones, antivirals, peptides, proteins, chemotherapeutics, small molecules, vitamins, co-factors, nucleosides, nucleotides, oligonucleotides, enzymatic nucleic acids, antisense nucleic acids, triplex forming oligonucleotides, 2,5-A chimeras, siNA, siRNA, miRNA, RNAi inhibitors, dsRNA, allozymes, aptamers, decoys and analogs thereof. Biologically active molecules of the invention also include molecules capable of modulating the pharmacokinetics and/or pharmacodynamics of other biologically active molecules, for example, lipids and polymers such as polyamines, polyamides, polyethylene glycol and other polyethers. In certain embodiments, the term biologically active molecule is used interchangeably with the term "macromolecule".

The term "macromolecule" as used herein refers to large molecules (molecular weight greater than 1000 daltons) exemplified by, but not limited to, peptides, proteins, and oligonucleotides and polynucleotides of biological or synthetic origin.

The term "peptide" as used herein refers to a compound made up of a single chain of D- or L-amino acids or a mixture of D- and L-amino acids joined by peptide bonds. Preferably, peptides contain at least two amino acid residues and are less than about 50 amino acids in length.

The term "protein" as used herein refers to a compound that is composed of linearly arranged amino acids attached by peptide bonds, but in contrast to peptides, has a well-defined conformation. Proteins, as opposed to peptides, preferably contain chains of 50 or more amino acids.

The term "polypeptide" as used herein refers to a polymer of at least two amino acid residues and which contains one or more peptide bonds. Polypeptides encompass peptides and proteins, regardless of whether the polypeptide has a well-defined conformation.

The term "nucleic acid" as used herein refers to oligonucleotides or polynucleotides, such as deoxyribonucleic acid (DNA) and ribonucleic acid (RNA), as well as analogs of either RNA or DNA, for example made from nucleotide analogs any of which are in single or double stranded form.

Amino acid residues are herein referred to by their standard single-letter or three-letter notations or by their full names: A, Ala, alanine; C, Cys, cysteine; D, Asp, aspartic acid; E, Glu, glutamic acid; F, Phe, phenylalanine; G, Gly, glycine; H, His, histidine; I, Ile, isoleucine; K, Lys, lysine; L, Leu, leucine; M, Met, methionine; N, Asn, asparagine; P, Pro, proline; Q, Gln, glutamine; R, Arg, arginine; S, Ser, serine; T, Thr, threonine; V, Val, valine; W, Trp, tryptophan; X, Hyp, hydroxyproline; and Y, Tyr, tyrosine.

The term "macromolecule transduction domain (MTD)" as used herein refers to a peptide that facilitates the transport of macromolecules into a cell.

The term "isolated" polynucleotide as used herein refers to a polynucleotide that is substantially free of the sequences with which it is associated in nature. By substantially free is meant at least 50%, preferably at least 70%, more preferably at least 80%, and even more preferably at least 90% free of the materials with which it is associated in nature. As used herein, an "isolated" polynucleotide also refers to recombinant polynucleotides, which, by virtue of origin or manipulation: (1) are not associated with all or a portion of a polynucleotide with which it is associated in nature, (2) are linked to a polynucleotide other than that to which it is linked in nature, or (3) does not occur in nature.

The term "operatively attached" as used herein means that DNA fragments of a promoter, a MTD peptide or other genes are sufficiently connected to direct and regulate the expression of genes.

The term "homology" or "similarity" as used herein refers to the likeness or the percentage of identity between two sequences (e.g., polynucleotide or polypeptide sequences). Typically, a higher similarity in sequences means more similarity in the physical or chemical properties or biological activities.

Unless defined otherwise, all technical and scientific terms used herein have the same meaning as commonly understood by one of ordinary skill in the art to which this invention belongs. Although any method and material similar or equivalent to those described herein can also be used in the practice or testing of the present invention, specific methods and materials are now described. All publications mentioned herein are incorporated herein by reference in their entirety to disclose and describe the methods and/or materials in connection with which the publications are cited.

It must be noted that as used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### [Examples]

The following examples are presented to aid practitioners of the invention, provide experimental support for the invention, and to provide model protocols. Parts of the examples as indicated in the examples do not form part of the invention but are useful for the understanding of the invention. In no way are these examples to be understood to limit the invention.

### Example 1 - Identification of Novel Macromolecule Transduction Domain Peptides

In order to identify novel macromolecule transduction domain (MTD) candidates that can potentially penetrate the plasma membrane of live cells, secreted proteins having a signal sequence-like domain were selected from the PubMed Entrez Protein Database by using several key words, including "hydrophobic region of signal sequence," "signal sequence hydrophobic region," "signal sequence of secreted protein," "hydrophobic signal sequence," and "hydrophobic domain of secreted protein." As a result, more than 1,500 secreted proteins having a signal sequence-like domain were selected.

All of the selected secreted proteins having a signal sequence-like domain were subjected to hydropathy analysis to determine whether they contain a single hydrophobic region at their N-terminus. Subsequently, they were subjected to a computer-aided genomic and proteomic information analysis, i.e., the SOSUI system (as described on http://bp.nuap.nagoyau.ac.jp/sosui), to determine whether they form a helix structure. Since the SOSUI system currently requires peptides to be analyzed to have at least about 20 amino acids in length, additional amino acids derived from the N-terminal domain of enhanced green fluorescent protein were added to the end of the signal sequence-liked domain. When the SOSUI system is used, 220 peptide sequences having a single hydrophobic region at their N-terminus and forming a transmembrane helix structure were selected.

To optimize the selected peptide sequences as a signal sequence, empirical modifications were made to them as follows: first, hydrophilic, nonpolar, and positively or negatively charged amino acids were removed from the selected peptide sequences and, then, various combinations of five hydrophobic amino acids (i.e., alanine: A, valine: V, proline: P, leucine: L, isoleucine: I) were created empirically.

After that, non-hydrophobic amino acids at the left or right side of the peptide sequences were eliminated to minimize the size while maintaining their hydrophobic region, followed by adding or replacing non-hydrophobic amino acids at the middle or right side of the peptide sequences with proline, to thereby provide flexibility to the peptide. Finally, the number of repeated hydrophobic amino acids was minimized to reduce the length of the peptide sequence. The resulting peptides as MTD candidates exhibited hydrophobicity and flexibility, have relatively short amino acids (7 to 17 in length), and form a helix structure.

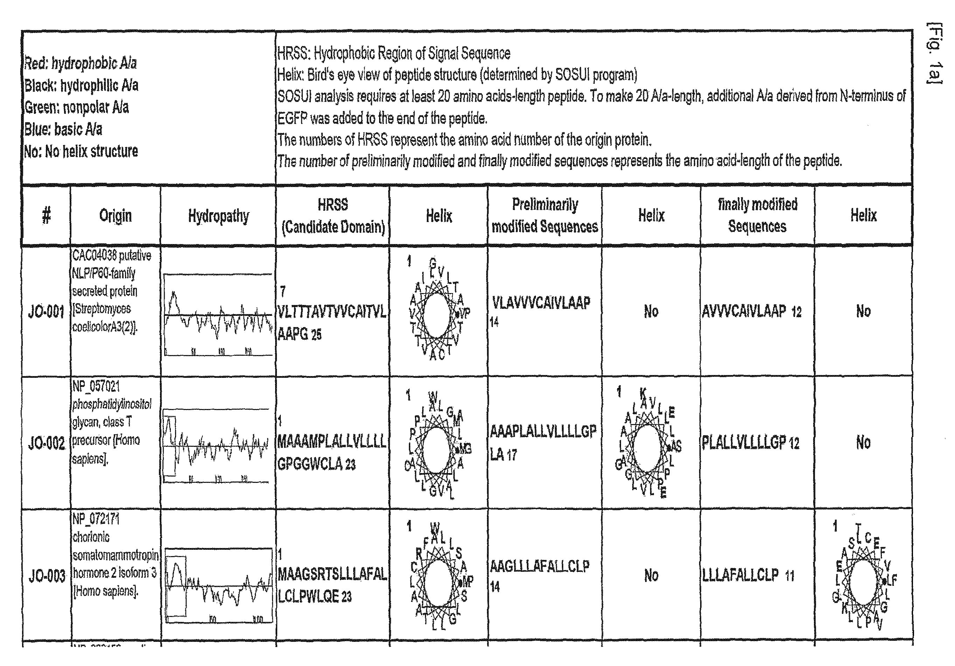

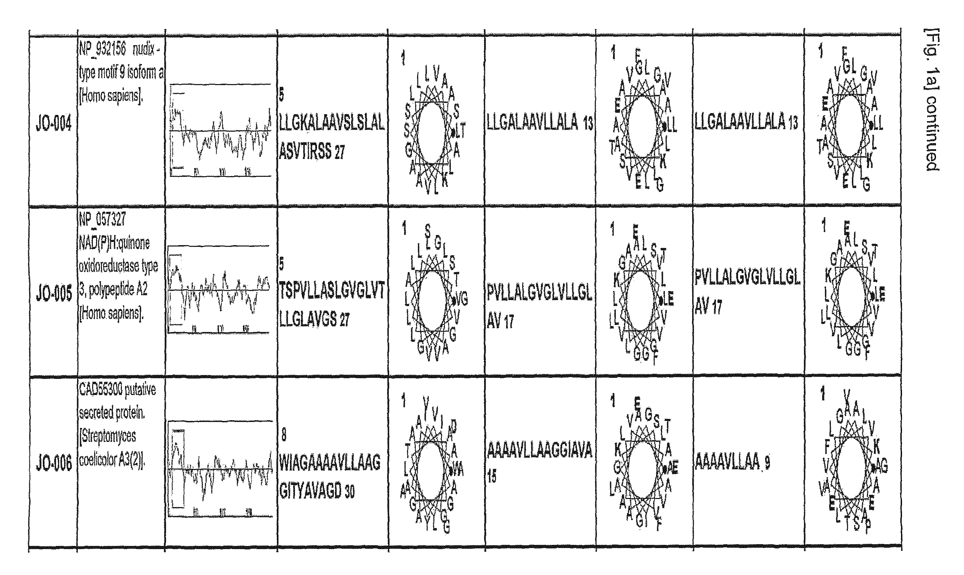

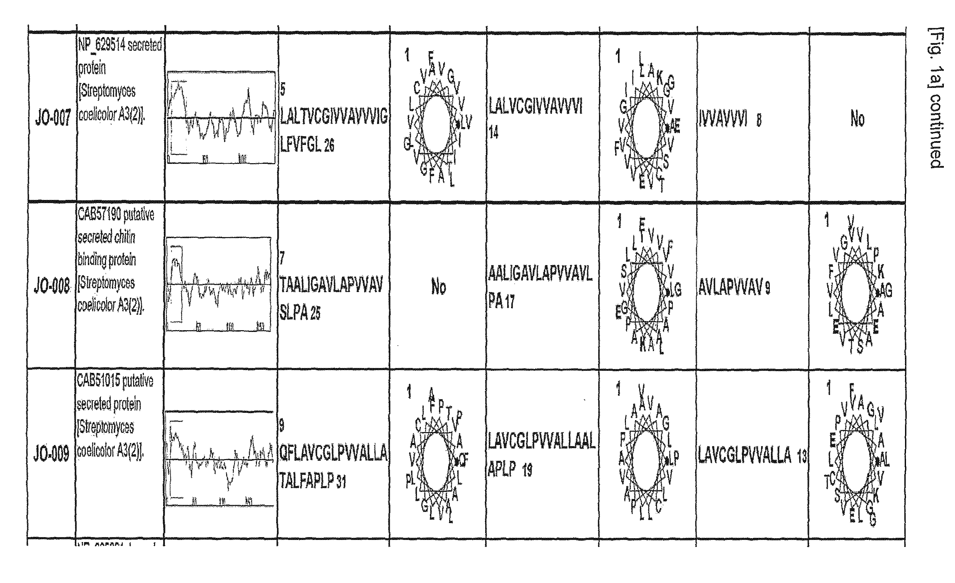

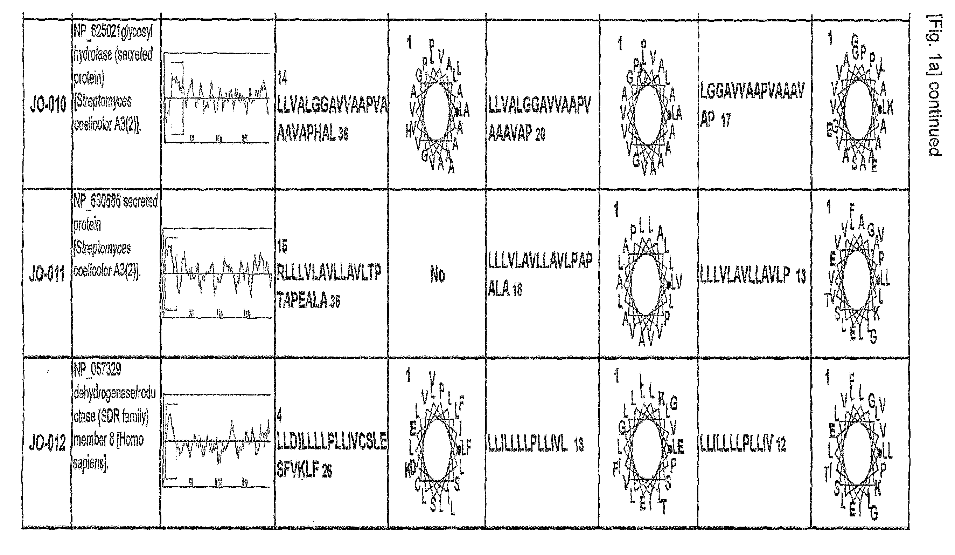

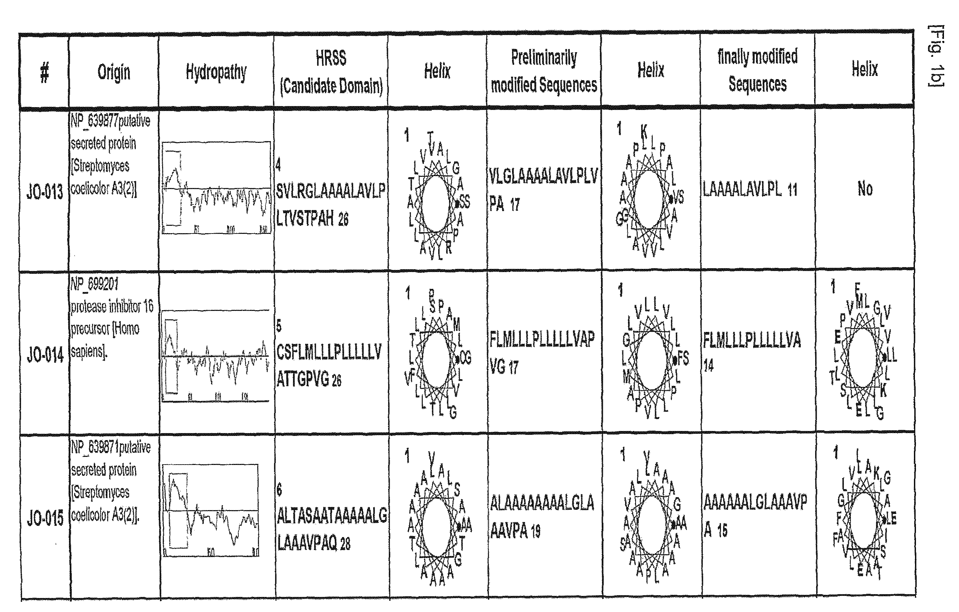

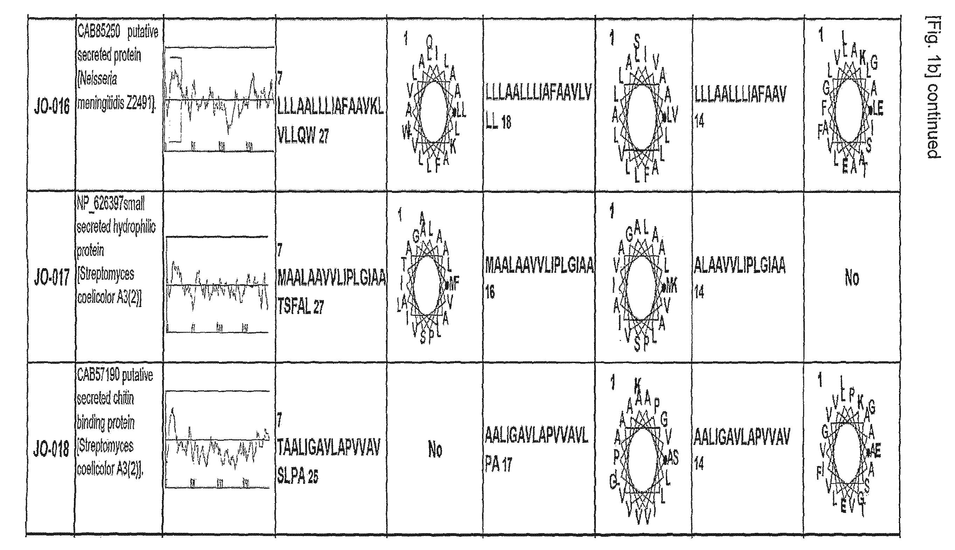

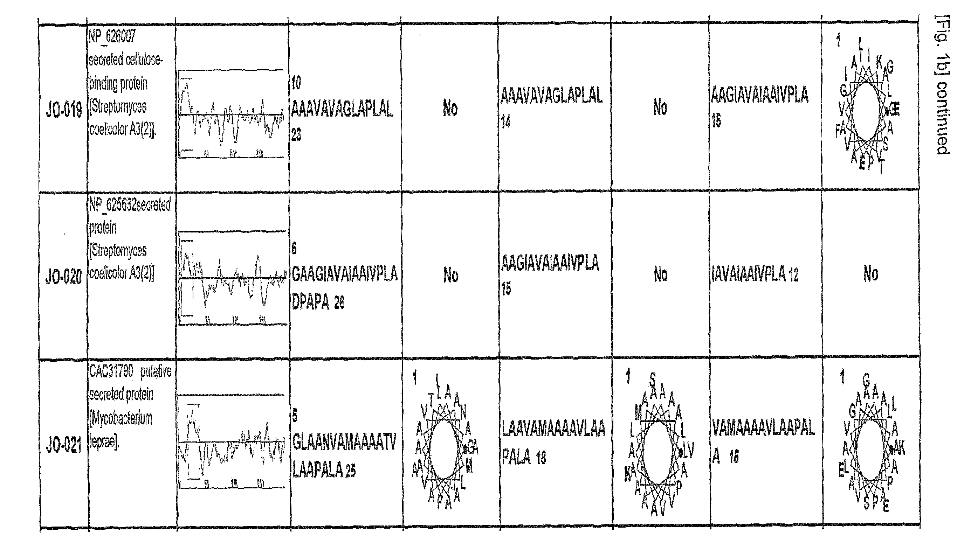

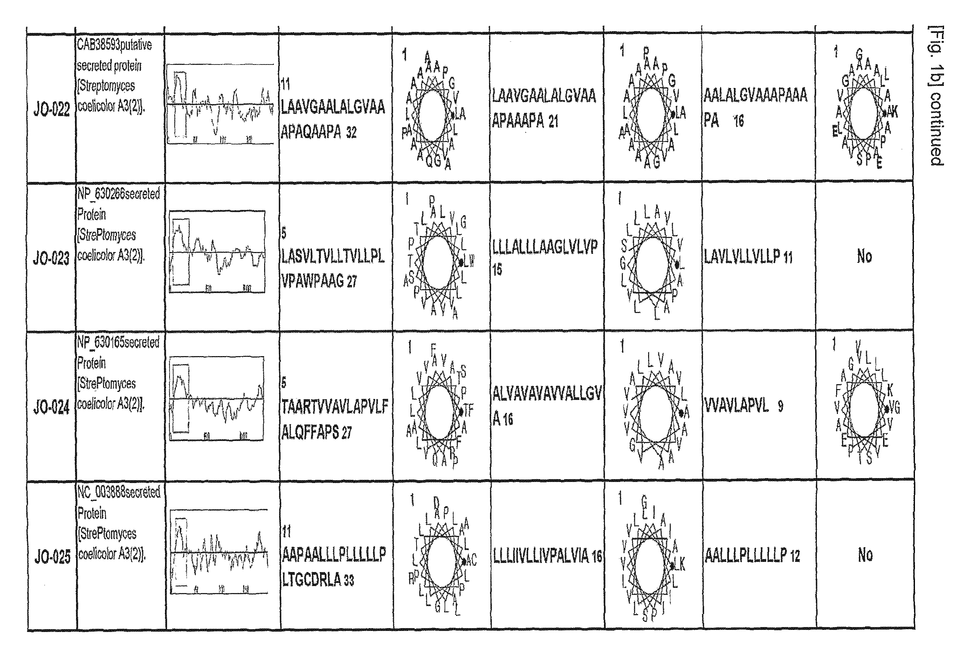

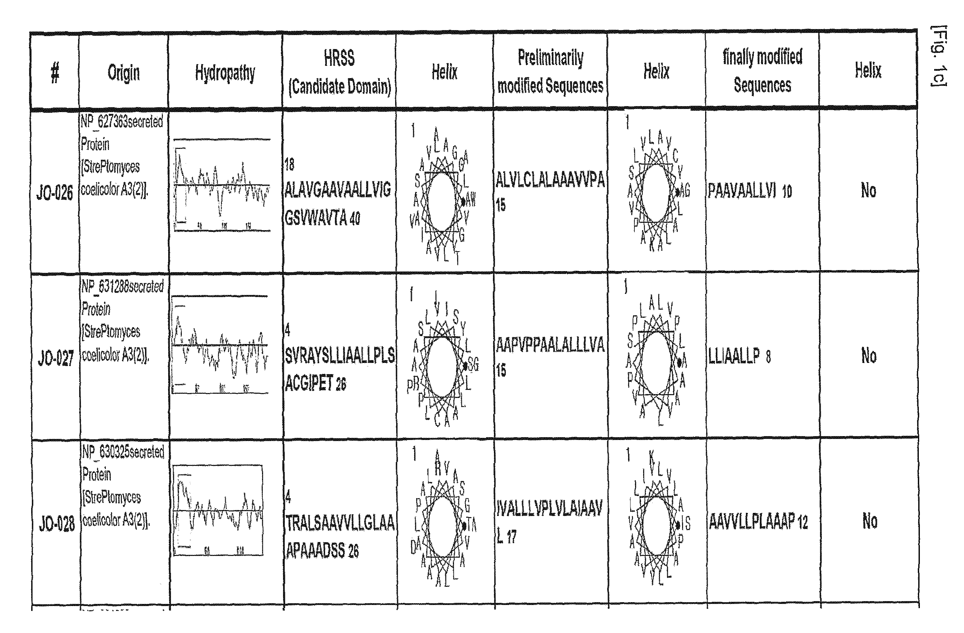

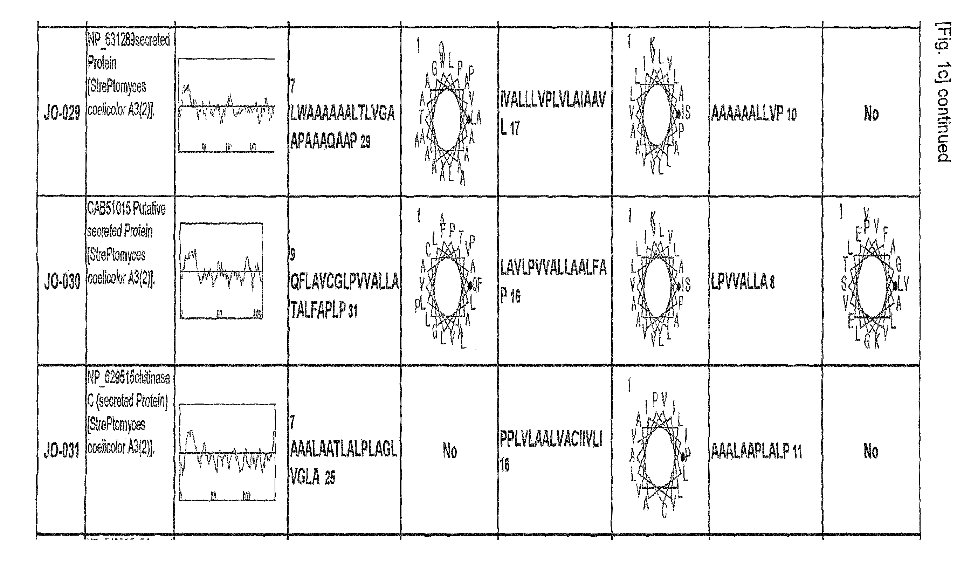

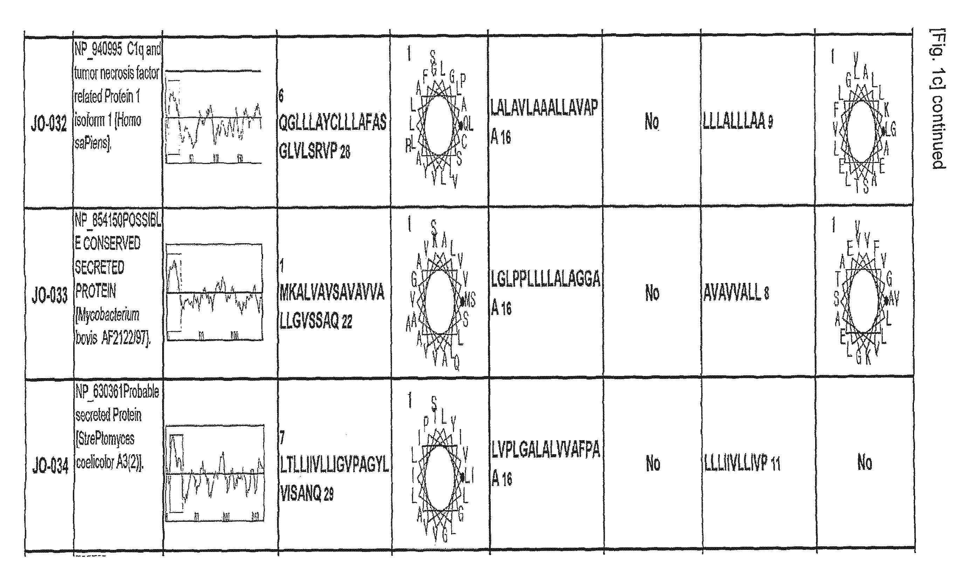

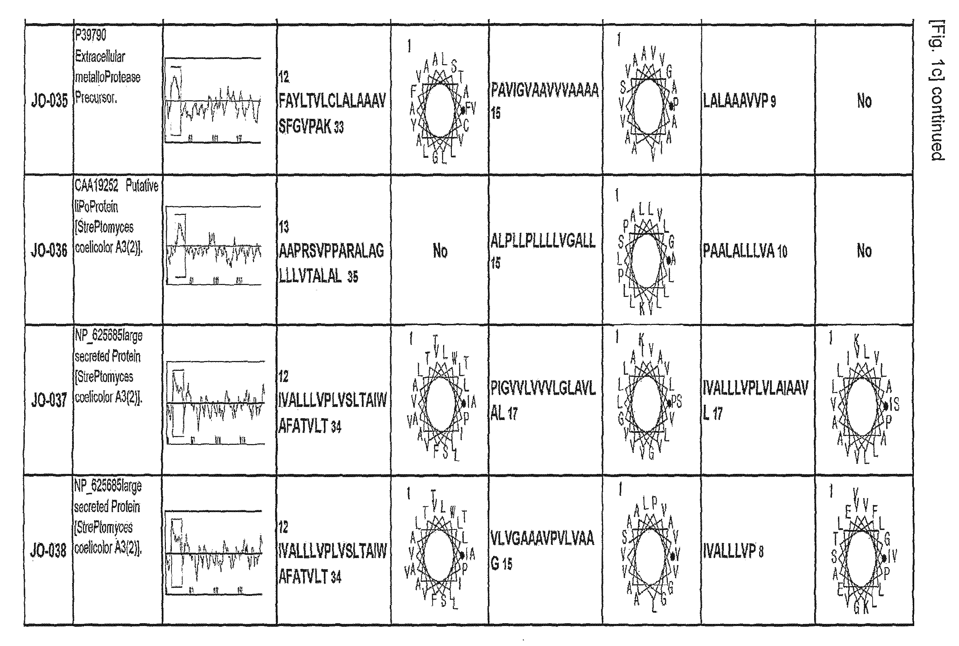

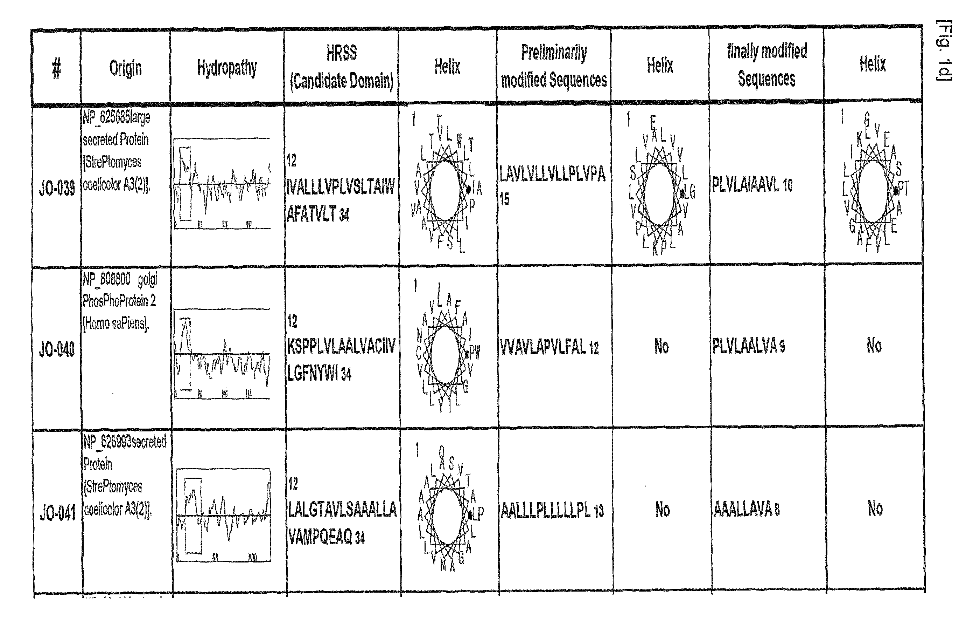

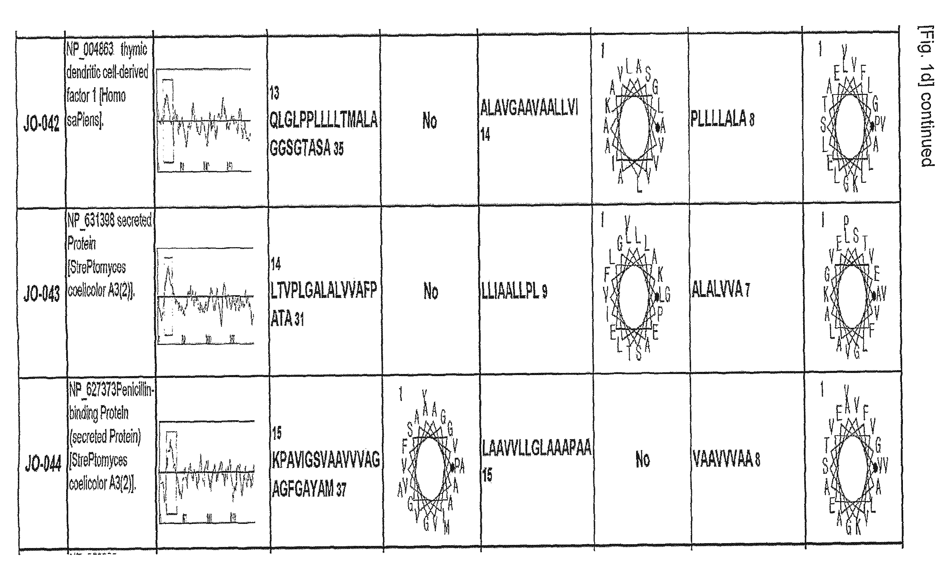

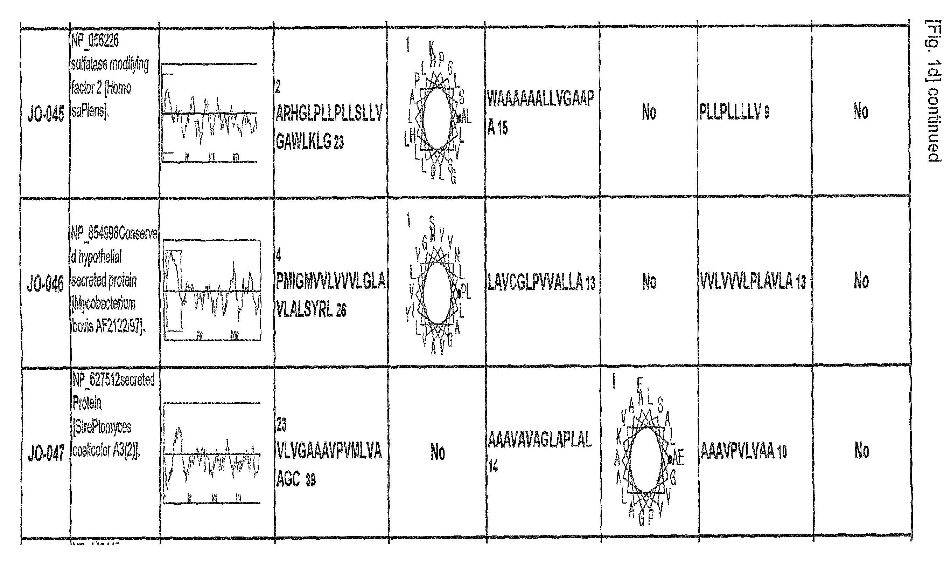

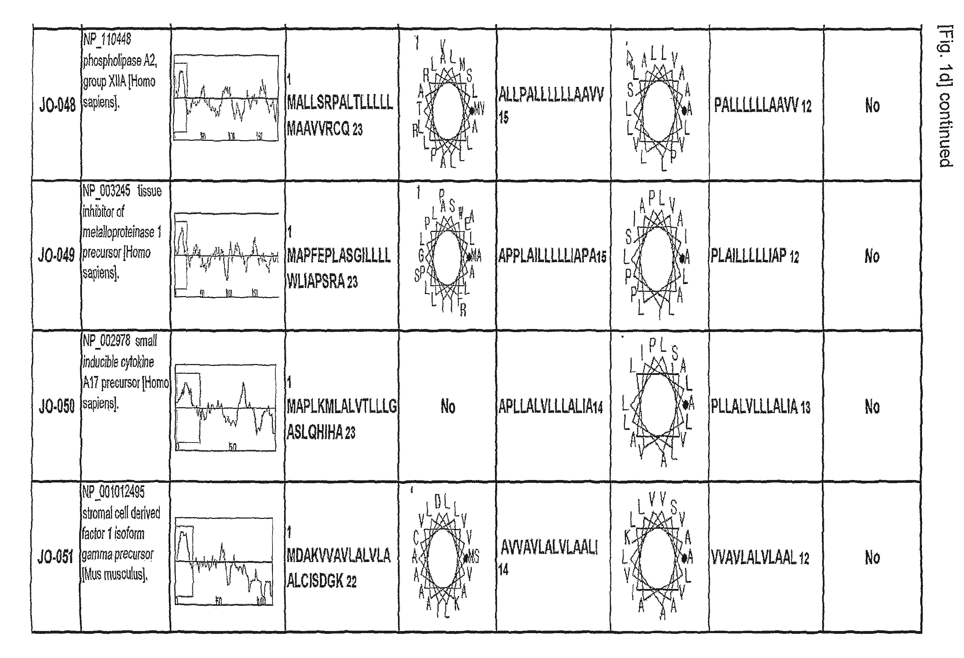

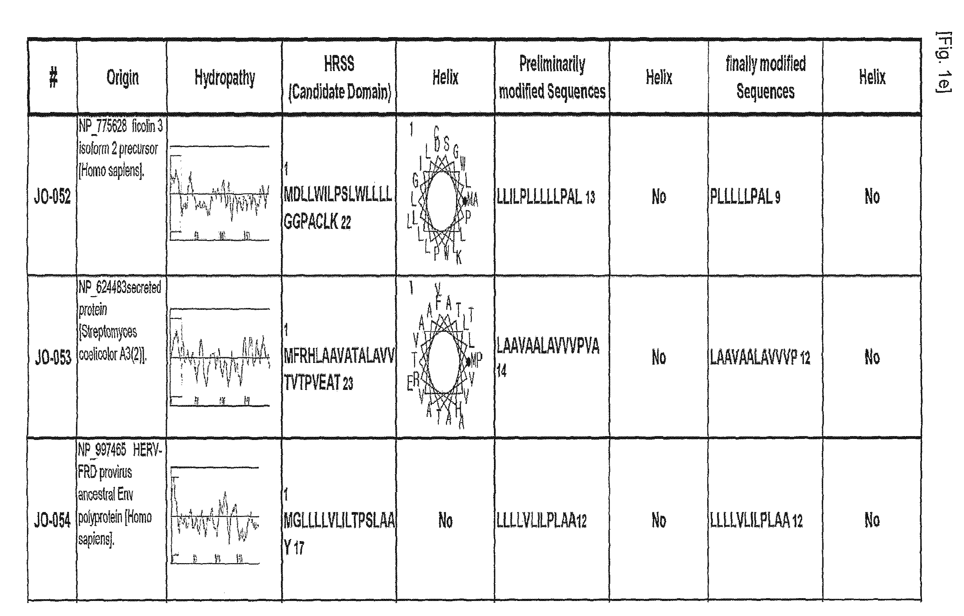

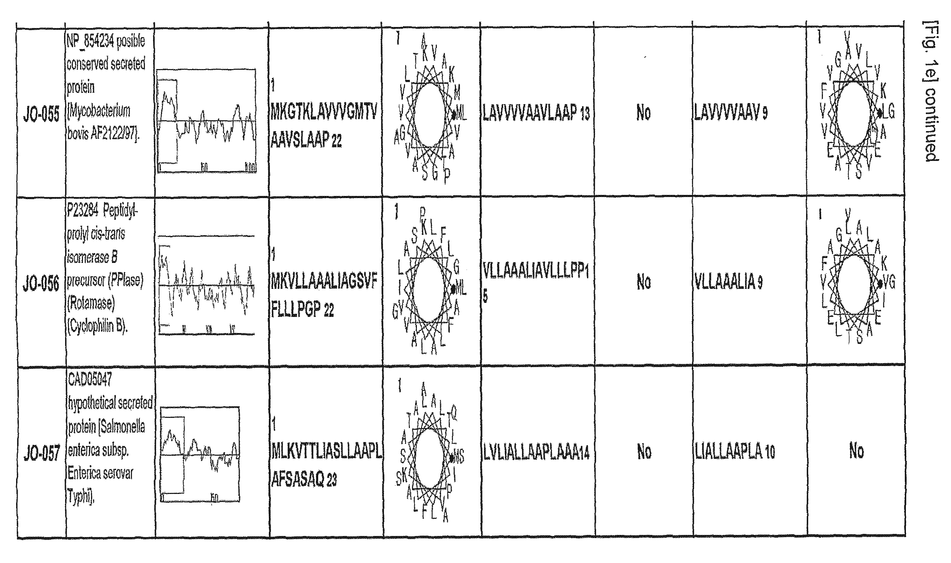

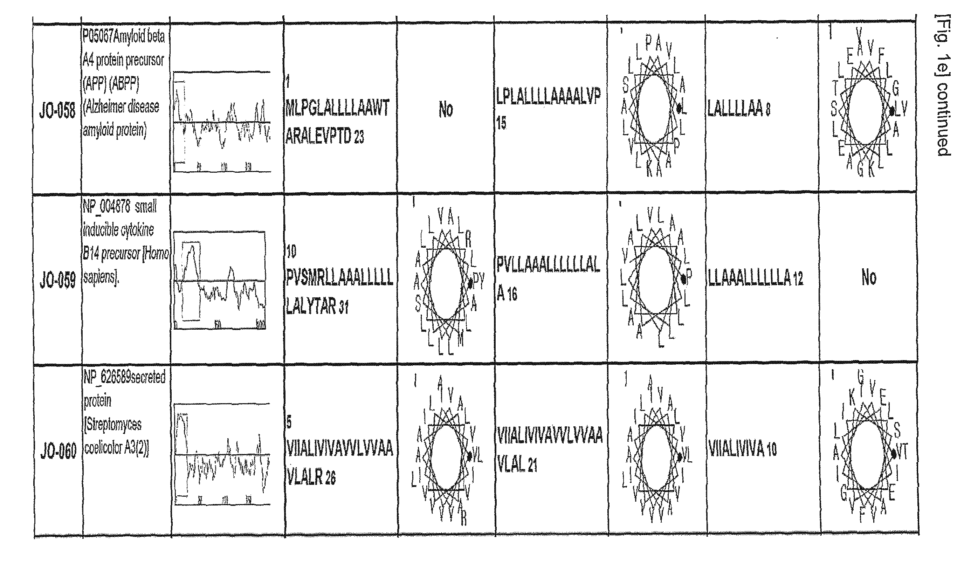

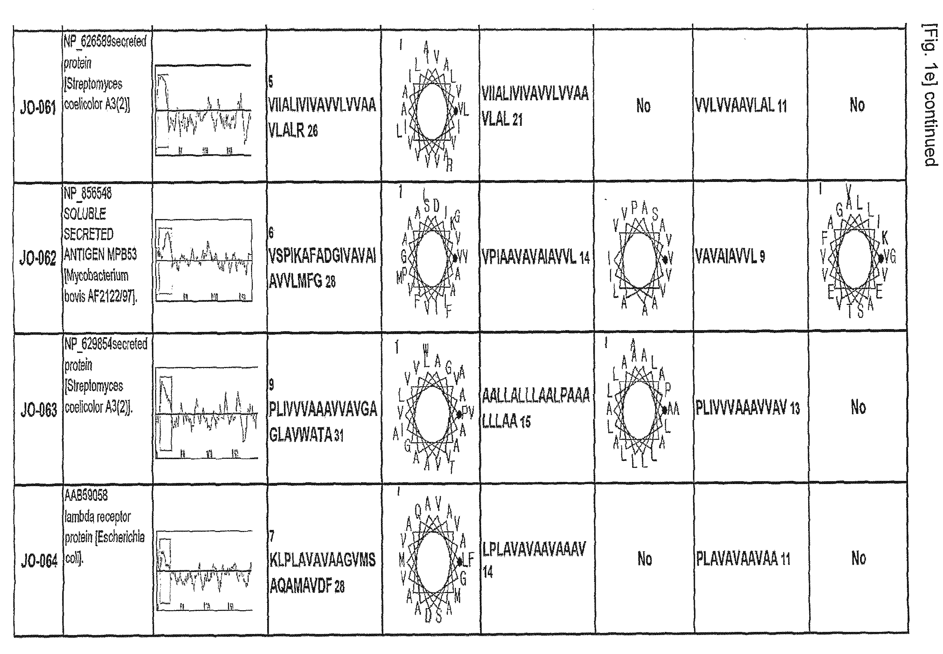

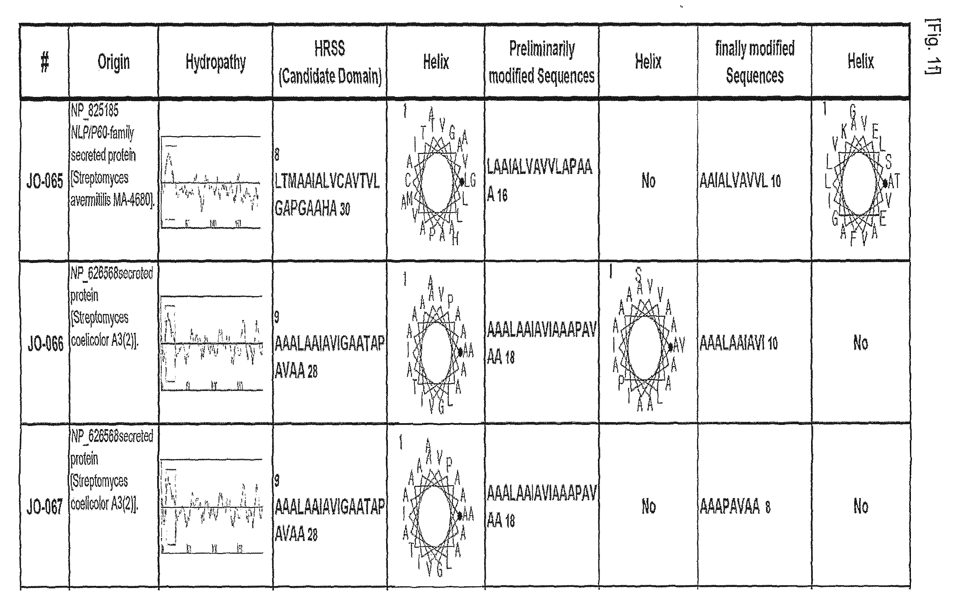

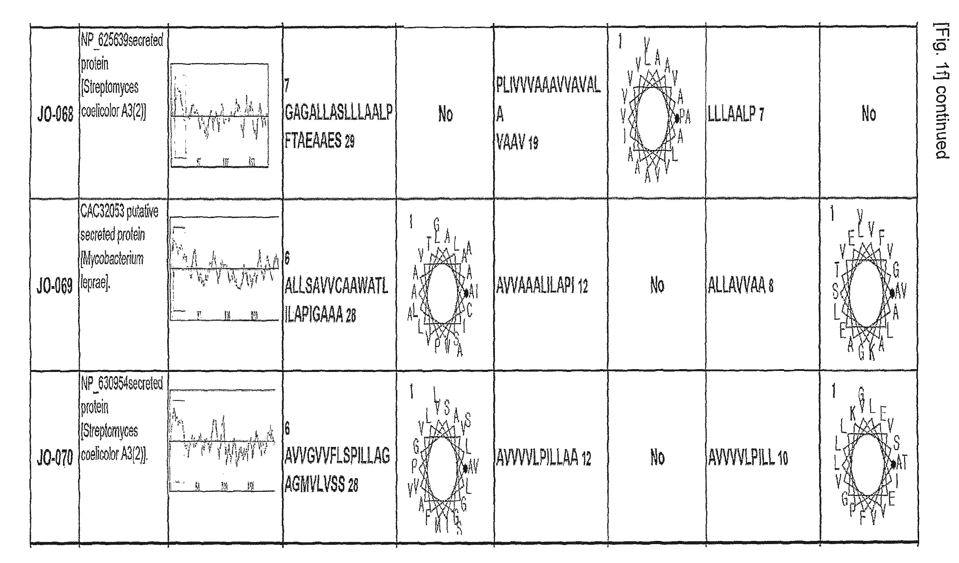

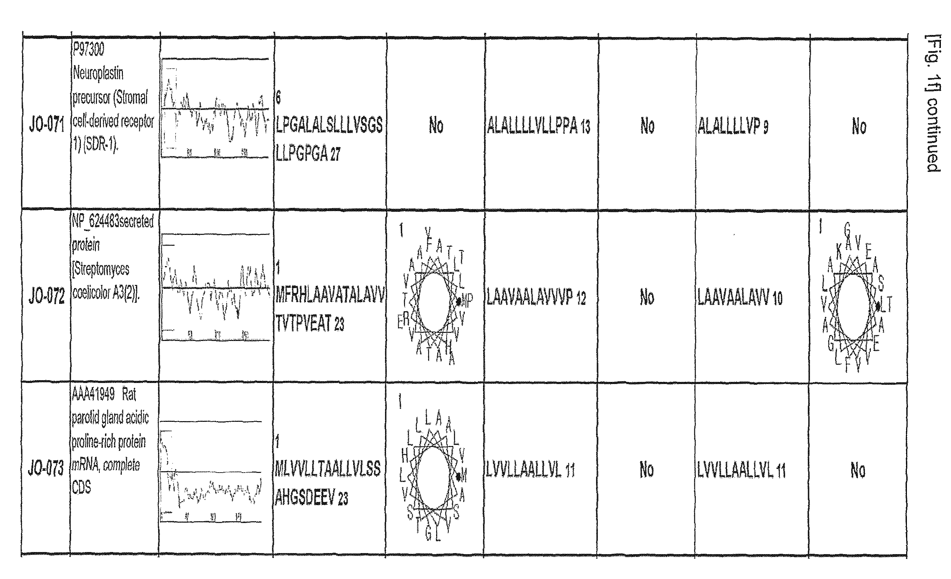

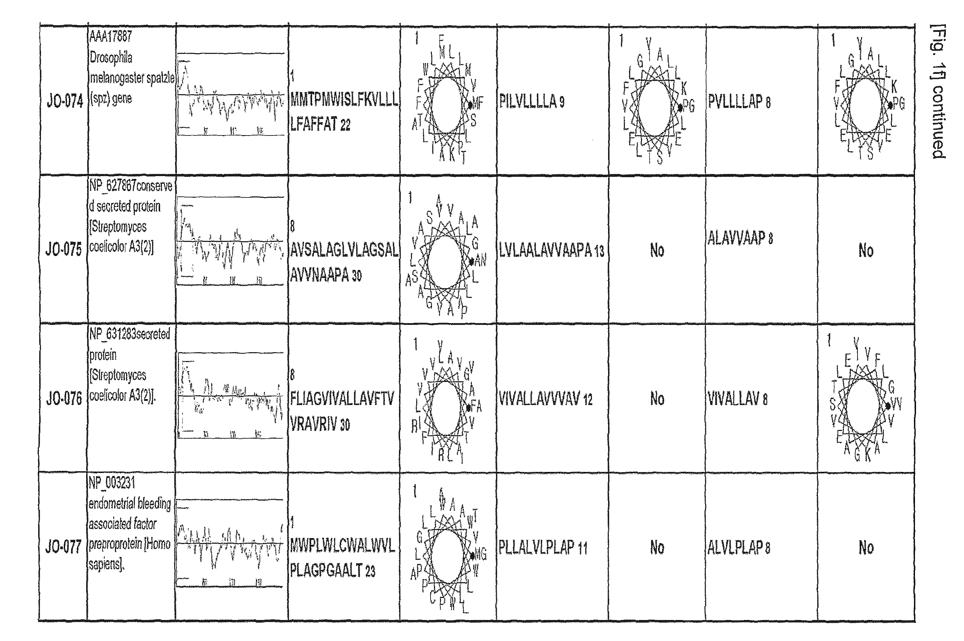

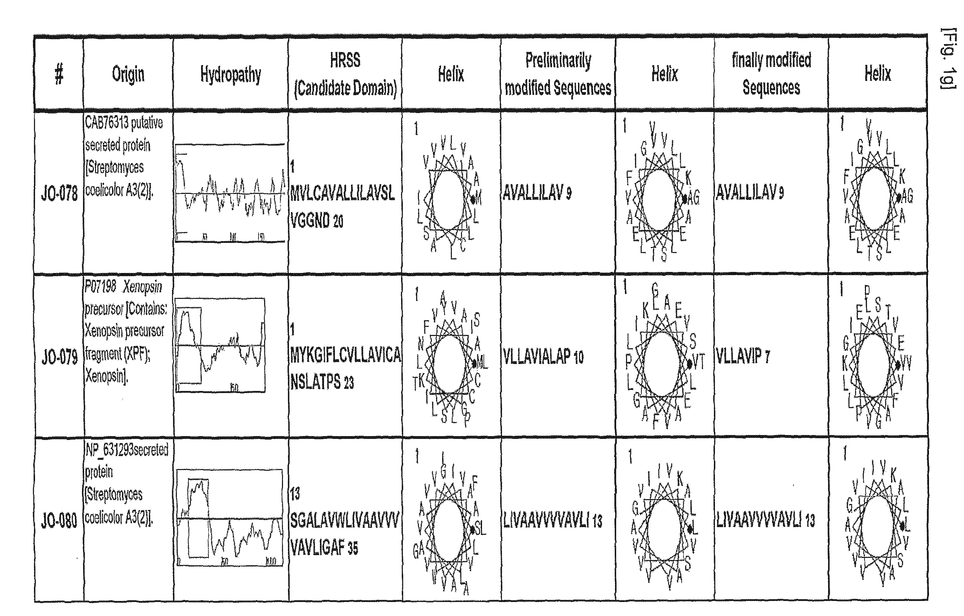

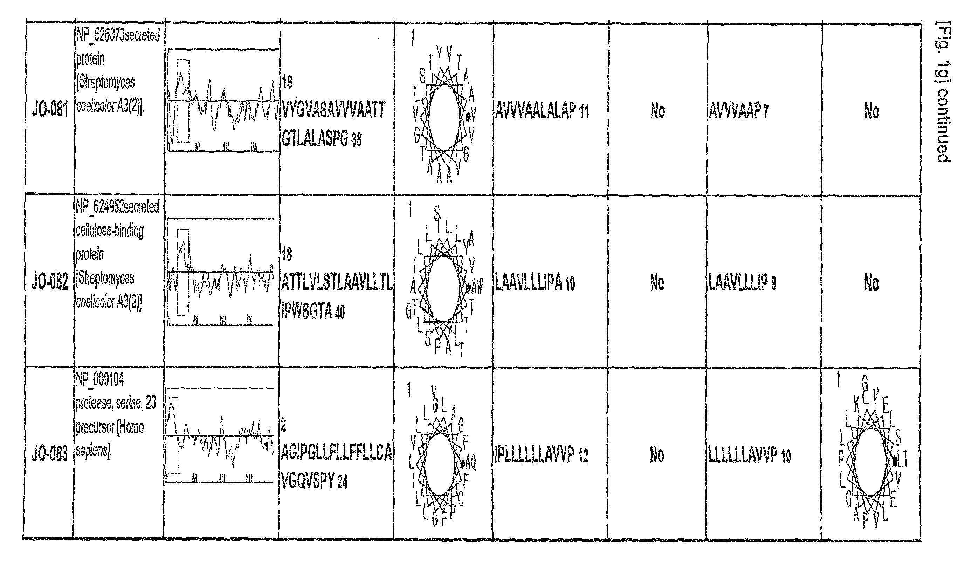

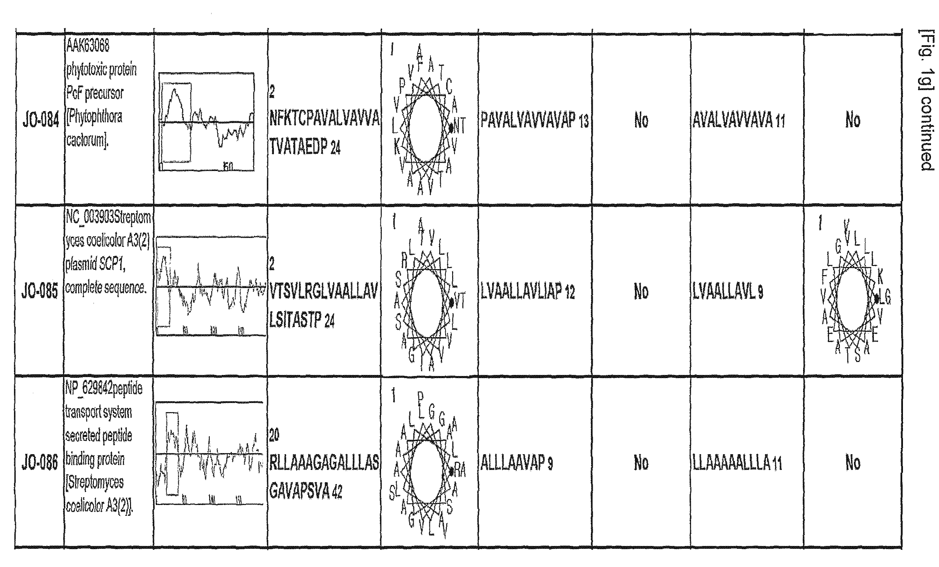

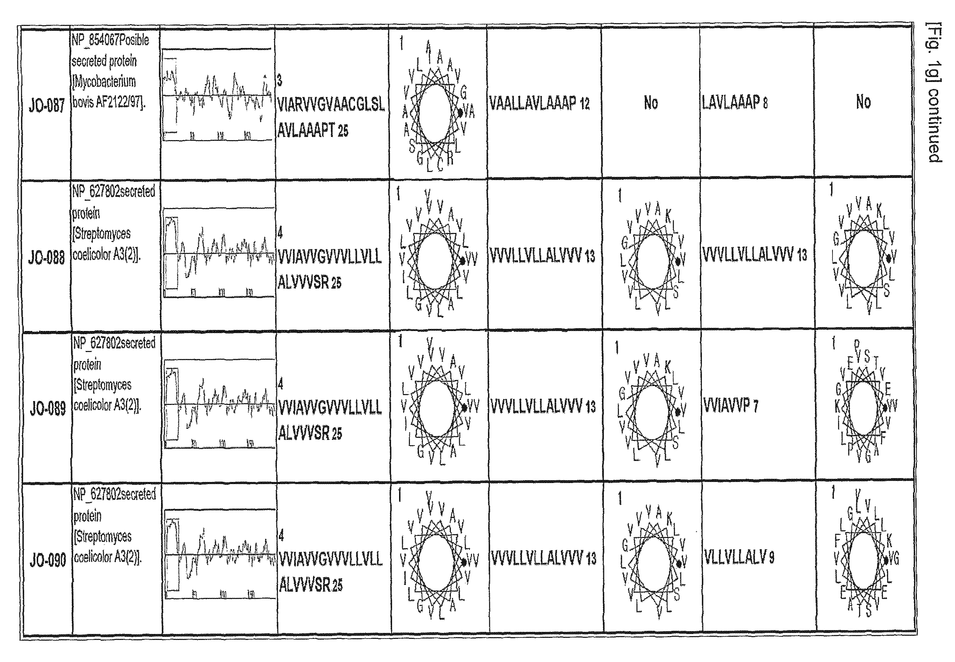

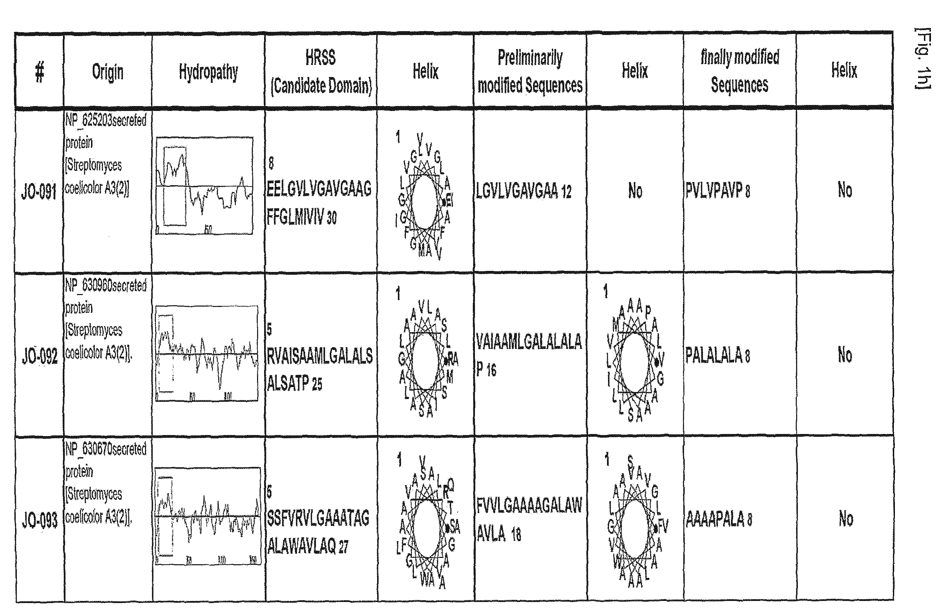

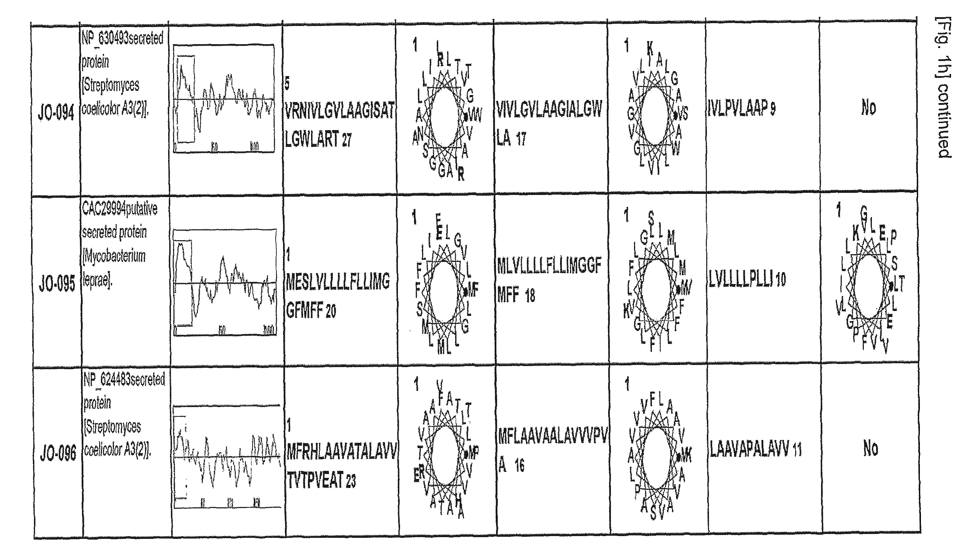

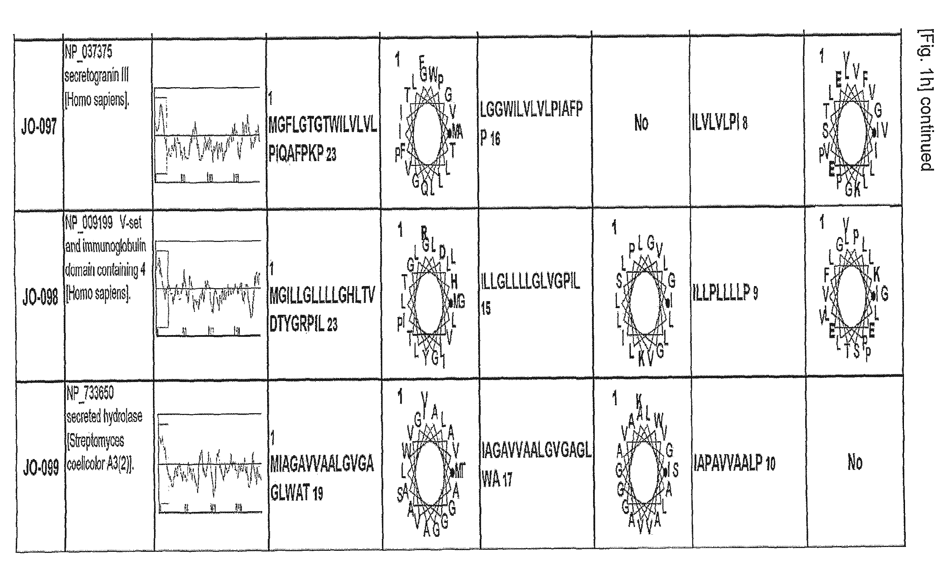

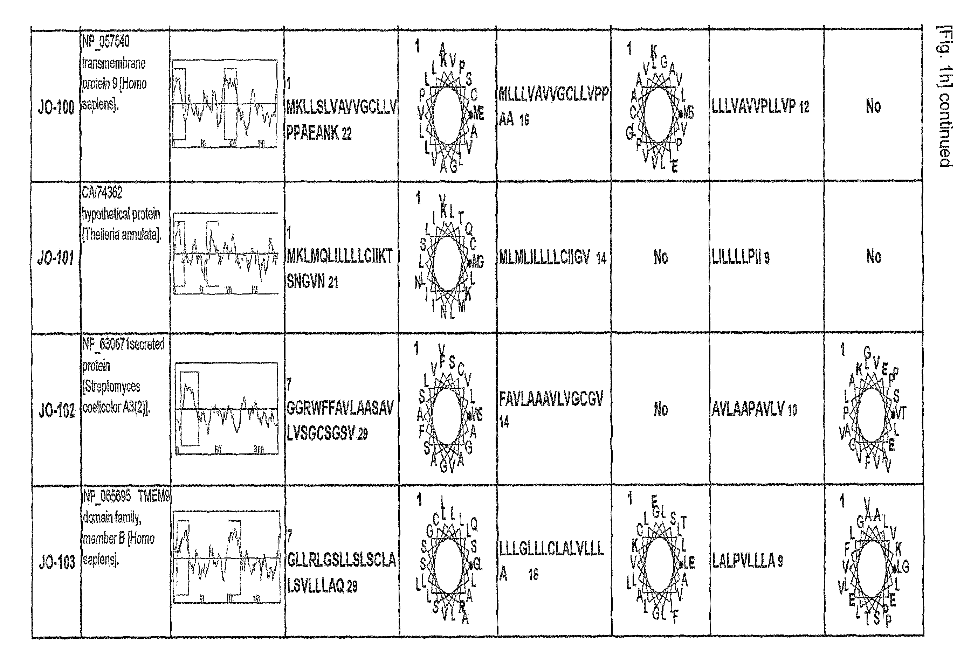

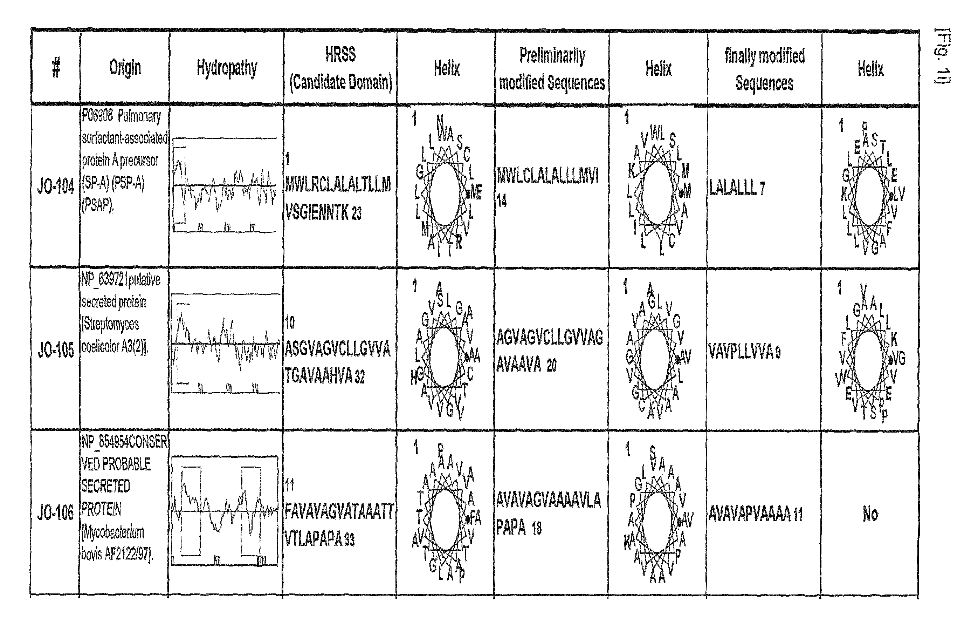

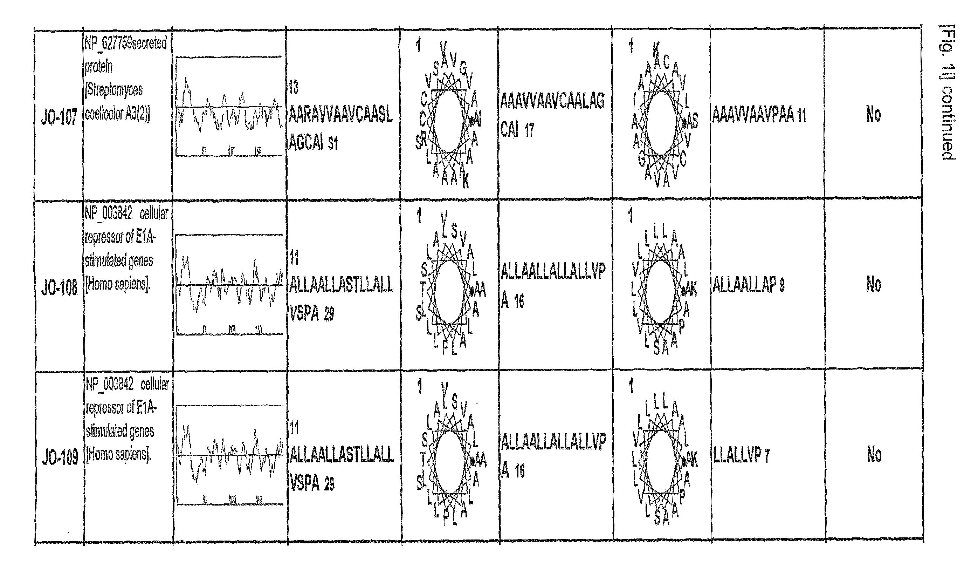

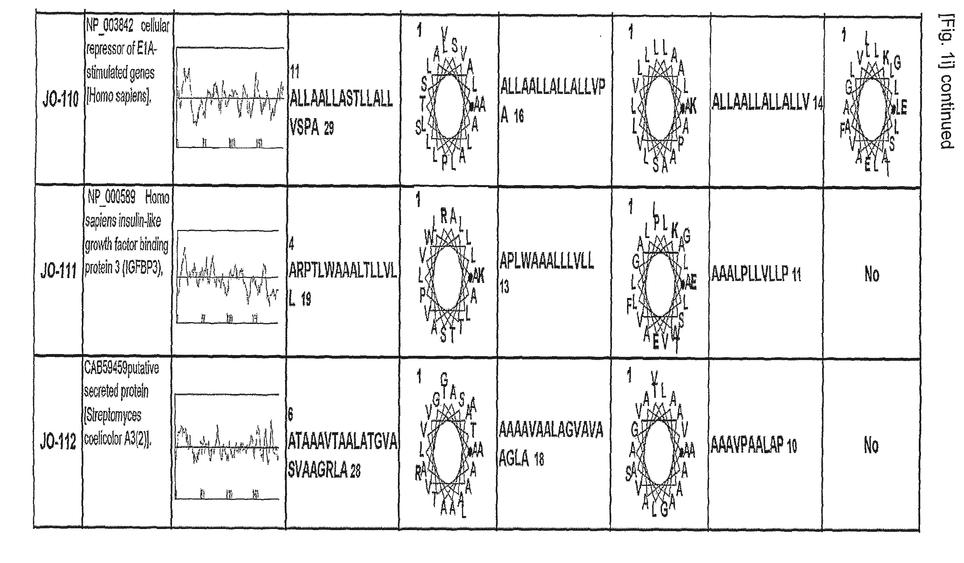

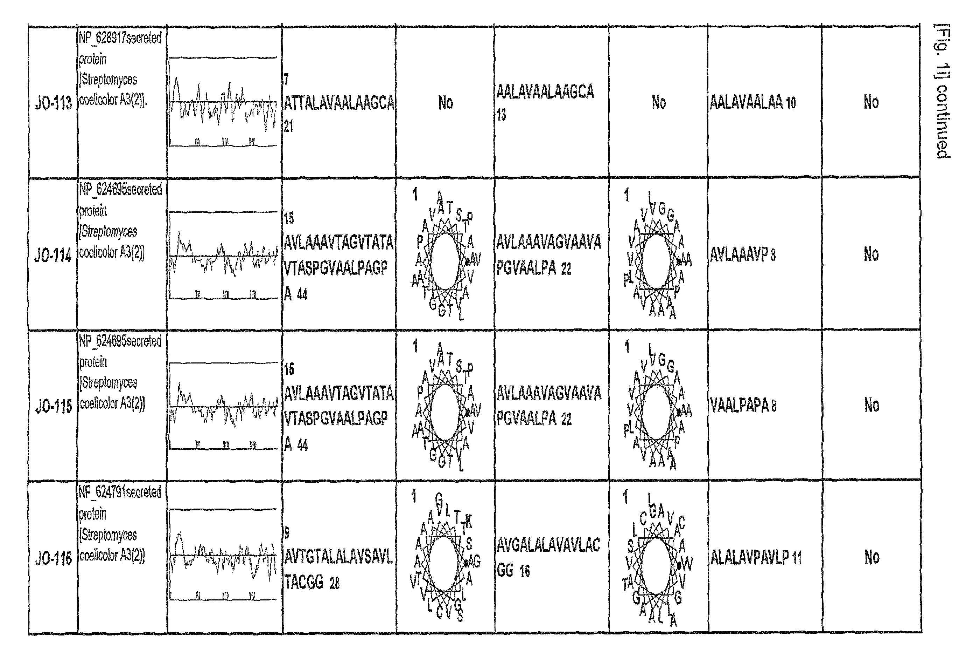

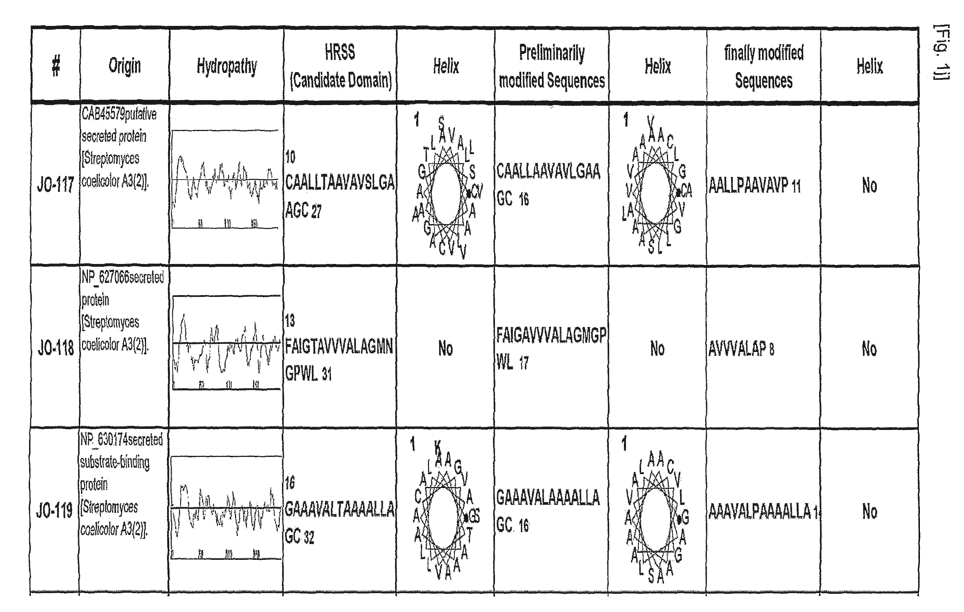

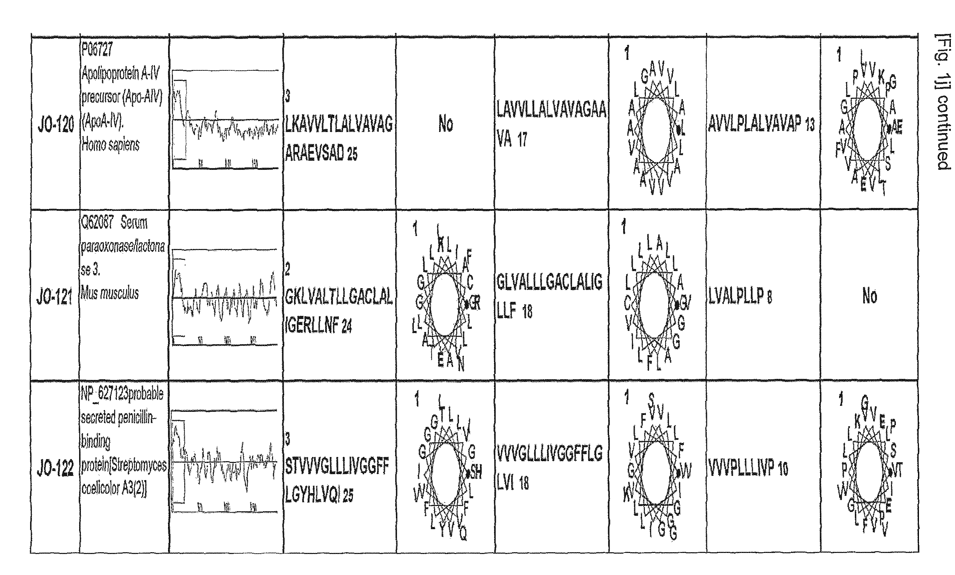

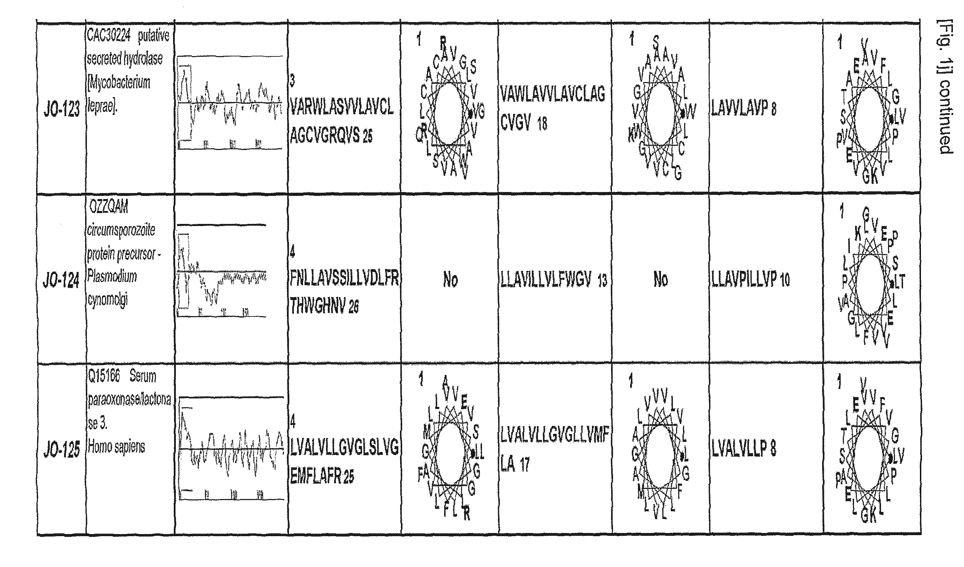

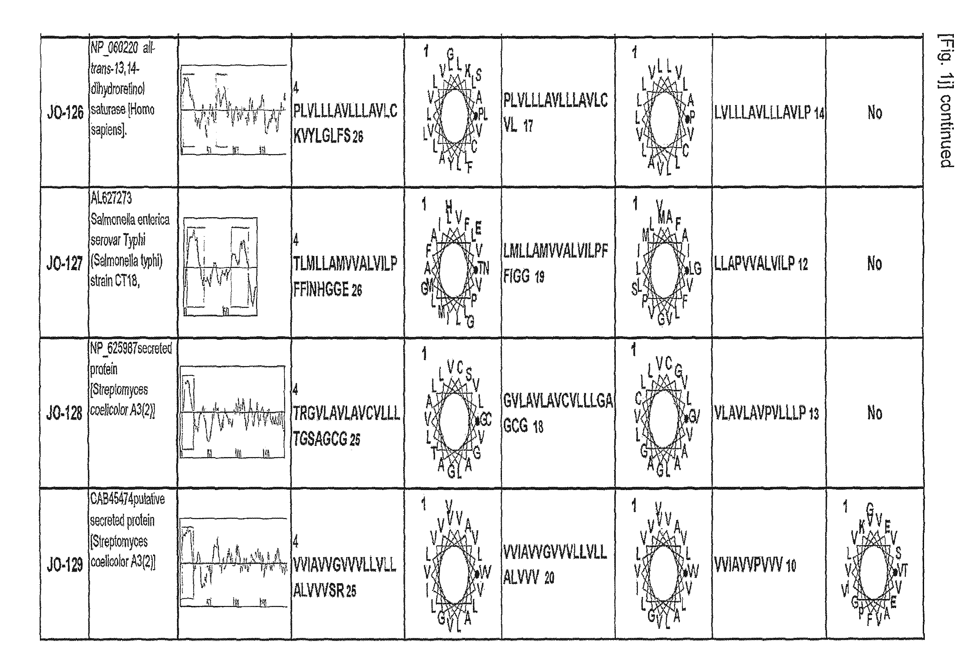

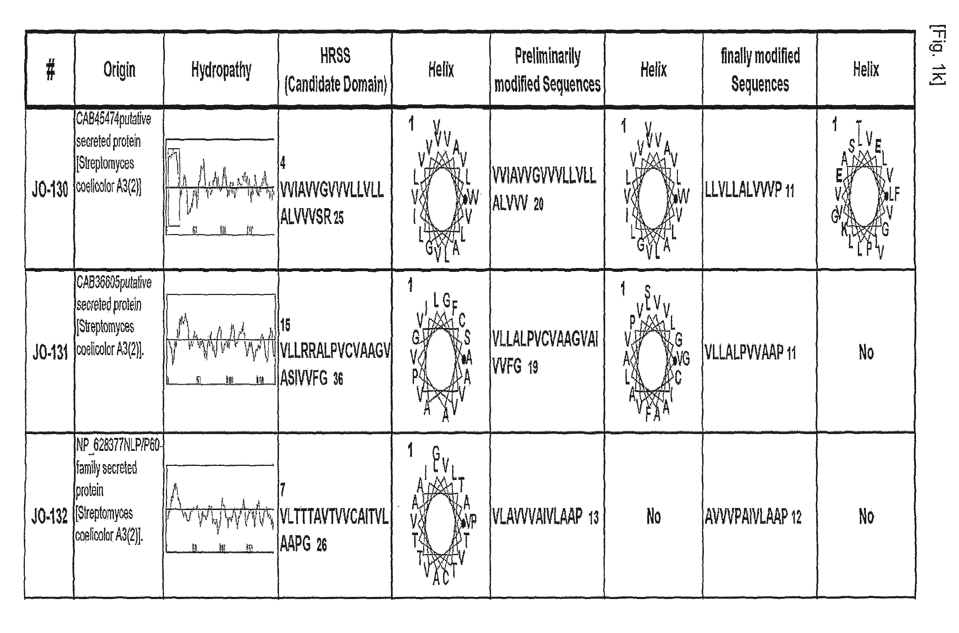

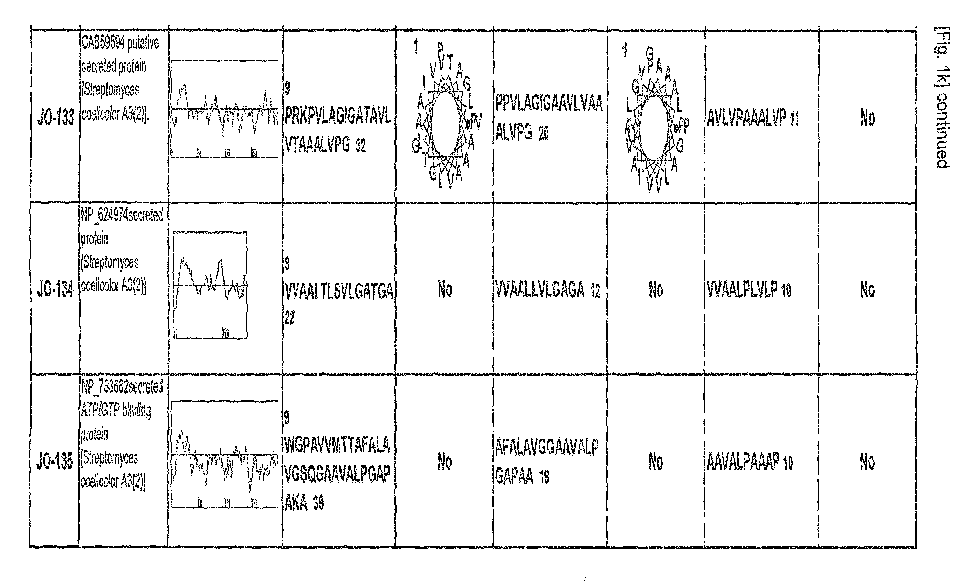

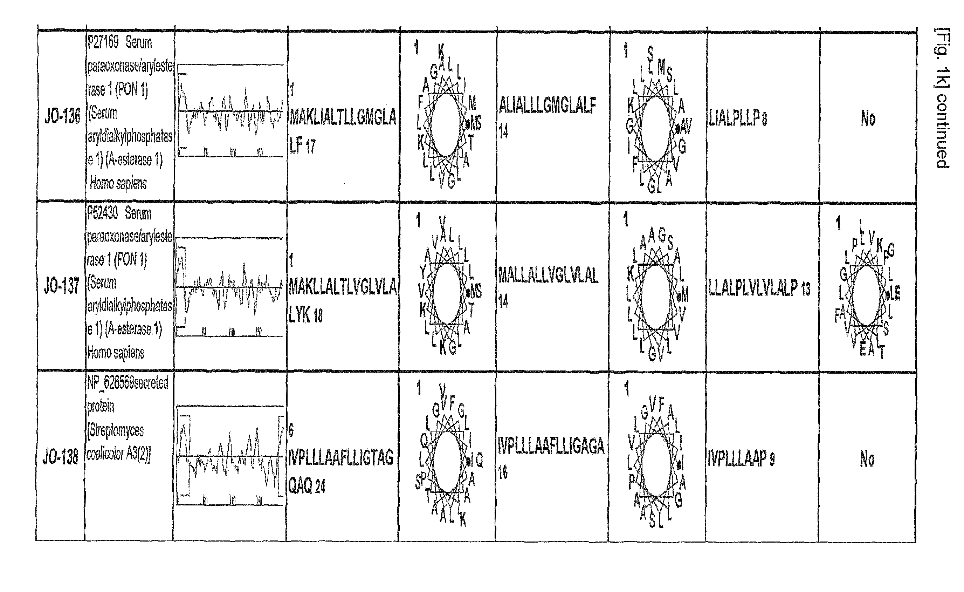

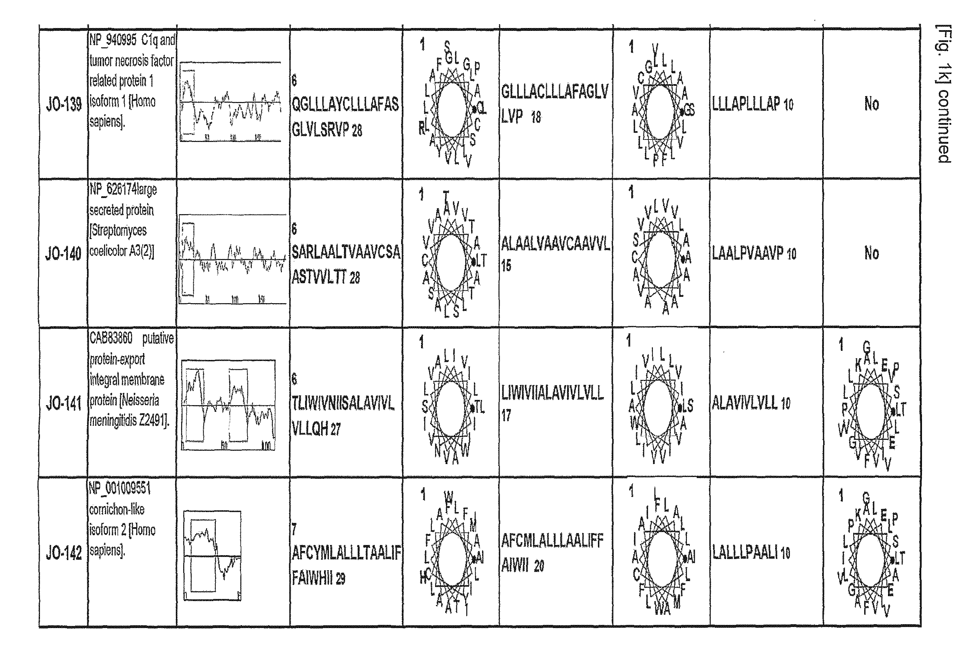

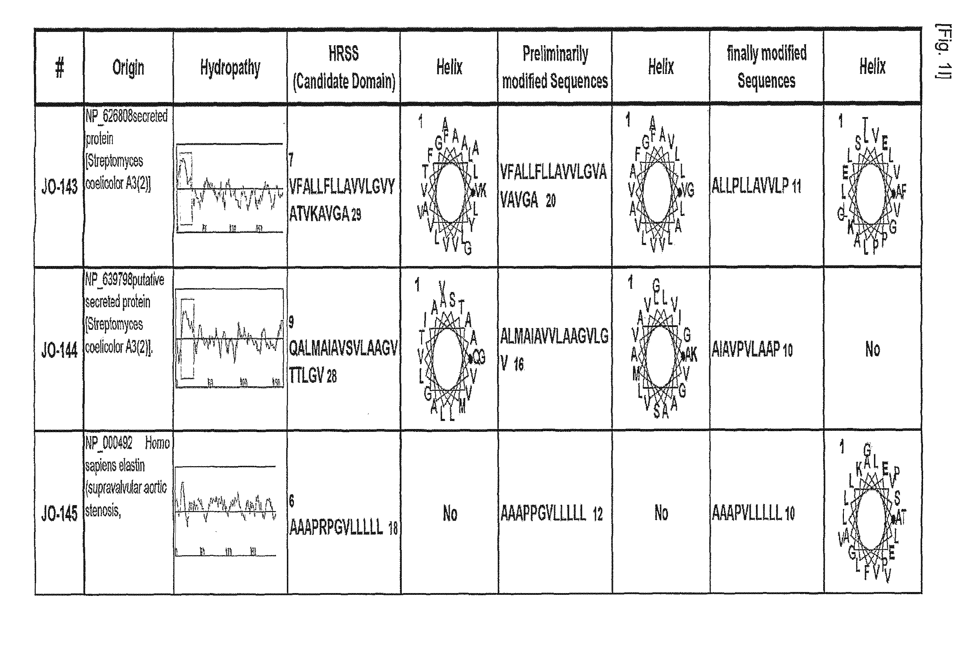

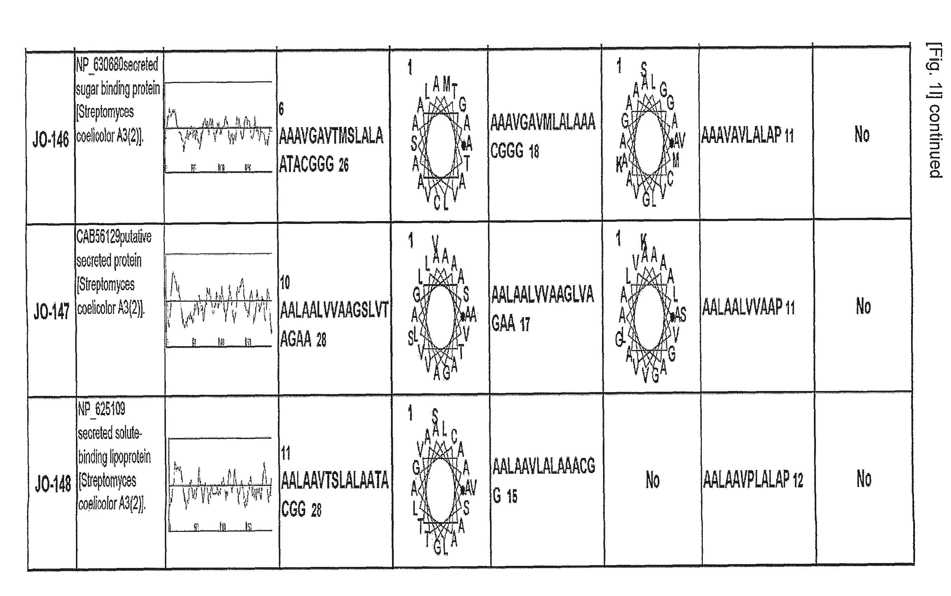

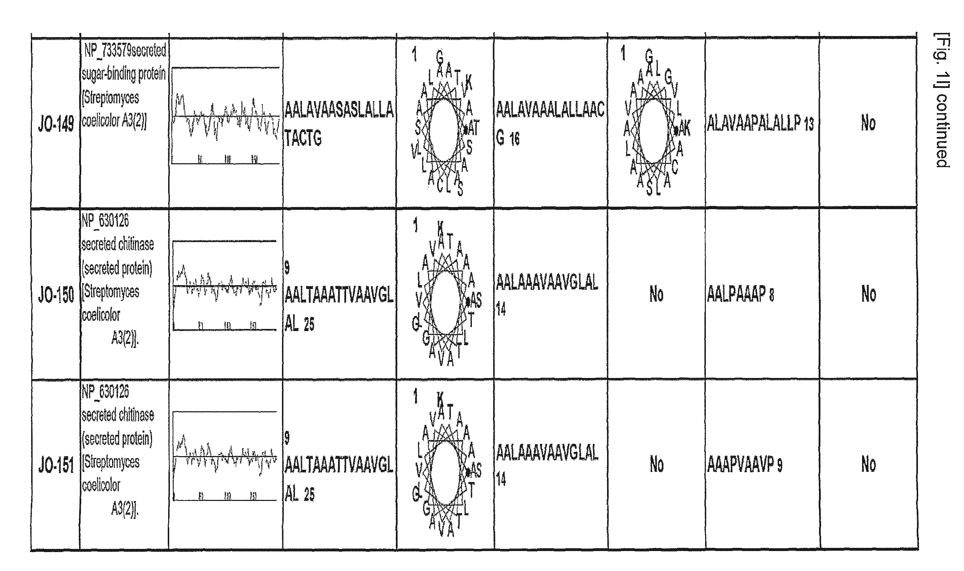

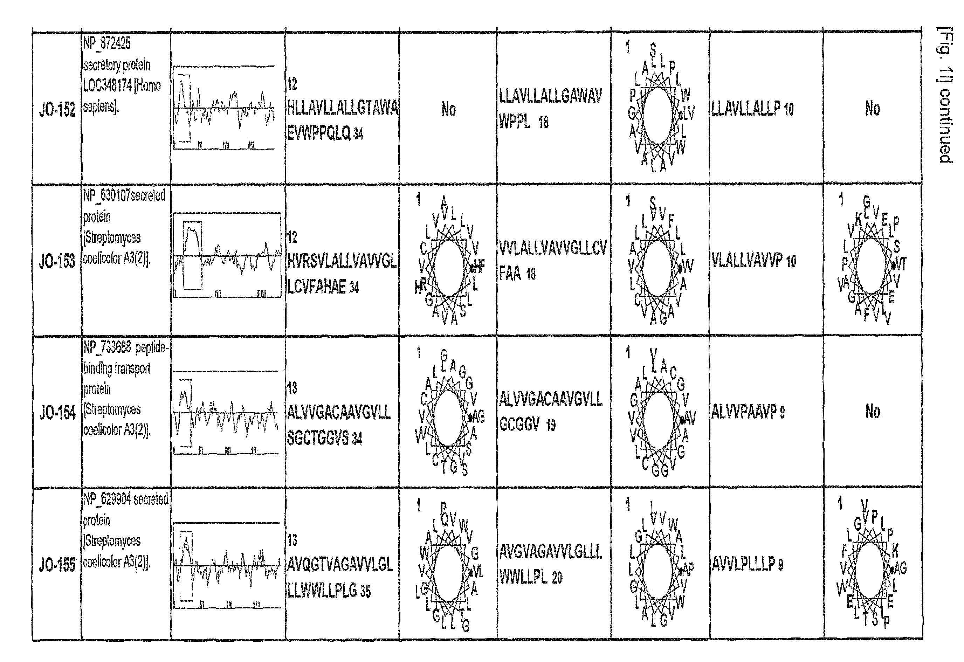

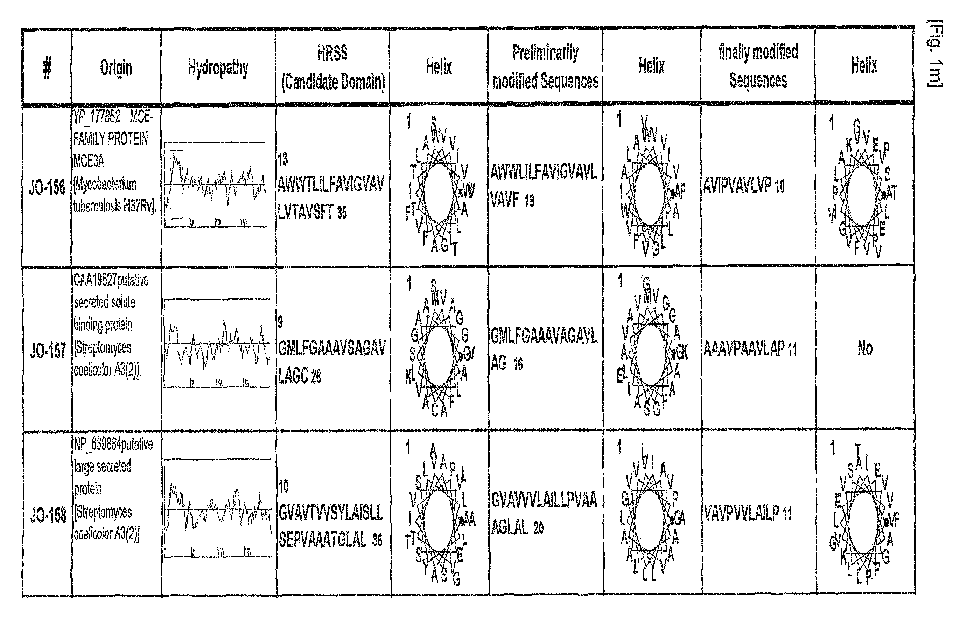

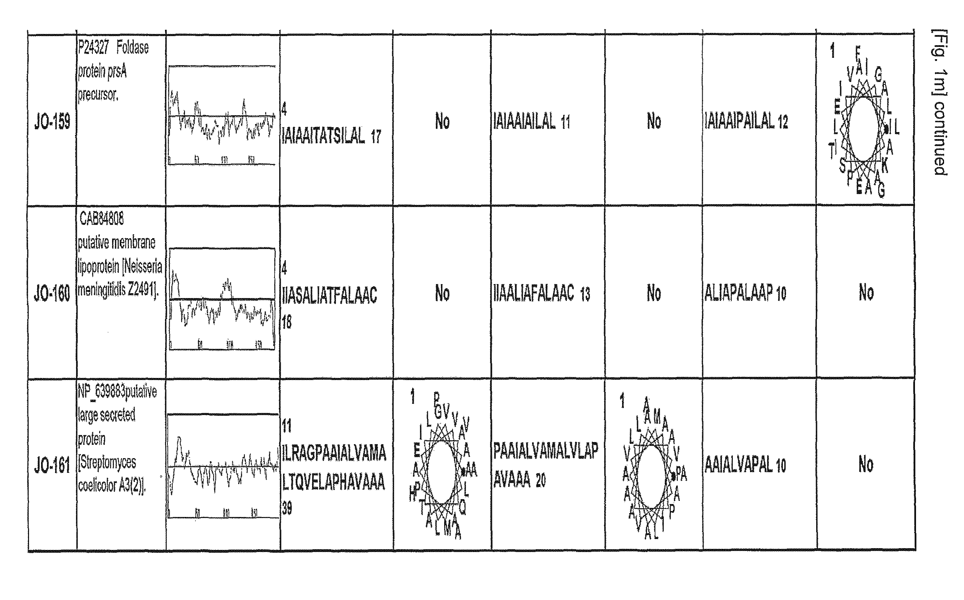

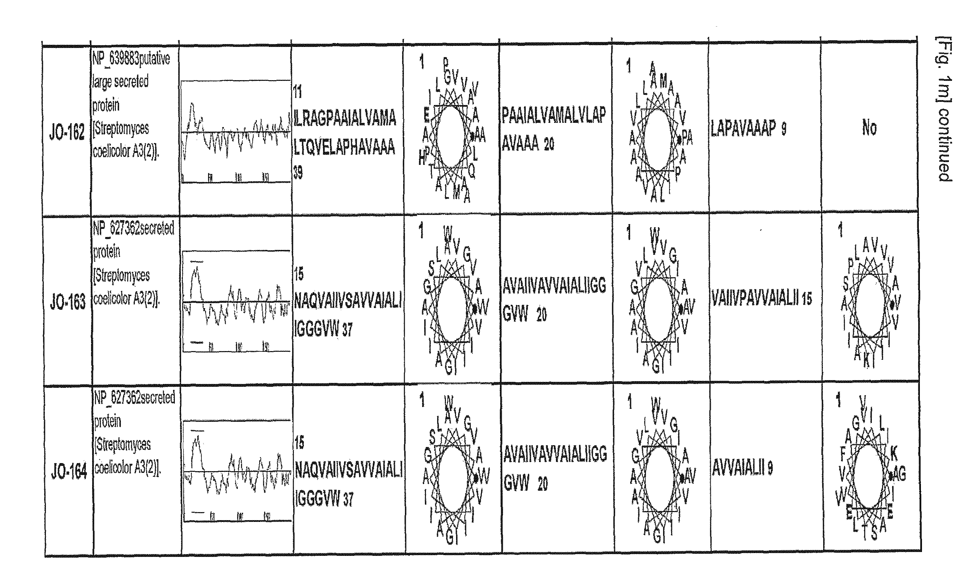

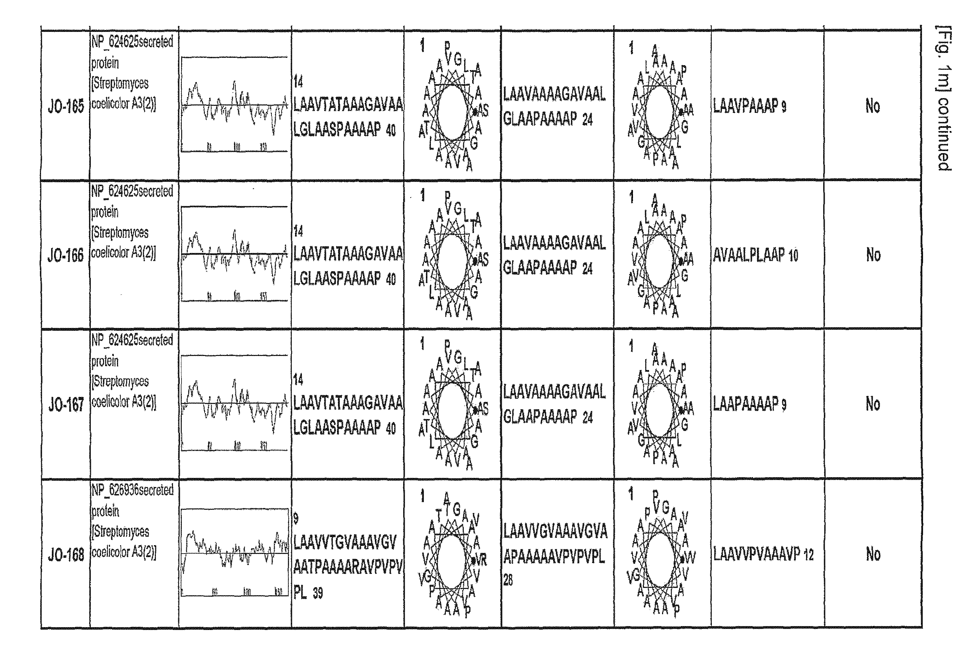

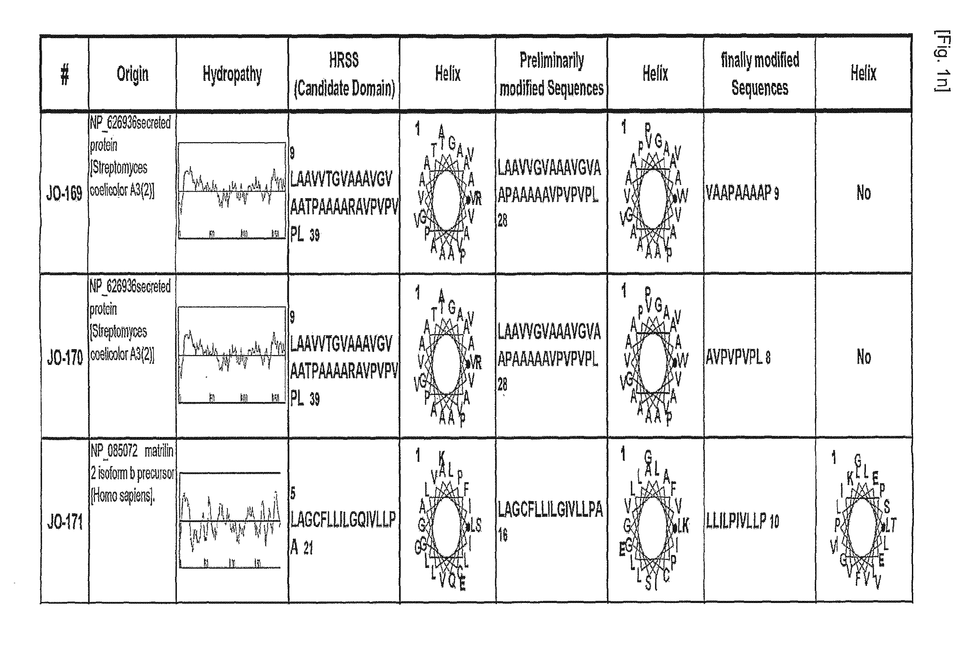

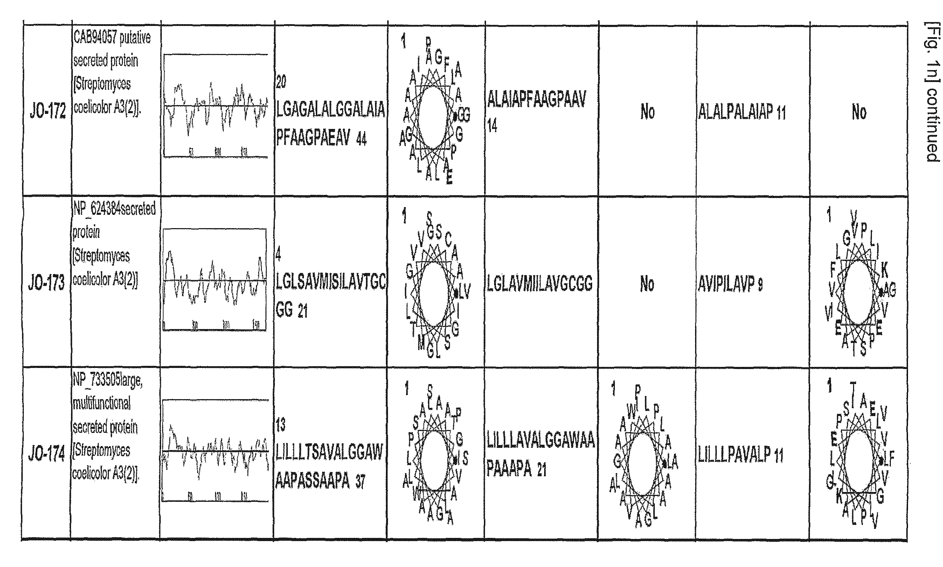

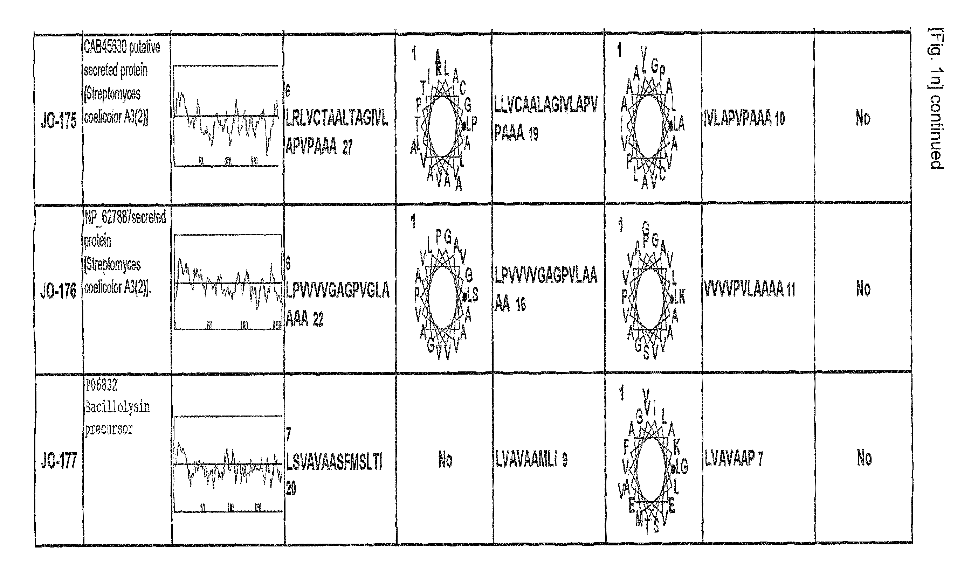

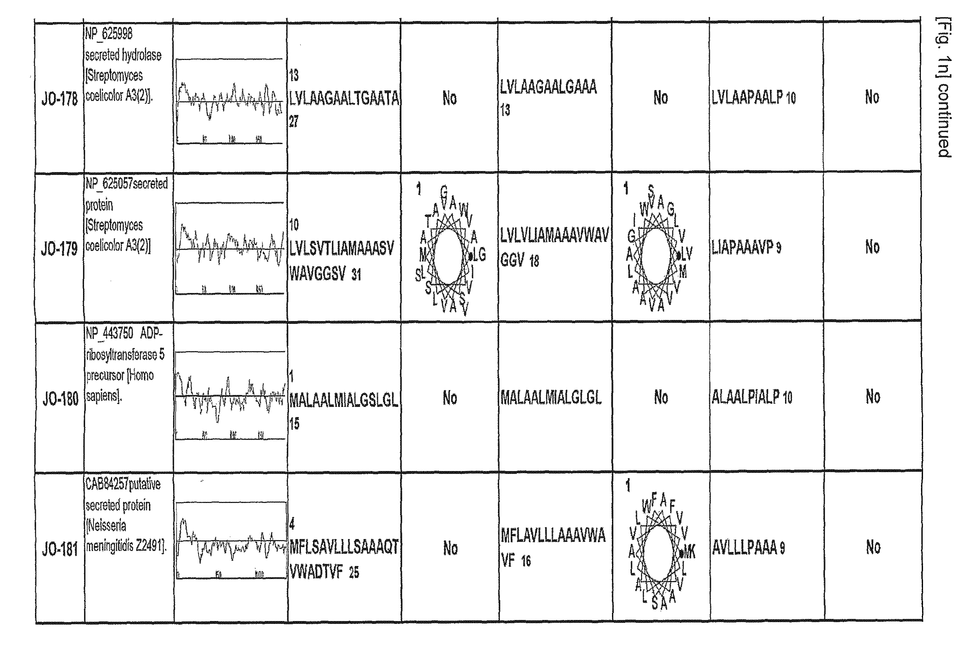

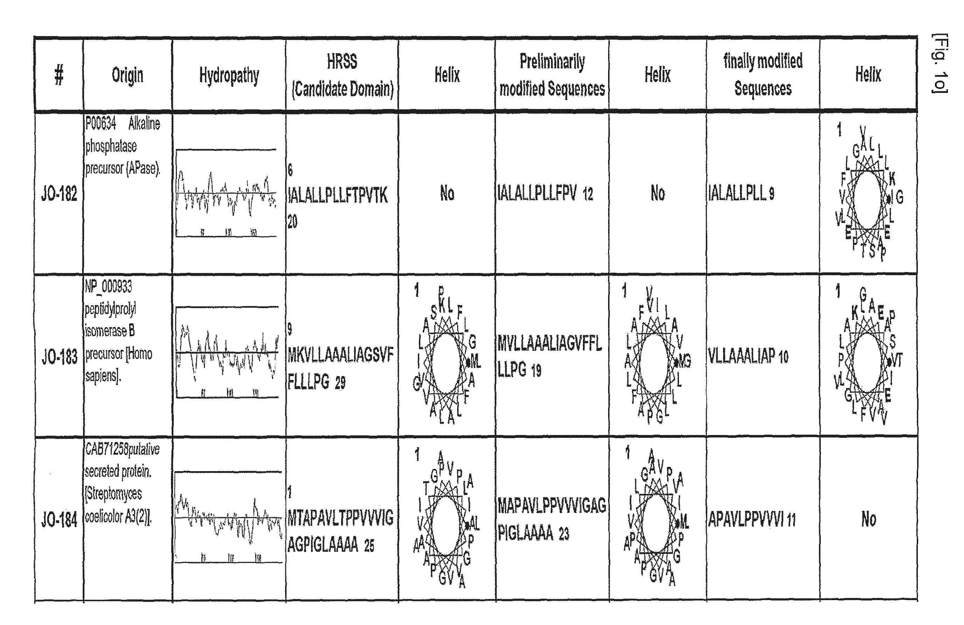

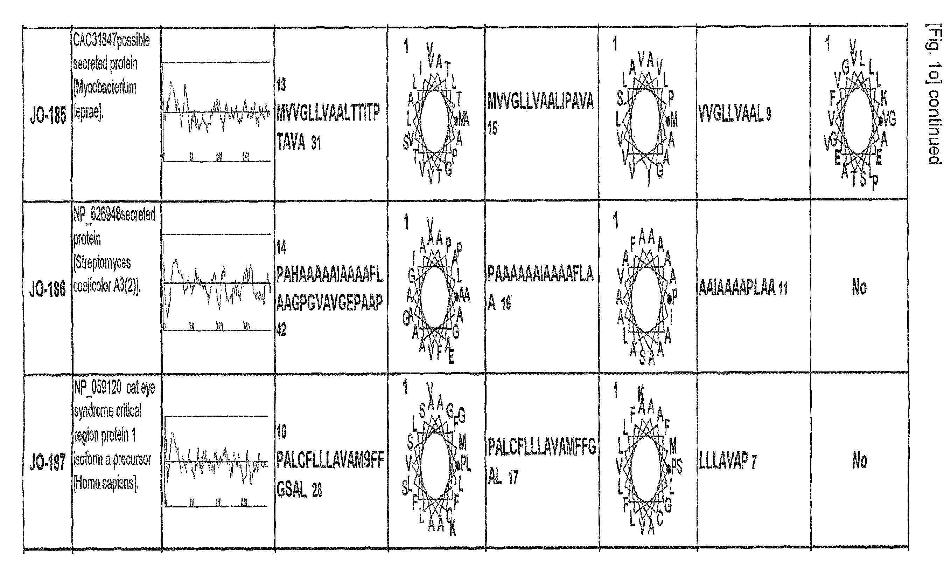

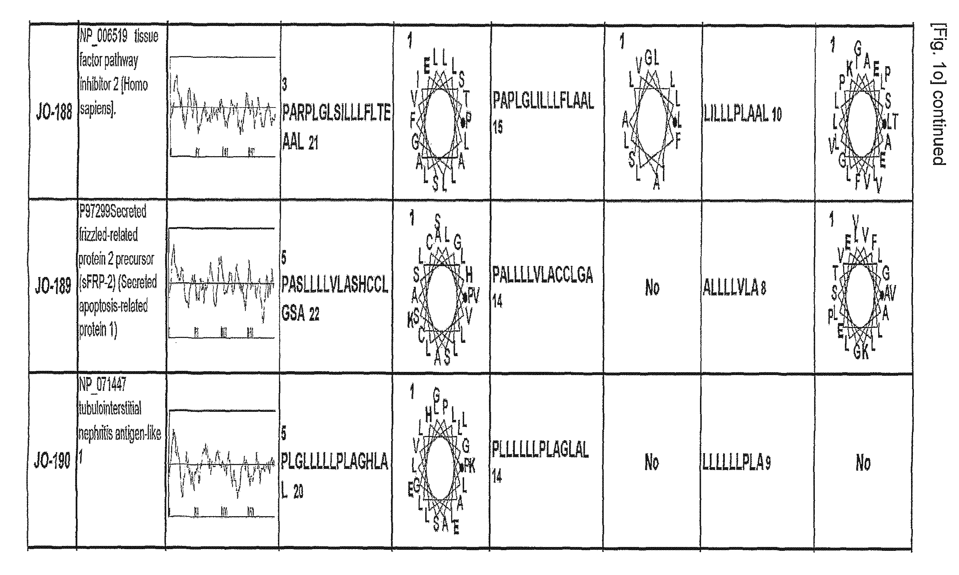

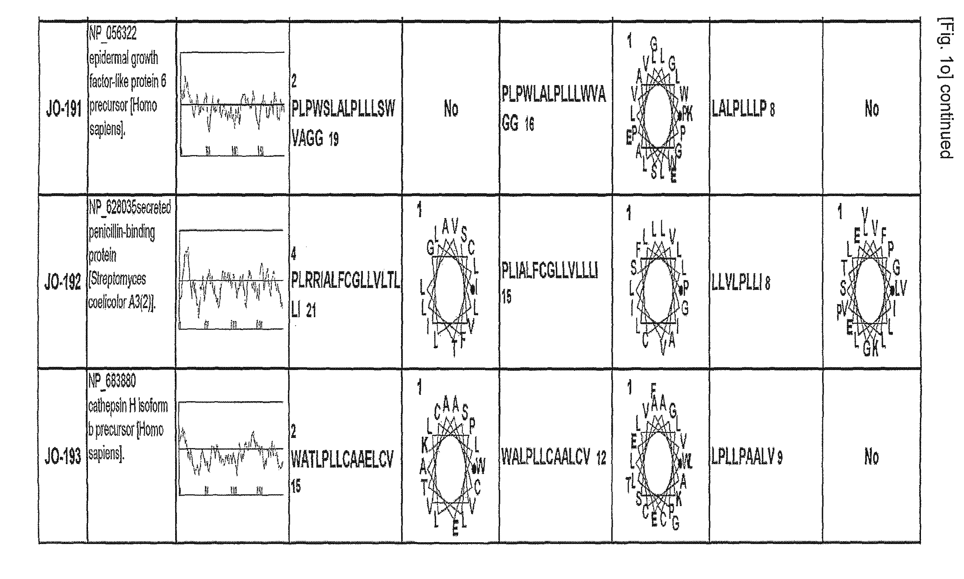

According to the method described above, novel 193 MTD peptides having amino acid sequences represented by SEQ ID NOs: 01 to 193, respectively, and are capable of facilitating the traverse of biologically active molecules across the cell membrane were identified. The 193 MTDs were designated as JO-001 to JO-193, respectively, and their structural characteristics and sequences are shown in Figs. 1a to 1o. The MTD of the present invention is designated as JO-173.

### Example 2 - Expression of Recombinant Proteins Fused to MTDs

Having demonstrated the feasibility of the cellular import of MTD peptides identified in Example 1 above, the enhanced green fluorescent protein (EGFP) was used as a full-length protein cargo molecule. Green fluorescent protein (GFP) is a protein cloned from the jellyfish, *Aquorea Victoria.* GFP is one of the most widely used reporter proteins and produces a green light when illuminated with blue or UV light (Inouye et al., FEBS Letters 351(2): 211-14 (1994)). In one embodiment of the present invention, a commercially available GFP expression vector, pEGFP-C1 (Clontech), is used. The EGFP protein encoded by pEGFP-C1 is a mutant of wild-type GFP, which has been modified to produce a stronger green light. Further, when a foreign gene is inserted into the multiple cloning sites of the pEGFP-C1 vector, the inserted foreign gene is expressed in the form of a recombinant protein with EGFP.

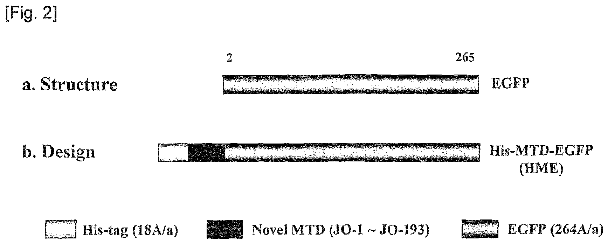

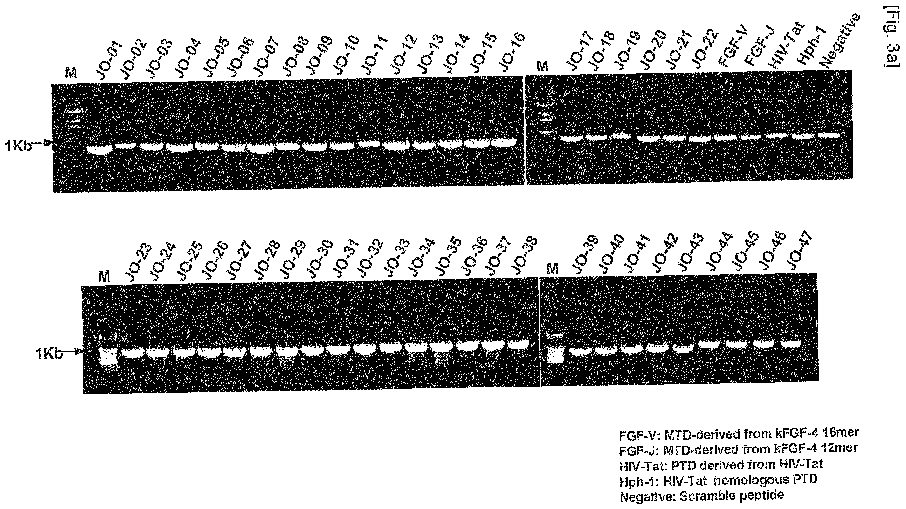

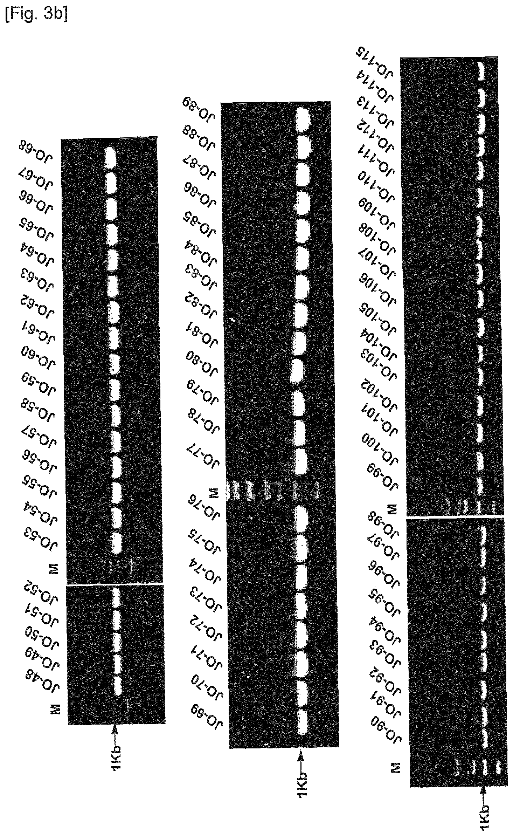

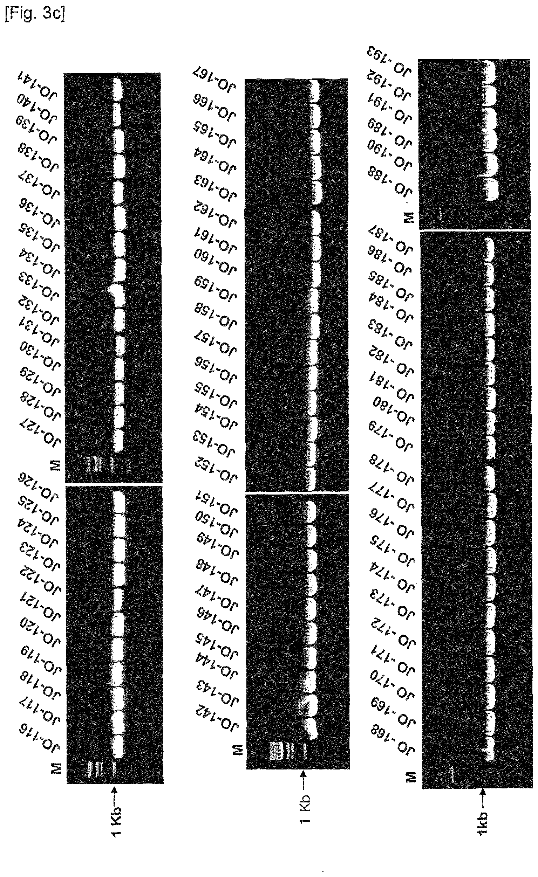

In order to construct expression vectors for EGFP proteins fused to each novel MTD (His-MTD-EGFP: HME) (Fig. 2), a polymerase chain reaction (PCR) was conducted by using the oligonucleotides described in Tables 1 and 2 below as a primer set (forward primers SEQ ID NOs: 393 to 585 for MTD JO-01 to JO-193, respectively; reverse primer SEQ ID NO: 586 for MTD JO-01 to JO-193) and the pEGFP-C1 plasmid as a template. The conditions for the PCR were 30 cycles of 45 seconds at 95°C, 45 seconds at 68°C, and 1 minute at 72°C after initial denaturation of 5 minutes at 95°C, and final extension of 5 minutes at 72°C. The amplified PCR product was digested with restriction enzyme *Nde*I and subjected to agarose gel electrophoresis. An MTD-derived from kFGF-4 16 mer (FGF-V, AAVALLPAVLLALLAP, Lin et al., J. Biol. Chem. 275: 16774-16778, 2000; Veach et al., J. Biol. Chem. 279: 11425-11431, 2004), an MTD-derived from kFGF-4 12 mer (FGF-J, AAVLLPVLLAAP, Jo et al., Nat. Biotech. 19: 929-933, 2001; Jo et al., Nat. Med. 11: 892-898, 2005(SEQ ID NO: 389), a protein transduction domain derived from HIV-Tat (HIV-Tat, YGRKKRRQRRR, Schwarze et al., Science 285: 1569-1572, 1999), and HIV-Tat homologous (Hph-1, YARVRRRGPRR) were used as positive controls, and a scramble peptide (SEQ ID NO: 387) was used as a negative control. As shown in Figs. 3a to 3c, it was confirmed that the DNA fragments encoding MTDs-EGFP were successfully amplified.

**<Table 1>**

| PCR forward primer sequences for each MTD-EGFP protein, wherein the primer sequence of JO-173 refers to the present invention | | |
|---|---|---|
| MTD | Sequence | SEQ ID NO |
| JO-01 | | 393 |
| JO-02 | | 394 |
| JO-03 | | 395 |
| JO-04 | | 396 |
| JO-05 | | 397 |
| JO-06 | | 398 |
| JO-07 | | 399 |
| JO-08 | | 400 |
| JO-09 | | 401 |
| JO-10 | | 402 |
| | | |
| JO-11 | | 403 |
| JO-12 | | 404 |
| JO-13 | | 405 |
| JO-14 | | 406 |
| JO-15 | | 407 |
| JO-16 | | 408 |
| JO-17 | | 409 |
| JO-18 | | 410 |
| JO-19 | | 411 |
| JO-20 | | 412 |
| JO-21 | | 413 |
| JO-22 | | 414 |
| JO-23 | | 415 |
| JO-24 | | 416 |
| JO-25 | | 417 |
| JO-26 | | 418 |
| JO-27 | | 419 |
| JO-28 | | 420 |
| JO-29 | | 421 |
| JO-30 | | 422 |
| JO-31 | | 423 |
| JO-32 | | 424 |
| JO-33 | | 425 |
| JO-34 | | 426 |
| JO-35 | | 427 |
| | | |
| JO-36 | | 428 |
| JO-37 | | 429 |
| JO-38 | | 430 |
| JO-39 | | 431 |
| JO-40 | | 432 |
| JO-41 | | 433 |
| JO-42 | | 434 |
| JO-43 | | 435 |
| JO-44 | | 436 |
| JO-45 | | 437 |
| JO-46 | | 438 |
| JO-47 | | 439 |
| JO-48 | | 440 |
| JO-49 | | 441 |
| JO-50 | | 442 |
| JO-51 | | 443 |
| JO-52 | | 444 |
| JO-53 | | 445 |
| JO-54 | | 446 |
| JO-55 | | 447 |
| JO-56 | | 448 |
| JO-57 | | 449 |
| JO-58 | | 450 |
| JO-59 | | 451 |
| JO-60 | | 452 |
| | | |
| JO-61 | | 453 |
| JO-62 | | 454 |
| JO-63 | | 455 |
| JO-64 | | 456 |
| JO-65 | | 457 |
| JO-66 | | 458 |
| JO-67 | | 459 |
| JO-68 | | 460 |
| JO-69 | | 461 |
| JO-70 | | 462 |
| JO-71 | | 463 |
| JO-72 | | 464 |
| JO-73 | | 465 |
| JO-74 | | 466 |
| JO-75 | | 467 |
| JO-76 | | 468 |
| JO-77 | | 469 |
| JO-78 | | 470 |
| JO-79 | | 471 |
| JO-80 | | 472 |
| JO-81 | | 473 |
| JO-82 | | 474 |
| JO-83 | | 475 |
| JO-84 | | 476 |
| JO-85 | | 477 |
| | | |
| JO-86 | | 478 |
| JO-87 | | 479 |
| JO-88 | | 480 |
| JO-89 | | 481 |
| JO-90 | | 482 |
| JO-91 | | 483 |
| JO-92 | | 484 |
| JO-93 | | 485 |
| JO-94 | | 486 |
| JO-95 | | 487 |
| JO-96 | | 488 |
| JO-97 | | 489 |
| JO-98 | | 490 |
| JO-99 | | 491 |
| JO-100 | | 492 |
| JO-101 | | 493 |
| JO-102 | | 494 |
| JO-103 | | 495 |
| JO-104 | | 496 |
| JO-105 | | 497 |
| JO-106 | | 498 |
| JO-107 | | 499 |
| JO-108 | | 500 |
| JO-109 | | 501 |
| JO-110 | | 502 |
| | | |
| JO-111 | | 503 |
| JO-112 | | 504 |
| JO-113 | | 505 |
| JO-114 | | 506 |
| JO-115 | | 507 |
| JO-116 | | 508 |
| JO-117 | | 509 |
| JO-118 | | 510 |
| JO-119 | | 511 |
| JO-120 | | 512 |
| JO-121 | | 513 |
| JO-122 | | 514 |
| JO-123 | | 515 |
| JO-124 | | 516 |
| JO-125 | | 517 |
| JO-126 | | 518 |
| JO-127 | | 519 |
| JO-128 | | 520 |
| JO-129 | | 521 |
| JO-130 | | 522 |
| JO-131 | | 523 |
| JO-132 | | 524 |
| JO-133 | | 525 |
| JO-134 | | 526 |
| JO-135 | | 527 |
| | | |
| JO-136 | | 528 |
| JO-137 | | 529 |
| JO-138 | | 530 |
| JO-139 | | 531 |
| JO-140 | | 532 |
| JO-141 | | 533 |
| JO-142 | | 534 |
| JO-143 | | 535 |
| JO-144 | | 536 |
| JO-145 | | 537 |
| JO-146 | | 538 |
| JO-147 | | 539 |
| JO-148 | | 540 |
| JO-149 | | 541 |
| JO-150 | | 542 |
| JO-151 | | 543 |
| JJO-152 | | 544 |
| JO-153 | | 545 |
| JO-154 | | 546 |
| JO-155 | | 547 |
| JO-156 | | 548 |
| JO-157 | | 549 |
| JO-158 | | 550 |
| JO-159 | | 551 |
| JO-160 | | 552 |
| | | |
| JO-161 | | 553 |
| JO-162 | | 554 |
| JO-163 | | 555 |
| JO-164 | | 556 |
| JO-165 | | 557 |
| JO-166 | | 558 |
| JO-167 | | 559 |
| JO-168 | | 560 |
| JO-169 | | 561 |
| JO-170 | | 562 |
| JO-171 | | 563 |
| JO-172 | | 564 |
| JO-173 | | 565 |
| JO-174 | | 566 |
| JO-175 | | 567 |
| JO-176 | | 568 |
| JO-177 | | 569 |
| JO-178 | | 570 |
| JO-179 | | 571 |
| JO-180 | | 572 |
| JO-181 | | 573 |
| JO-182 | | 574 |
| JO-183 | | 575 |
| JO-184 | | 576 |
| JO-185 | | 577 |
| | | |
| JO-186 | | 578 |
| JO-187 | | 579 |
| JO-188 | | 580 |
| JO-189 | | 581 |
| JO-190 | | 582 |
| JO-191 | | 583 |
| JO-192 | | 584 |
| JO-193 | | 585 |

**<Table 2>**

| PCR reverse primer sequence for each MTD-EGFP protein | | |
|---|---|---|
| MTD | Sequence | SEQ ID NO |
| JO-1∼ JO-193 | 3'-TTA TCT AGA TCC GGT GGA TCC CGG GCC-5' | 586 |

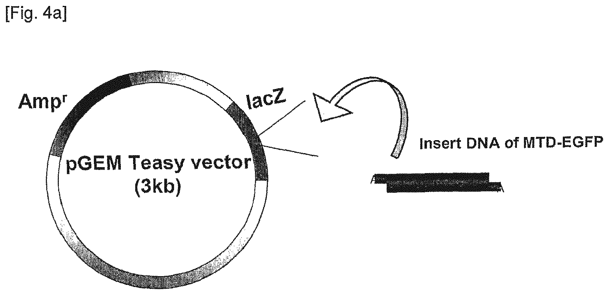

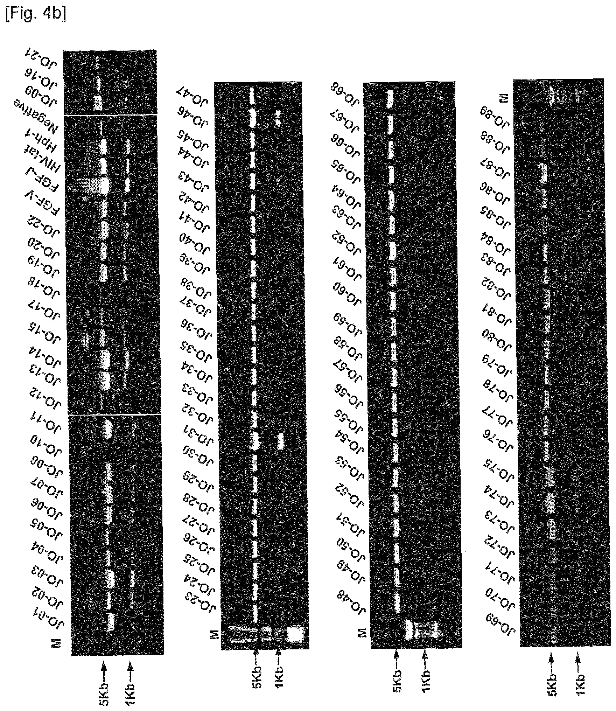

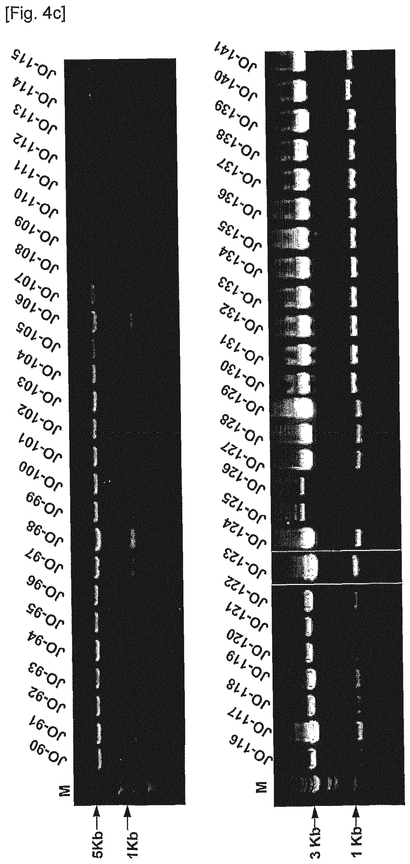

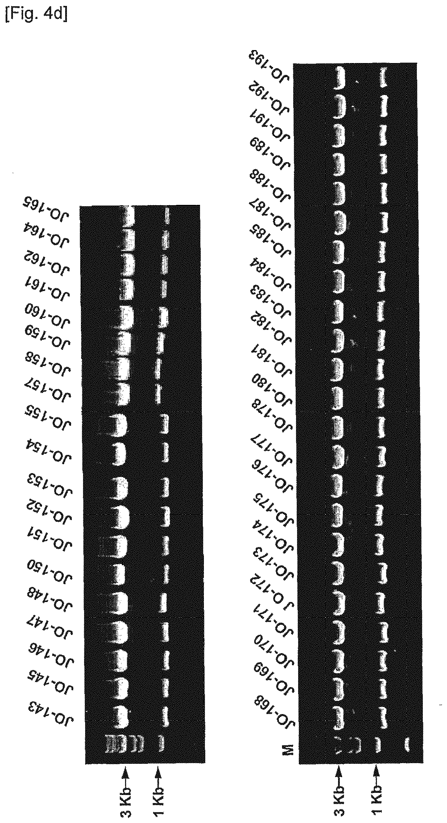

Referring to Fig. 4a, each of the PCR amplified products was subcloned into the pGEM-Teasy vector (Promega, Madison WI, USA) with a T4 DNA ligase, according to the TA cloning method and then *E. coli* DH5α was transformed, where transformants were selected on LB plate media containing 50 µg/mL ampicillin, IPTG and X-gal. After the recombinant fragment inserted into pGEM-Teasy vector was isolated by treating with the restriction enzyme *Nde*I, it was subjected to 0.8 % agarose gel electrophoresis. As a result, DNA fragments of about 1 kb and vector fragments of about 3 kb were detected, which confirms that the insert DNA of MTD-EGFP was appropriately subcloned into pGEM-Teasy vector (Figs. 4b to 4d).

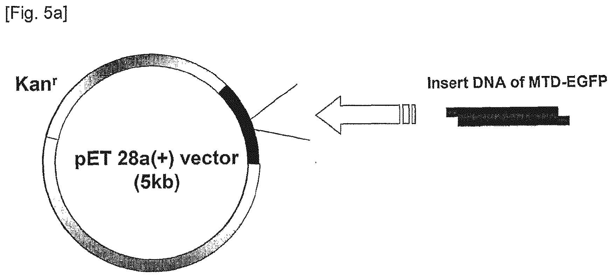

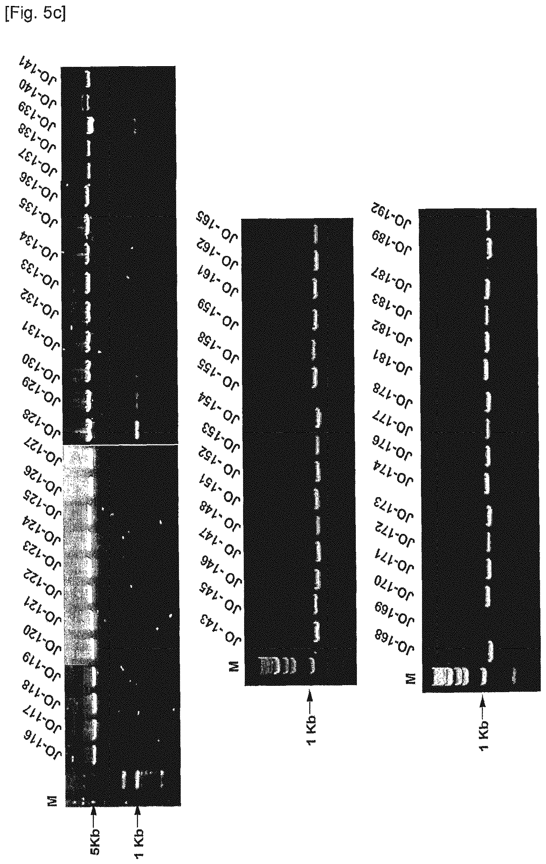

As described in Fig. 5a, each of the isolated insert DNA fragments encoding MTDs-EGFP was cloned into the *E. coli* expression plasmid pET-28a(+) (commercially available from Novagen, Madison, WI). The pET-28a(+) plasmids are designed to facilitate His-tag fusions at either the N- or C-terminus and to provide strong expression of the genes in *E. coli* from the T7 phage promoter. At the 3' end of each MTD-EGFP encoding gene, the coding sequence was fused in frame at the *NdeI* site to the His-tag sequence followed by a translation stop codon, which results in the production of MTD-EGFP recombinant proteins with six histidine residues added to the C-terminus for the sake of easy purification on nickel columns.

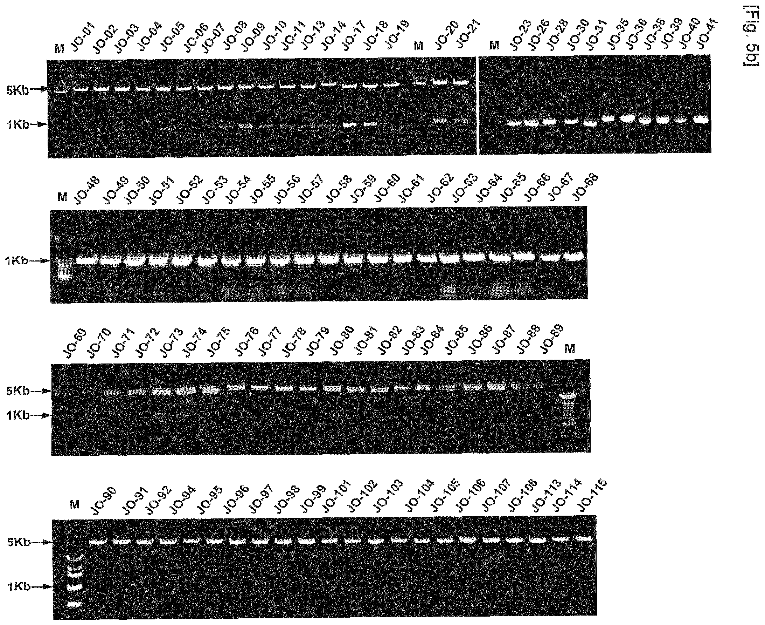

After the clones were treated with the restriction enzyme *NdeI* and subjected to agarose gel electrophoresis, it was verified that DNA fragments of about 1 kb and vector fragments of about 5 kb were detected, which confirms the cloning of the insert DNA of MTD-EGFP into pET-28a(+) vector, as shown in Figs. 5b to 5c. Further, out of the 193 novel MTDs, 148 MTDs were found to be successfully cloned for the expression of His-MTD-EGFP recombinant proteins. The present invention refers to MTD of SEQ ID NO: 173.

### Example 3 - Inducible Expression and Purification of Recombinant Proteins Fused to MTDs

To express the cell permeable recombinant proteins fused to MTDs prepared as described in Example 2 above, the expression vectors comprising the His-MTD-EGFP recombinant proteins were transfected in the BL21 (DE3), BL21-Gold (DE3), BL21-CodonPlus (DE3) and BL21-GoldpLysS (DE3) strains, respectively. An EGFP expression vector including kFGF4-derived MTD (SEQ ID NO: 387) was used as a positive control, while an EGFP expression vector fused to a scramble peptide (SEQ ID NO: 389) having no function was used as a negative control. The present invention refers to recombinant proteins fused to MTDs comprising SEQ ID NO: 173.

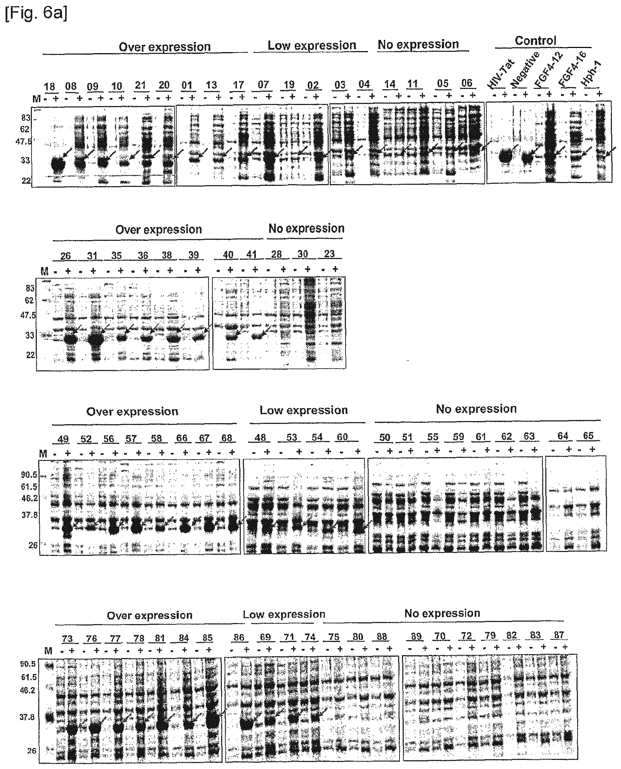

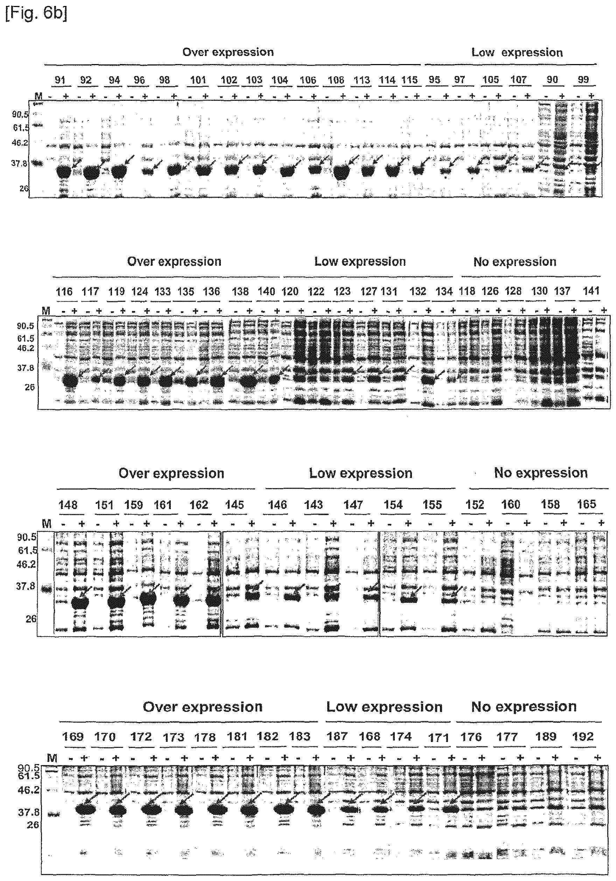

After the transfection, cells were grown at 37°C in an LB medium containing kanamycin (30 µg/ml) with vigorous shaking until the optical density 600 (OD₆₀₀) reached between 0.4 and 0.6. IPTG (isoprophyl-β-D-thiogalactoside) was then added thereto at a final concentration of 0.6 mM to induce the expression of the His-MTD-EGFP recombinant proteins. Protein induction was prolonged for 3 hours at 37°C. His-MTD-EGFP recombinant proteins expressed in *E. coli* with IPTG were loaded on a SDS-PAGE gel, stained with Coomassie Brilliant Blue, and then destained. As illustrated in Figs. 6a and 6b, except for some His-MTD-EGFP recombinant proteins expressed in BL21-GoldpLysS (DE3) strains, most His-MTD-EGFP recombinant proteins were expressed at a high level in BL21-CodonPlus (DE3). Several His-MTD-EGFP recombinant proteins were not expressed.

The inducible expression of His-MTD-EGFP recombinant proteins in an *E. coli* system leads to the formation of insoluble aggregates, which are known as inclusion bodies. To completely solubilize these inclusion bodies, all of the above expressed proteins were denatured by dissolving them in 8 M urea. Denatured His-MTD-EGFP recombinant proteins were purified by histidine tag affinity chromatography, using a nickel nitrilotriacetate resin (Qiagen, Hilden, Germany). Since strong denaturants, such as 8 M urea, completely solubilize the inclusion bodies, the purification method was carried out under pH-dependent denaturing conditions.

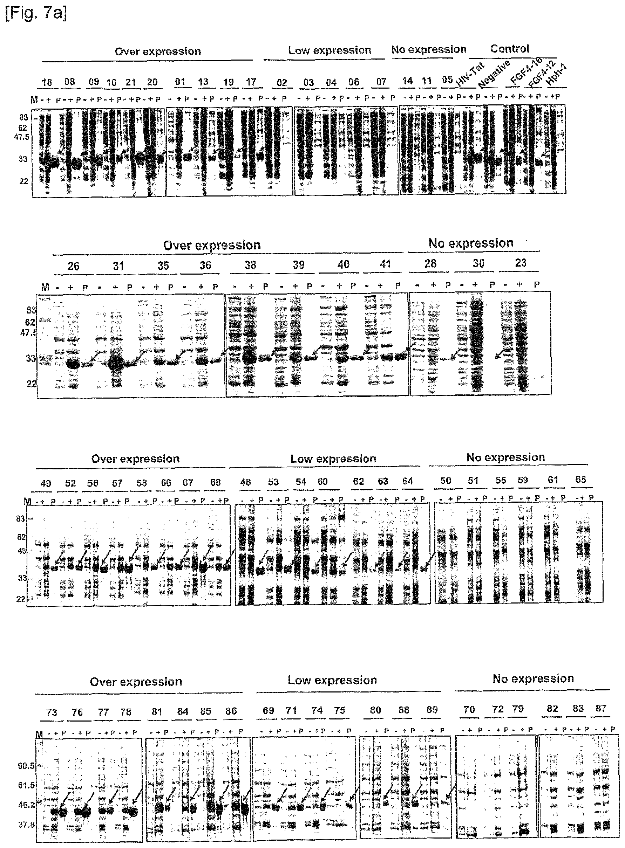

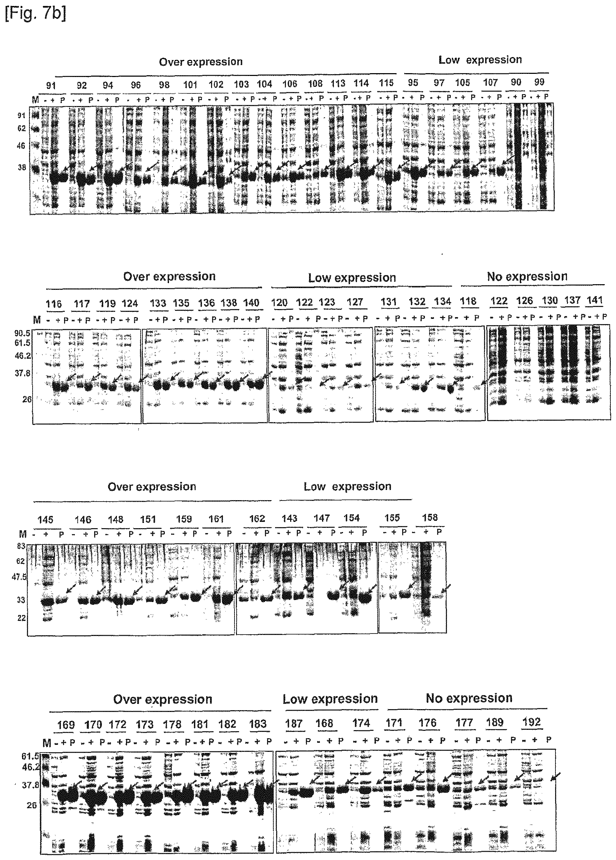

The *E. coli* culture solutions were harvested by centrifugation at 4,000 ×g for 20 minutes, re-suspended in a lysis buffer (100 mM NaH₂PO₄, 10 mM Tris·Cl, 8 M urea, pH 8.0), and subjected to ultrasonication on ice using a sonicator equipped with a probe. The cell lysates were centrifuged at 7,000 ×g for 20 minutes, so as to separate the supernatant and the cellular debris pellet. The supernatant was taken out and then incubated with a Ni-NTA resin equilibrated with the lysis buffer by gently shaking (using a rotary shaker) for 2 hours to overnight. After washing with a washing buffer (100 mM NaH₂PO₄, 10 mM Tris·Cl, 8 M urea, pH 6.3) five times, the proteins bound to the resin were eluted with an elution buffer (100 mM NaH₂PO₄, 10 mM Tris·Cl, 8 M urea, pH 4.5). The His-MTD-EGFP recombinant proteins purified under the denaturing conditions described above were analyzed on a SDS-PAGE gel and stained with Coomassie Brilliant Blue, where the results thereof are shown in Figs. 7a and 7b.

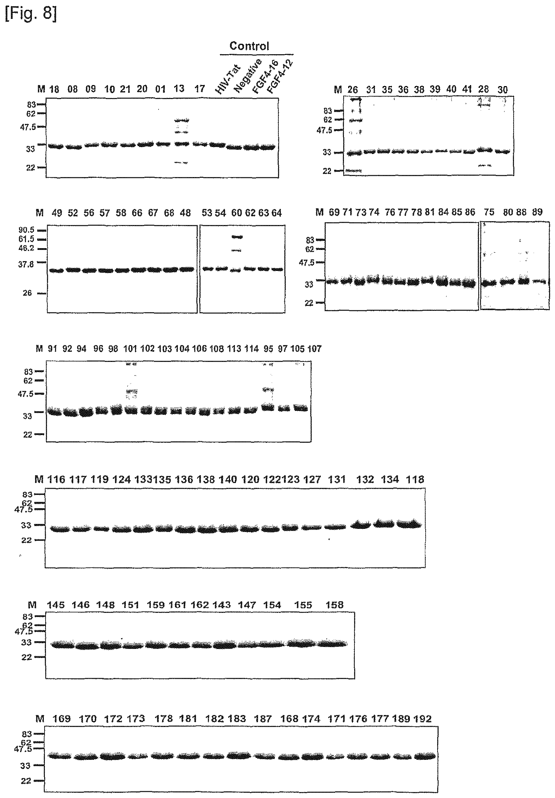

In order to renature the His-MTD-EGFP recombinant proteins purified above, the denatured proteins were refolded by removing the denaturant. Urea was removed from the proteins by dialyzing them against a refolding buffer (0.55 M Guanidine HCl, 0.44 M L-Arginine, 50 mM Tris-HCl, 150 mM NaCl, 1 mM EDTA, 100 mM NDSB, 2 mM Glutathione Oxidized, and 0.2 mM Glutathione Reduced). All of the refolded recombinant proteins were dialyzed for 9 hours against a physiological buffer, such as a cell culture medium (e.g., Dulbecco's modified Eagle's Medium: DMEM). After the replacement of the refolding buffer with DMEM, the cell permeabilities of all of the purified recombinant proteins were ready to be determined *in vitro* and *in vivo.* According to the SDS-PAGE analysis results shown in Fig. 8, out of the 148 transformants established as described in Example 2 above, the inducible expression of His-MTD-EGFP recombinant proteins was visually detected in 112 transformants, whereas 4 His-MTD-EGFP recombinant proteins were not expressed enough to be visually detected but were sufficiently purified for cell permeability analysis. The 116 His-MTD-EGFP recombinant proteins were prepared in a soluble form.

### Example 4 - Determination of Quantitative Cell Permeability of Recombinant Proteins Fused to MTDs

In order to quantitatively determine the cell permeability of the His-MTD-EGFP recombinant proteins, the 116 recombinant proteins purified in a soluble form as described in Example 3 above were mixed with 0.7 µg/µl of fluorescein isothiocyanate (FITC) and reacted at room temperature for 1 hours by stirring. The reaction solution was subjected to a dialysis against Dulbecco's modified Eagle's medium (DMEM; WelGENE Inc., Korea) for 2 days until the FITC was completely removed to thereby obtain FITC-conjugated recombinant proteins. RAW 264.7 cells derived from mouse macrophage were treated with 10 µM of the FITC-labeled protein. The RAW 264.7 cells were maintained in DMEM supplemented with 10% fetal bovine serum and 1% penicillin (500 mg/ml, WelGENE Inc.), and incubated at 37°C in a humidified atmosphere of 5% CO₂ in air. After the incubation, the cells were incubated with 10 µM of each of the FITC-conjugated recombinant protein prepared above for 1 hour at 37°C, followed by treating them with Trypsin/EDTA (T/E, Invitrogen, Carlsbad CA, USA) to remove cell surface bound proteins and washing with cold PBS three times.

The FITC-conjugated His-MTD-EGFP recombinant proteins were subjected to fluorescence-activated cell sorting (FACS) analysis (FACS Calibur, Beckton-Dickinson, San Diego CA, USA). For each sample, the cells (1×10⁴) were analyzed by using the CellQuest Pro cytometric analysis software. Each experiment was conducted at least twice. The cell permeable potency of each His-MTD-EGFP recombinant protein fused to novel MTDs (JO-01 to JO-193) was visually compared to that of a positive control protein fused to kFGF4-derived MTD.

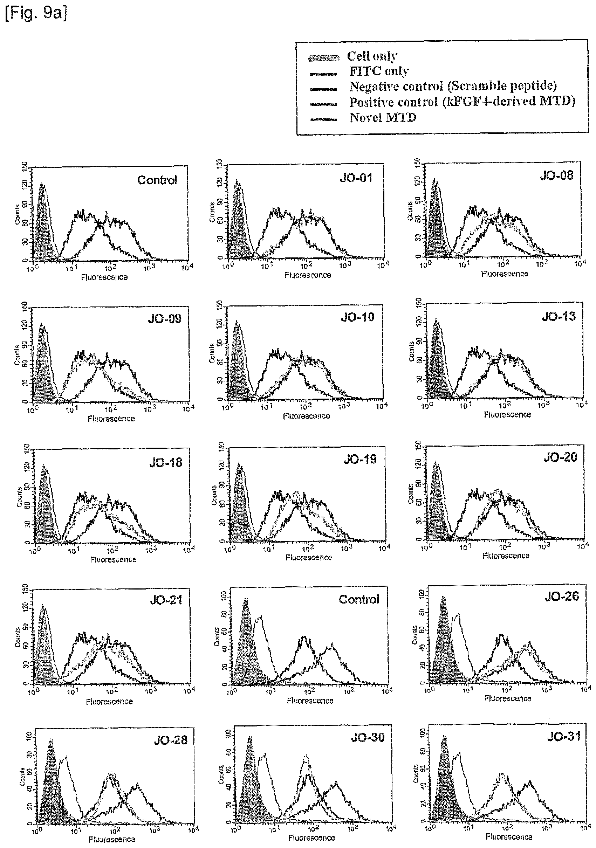

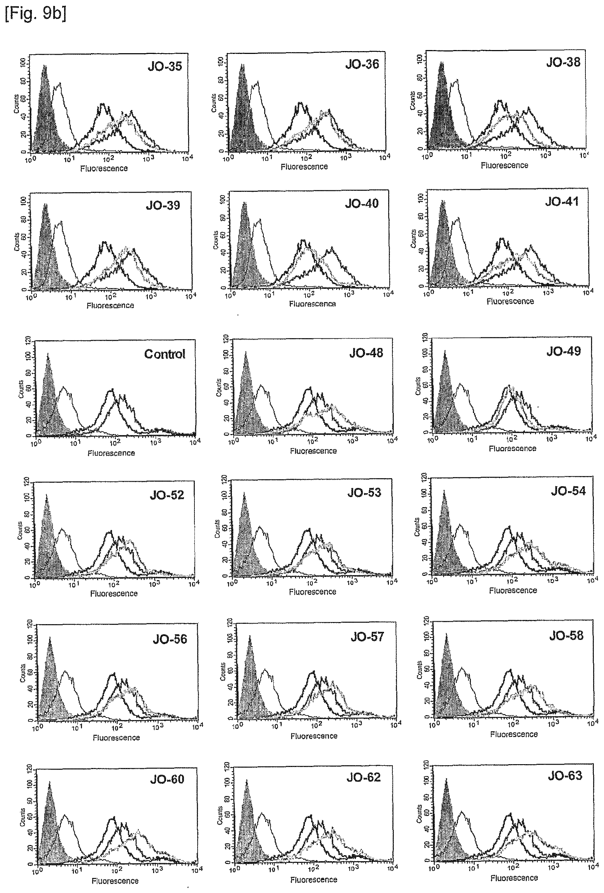

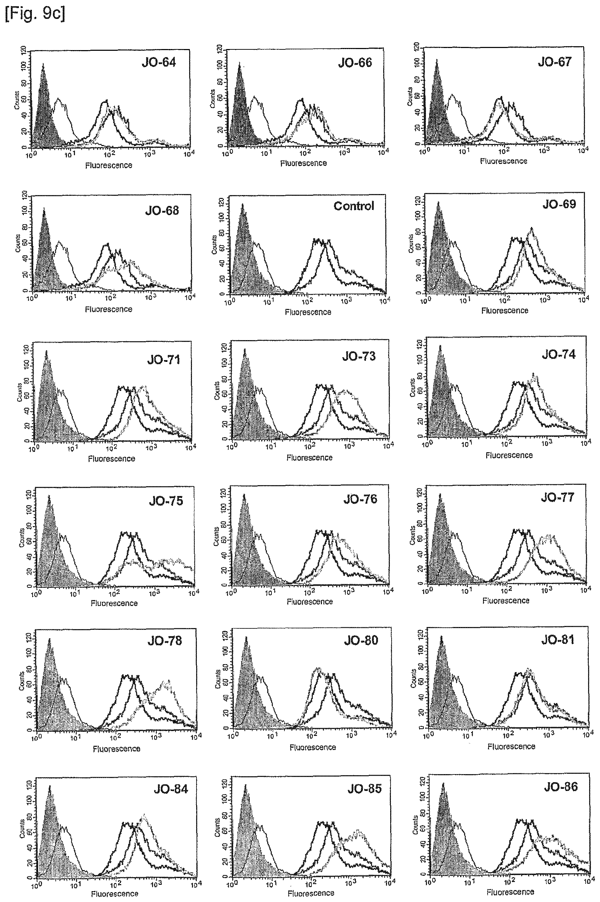

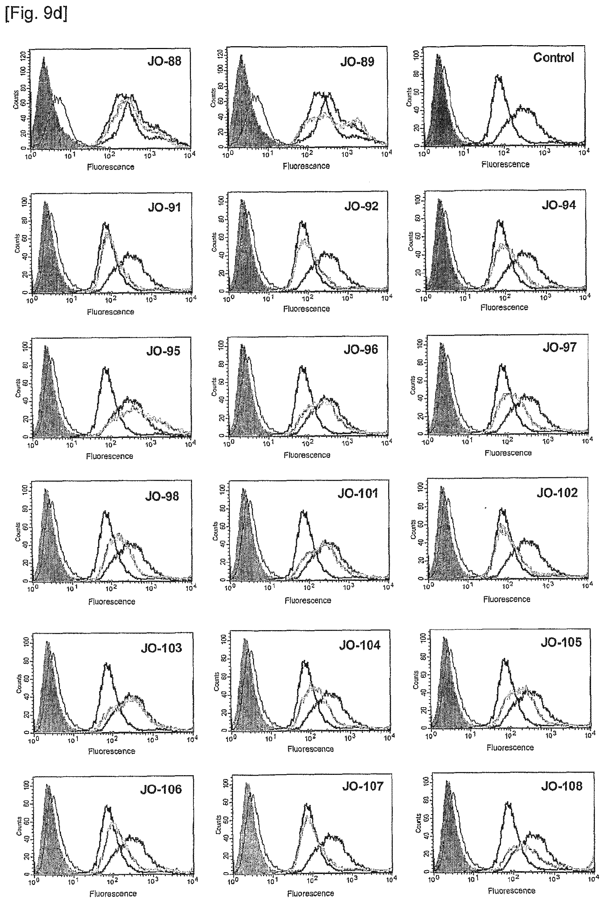

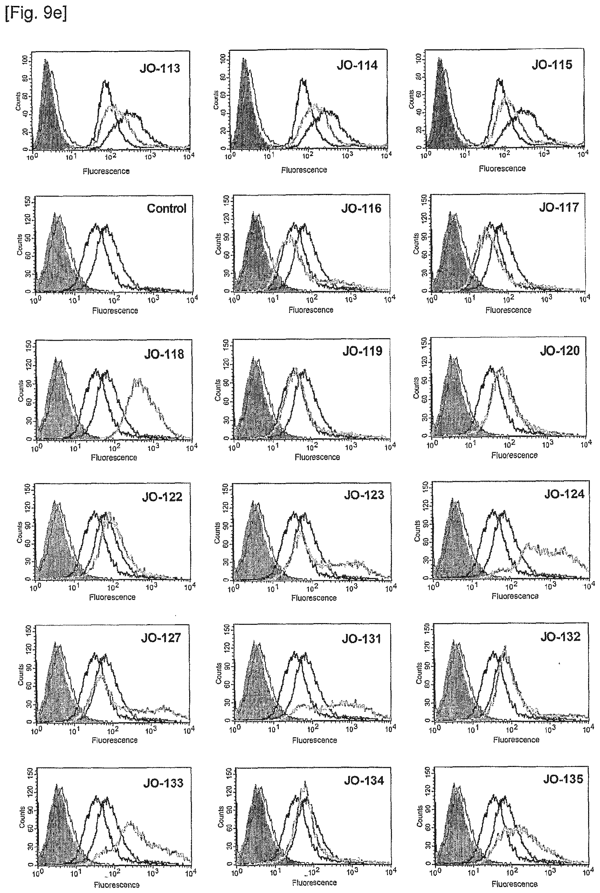

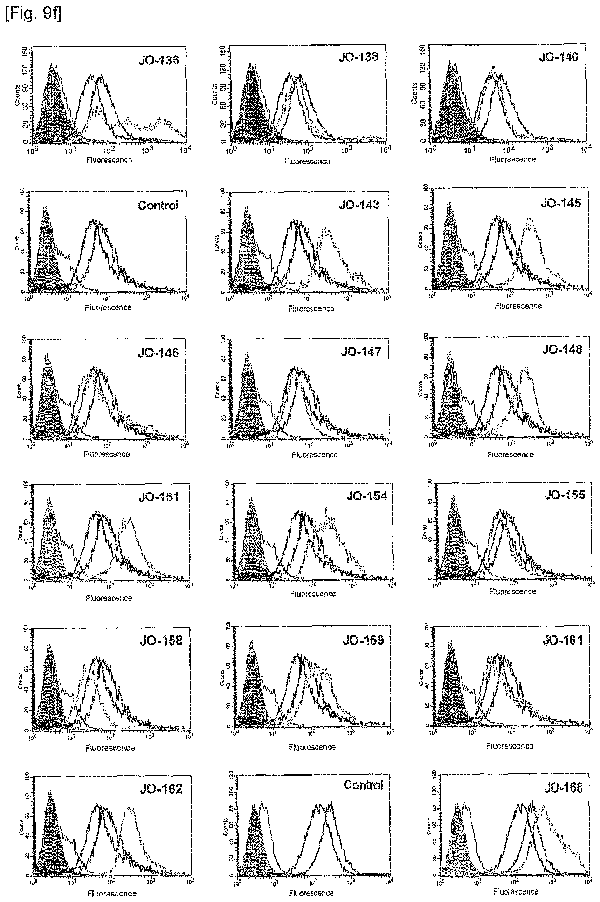

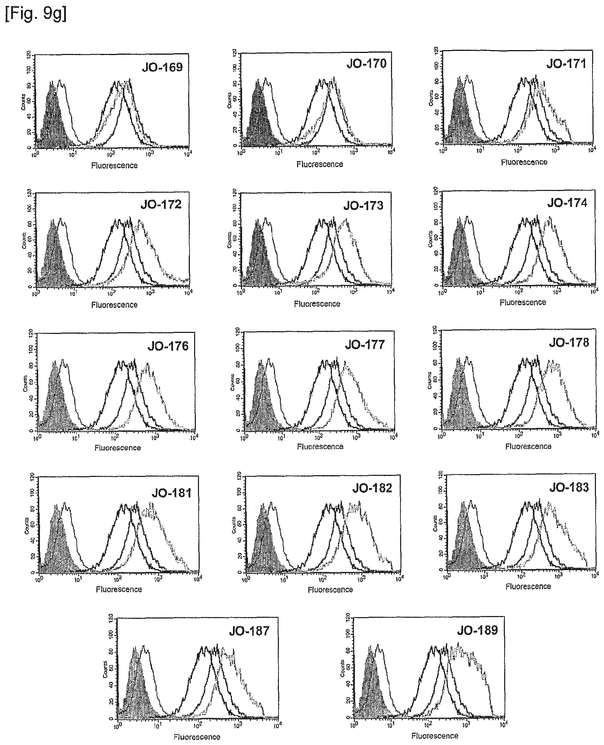

Figs. 9a to 9g show the results of a flow cytometry analysis where the gray filled curve represents cell only, the black curve represents FITC only, the blue curve represents the cell permeability of a negative control (scramble peptide), the red curve represents the cell permeability of a positive control (kFGF4-derived MTD), and the green curve represents the cell permeability of each recombinant protein.

In order to evaluate the cell permeability of each MTD in mammalian cells, the cellular uptake efficiencies of the His-MTD-EGFP recombinant proteins were compared with those of the positive control protein fused to kFGF4-derived MTD and the negative control protein fused to a scramble peptide, by evaluating the change in median fluorescence after incubation with each FITC-conjugated His-MTD-EGFP recombinant protein.

Referring to the results shown in Figs. 9a to 9g, none of the cells treated with 10 µM of the FITC-conjugated His-MTD-EGFP proteins for 1 hour at 37°C underwent a change in median fluorescence such that the median fluorescence after treatment was significantly lower than that of the cells treated with the negative control. In contrast, 80 His-MTD-EGFP recombinant proteins, out of the 111 proteins that were tested, exhibited 0.5-fold (50%) or higher fluorescence signal than that of the positive control. These results suggest that some of the newly developed novel MTDs fused with the cargo molecule EGFP exhibit significantly high levels of plasma membrane-penetrating ability which is enough to deliver macromolecules, such as protein, into live cells. The present invention refers to the His-MTD-EGFP recombinant protein, wherein the MTD comprises the amino acid sequence of SEQ ID NO: 173.

### Example 5 - Determination of Cell Permeability and Intracellular Localization of Recombinant Proteins Fused to MTDs

Out of the 111 FITC-conjugated His-MTD-EGFP recombinant proteins tested by flow cytometry, 60 His-MTD-EGFP recombinant proteins were selected for visualization of cell permeability and intracellular localization thereof. Among them, 50 His-MTD-EGFP recombinant proteins exhibited higher cell permeability than 0.5-fold as compared with the positive control protein fused to kFGF4-derived MTD.

NIH 3T3 cells were treated without (cell only) or with FITC (FITC only), or FITC-conjugated recombinant proteins such as a negative control (His-scramble peptide-EGFP), a positive control (His-kFGF4-derived MTD-EGFP) or the recombinant proteins fused to novel MTDs (His-MTDs-EGFP), and visualized by confocal laser scanning microscopy. NIH 3T3 cells were cultured for 24 hours in an 8-well chamber slide (LabTek, Nalgen Nunc, Rochester NY, USA). Cells were maintained in DMEM supplemented with 10% fetal bovine serum, 1% penicillin and streptomycin in 5% CO₂ at 37°C. The cells were washed with PBS three times, and then treated for 1 hour with DMEM, DMEM plus free FITC, or DMEM containing 10 µM FITC-conjugated recombinant proteins in 5% CO₂ at 37°C. One hour after the treatment, the cells were fixed in 4% paraformaldehyde (PFA) for 20 minutes at room temperature for observation.

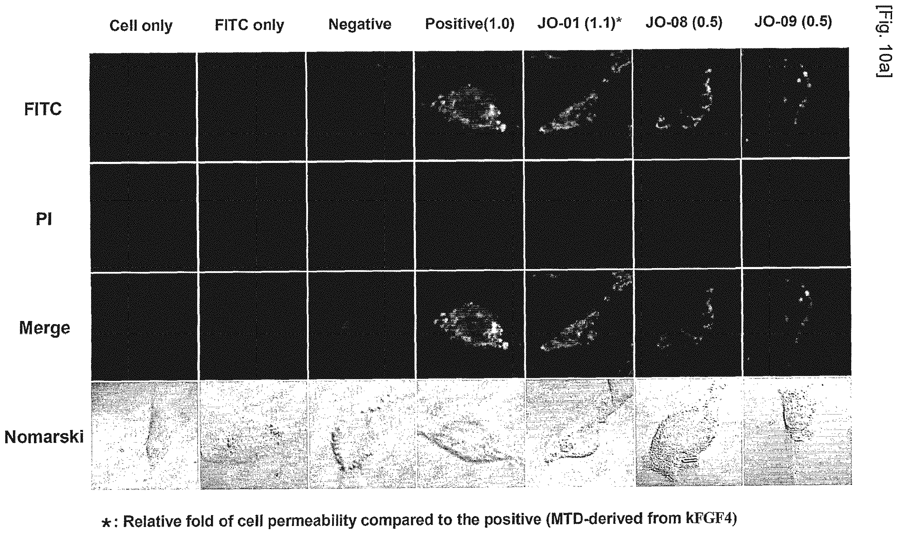

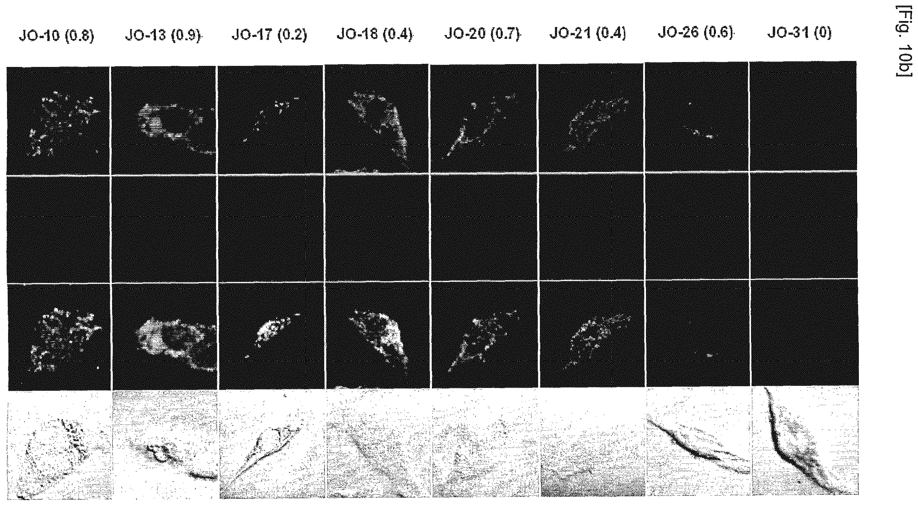

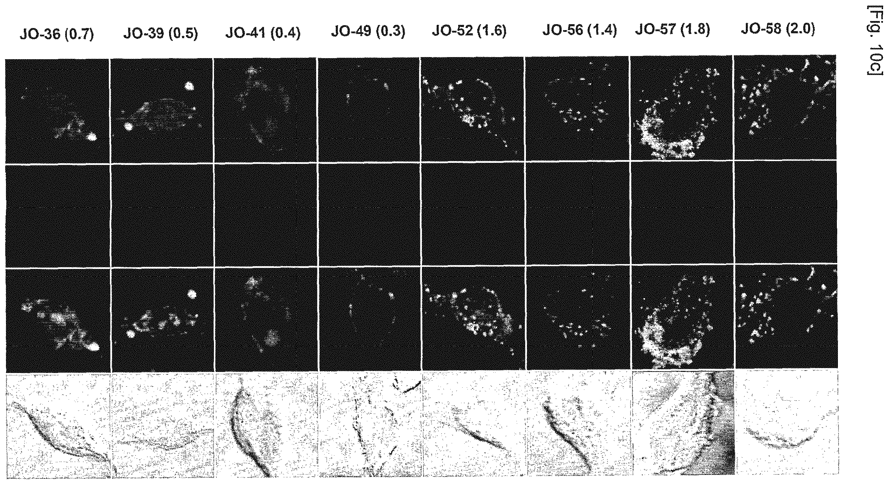

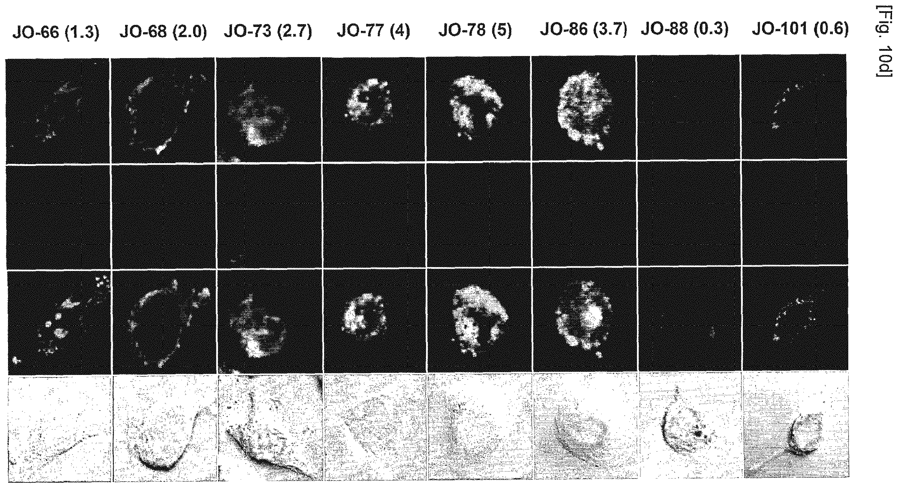

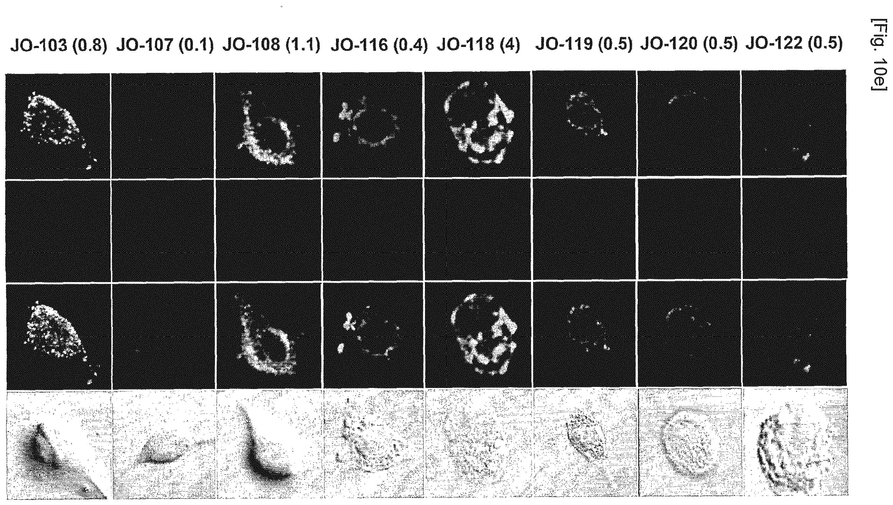

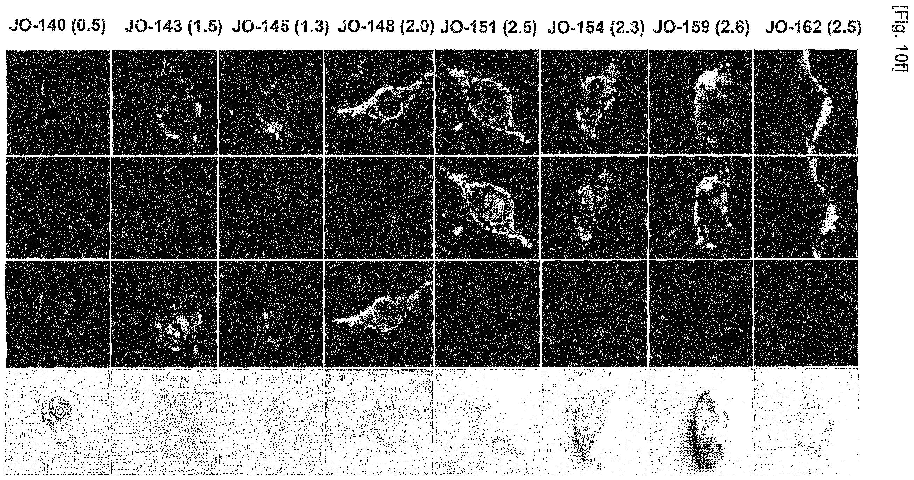

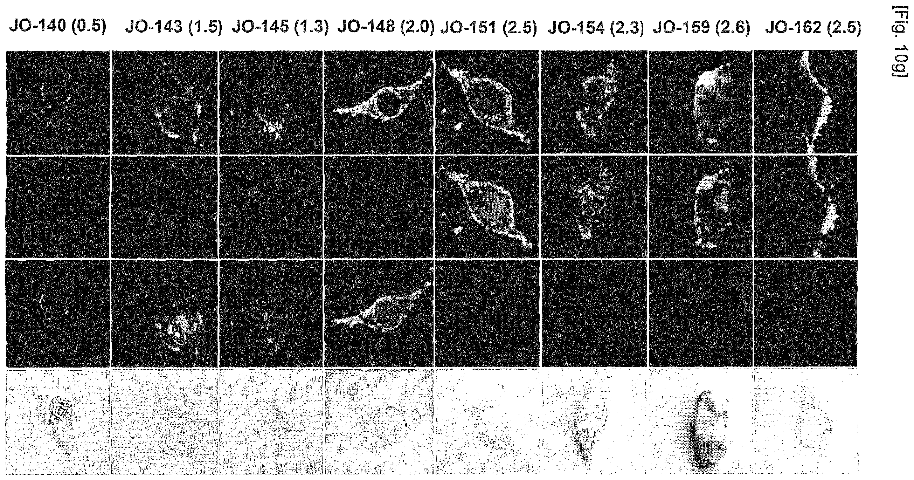

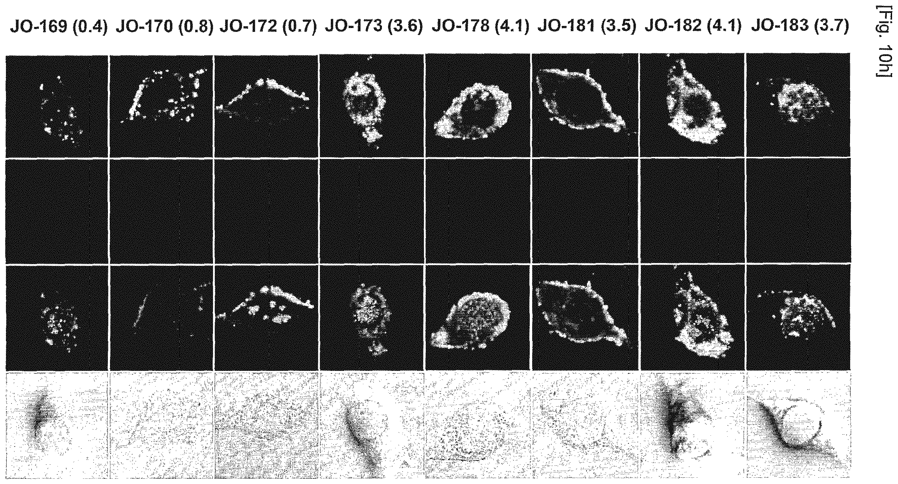

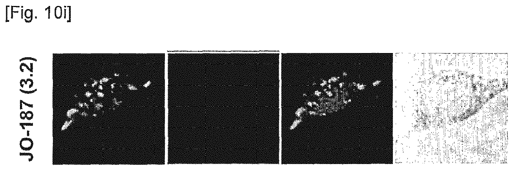

For the direct detection of FITC-conjugated recombinant proteins that were internalized, the cells were washed with PBS three times and counterstained with a nuclear fluorescent stain solution, propidium iodide (PI, Sigma-Aldrich, St Louis MO, USA), at a concentration of 1 µg/ml. After PI staining for 5 minutes, the cells were washed with PBS three times and fixed by polyvinyl alcohol mountain medium with DABCO (Fluca, St Louis MO, USA). The intracellular distribution of the fluorescence was determined at the middle of a single cell analyzed by confocal laser scanning microscopy, where the results are shown in Figs. 10a to 10i. Parameters specific for each fluorochrome were followed as FITC: excited at 488 nm light, detected with a 530 nm bandpass filter.

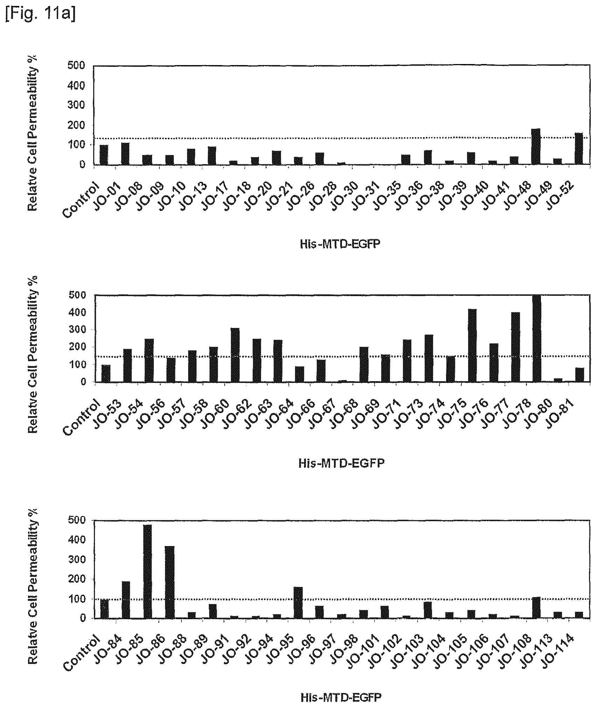

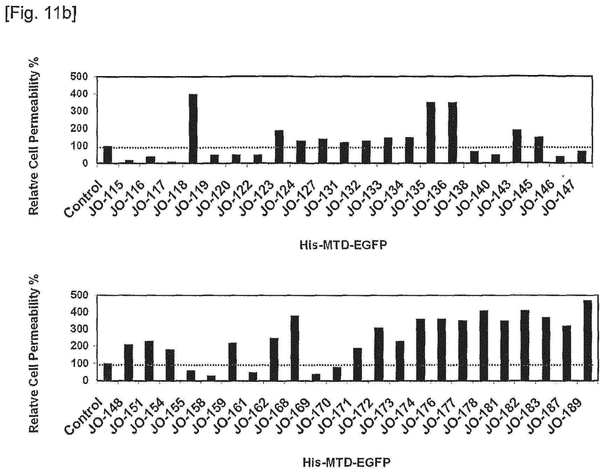

Surprisingly, as shown in Figs. 10a to 10i, 11a, and 11b, the FITC-conjugated His-MTD-EGFP recombinant proteins were well distributed largely in either the cytoplasm or the nucleus (JO-13, -18, -49, -58, -68, -101, -108, - 116, -118, -122, -123, -127, -132, -133, -136, -138, -140, -148, -162, -169, -170, and -172) or both, as compared with the negative control protein fused to a scramble peptide. Ten MTDs (JO-17, -18, -21, -31, -41, -49, -88, -107, -116, and -169), which exhibited lower cell permeability than 0.5-fold as compared with the positive control (1.0-fold), showed weak intracellular localization ability after an 1 hour treatment. These results completely coincided with those from the flow cytometry analysis.

### Example 6 - Determination of in vivo Tissue Distribution of Recombinant Proteins Fused to MTDs

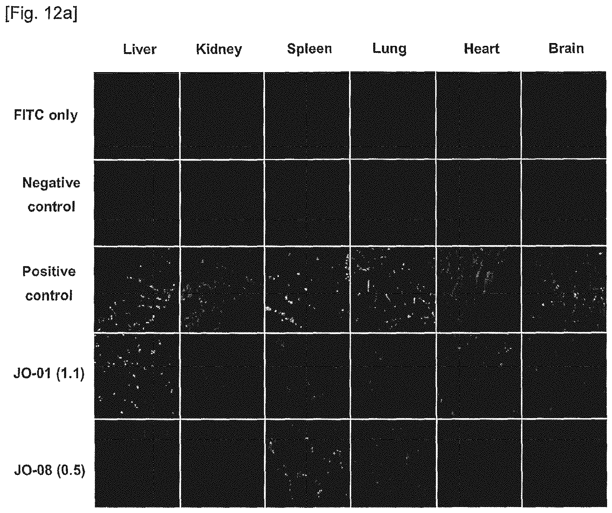

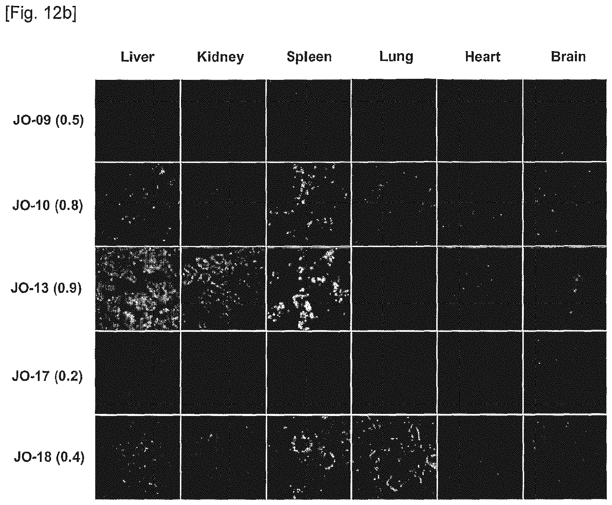

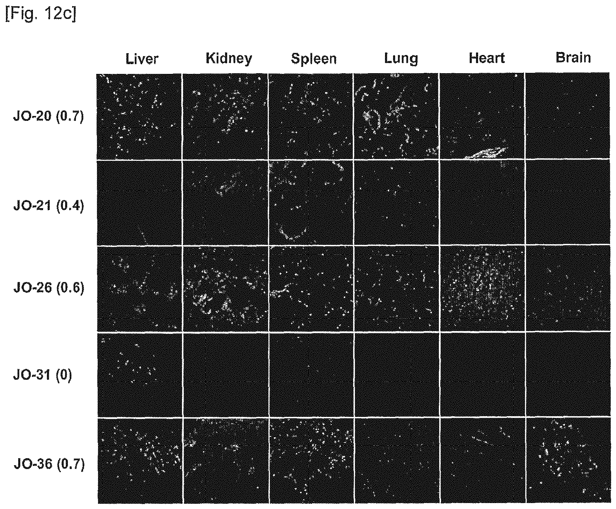

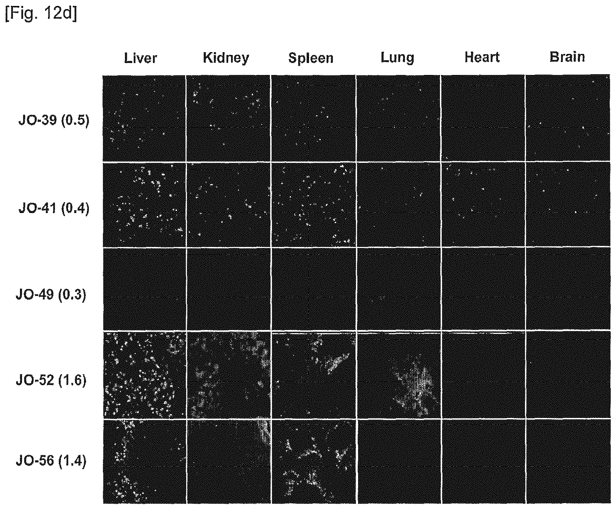

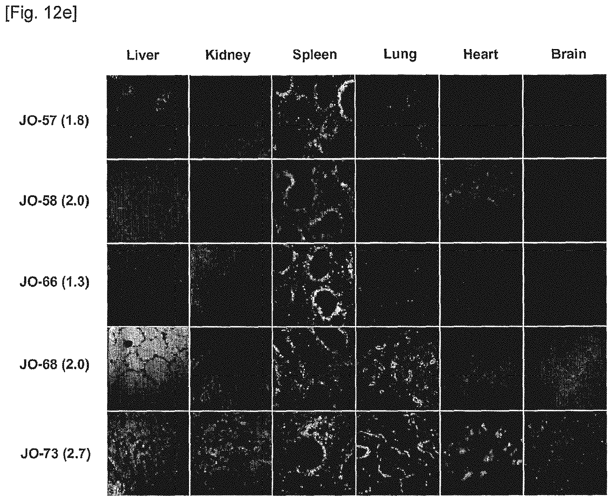

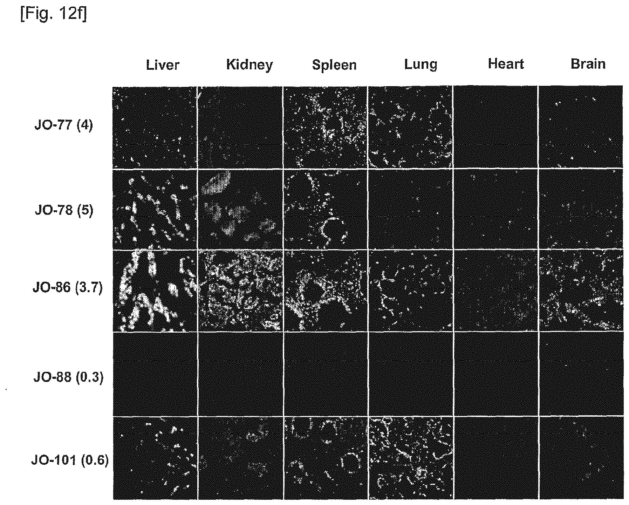

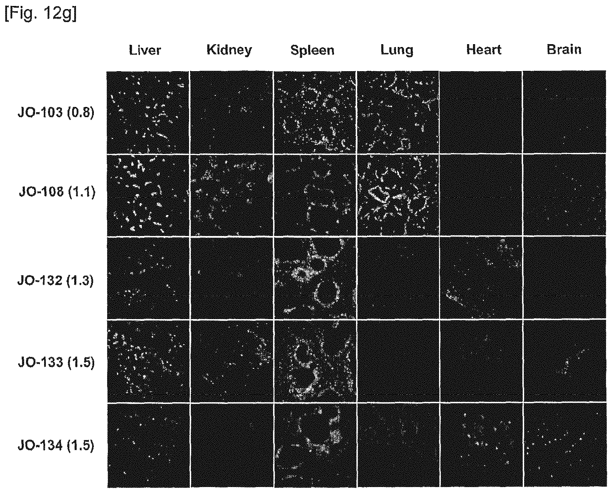

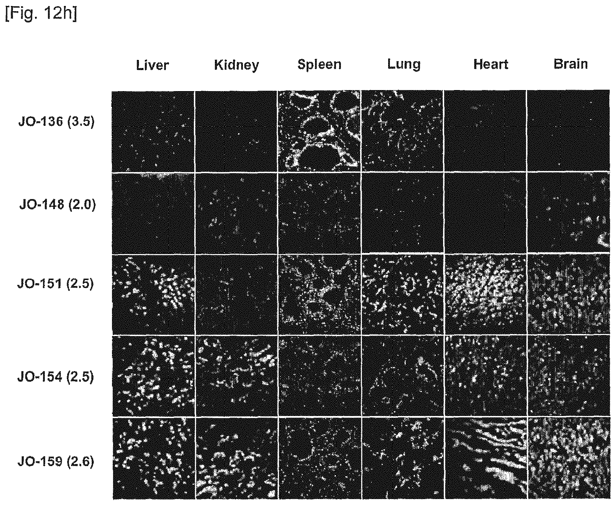

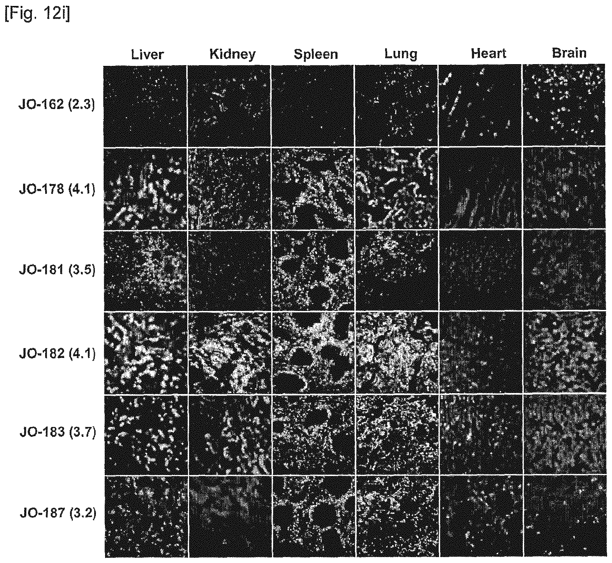

Forty-three recombinant proteins were selected from 111 FITC-conjugated recombinant proteins tested by flow cytometry for the analysis of *in vivo* tissue distribution ability. FITC only (DMEM plus free FITC), a negative control (FITC-conjugated recombinant protein fused to a scramble peptide), a positive control (FITC-conjugated recombinant protein fused to a kFGF4-derived MTD), or the His-MTD-EGFP recombinant proteins (FITC-conjugated recombinant proteins fused to selected novel MTDs) were intraperitoneally injected into Balb/c mice (6 weeks, female, Central Lab. Animal Inc., Korea) at a dose of 300 µg/500 µl/mouse, respectively. After 2 hours, six organs (i.e., liver, kidney, spleen, lung, heart, and brain) of each mouse were extracted, washed with PBS, and quickly frozen with an O.C.T. compound (Sakura, Japan) on dry ice. Cryosections (20 µm thickness) of each organ were prepared by a microtome cryostat (Sakura), placed on glass slides and, then, mounted in a Vectashield mounting medium (Vecta lab, Burlingame CA, USA). *In vivo* tissue distribution of selected novel MTDs was analyzed by using a fluorescence microscope (Nikon, Japan), where the results are shown in Figs. 12a to 12i.

In the negative control, there was no significant level of fluorescence. In contrast, the positive control protein fused to kFGF4-derived MTD showed well distributed fluorescence activity in all of the organs. With respect to the mice injected with the recombinant proteins fused to selected novel MTDs, most MTDs having 0.5-fold or higher cell permeability than the positive control as determined by flow cytometry showed relatively strong *in vivo* tissue distribution ability in all of the organs, although there were some minor differences among the organs. In particular, JO-13 (1.3-fold) and JO-133 (1.5-fold) MTDs were distributed well in all of the organs except the brain. These results indicate that the novel MTDs demonstrated their strong cell permeability and/or intracellular localization ability by flow cytometry and confocal laser scanning microscopy and can effectively mediate macromolecule intracellular transduction *in vitro* and *in vivo.*

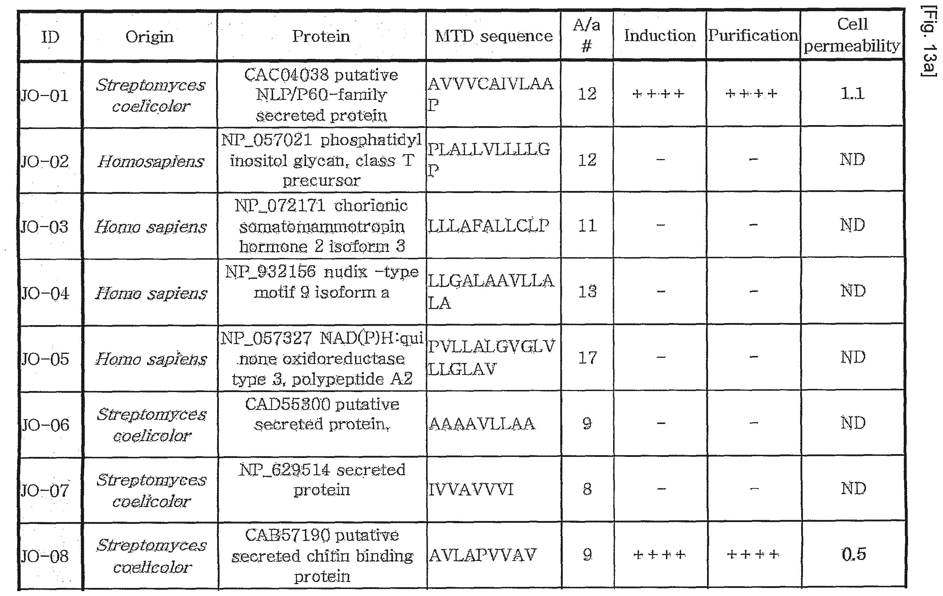

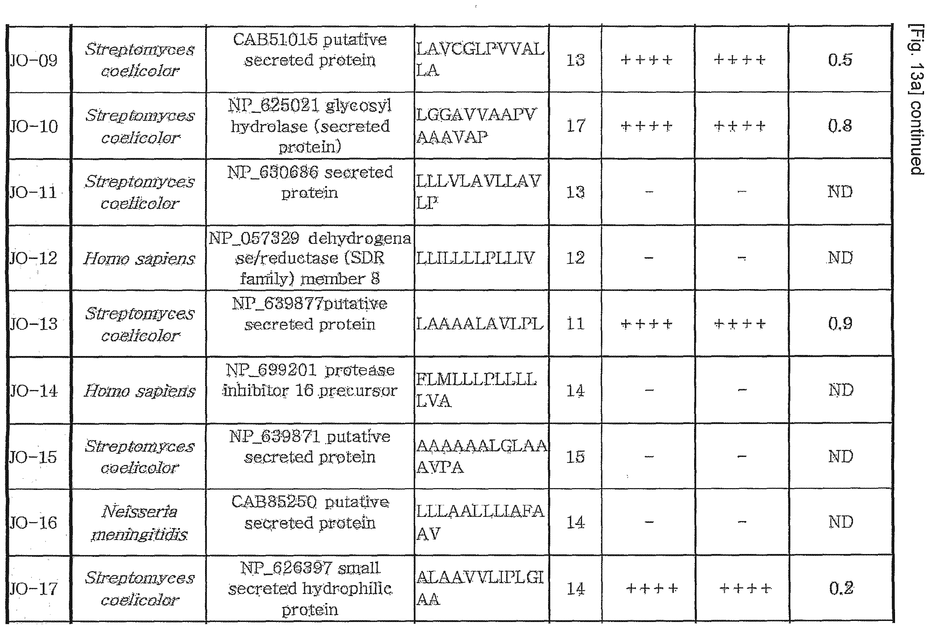

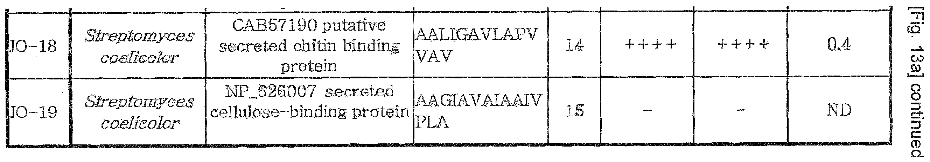

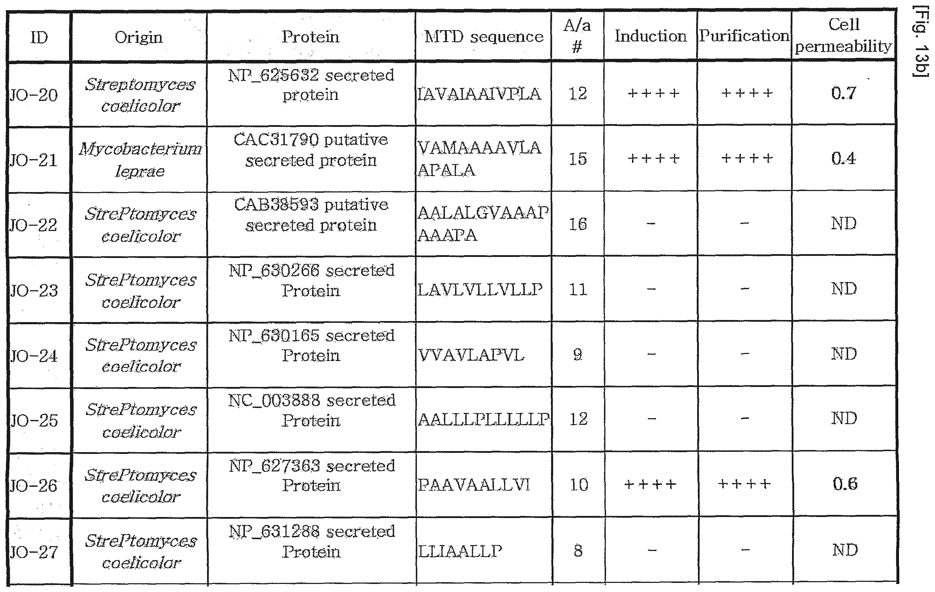

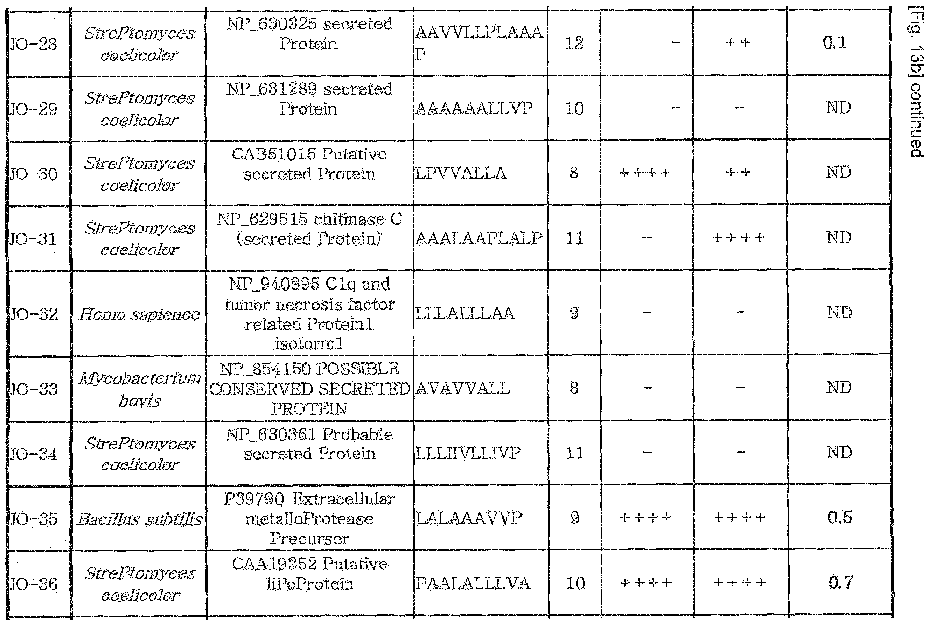

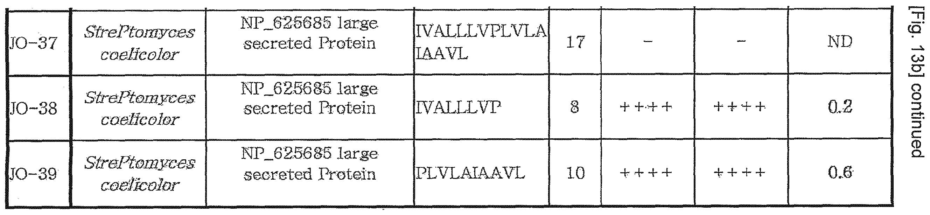

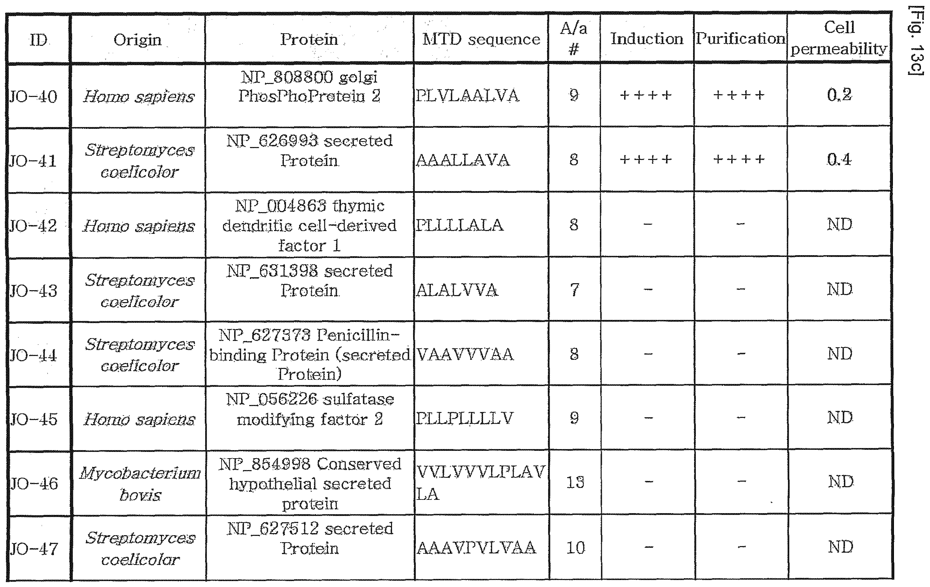

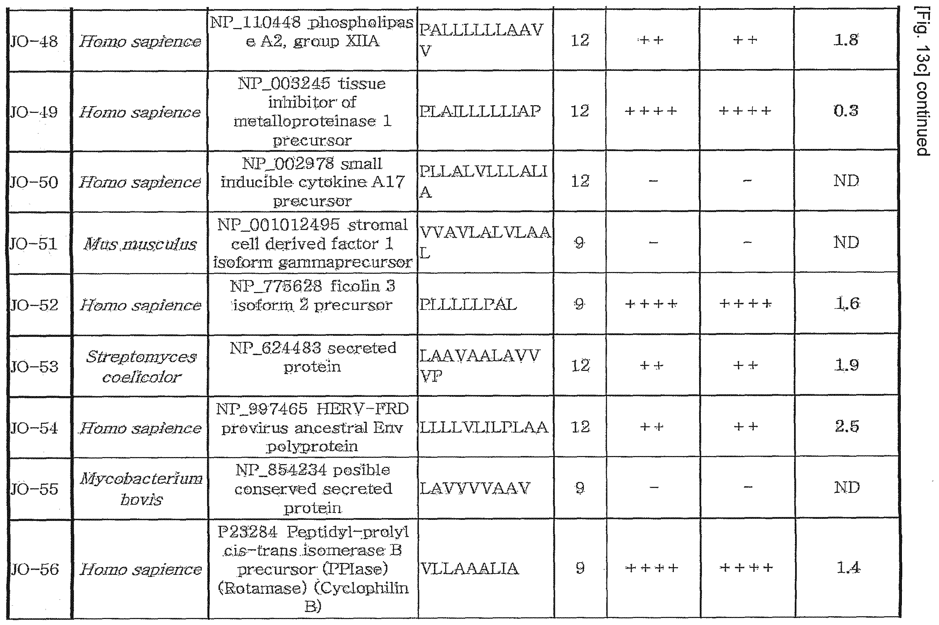

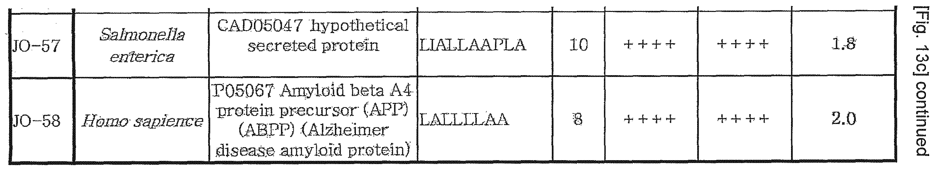

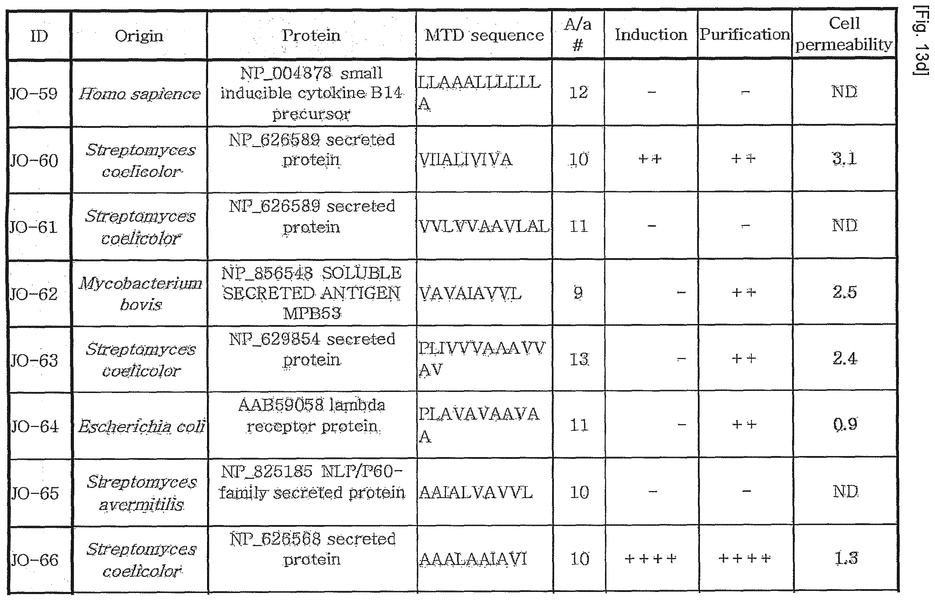

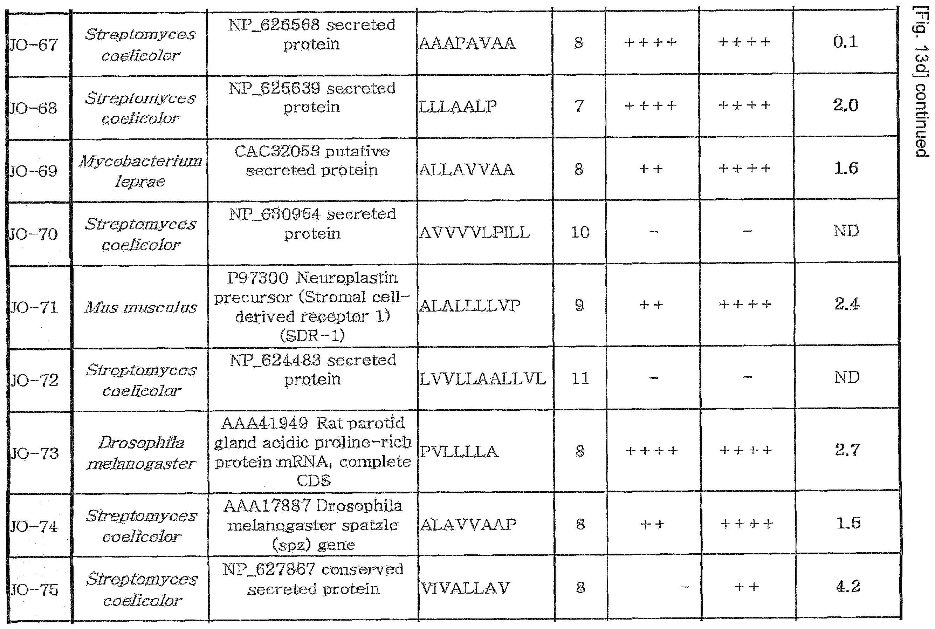

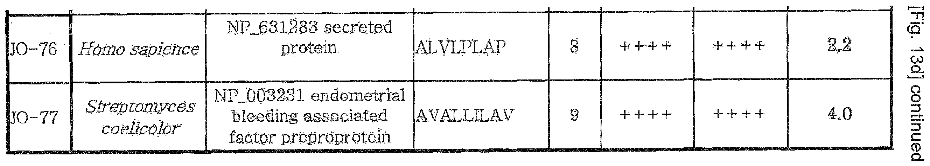

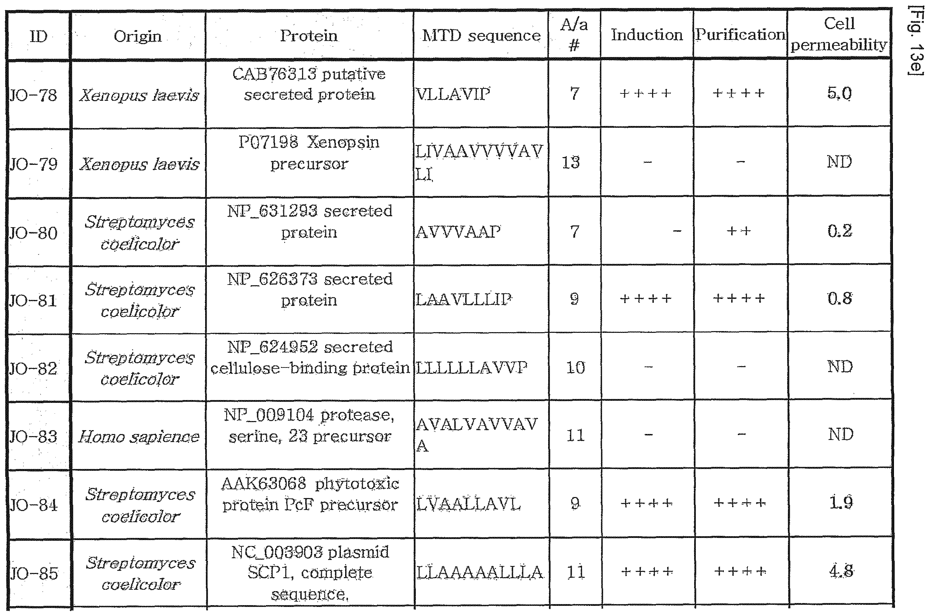

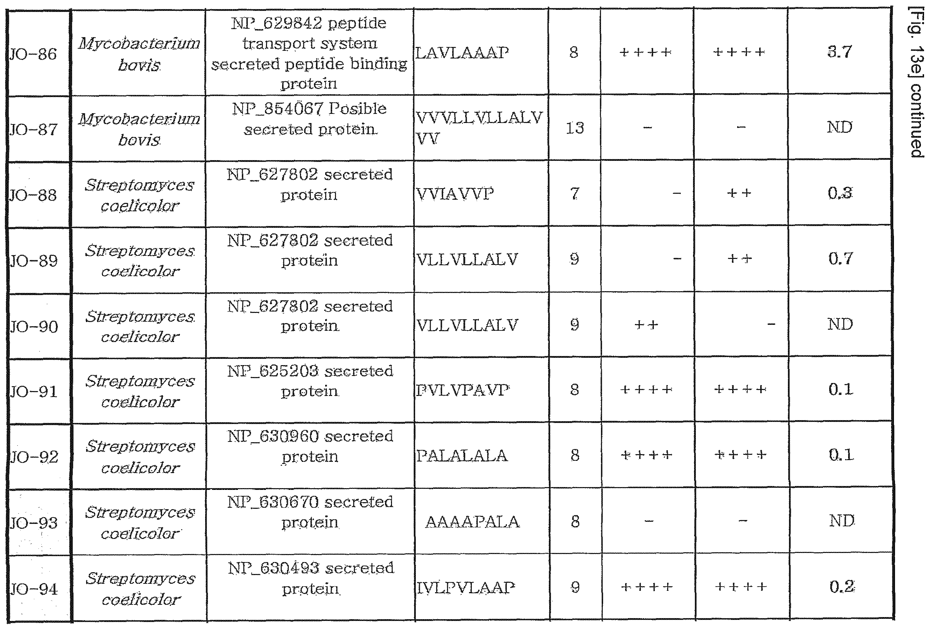

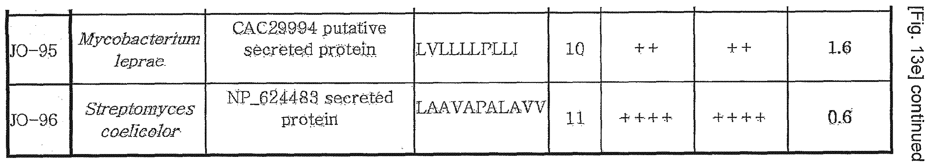

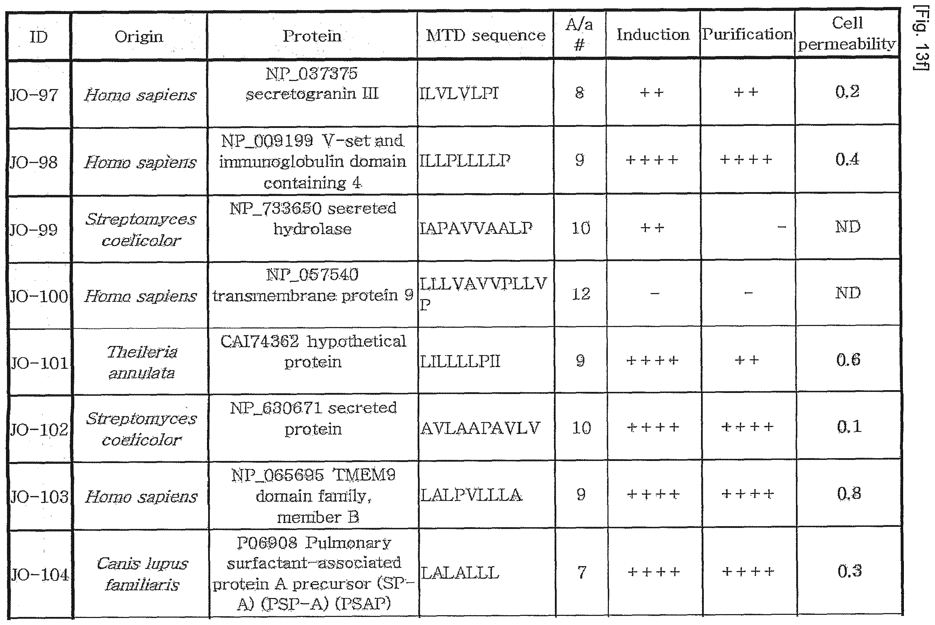

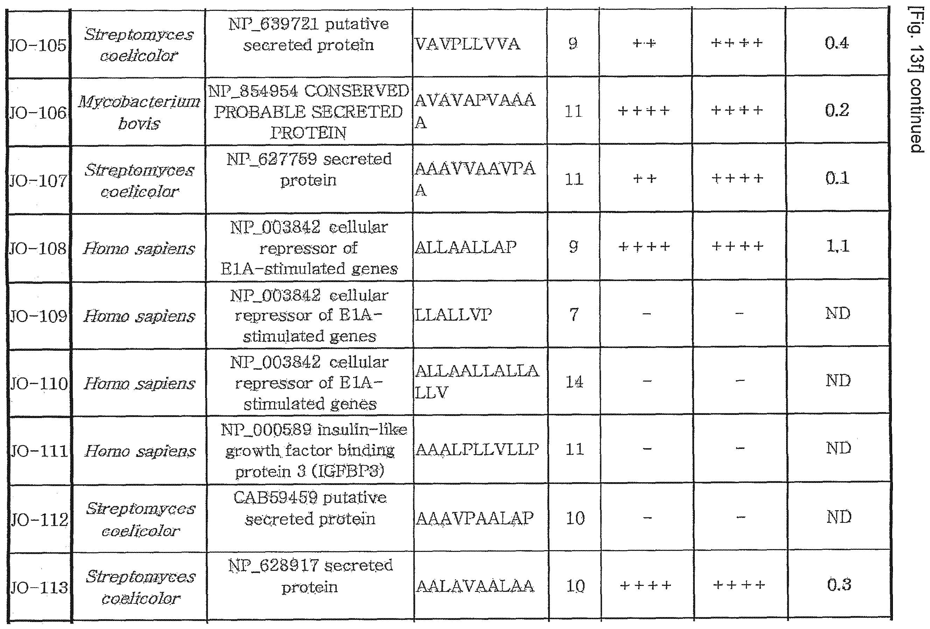

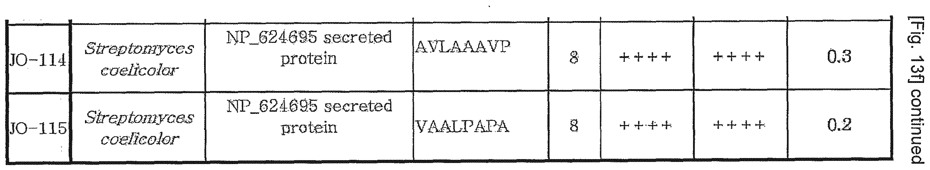

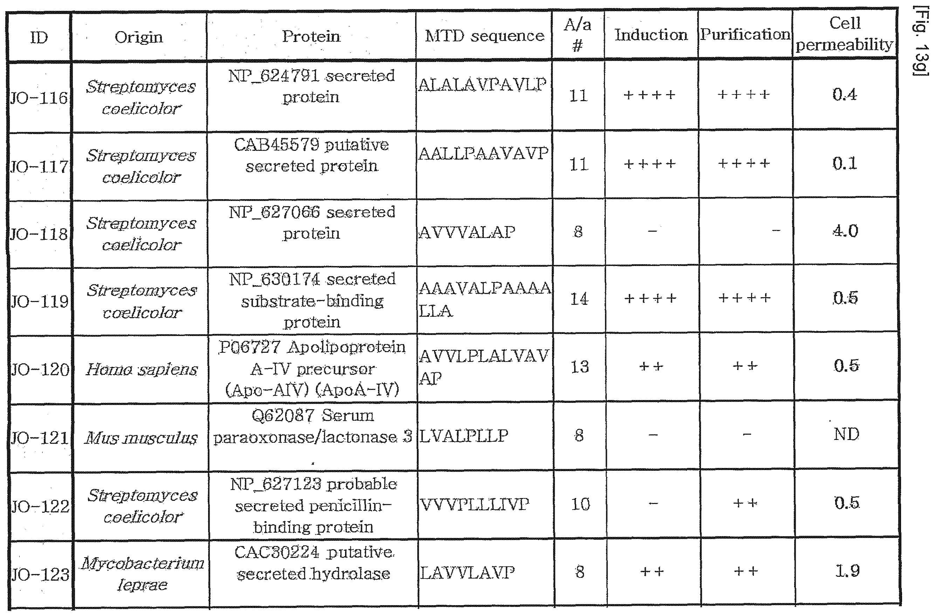

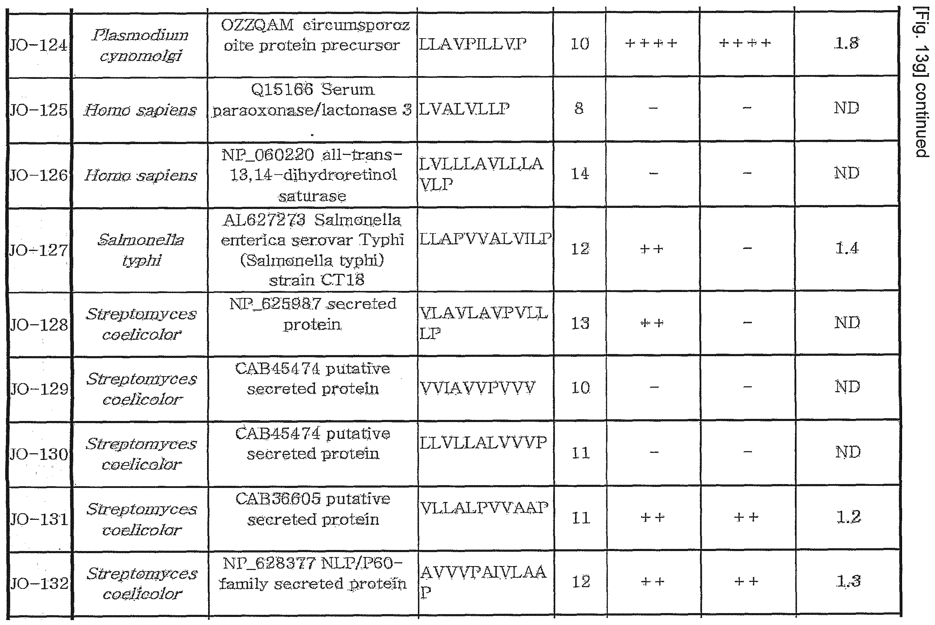

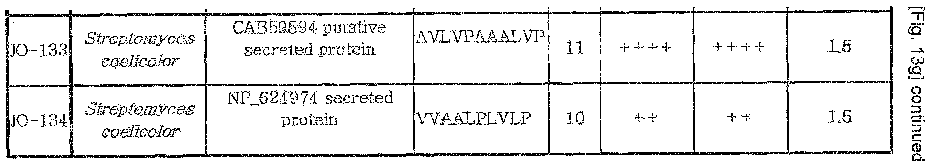

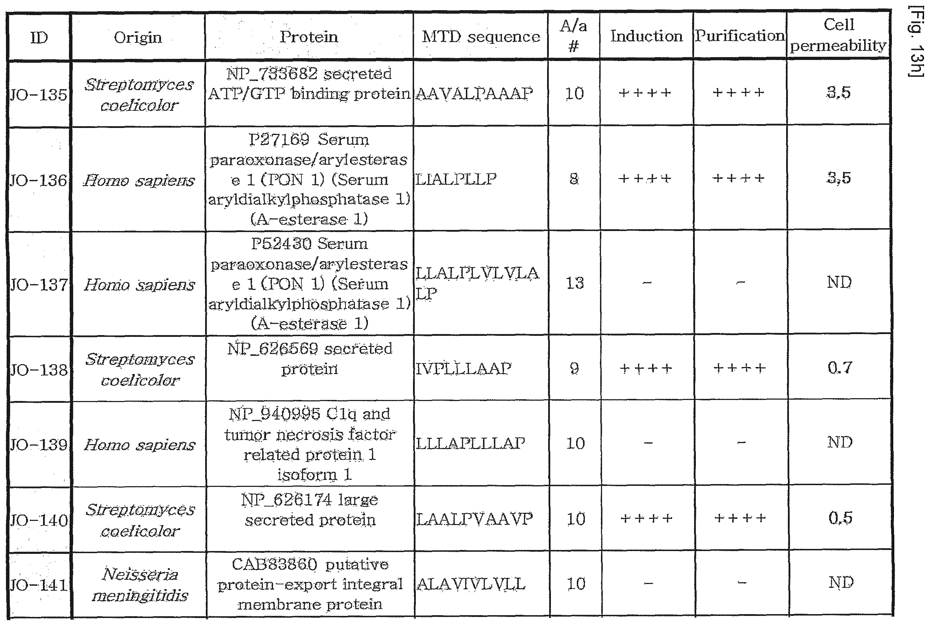

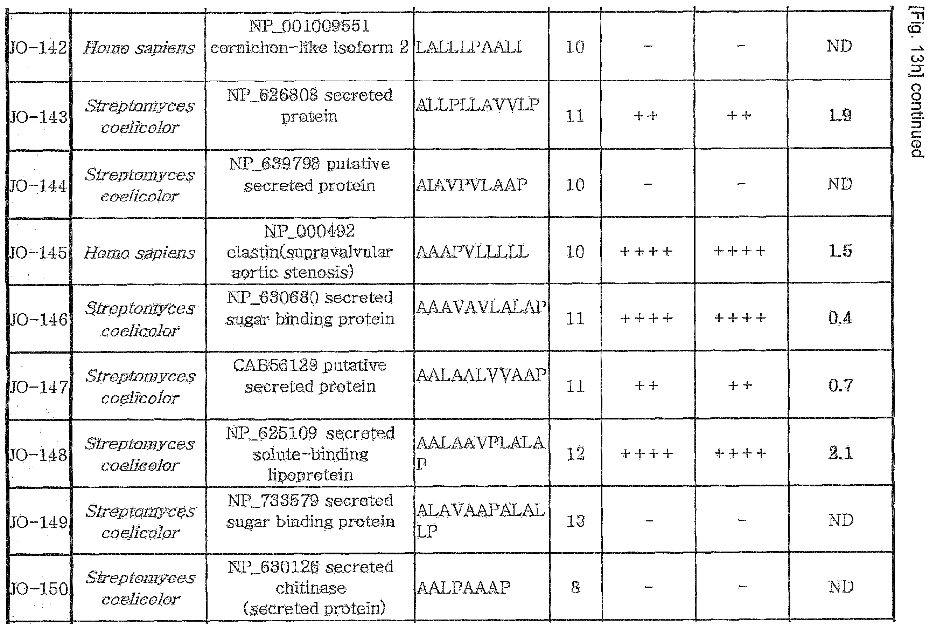

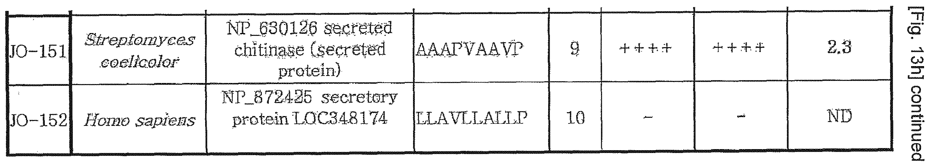

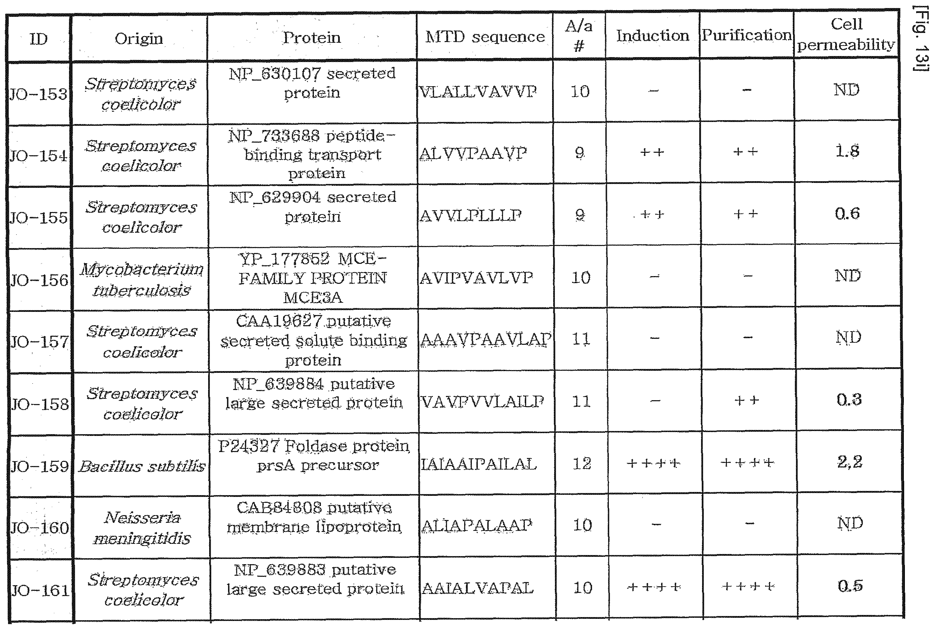

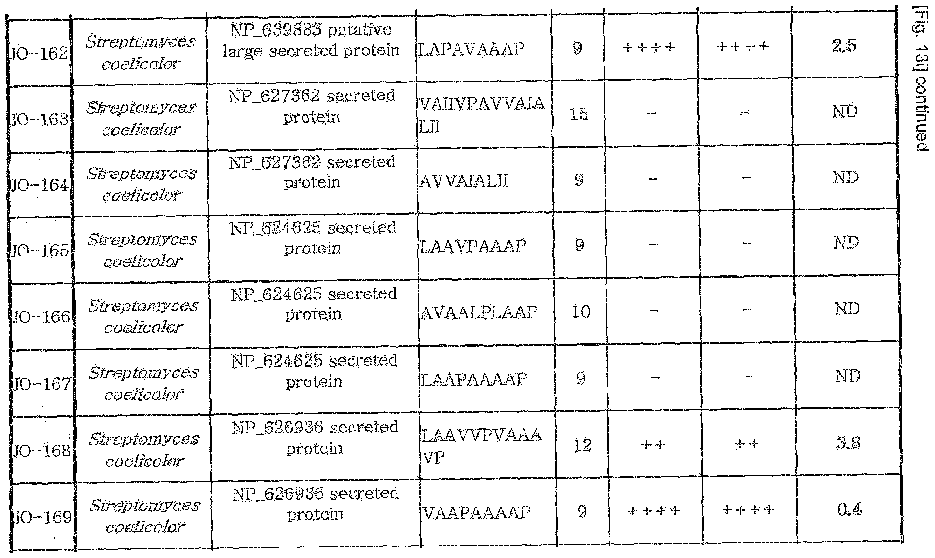

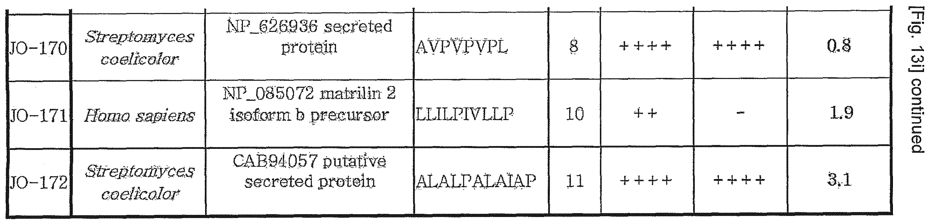

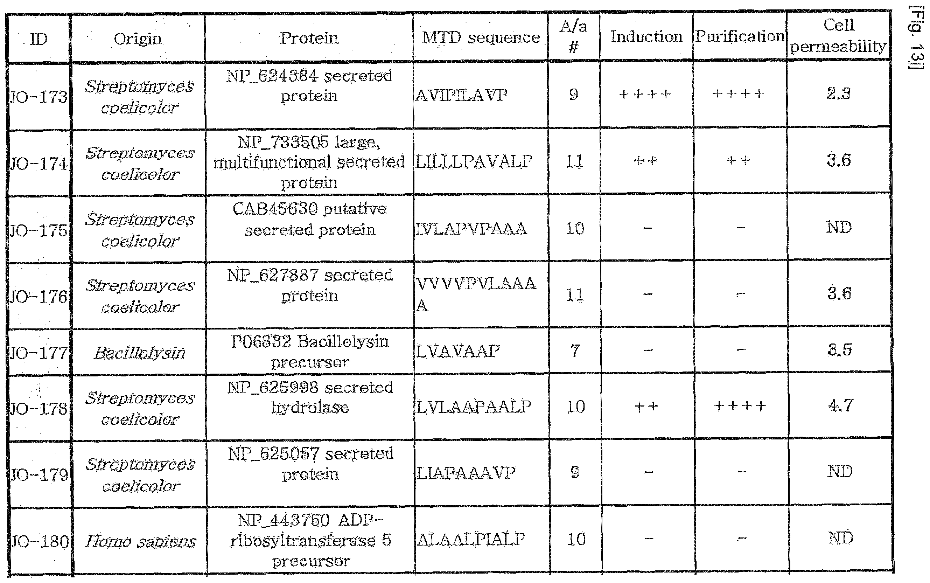

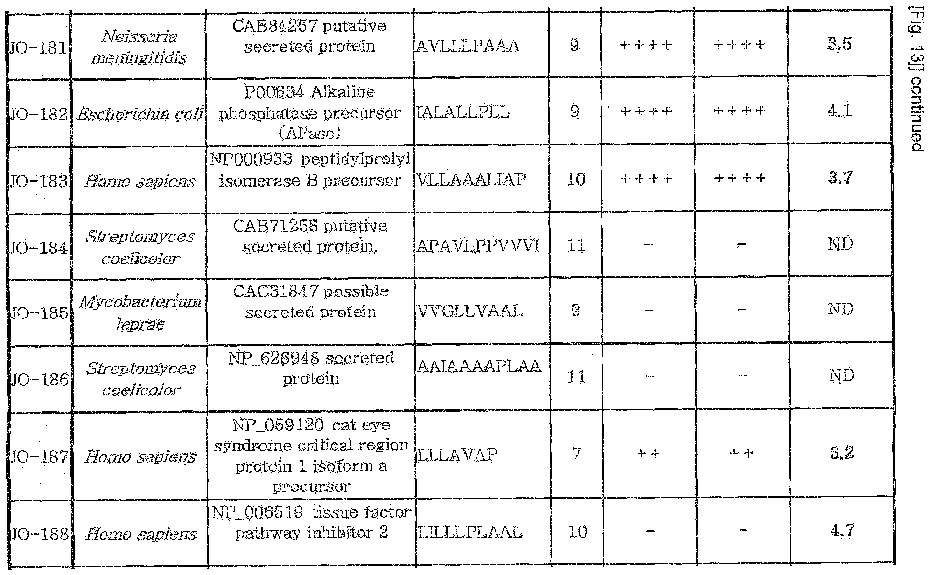

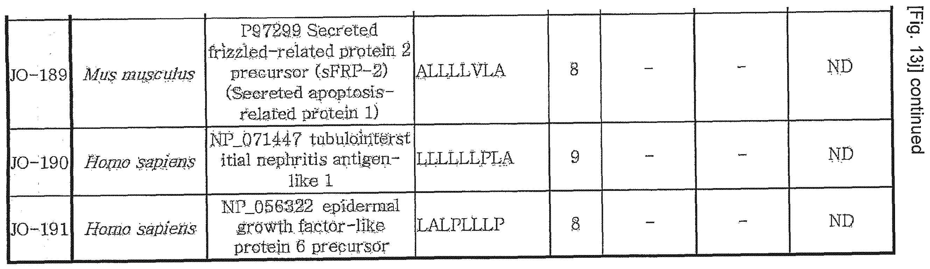

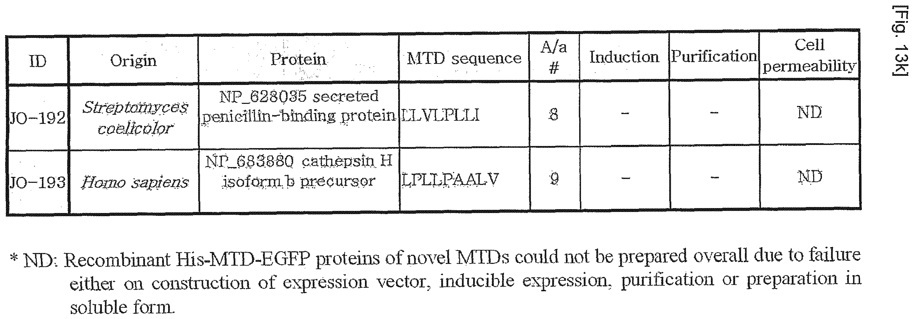

In summary, the macromolecule transduction domain (MTD) peptides described, wherein peptides comprising comprising an amino acid sequence of SEQ ID NO: 173belong to the present invention have the characteristics of inducible protein expression, purification and cell permeability as represented in Figs. 13a to 13k.

The present invention has been described in detail with reference to specific embodiments thereof. However, it will be appreciated by those skilled in the art that changes may be made in these embodiments without departing from the principles.

### Features of the parent application

### [Feature 1]

An isolated macromolecule transduction domain (MTD) peptide capable of mediating the transport of a biologically active molecule into a cell, wherein the peptide has an amino acid sequence of SEQ ID NO: 173.

### [Feature 2]

An isolated polynucleotide encoding the macromolecule transduction domain (MTD) peptide according to Feature 1.

### [Feature 3]

The isolated polynucleotide according to Feature 2, wherein the polynucleotide has a nucleotide sequence of SEQ ID NO: 366.

### [Feature 4]

A method of identifying a macromolecule transduction domain (MTD) peptide comprising an amino acid sequence of SEQ ID NO: 173 capable of mediating the transport of a biologically active molecule into a cell, comprising:
identifying secreted proteins having a signal sequence-like domain from multiple amino acid sequence databases;
selecting from said identified secreted proteins hydrophobic peptides having a single hydrophobic region at their N-terminus and forming a helix structure; and
optimizing and minimizing said selected hydrophobic peptides under conditions effective to produce peptides suitable for use as an MTD peptide.

### [Feature 5]

The method according to Feature 4, wherein the signal sequence-like domain is selected from the group consisting of penetratin (Arg-Gln-Ile-Lys-Ile-Trp-Phe-Gln-Asn-Arg-Arg-Met-Lys-Trp-Lys-Lys), the Tat peptide derived from the transactivating protein Tat of HIV-1 (Arg-Lys-Lys-Arg-Arg-Gln-Arg-Arg-Pro), transportan (Gly-Trp-Thr-Leu-Asn-Ser-Ala-Gly-Tyr-Leu-Leu-Gly-Lys-Ile-Asn-Lys-Ala-Leu-Ala-Ala-Leu-Ala-Lys-Lys-Ile-Leu), Buforin II (Thr-Arg-Ser-Ser-Arg-Ala-Gly-Leu-Gln-Phe-Arg-Val-Gly-Arg-Val-His-Arg-Leu-Leu-Arg-Lys), MAP (model amphiphatic peptide: Lys-Leu-Ala-Leu-Lys-Ala-Ser-Leu-Lys-Ala-Leu-Lys-Ala-Ala-Leu-Lys-Leu-Ala), k-FGF (Ala-Ala-Val-Ala-Leu-Leu-Pro-Ala-Val-Leu-Leu-Ala-Leu-Leu-Ala-Pro), Ku 70 (Val-Pro-Met-Leu-Lys-Pro-Met-Leu-Lys-Glu), prion (Met-Ala-Asn-Leu-Gly-Tyr-Trp-Leu-Leu-Ala-Leu-Phe-Val-Thr-Met-Trp-Thr-Asp-Val-Gly-Leu-Cys-Lys-Lys-Arg-Pro-Lys-Pro), pVEC (Leu-Leu-Ile-Ile-Leu-Arg-Arg-Arg-Ile-Arg-Lys-Gln-Ala-His-Ala-His-Ser-Lys), pep-1 (Lys-Glu-Thr-Trp-Trp-Glu-Thr-Trp-Trp-Thr-Glu-Trp-Ser-Gln-Pro-Lys-Lys-Lys-Arg-Lys-Val), SynB1 (Arg-Gly-Gly-Arg-Leu-Ser-Tyr-Ser-Arg-Arg-Arg-Phe-Ser-Thr-Ser-Thr-Gly-Arg), pep-7 (Ser-Asp-Leu-Trp-Glu-Met-Met-Met-Val-Ser-Leu-Ala-Cys-Gln-Tyr), and HN-1 (Thr-Ser-Pro-Leu-Leu-Ile-His-Asn-Gly-Gln-Lys-Leu).

### [Feature 6]

The method according to Feature 4, wherein the amino acid sequence database is selected from the group consisting of the PubMed Entrez Protein Database.

### [Feature 7]

The method according to Feature 4, wherein said optimizing and minimizing step is carried out by the following steps:
eliminating non-hydrophobic amino acids at the left or right side of the selected hydrophobic peptide to minimize the peptide length while maintaining the hydrophobic region thereof;
replacing non-hydrophobic amino acids at the middle or right side of the selected hydrophobic peptide with a hydrophobic amino acid, proline, to provide flexibility; and
minimizing the number of repeated hydrophobic amino acids to reduce the length of the peptide.

### [Feature 8]

The method according to Feature 4, wherein the MTD peptide produced as a result of said optimizing and minimizing exhibits cell permeability, hydrophobicity, and flexibility, has about 7 to about 17 amino acids in length, and forms a helix structure.

### [Feature 9]

An isolated recombinant protein having cell permeability comprising:
a macromolecule transduction domain (MTD) peptide comprising an amino acid sequence of SEQ ID NO: 173; and
a biologically active molecule.

### [Feature 10]

The isolated recombinant protein according to Feature 9, wherein the biologically active molecule is selected from the group consisting of proteins, polypeptides, and peptides.

### [Feature 11]

The isolated recombinant protein according to Feature 9, wherein the biologically active molecule is selected from the group consisting of growth factors, enzymes, transcription factors, toxins, antigenic peptides, antibodies, and antibody fragments.

### [Feature 12]

The isolated recombinant protein according to Feature 11, wherein the biologically active molecule is selected from the group consisting of enzymes, hormones, transport proteins, immunoglobulin, antibodies, structural proteins, motor function proteins, receptors, signaling proteins, storage proteins, membrane proteins, transmembrane proteins, internal proteins, external proteins, secret proteins, virus proteins, native proteins, glicoproteins, cleaved proteins, proteins with disulfide bonds, protein complexes, chemically modified proteins and prions.

### [Feature 13]

The isolated recombinant protein according to Feature 9, wherein the biologically active molecule is selected from the group consisting of nucleic acids, coding nucleic acid sequences, mRNAs, antisense RNA molecules, carbohydrates, lipids, and glycolipids.

### [Feature 14]

The isolated recombinant protein according to Feature 9, wherein the biologically active molecule is a therapeutic agent.

### [Feature 15]

The isolated recombinant protein according to Feature 14, wherein the biologically active molecule is selected from the group consisting of therapeutic drugs and toxic chemicals.

### [Feature 16]

A method of genetically engineering a biologically active molecule having cell permeability comprising attaching a macromolecule transduction domain (MTD) peptide to a biologically active molecule under conditions effective to produce a biologically active molecule having cell permeability, wherein the MTD peptide comprises the amino acid sequence of SEQ ID NO: 173.

### [Feature 17]

The method according to Feature 16, wherein said attaching comprises attaching a MTD peptide to the N-terminal, C-terminal, both or middle of the biologically active molecule.

### [Feature 18]

The method according to Feature 16, wherein said attaching comprises attaching a MTD peptide to the biologically active molecule by a peptide bond.

### [Feature 19]

The method according to Feature 16, wherein said attaching comprises attaching a MTD peptide to the biologically active molecule by a covalent bond.

### [Feature 20]

A method of transporting a biologically active molecule into a cell in a subject comprising administering to a subject a cell permeable recombinant protein comprising a macromolecule transduction domain (MTD) peptide attached to a biologically active molecule, wherein the MTD peptide comprises an amino acid sequence of SEQ ID NO: 173.

### [Feature 21]

Use of a macromolecule transduction domain (MTD) peptide for drug delivery, wherein the MTD peptide comprises an amino acid sequence of SEQ ID NO: 173.

### [Feature 22]

Use of a macromolecule transduction domain (MTD) peptide for vaccine administration, wherein the MTD peptide comprises an amino acid sequence of SEQ ID NO: 173.

### [Feature 23]

Use of a macromolecule transduction domain (MTD) peptide for protein therapy, wherein the MTD peptide comprises an amino acid sequence of SEQ ID NO: 173.

### [Feature 24]

Use of a macromolecule transduction domain (MTD) peptide for gene therapy, wherein the MTD peptide comprises an amino acid sequence of SEQ ID NO: 173.

## Claims

1. An isolated macromolecule transduction domain (MTD) peptide capable of mediating the transport of a biologically active molecule into a cell, wherein the peptide comprises an amino acid sequence of SEQ ID NO: 173, wherein said MTD peptide is characterized as having a single hydrophobic region at its N-terminus, forming a helix structure, exhibiting flexibility, and having 7 to 17 amino acids in length.

2. An isolated polynucleotide encoding the macromolecule transduction domain (MTD) peptide according to claim 1.

3. The isolated polynucleotide according to claim 2, wherein the polynucleotide has a nucleotide sequence of SEQ ID NO: 366.

4. A method of identifying the peptide of Claim 1 capable of mediating the transport of a biologically active molecule into a cell, comprising:
identifying secreted proteins having a signal sequence-like domain from multiple amino acid sequence databases;
selecting from said identified secreted proteins hydrophobic peptides having a single hydrophobic region at their N-terminus and which form a helix structure; and
optimizing and minimizing said selected hydrophobic peptides under conditions effective to produce peptides suitable for use as an MTD peptide,
wherein said optimizing and minimizing step is carried out by the following step:
eliminating non-hydrophobic amino acids at the left or right side of the sequences of the selected hydrophobic peptide to minimize the peptide length while maintaining the hydrophobic region thereof;
replacing non-hydrophobic amino acids at the middle or right side of the sequences of the selected hydrophobic peptide with a hydrophobic amino acid, proline, to provide flexibility; and
minimizing the number of repeated hydrophobic amino acids to reduce the length of the peptide.

5. The method according to claim 4, wherein the signal sequence-like domain is selected from the group consisting of penetratin (Arg-Gln-Ile-Lys-Ile-Trp-Phe-Gln-Asn-Arg-Arg-Met-Lys-Trp-Lys-Lys), the Tat peptide derived from the transactivating protein Tat of HIV-I (Arg-Lys-Lys-Arg-Arg-Gln-Arg-Arg-Pro),transportan (Gly-Trp-Thr-Leu-Asn-Ser-Ala-Gly-Tyr-Leu-Leu-Gly-Lys-Ile-Asn-Lys-Ala-Leu-Ala-Ala-Leu-Ala-Lys-Lys-Ile-Leu), Buforin II(Thr-Arg-Ser-Ser-Arg-Ala-Gly-Leu-Gln- Phe-Arg-Val-Gly-Arg-Val-His-Arg-Leu-Leu-Arg-Lys), MAP(model amphiphatic peptide: Lys-Leu-Ala-Leu-Lys-Ala-Ser-Leu-Lys-Ala-Leu-Lys-Ala-Ala-Leu-Lys-Leu-Ala), k-FGF(Ala-Ala-Val-Ala-Leu-Leu-Pro-Ala-Val-Leu-Leu-Ala-Leu-Leu-Ala-Pro), Ku70 (Val-Pro-Met-Leu-Lys-Pro-Met-Leu-Lys-Glu), prion(Met-Ala-Asn-Leu-Gly-Tyr-Trp-Leu-Leu-Ala-Leu-Phe-Val-Thr-Met-Trp-Thr-Asp-Val-Gly-Leu-Cys-Lys-Lys-Arg-Pro-Lys-Pro),pVEC(Leu-Leu-Ile-Ile-Leu-Arg-Arg-Arg-Ile-Arg-Lys-Gln-Ala-His-Ala-His-Ser-Lys),pep-1(Lys-Glu-Thr-Trp-Trp-Glu-Thr-Trp-Trp-Thr-Glu-Trp-Ser-Gln-Pro-Lys-Lys-Lys-Arg-Lys-Val),SynB1(Arg-Gly-Gly-Arg-Leu-Ser-Tyr-Ser-Arg-Arg-Arg-Phe-Ser-Thr-Ser-Thr-Gly-Arg), pep-7(Ser-Asp-Leu-Trp-Glu-Met-Met-Met-Val-Ser-Leu-Ala-Cys-Gln-Tyr), and HN-1(Thr-Ser-Pro-Leu-Leu-Ile-His-Asn-Gly-Gln-Lys-Leu).

6. The method according to claim 4, wherein the amino acid sequence database is selected from the group consisting of the Pubmed Entrez Protein Database.

7. An isolated recombinant protein having cell permeability comprising:
a macromolecule transduction domain (MTD) peptide comprising an amino acid sequence of SEQ ID NO: 173, wherein said MTD peptide is characterized as having a single hydrophobic region at its N-terminus, forming a helix structure, exhibiting flexibility, and having 7 to 17 amino acids in length; and a biologically active molecule.

8. The isolated recombinant protein according to claim 7, wherein the biologically active molecule is selected from the group consisting of proteins, polypeptides, and peptides, and is preferably selected from the group consisting of growth factors, enzymes, transcription factors, toxins, antigenic peptides, antibodies, and antibody fragments, and is even more preferably selected from the group consisting of enzymes, hormones, transport proteins, immunoglobulin, antibodies, structural proteins, motor function proteins, receptors, signaling proteins, storage proteins, membrane proteins, transmembrane proteins, internal proteins, external proteins, secret proteins, virus proteins, native proteins, glycoproteins, cleaved proteins, proteins with disulfide bonds, protein complexes, chemically modified proteins and prions.

9. The isolated recombinant protein according to claim 7, wherein the biologically active molecule is selected from the group consisting of nucleic acids, coding nucleic acid sequences, mRNAs, antisense RNA molecules, carbohydrates, lipids, and glycolipids.

10. The isolated recombinant protein according to claim 7, wherein the biologically active molecule is a therapeutic agent, preferably selected from the group consisting of therapeutic drugs and toxic chemicals.

11. A method of genetically engineering a biologically active molecule having cell permeability comprising attaching a macromolecule transduction domain (MTD) peptide to a biologically active molecule under conditions effective to produce a biologically active molecule having cell permeability, wherein the MTD peptide comprises the amino acid sequence of SEQ ID NO: 173, wherein said MTD peptide is characterized as having a single hydrophobic region at its N-terminus, forming a helix structure, exhibiting flexibility, and having 7 to 17 amino acids in length.

12. The method according to claim 11, wherein said attaching comprises:
(i) attaching the MTD peptide to the N-terminal, C-terminal, both or middle of the biologically active molecule; or
(ii) attaching the MTD peptide to the biologically active molecule by a peptide bond; or
(iii) attaching the MTD peptide to the biologically active molecule by a covalent bond.

13. A cell permeable recombinant protein comprising a macromolecule transduction domain (MTD) peptide attached to a biologically active molecule, wherein the MTD peptide comprises the amino acid sequence of SEQ ID NO: 173, wherein said MTD peptide is characterized as having a single hydrophobic region at its N-terminus, forming a helix structure, exhibiting flexibility, and having 7 to 17 amino acids in length, for use in transporting a biologically active molecule into a cell in a subject.

14. A macromolecule transduction domain (MTD) peptide comprising an amino acid sequence of SEQ ID NO: 173, wherein said MTD peptide is characterized as having a single hydrophobic region at its N-terminus, forming a helix structure, exhibiting flexibility, and having 7 to 17 amino acids in length, for use in:
(i) drug delivery; or
(ii) vaccine administration; or
(iii) protein therapy; or
(iv) gene therapy.

## Patentansprüche

1. Isoliertes Makromolekültransduktionsdomänen- (MTD) Peptid, fähig, den Transport eines biologisch aktiven Moleküls in eine Zelle zu vermitteln, wobei das Peptid eine Aminosäuresequenz von SEQ ID NO: 173 umfasst, wobei das MTD-Peptid **dadurch gekennzeichnet ist, dass** es eine einzige hydrophobe Region an seinem N-Terminus hat, ausbildend eine Flexibilität aufweisende Helixstruktur und mit einer Länge von 7 bis 17 Aminosäuren.

2. Isoliertes Polynukleotid, kodierend das Makromolekültransduktionsdomänen-(MTD) Peptid gemäß Anspruch 1.

3. Isoliertes Polynukleotid gemäß Anspruch 2, wobei das Polynukleotid eine Nukleotidsequenz von SEQ ID NO: 366 hat.

4. Verfahren zum Identifizieren des Peptids von Anspruch 1, fähig, den Transport eines biologisch aktiven Moleküls in eine Zelle zu vermitteln, umfassend:
Identifizieren sekretierter Proteine mit einer Signalsequenz-ähnlichen Domäne von mehreren Aminosäuresequenzdatenbanken;
Auswählen aus den identifizierten, sekretierten Proteinen hydrophobe Peptide mit einer einzigen hydrophoben Region an ihrem N-Terminus und die eine Helixstruktur ausbilden, und
Optimieren und Minimieren der ausgewählten hydrophoben Peptide unter Bedingungen, die wirksam sind, Peptide, geeignet zur Verwendung als ein MTD-Peptid, herzustellen,
wobei der Schritt vom Optimieren und Minimieren durch den folgenden Schritt ausgeführt wird:
Eliminieren nicht-hydrophober Aminosäuren an der linken oder rechten Seite von den Sequenzen der ausgewählten hydrophoben Peptide, um die Peptidlänge unter Beibehaltung der hydrophoben Region davon zu minimieren;
Ersetzen nicht-hydrophober Aminosäuren in der Mitte oder rechten Seite von den Sequenzen der ausgewählten, hydrophoben Peptide mit einer hydrophoben Aminosäure, Prolin, um Flexibilität bereitzustellen; und
Minimieren der Anzahl von wiederholten hydrophoben Aminosäuren, um die Länge des Peptids zu reduzieren.

5. Verfahren gemäß Anspruch 4, wobei die Signalsequenz-ähnliche Domäne ausgewählt ist aus der Gruppe, bestehend aus Penetratin (Arg-Gln-Ile-Lys-Ile-Trp-Phe-Gln-Asn-Arg-Arg-Met-Lys-Trp-Lys-Lys), dem Tat-Peptid, abgeleitet von dem transaktivierenden Protein Tat von HIV-I (Arg-Lys-Lys-Arg-Arg-Gln-Arg-Arg-Pro), Transportan (Gly-Trp-Thr-Leu-Asn-Ser-Ala-Gly-Tyr-Leu-Leu-Gly-Lys-Ile-Asn-Lys-Ala-Leu-Ala-Ala-Leu-Ala-Lys-Lys-Ile-Leu), Buforin II (Thr-Arg-Ser-Ser-Arg-Ala-Gly-Leu-Gln- Phe-Arg-Val-Gly-Arg-Val-His-Arg-Leu-Leu-Arg-Lys), MAP (Modell amphiphatisches Peptid: Lys-Leu-Ala-Leu-Lys-Ala-Ser-Leu-Lys-Ala-Leu-Lys-Ala-Ala-Leu-Lys-Leu-Ala), k-FGF (Ala-Ala-Val-Ala-Leu-Leu-Pro-Ala-Val-Leu-Leu-Ala-Leu-Leu-Ala-Pro), Ku70 (Val-Pro-Met-Leu-Lys-Pro-Met-Leu-Lys-Glu), Prion (Met-Ala-Asn-Leu-Gly-Tyr-Trp-Leu-Leu-Ala-Leu-Phe-Val-Thr-Met-Trp-Thr-Asp-Val-Gly-Leu-Cys-Lys-Lys-Arg-Pro-Lys-Pro), pVEC (Leu-Leu-Ile-Ile-Leu-Arg-Arg-Arg-Ile-Arg-Lys-Gln-Ala-His-Ala-His-Ser-Lys), Pep-1 (Lys-Glu-Thr-Trp-Trp-Glu-Thr-Trp-Trp-Thr-Glu-Trp-Ser-Gln-Pro-Lys-Lys-Lys-Arg-Lys-Val), SynB1 (Arg-Gly-Gly-Arg-Leu-Ser-Tyr-Ser-Arg-Arg-Arg-Phe-Ser-Thr-Ser-Thr-Gly-Arg), Pep-7 (Ser-Asp-Leu-Trp-Glu-Met-Met-Met-Val-Ser-Leu-Ala-Cys-Gln-Tyr), und HN-1 (Thr-Ser-Pro-Leu-Leu-Ile-His-Asn-Gly-Gln-Lys-Leu).

6. Verfahren gemäß Anspruch 4, wobei die Aminosäuresequenzdatenbank ausgewählt ist aus der Gruppe, bestehend aus der Pubmed Entrez Proteindatenbank.

7. Isoliertes, rekombinantes Protein mit Zellpermeabilität, umfassend:
ein Makromolekültransduktionsdomänen- (MTD) Peptid, umfassend eine Aminosäuresequenz von SEQ ID NO: 173, wobei das MTD-Peptid **dadurch gekennzeichnet ist, dass** es eine einzige hydrophobe Region an seinem N-Terminus hat, ausbildend eine Flexibilität aufweisende Helixstruktur und mit einer Länge von 7 bis 17 Aminosäuren; und ein biologisch aktives Molekül.

8. Isoliertes, rekombinantes Protein gemäß Anspruch 7, wobei das biologisch aktive Molekül ausgewählt ist aus der Gruppe, bestehend aus Proteinen, Polypeptiden, und Peptiden, und bevorzugt ausgewählt ist aus der Gruppe, bestehend aus Wachstumsfaktoren, Enzymen, Transkriptionsfaktoren, Toxinen, antigenen Peptiden, Antikörpern, und Antikörperfragmenten, und noch bevorzugter ausgewählt ist aus der Gruppe, bestehend aus Enzymen, Hormonen, Transportproteinen, Immunglobulin, Antikörpern, Strukturproteinen, Motorfunktionsproteinen, Rezeptoren, Signalproteinen, Speicherproteinen, Membranproteinen, Transmembranproteinen, internen Proteinen, externen Proteinen, sekretorischen Proteinen, viralen Proteinen, nativen Proteinen, Glycoproteinen, gespaltenen Proteinen, Proteinen mit Disulfidbrücken, Proteinkomplexen, chemisch modifizierten Proteinen und Prionen.

9. Isoliertes, rekombinantes Protein gemäß Anspruch 7, wobei das biologisch aktive Molekül ausgewählt ist aus der Gruppe, bestehend aus Nukleinsäuren, kodierenden Nukleinsäuresequenzen, mRNAs, Antisense-RNA-Molekülen, Kohlenhydraten, Lipiden und Glykolipiden.

10. Isoliertes, rekombinantes Protein gemäß Anspruch 7, wobei das biologisch aktive Molekül ein therapeutisches Agens ist, bevorzugt ausgewählt aus der Gruppe, bestehend aus therapeutischen Medikamenten und toxischen Chemikalien.

11. Verfahren zum gentechnischen Verändern eines biologisch aktiven Moleküls mit Zellpermeabilität, umfassend Anbringen eines Makromolekültransduktionsdomänen-(MTD) Peptids an ein biologisch aktives Molekül unter Bedingungen, die wirksam sind, ein biologisch aktives Molekül mit Zellpermeabilität herzustellen, wobei das MTD-Peptid die Aminosäuresequenz von SEQ ID NO: 173 umfasst, wobei das MTD-Peptid **dadurch gekennzeichnet ist, dass** es eine einzige hydrophobe Region an seinem N-Terminus hat, ausbildend eine Flexibilität aufweisende Helixstruktur, und mit einer Länge von 7 bis 17 Aminosäuren.

12. Verfahren gemäß Anspruch 11, wobei das Anbringen umfasst:
(i) Anbringen des MTD-Peptids an den N-Terminus, C-Terminus, beide oder Mitte des biologisch aktiven Moleküls; oder
(ii) Anbringen des MTD-Peptids an das biologisch aktive Molekül durch eine Peptidbindung; oder
(iii) Anbringen des MTD-Peptids an das biologisch aktive Molekül durch eine kovalente Bindung.

13. Zellpermeables, rekombinantes Protein, umfassend ein Makromolekültransduktionsdomänen- (MTD) Peptid, angebracht an ein biologisch aktives Molekül, wobei das MTD-Peptid die Aminosäuresequenz von SEQ ID NO: 173 umfasst, wobei das MTD-Peptid **dadurch gekennzeichnet ist, dass** es eine einzige hydrophobe Region an seinem N-Terminus hat, ausbildend eine Flexibilität aufweisende Helixstruktur und mit einer Länge von 7 bis 17 Aminosäuren, zur Verwendung beim Transportieren eines biologisch aktiven Moleküls in eine Zelle in einem Individuum.

14. Makromolekültransduktionsdomänen- (MTD) Peptid, umfassend eine Aminosäuresequenz von SEQ ID NO: 173, wobei das MTD-Peptid **dadurch gekennzeichnet ist, dass** es eine einzige hydrophobe Region an seinem N-Terminus hat, ausbildend eine Flexibilität aufweisende Helixstruktur und mit einer Länge von 7 bis 17 Aminosäuren, zur Verwendung in:
(i) Medikamentenfreisetzung; oder
(ii) Impfstoffverabreichung; oder
(iii) Proteintherapie; oder
(iv) Gentherapie.

## Revendications

1. Peptide de domaine de transduction macromoléculaire (DTM) isolé capable de médier le transport d'une molécule biologiquement active dans une cellule, où le peptide comprend une séquence d'acides aminés de SEQ ID NO : 173, où ledit peptide DTM est **caractérisé en ce qu'**il comporte une région hydrophobe unique à son extrémité N-terminale, forme une structure en hélice, présente une flexibilité, et a une longueur de 7 à 17 acides aminés.

2. Polynucléotide isolé codant pour le peptide de domaine de transduction macromoléculaire (DTM) selon la revendication 1.

3. Polynucléotide isolé selon la revendication 2, où le polynucléotide a une séquence nucléotidique de SEQ ID NO : 366.

4. Procédé d'identification du peptide selon la revendication 1 capable de médier le transport d'une molécule biologiquement active dans une cellule, comprenant :
l'identification des protéines sécrétées comportant un domaine de type séquence signal parmi de multiples banques de données de séquences d'acides aminés ;
la sélection parmi lesdites protéines sécrétées identifiées des peptides hydrophobes comportant une région hydrophobe unique à leur extrémité N-terminale et qui forment une structure en hélice ; et
l'optimisation et la minimisation desdits peptides hydrophobes sélectionnés dans des conditions efficaces pour produire des peptides appropriés pour une utilisation en tant que peptide DTM,
dans lequel ladite étape d'optimisation et de minimisation est réalisée par l'étape suivante :
l'élimination des acides aminés non hydrophobes du côté gauche ou droit des séquences du peptide hydrophobe sélectionné pour minimiser la longueur du peptide tout en maintenant sa région hydrophobe ;
le remplacement des acides aminés non hydrophobes au milieu ou du côté droit des séquences du peptide hydrophobe sélectionné par un acide aminé hydrophobe, la proline, pour fournir une flexibilité ; et
la minimisation du nombre des acides aminés hydrophobes répétés pour réduire la longueur du peptide.

5. Procédé selon la revendication 4, dans lequel le domaine de type séquence signal est choisi dans le groupe constitué de la pénétratine (Arg-Gln-Ile-Lys-Ile-Trp-Phe-Gln-Asn-Arg-Arg-Met-Lys-Trp-Lys-Lys), le peptide Tat dérivé de la protéine de transactivation Tat du VIH-I (Arg-Lys-Lys-Arg-Arg-Gln-Arg-Arg-Pro), le transportane (Gly-Trp-Thr-Leu-Asn-Ser-Ala-Gly-Tyr-Leu-Leu-Gly-Lys-Ile-Asn-Lys-Ala-Leu-Ala-Ala-Leu-Ala-Lys-Lys-Ile-Leu), la buforine II (Thr-Arg-Ser-Ser-Arg-Ala-Gly-Leu-Gln-Phe-Arg-Val-Gly-Arg-Val-His-Arg-Leu-Leu-Arg-Lys), le MAP (peptide amphipathique modèle : Lys-Leu-Ala-Leu-Lys-Ala-Ser-Leu-Lys-Ala-Leu-Lys-Ala-Ala-Leu-Lys-Leu-Ala), le k-FGF (Ala-Ala-Val-Ala-Leu-Leu-Pro-Ala-Val-Leu-Leu-Ala-Leu-Leu-Ala-Pro), Ku70 (Val-Pro-Met-Leu-Lys-Pro-Met-Leu-Lys-Glu), le prion (Met-Ala-Asn-Leu-Gly-Tyr-Trp-Leu-Leu-Ala-Leu-Phe-Val-Thr-Met-Trp-Thr-Asp-Val-Gly-Leu-Cys-Lys-Lys-Arg-Pro-Lys-Pro), pVEC (Leu-Leu-Ile-Ile-Leu-Arg-Arg-Arg-Ile-Arg-Lys-Gln-Ala-His-Ala-His-Ser-Lys), pep-1 (Lys-Glu-Thr-Trp-Trp-Glu-Thr-Trp-Trp-Thr-Glu-Trp-Ser-Gln-Pro-Lys-Lys-Lys-Arg-Lys-Val), SynB1 (Arg-Gly-Gly-Arg-Leu-Ser-Tyr-Ser-Arg-Arg-Arg-Phe-Ser-Thr-Ser-Thr-Gly-Arg), pep-7 (Ser-Asp-Leu-Trp-Glu-Met-Met-Met-Val-Ser-Leu-Ala-Cys-Gln-Tyr), et HN-1 (Thr-Ser-Pro-Leu-Leu-Ile-His-Asn-Gly-Gln-Lys-Leu).

6. Procédé selon la revendication 4, dans lequel la base de données de séquences d'acides aminés est choisie dans le groupe constitué des banques de données Pubmed, Entrez, Protein.

7. Protéine recombinante isolée présentant une perméabilité cellulaire comprenant :
un peptide de domaine de transduction macromoléculaire (DTM) comprenant une séquence d'acides aminés de SEQ ID NO : 173, dans laquelle ledit peptide DTM est **caractérisé en ce qu'**il comporte une région hydrophobe unique à son extrémité N-terminale, forme une structure en hélice, présente une flexibilité, et a une longueur de 7 à 17 acides aminés ; et une molécule biologiquement active.

8. Protéine recombinante isolée selon la revendication 7, dans laquelle la molécule biologiquement active est choisie dans le groupe constitué de protéines, de polypeptides, et de peptides, et est choisie de préférence dans le groupe constitué de facteurs de croissance, d'enzymes, de facteurs de transcription, de toxines, de peptides antigéniques, d'anticorps, et de fragments d'anticorps, et est choisie de façon même davantage préférée dans le groupe constitué d'enzymes, d'hormones, de protéines de transport, d'immunoglobulines, d'anticorps, de protéines structurales, de protéines de la fonction motrice, de récepteurs, de protéines de signalisation, de protéines de stockage, de protéines membranaires, de protéines transmembranaires, de protéines internes, de protéines externes, de protéines sécrétées, de protéines virales, de protéines natives, de glycoprotéines, de protéines clivées, de protéines avec des liaisons disulfures, de complexes de protéines, de protéines chimiquement modifiées et de prions.

9. Protéine recombinante isolée selon la revendication 7, dans laquelle la molécule biologiquement active est choisie dans le groupe constitué d'acides nucléiques, de séquences d'acide nucléique codantes, d'ARNm, de molécules d'ARN antisens, de glucides, de lipides, et de glycolipides.

10. Protéine recombinante isolée selon la revendication 7, dans laquelle la molécule biologiquement active est un agent thérapeutique, choisi de préférence dans le groupe constitué de médicaments thérapeutiques et d'agents chimiques toxiques.

11. Procédé de modification par génie génétique d'une molécule biologiquement active présentant une perméabilité cellulaire comprenant la fixation d'un peptide de domaine de transduction macromoléculaire (DTM) à une molécule biologiquement active dans des conditions efficaces pour produire une molécule biologiquement active présentant une perméabilité cellulaire, dans lequel le peptide DTM comprend la séquence d'acides aminés de SEQ ID NO : 173, dans lequel ledit peptide DTM est **caractérisé en ce qu'**il comporte une région hydrophobe unique à son extrémité N-terminale, forme une structure en hélice, présente une flexibilité, et a une longueur de 7 à 17 acides aminés.

12. Procédé selon la revendication 11, dans lequel ladite fixation comprend :
(i) la fixation du peptide DTM à l'extrémité N-terminale, à l'extrémité C-terminale, aux deux ou au milieu de la molécule biologiquement active ; ou
(ii) la fixation du peptide DTM à la molécule biologiquement active par une liaison peptidique ; ou
(iii) la fixation du peptide DTM à la molécule biologiquement active par une liaison covalente.

13. Protéine recombinante présentant une perméabilité cellulaire comprenant un peptide de domaine de transduction macromoléculaire (DTM) fixé à une molécule biologiquement active, dans laquelle le peptide DTM comprend la séquence d'acides aminés de SEQ ID NO : 173, dans laquelle ledit peptide DTM est **caractérisé en ce qu'**il comporte une région hydrophobe unique à son extrémité N-terminale, forme une structure en hélice, présente une flexibilité, et a une longueur de 7 à 17 acides aminés, pour une utilisation dans le transport d'une molécule biologiquement active dans une cellule chez un sujet.

14. Peptide de domaine de transduction macromoléculaire (DTM) comprenant une séquence d'acides aminés de SEQ ID NO : 173, où ledit peptide DTM est **caractérisé en ce qu'**il comporte une région hydrophobe unique à son extrémité N-terminale, forme une structure en hélice, présente une flexibilité, et a une longueur de 7 à 17 acides aminés, destiné à une utilisation dans :
(i) la libération de médicaments ; ou
(ii) l'administration de vaccins ; ou
(iii) une protéinothérapie ; ou
(iv) une thérapie génique.
